# EUROPEAN PATENT SPECIFICATION

(11) **EP 1 363 925 B1**
(45) Date of publication and mention of the grant of the patent: **15.11.2006**
(21) Application number: 01271380.6
(22) Date of filing: 20.12.2001
(51) Int. Cl.: C07H 17/08, A61K 31/70

(54) **MACROLIDE ANTIBIOTICS**
MAKROLIDANTIBIOTIKA
ANTIBIOTIQUES A BASE DE MACROLIDES

(30) Priority: 21.12.2000 GB 0031309; 01.11.2001 GB 0126277; 01.11.2001 GB 0126276
(43) Date of publication of application: 26.11.2003
(73) Proprietor: GLAXO GROUP LIMITED, Greenford, Middlesex UB6 ONN (GB); PLIVA D.D., 10000 Zagreb (HR)
(72) Inventor: ANDREOTTI, Daniele, GlaxoSmithKline SpA, I-37100 Verona (IT); ARISTA, Luca, GlaxoSmithKline SpA, I-37100 Verona (IT); BIONDI, Stefano, GlaxoSmithKline SpA, I-37100 Verona (IT); CARDULLO, Francesca, GlaxoSmithKline SpA, I-37100 Verona (IT); DAMIANI, Frederica, GlaxoSmithKline SpA, I-37100 Verona (IT); LOCIURO, Sergio, GlaxoSmithKline SpA, I-37100 Verona (IT); MARCHIORO, Carla, GlaxoSmithKline SpA, I-37100 Verona (IT); MERLO, Giancarlo, GlaxoSmithKline SpA, I-37100 Verona (IT); MINGARDI, Anna, GlaxoSmithKline SpA, I-37100 Verona (IT); NICCOLAI, Daniela, GlaxoSmithKline SpA, I-37100 Verona (IT); PAIO, Alfredo, GlaxoSmithKline SpA, I-37100 Verona (IT); PIGA, Elisabetta, GlaxoSmithKline SpA, I-37100 Verona (IT); POZZAN, Alfonso, GlaxoSmithKline SpA, I-37100 Verona (IT); SERI, Catia, GlaxoSmithKline SpA, I-37100 Verona (IT); TARSI, Luca, GlaxoSmithKline SpA, I-37100 Verona (IT); TERRENI, Silvia, GlaxoSmithKline SpA, I-37100 Verona (IT); TIBASCO, Jessica, GlaxoSmithKline SpA, I-37100 Verona (IT); CIRACO, Manuela, c/o BPB Italia S.p.A., 20090 Settala (IT)
(74) Representative: Gladwin, Amanda Rachel
(86) International application number: PCT/GB2001/005665
(87) International publication number: WO 2002/050091

(56) References cited:
- EP-A- 1 114 826
- WO-A-00/44761
- WO-A-99/21869
- WO-A-99/21870
- FR-A- 2 732 684
- US-A- 5 747 467

## Description

The present invention relates to novel semi-synthetic macrolides having antibacterial activity. More particularly this invention relates to 11,12 γ lactone ketolides, to processes for their preparation, to compositions containing them and to their use in medicine.

EP 1114826 inter alia generically discloses macrolide compounds of formula (A) having antibacterial activity 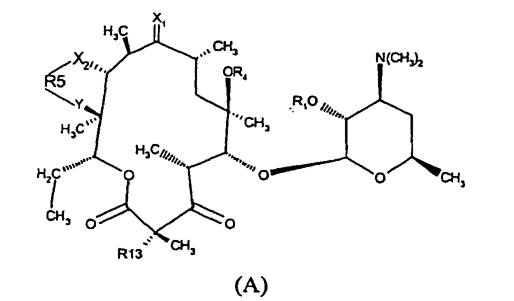 wherein R₁ is hydrogen or a hydroxyl protecting group; R₄ is inter alia an optionally substituted C₁₋₁₀ alkyl, X₁ is inter alia oxygen, X₂ is inter alia CH₂, Y is NH, O or S, R₅ is inter alia C(O) and R₁₃ is hydrogen or halo.

We have now found novel 11,12 γ lactone ketolides having antibacterial activity.

Thus, the present invention provides compounds of general formula (I) 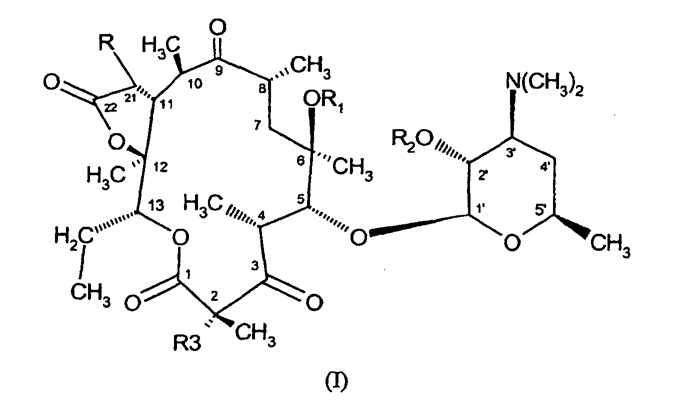 wherein
R is (CH₂)ₙA-X-R₄ or (CH₂)ₙR₅;
A is a group selected from -N(R₆)-, -N[C(O)R₆]-, -N(R₆)C(O)-, -N(R₆)S(O)₂-, N(R₆)C(O)O-, -N=C(R₆)- or -N(R₆)C(Y)N(R₇)-;
R₁ is C₁₋₆ alkyl or C₃₋₆ alkenyl;
R₂ is hydrogen or acyl;
R₃ is hydrogen or halogen;
X is a bond, a C₁₋₁₀ alkylene, a C₂₋₁₀ alkenylene or a C₂₋₁₀ alkynylene chain wherein said chains are:
   i) optionally interrupted by a bivalent radical group selected from -O-, -N(R₈)-,-C(O)-, -N(R₈)C(Y)N(R₉)-, -S(O)m-, -N(R₈)C(O)-, -C(O)N(R₈)-, -N(R₈)C(O)C(O)-, -C(O)O- or -C(NOR₆)- and/or
   ii) optionally substituted by one or two groups selected from:
      C₁₋₄ alkyl, oxo, C₁₋₄ alkoxy, halogen, cyano, phenoxy, hydroxy, NR₈R₉, N(R₈)C(O)R₉, =NOR₆, NR₈C(Y)NR₉ or optionally substituted phenyl;
R₄ is selected from:
   hydrogen,
   optionally substituted phenyl,
   optionally substituted C₃₋₇ cycloalkyl,
   optionally substituted 9 to 10 membered fused bicyclic carbocyclic,
   optionally substituted 5 or 6 membered heteroaryl in which the 5-membered heteroaryl contains at least one heteroatom selected from oxygen, sulphur or nitrogen and the 6-membered heteroaryl group contains from 1 to 3 nitrogen atoms,
   optionally substituted 5-6 membered heterocyclic,
   or
R₄ is an optionally substituted 9 or 10 membered fused bicyclic heterocyclic having at least one heteroatom selected from oxygen, sulphur or nitrogen wherein optional substituents, for said ring systems, are defined hereinafter in the specification;
R₅ is a 5 or 6 membered heterocyclic containing at least one nitrogen, optionally substituted by one or two groups selected from oxo or 9 or 10 membered fused bicyclic heterocyclic having at least one heteroatom selected from oxygen, sulphur or nitrogen;
R₆ and R₇ are independently hydrogen, C₁₋₄ alkyl or phenyl which is optionally substituted by one or two C₁₋₄ alkyl groups;
R₈ and R₉ are independently hydrogen, phenyl (which may be substituted by one or two C₁₋₄ alkyl) or R₈ and R₉ are independently C₁₋₄ alkyl which is optionally substituted by 1 or 2 groups selected from:
   phenyl, C₁₋₄ alkoxy,
   cyano,
   5-membered heteroaryl containing 1 or 2 heteroatoms selected from oxygen, sulphur or nitrogen or the 6-membered heteroaryl group contains from 1 to 3 nitrogen atoms,
   hydroxy,
   oxo,
   carboxy;
Y is an oxygen or a sulphur atom;
n is 0 or an integer from 1 to 3;
m is 0, 1 or 2;
and pharmaceutically acceptable salts and solvates thereof.

Yet a further embodiment of the invention provides compounds of formula (I) and pharmaceutically acceptable salts and solvates thereof, 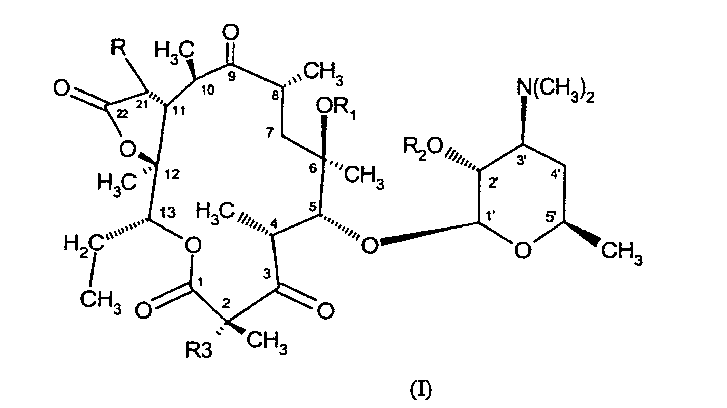 wherein
R is (CH₂)ₙA(CH₂)ₘR₄;
R₁ is C₁₋₆ alkyl or C₃₋₆ alkenyl;
R₂ is hydrogen or acyl;
R₃ is hydrogen or halogen;
R₄ is selected from:
   hydrogen;
   optionally substituted phenyl;
   optionally substituted 9 to 10 membered aromatic fused bicyclic carbocyclic ring;
   optionally substituted 5 or 6 membered heteroaryl in which the 5-membered heteroaryl contains at least one heteroatom selected from oxygen, sulphur or nitrogen and the 6-membered heteroaryl group contains from 1 to 3 nitrogen atoms; or
   R₄ is optionally substituted fused bicyclic heteroaryl groups containing 9 or 10 ring members having at least one heteroatom selected from oxygen, sulphur or nitrogen;
A is a bond or a group selected from N(R₅), N[C(O)R₅], N(R₅)C(O), N(R₅)S(O₂), N(R₅)C(O)O, N=C(R₆) or N(R₅)C(X)N(R₆);
R₅ and R₆ are independently hydrogen, phenyl, or C₁₋₄ alkyl;
X is an oxygen or a sulphur atom;
n or m are independently 0 or an integer from 1 to 5 with the proviso that the sum of n and m is 0 or an integer from 1 to 5;
and pharmaceutically acceptable salts and solvates thereof.

Suitable pharmaceutically acceptable salts of the compounds of general formula (I) include acid addition salts formed with pharmaceutically acceptable organic or inorganic acids, for example hydrochlorides, hydrobromides, sulphates, alkyl- or arylsulphonates (e.g. methanesulphonates or p-toluenesulphonates), phosphates, acetates, citrates, succinates, tartrates, fumarates and maleates.
The compound of formula (I) and salts thereof may form solvates and the invention includes all such solvates. The solvates may, for example, be hydrates.

References hereinafter to a compound according to the invention include both compounds of formula (I) and their pharmaceutically acceptable acid addition salts together with pharmaceutically acceptable solvates.

In the general formula (I) as drawn, the solid wedge shaped bond indicates that the bond is above the plane of the paper. The broken bond indicates that the bond is below the plane of the paper.

It will be appreciated by those skilled in the art that the compounds of formula (I) when R is not hydrogen contain at least one chiral centre (namely the carbon atom shown as 21 in formula (I)) and this may be represented by the formulae (1a) and (1b).

The configuration for the carbon atom shown as 21 in formula la is hereinafter referred to as the β configuration and in formula 1b as the 21α configuration.

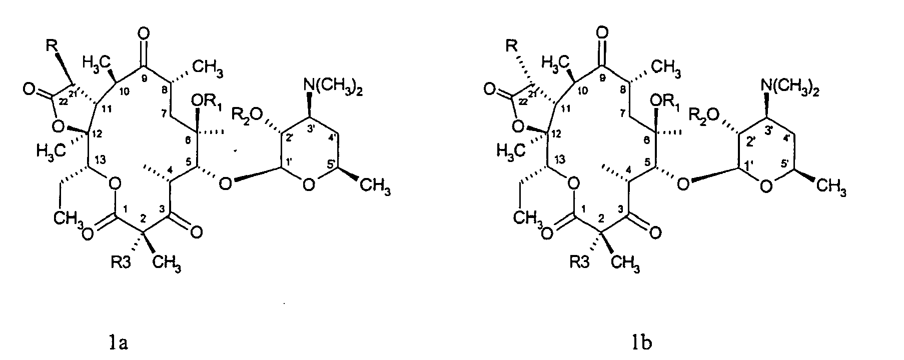

It is to be understood that the two diastereoisomers (1a, 1b) and mixtures thereof are encompassed within the scope of the present invention.

Compounds wherein R₂ represents a hydroxyl protecting group are in general intermediates for the preparation of other compounds of formula (I).

When the group OR₂ is a protected hydroxyl group this is a non-toxic protecting group, conveniently OR₂ is an acyloxy group (i.e. acetoxy or benzyloxy).

The term C₁₋₄ alkyl as used herein as a group or a part of the group refers to a straight or branched alkyl group containing from 1 to 4 carbon atoms; examples of such groups include methyl, ethyl, propyl, isopropyl, n-butyl, isobutyl and tert-butyl.

The term C₁₋₁₀ alkylene chain refers to straight or branched chain containing from 1 to 10 carbon atoms examples of such group include, but are not limited to methylene, ethylene, propylene, isopropylene, n-butylene, isobutylene, tert-butylene, pentylene, n-heptylene, n-octylene, n-nonylene and n-decylene.

The term C₂₋₁₀ alkenylene chain refers to a straight or branched alkylene chain containing from 2 to 10 carbon atoms and having at least one double bond; examples of such groups include ethylene, 2-propenylene, 1-propenylene, isopropenylene, 2-butenylene, 2-pentenylene, 2-hexenylene and the like.

The term C₂₋₁₀ alkynylene chain refers to a straight or branched alkylene chain containing from 2 to 12 carbon atoms and having at least one triple bond; examples of such groups include ethynylene, 2-propynylene, 1-propynylene, isopropynylene, 2-butynylene, 2-pentynylene, 2-hexenylene and the like.

The term halogen refers to a fluorine, chlorine, bromine or iodine atom.

When R₄ is a 5 or 6 membered heteroaryl group according to the invention this includes furanyl, thiophenyl, pyrrolyl, imidazolyl, thiazolyl, oxazolyl, pyrazolyl, isoxazolyl, isothiazolyl, 1,2,3-triazolyl, 1,2,3-oxadiazolyl, 1,2,3-thiadiazolyl, 1,2,4-triazolyl, 1,3,4-oxadiazolyl, 1,3,4-thiadiazolyl, 1,2,5-oxadiazolyl, 1,2,5-thiadiazolyl, pyridyl, pyridazinyl, pyrimidinyl, pyrazinyl, 1,2,4 oxadiazolyl, 1,2,5-triazinyl or 1,3,5-triazinyl and the like.

The term 9 to 10 membered fused bicyclic heterocyclic group refers to a 5,6/6,5 or 6,6 bicyclic ring system, containing at least one heteroatom selected from oxygen, sulphur or nitrogen, which may be saturated, unsaturated or aromatic. The term 9 to 10 membered fused bicyclic heterocyclic group also refers to a phenyl fused to one 5 or 6 membered heterocyclic group. Example of such groups include benzofuranyl, benzothiophenyl, indolyl, benzoxazolyl, 3H-imidazo[4,5-c]pyridin-yl, dihydrophthazinyl, 1H-imidazo[4,5-c]pyridin-1-yl, imidazo[4,5-b]pyridyl, 1,3 benzo[1,3]dioxolyl, 2H-chromanyl, isochromanyl, 5-oxo-2,3 dihydro-5H-[1,3]thiazolo[3,2-a]pyrimidyl, 1,3-benzothiazolyl, 1,4,5,6 tetrahydropyridaziyl, 1,2,3,4,7,8 hexahydropteridinyl, 2-thioxo2,3,6,9-tetrahydro-1H-purin-8-yl, 3,7-dihydro-1H-purin-8-yl, 3,4 dihydropyrimidin-1-yl, 2,3 -dihydro-1,4-benzodioxinyl, benzo[1,3]dioxolyl, 2H-chromenyl, chromanyl, 3,4-dihydrophthalazinyl, 2,3 dihydro-1H-indolyl, 1,3-dihydro-2H-isoindol-2-yl, 2,4,7-trioxo-1,2,3,4,7,8-hexahydropteridinyl, thieno[3,2-d]pyrimidinyl, 4-oxo-4,7-dihydro-3H-pyrrolo[2,3-d]pyrimidinyl, 1,3 dimethyl-6-oxo-2-thioxo-2,3,6,9-tetrahydro-1H-purinyl, 1,2 dihydroisoquinolinyl, 2-oxo-1,3-benzoxazolyl, 2,3-dihydro-5H-1,3-thiazolo[3,2-a]pyrimidinyl, 5,6,7,8-tetrahydro-quinazolinyl, 4-oxochromanyl, 1,3-benzothiazolyl, benzimidazolyl, benzotriazolyl, purinyl, furylpyridyl, thiophenylpyrimidyl, thiophenylpyridyl, pyrrolylpiridyl, oxazolylpyridyl, thiazolylpiridyl, 3,4 dihydropyrimidin-1-yl imidazolylpiridyl, quinoliyl, isoquinolinyl, quinazolinyl, quinoxalinyl , naphthyridinyl, pyrazolyl[3.4]pyridine, 1,2 di hydroisoquinolinyl, cinnolinyl, 2,3dihydro-benzo[1,4]dioxin-6-yl, 4,5.6,7-tetrahydro-benzo[b]thiophenyl-2-yl, 1.8naphthyridinyl, 1,6naphthyridinyl, 3,4 dihydro-2H-1,4-benzothiazine, 4,8-Dihydroxy-quinolinyl, 1-oxo-1,2-dihydro-isoquinolinyl or 4-phenyl-[1,2,3]thiadiazolyl and the like.

The term 5 or 6 membered heterocyclic group refers to 5 or 6 ring member containig at least one heteroatom selected from oxygen, sulphur or nitrogen, which may be saturated, unsaturated. Examples of such groups include piperidyl, 2-oxodihydrofuranyl, piperazinyl, morpholinyl, pyrazolidinyl, 1,2 dihydro-3H-pyrazolyl ,imidazolidinyl or pyrrolidinyl and the like.

The term 9 to 10 membered fused bicyclic carbocyclic group refers to a 5,6/6,5 or 6,6 bicyclic carbocyclic ring system which may be saturated, unsaturated or aromatic. It also refers to a phenyl fused to one 5 or 6 membered saturated or unsaturated carbocyclic group. Examples of such groups include naphthyl, 1, 2, 3, 4 tetrahydronaphthyl, indenyl or indanyl and the like.

The term optionally substituted phenyl, optionally substituted 5-6 membered heterocyclic group, optionally substituted 9 to 10 membered fused bicyclic carbocyclic group, optionally substituted 9 to 10 membered fused bicyclic heterocyclic group or optionally substituted 5 or 6 membered heteroaryl group this refers to a 5-6 membered heterocyclic, a 9 to 10 membered fused bicyclic carbocyclic, a 9 to 10 fused bicyclic heterocyclic or 5 or 6 membered heteroaryl as defined above which is substituted by 1 to 4 groups, which may be the same or different, selected from (CH₂)ₚR₁₀ group wherein p is zero or an integer from 1 to 4 and R₁₀ is selected from:
halogen,
C₁₋₄alkoxy,
C₁₋₄alkyl,
hydroxy,
cyano,
nitro,
oxo,
trifluoromethyl,
carboxy,
NR₈R₉,
COR₈,
CONR₈R₉,
NHCOR₈,
NHSO₂R₈,
S(O)qR₆ (wherein q is 0 or an integer from 1 to 2),
phenyl (optionally substituted by halogen, C₁₋₄alkoxy or NR₈R₉);
phenoxy;
5-membered heteroaryl containing at least 1 heteroatoms selected from oxygen, sulphur or nitrogen and a 6-membered heteroaryl group containing at least 1 nitrogen atom which 5-6membered heteroaryl may be substituted by C₁₋₄alkyl or cyano.
   or
9 or 10 membered fused bicyclic heterocyclic .

When R₄ is an optionally substituted C₃₋₇ cycloalkyl, such a group is optionally substituted by 1 or 2 substituents which may be the same or different and selected from C₁₋₄ alkyl, halogen, cyano, nitro, trifluoromethyl and NR₆R₇.

When X is a C₁₋₁₀ alkylene, a C₂₋₁₀ alkynylene or a C₂₋₁₀ alkenylene chain which is interrupted by a bivalent radical group selected from -O- , NR₈-, -C(O)-, -N(R₈)C(Y)N(R₉)-, -S(O)m-, -N(R₈)C(O)-, -C(O)N(R₈)-, N(R₈)C(O)C(O)-, -C(O)O- or -C(NOR₆)-, this refers for example to C₁₋₁₀ alkylene-O-, C₁₋₁₀alkylene-NR₈C(Y)NR₉-, C₁₋₁₀alkylene-NR₈-, C₁₋₁₀ alkylene-C(O)-, C₁₋₁₀alkylene-S(O)m-, C₁₋₁₀ alkylene-NR₈C(O)-, C₁₋₁₀ alkylene-C(O)NR₈-, C₁₋₁₀ alkylene-N(R₈)C(O)C(O)-, C₁₋₁₀ alkylene-C(O)O-, C₁₋₁₀ alkylene C(NOR₆), C₂₋₁₀alkenylene-O-, C₂₋₁₀alkenylene-NR₈-, C₂₋₁₀alkenylene-C(O)-, C₂₋₁₀alkenylene-NR₈C(Y)NR₉-, C₂₋₁₀ alkenylene-S(O)m-, C₂₋₁₀ alkenylene-NR₈C(O)-, C₂₋₁₀ alkenylene-C(O)NR₈-, C₂₋₁₀ alkenylene-N(R₈)C(O)C(O)-, C₂₋₁₀ alkenylene-C(O)O-, C₂₋₁₀ alkenylene-C(NOR₆), C₂₋₁₀alkynylene-O-, C₂₋₁₀alkynylene-NR₈-, C_{2- 10}alkynylene-C(O)-, C₂₋₁₀alkynylene-NR₈C(Y)NR₉-, C₂₋₁₀alkynylene-S(O)m-, C₂₋₁₀alkynylene-NR₈C(O)-, C₂₋₁₀ alkynylene-C(O)NR₈-, C₂₋₁₀alkynylene-N(R₈)C(O)C(O)-, C₂₋₁₀ alkynylene-C(O)O-, C₂₋₁₀ alkynylene-C(NOR₆),
or this refers to a C₁₋₁₀ alkylene, a C₂₋₁₀ alkenylene or a C₂₋₁₀ alkynylene chain containing a bivalent radical group selected from:
-O-, NR₈-, -C(O)-, -NR₈C(Y)NR₉-, -S(O)m-, NR₈C(O)-, -C(O)NR₈-.

When A is -N(R₆)-, -N(R₆)S(O)₂- or -N(R₆)C(Y)N(R₇) and when X is an optionally substituted C₁₋₁₀ alkylene interrupted by a bivalent radical selected from -O-, -N(R₈)-,-N(R₈)C(Y)N(R₉)-, -S(O)m-, -N(R₈)C(O)- or -N(R₈)C(O)C(O)- said bivalent radicals are preferably linked to A group by an optionally substituted alkylene chain containing at least two carbon atoms.

When A is -N(R₆)-, N(R₆)S(O)₂- or -N(R₅)C(Y)N(R₇) and when X is an optionally substituted C₂₋₁₀ alkenylene or an optionally substituted C₂₋₁₀ alkynylene chain and when these chains are interrupted by a bivalent radical selected from -O-, -N(R₈)-,-N(R₈)C(Y)N(R₉)-, -S(O)m-, -N(R₈)C(O)- or -N(R₈)C(O)C(O)- said bivalent radicals are preferably linked to the A group by an optionally substituted alkenylene or alkynylene chain containing at least 4 carbon atoms and having -CH₂- as terminal groups.

A preferred group of compounds of formula (I) are those in which the carbon atom shown as 21 is in the β configuration.

R is preferably (CH₂)ₙA-X-R₄ or (CH₂)ₙR_{5.}

R₁ is preferably methyl or 2-propenyl.

R₂ is preferably hydrogen.

R₃ is preferably hydrogen or fluorine.

When R₄ is a 5 or 6 membered heteroaryl group this is preferably imidazolyl, imidazolyl, pyrazolyl, thiophenyl, 1,2,3-triazolyl, pyridinyl or furanyl.

When R₄ or R₅ is a 5 or 6 membered heterocyclic group this is preferably imidazolidinyl or pyrrolidinyl.

When R₄ is a 9 or 10 membered fused bicyclic heteroaryl group this is preferably quinolinyl, quinoxalinyl, indolyl, purinyl, 1,3 benzo[1,3]dioxolyl, benzothiazolyl, 1H-benzimidazol-yl 1,3-benzoxazoyl, 1H-pyrrolo[2,3-b]pyridinyl, 1,3-dihydro-2H-isoindolyl, 3H-imidazo[4,5-c]pyridin-3-yl, 3H-imidazo[4,5-b]pyridin-3-yl, 7H-purin-7-yl, 1H-imidazo[4,5-c]pyridin-1-yl, 4,5,6,7-tetrahydro-benzo[b]thiophen-2-yl.

R₅ is preferably 1-pyrrolidinyl which is optionally substituted by one oxo or benzo[1,3]dioxolyl.

X is preferably a C₁₋₅ alkylene, a C₂₋₅ alkenylene or a C₂₋₅ alkynylene chain wherein said chains are:
i) optionally interrupted by a bivalent radical group selected from -O-, -N(R₈)-,-C(O)-, -N(R₈)C(Y)N(R₉)-, -S(O)m-, -N(R₈)C(O)-, -C(O)N(R₈)-, -N(R₈)C(O)C(O)-, -C(O)O- or -C(NOR₆)-and/or
ii) optionally substituted by one or two groups selected from:
   C₁₋₄ alkyl, oxo, C₁₋₄ alkoxy, halogen, cyano, phenoxy, hydroxy, NR₈R₉, N(R₈)C(O)R₉, =NOR₆, NR₈C(Y)NR₉ or optionally substituted phenyl.

n is preferably 0 or 1.

Preferred compounds of the invention are those wherein A is selected from -NH-, -NHC(O)- or -NHC(Y)NH-. Within this class the compounds in which n is 0 or 1 are particular preferred.

A preferred class of compounds of formula (I) are those wherein X is a C₁₋₄ alkylene chain which is optionally interrupted by a bivalent radical selected from -O-, -NH-, -C(O)-,-NHC(O)-, -S(O)2- -S- and /or such a C₁₋₄ alkylene chain is optionally substituted by one group selected from NH₂, C₁₋₄ alkyl, oxo or N-OH.

A particularly preferred group of compounds of formula (I) is that wherein R₄ is phenyl (optionally substituted by 1 to 3 groups which may be the same or different selected from nitro, amino, methyl, C₁₋₄ alkoxy ie methoxy or hydroxy), 1-imidazolyl (optionally substituted by 1 to 3 groups which may be the same or different selected from pyridin-2-yl, pyridin-3-yl, pyridin-4-yl, phenyl, m-nitrophenyl, dichlorophenyl, C₁₋₄ alkyl i.e methyl, trifluoromethylphenyl, thiophen-2-yl, thiazol-2-yl), 3-trifluoramethylpyrazol-4-yl, 1-pyrazolyl (optionally substituted by 1 to 3 groups which may be the same or different selected from alogen (i.e. chlorine, fluorine), pyridin-2-yl, pyridin-4-yl, quinolin-2-yl, quinolin-4-yl, quinoxalin-2-yl, pyrimidin-4-yl, C₁₋₄ alkyl i.e methyl, 1,3 benzooxazol-2-yl, p-chloro phenyl, difluoro phenyl, pyrazin-2-yl thiazol-5-yl, 1H-indol-3-yl, 1H-indol-2-yl, 3-methoxy-quinoxalin-2-yl, 2-quinolinyl 3-quinolinyl, 4-quinolinyl, 4-pyridinyl, 3-pyridinyl(optionally substituted by one amino), 5methyl furan-2-yl, 3-thiophenyl, 6-methoxy-7H-purin-7-yl, quinoxalin-2-yl, 3-methoxy quinoxalin-2-yl 6-methoxy-2-oxol, 3-benzoxazol-3(2H)-yl, 1H-pyrrolo[2,3-b]pyridin-1-yl, 2-(methylthio)-1H-benzimidazol-1-yl, 1,3-dioxo-1,3-dihydro-2H-isoindol-2-yl, 6-methoxy-2-oxo-1,3-benzoxazol-3(2H)-yl, 3H-imidazo[4,5-b]pyridin-3-yl, 1,3-benzoxazol-2-yl, benzothiazol-2-yl, 1,3 benzo[1,3]dioxolyl, 3-(5-cyano-3,4-dimethylthien-2-yl)-1H-1,2,4-triazol-1-yl, 2,3-dihydro-benzo[1,4]dioxin-6-yl, 2,4-dimethyl-1,3-thiazol-5-yl or 4-oxo-4,5,6,7-tetrahydro-benzo[b]thiophen-2-yl.

A particularly preferred group of compounds of formula (I) are those wherein R₁ is methyl, R₂ or R₃ is hydrogen, A is -NH-, -NHC(O)-, X is C₁₋₄ alkylene chain which is optionally interrupted by a bivalent radical selected from -O-, -NH-, -C(O)-, -NHC(O)-, -S(O)2- -S- and /or such a C₁₋₄ alkylene chain is optionally substituted by one group selected from NH₂, C₁₋₄ alkyl, oxo or N-OH, R₄ is a group selected from 4-(pyridin-3-yl)-imidazol-1-yl, 4-(pyridin-3-yl)-imidazol-1-yl, quinoxalin-2-yl, quinoxalin-2-yl, quinolin-4-yl, quinoxalin-2-yl, 4-(2,3-dihydro-benzo[1,4]dioxin-6-yl, 4-oxo-4,5,6,7-tetrahydro-benzo[b]thiophen-2-yl, 4-methoxy-3-nitro-phenyl, 2-hydroxy-4,5-dimethoxy-phenyl, 3-hydroxy-4-methoxy-phenyl, 3,4-dimethoxy-phenyl, 4-hydroxy-3-methoxy-phenyl, 3-methoxy-quinoxalin-2-yl, 3-amino-4-methoxy-phenyl, 4-(pyridin-3-yl)-imidazol-1-yl, quinolin-4-yl, 4-pyrimidin-4-yl-pyrazol-1-yl, 2-(methylthio)-1H-benzimidazol-1-yl, 3-[3-(4-chlorophenyl)-1H-pyrazol-5-yl]propylamino)-methylene], 6-methoxy-2-oxo-1,3-benzoxazol-3(2H)-yl, 1H-pyrrolo[2,3-b]pyridin-1-yl, 3-(2,4-dimethyl-1,3-thiazol-5-yl)-1H-pyrazol-1-yl, 4-phenyl-1H-imidazol-1-yl, 4-pyridin-4-yl-1H-imidazol-1-yl, thiophen-2-yl, 3-(5-cyano-3,4-dimethylthien-2-yl)-1H-1,2,4-triazol-1-yl, quinolin-3-yl, 1,3-thiazol-2-yl and n is 0 or 1.

Particularly preferred compounds of the invention are selected from:
(11S, 21R)-3-decladinosyl-11,12-dideoxy-6-O-methyl-3-oxo-12,11-[oxycarbonyl-(4-(4-(pyridin-3-yl)-imidazol-1-yl)-butyramidomethyl)-methylene]-erythromycin A;
(11S, 21R)-3-decladinosyl-11,12-dideoxy-6-O-methyl-3-oxo-12,11-[oxycarbonyl-(3-(4-(pyridin-3-yl)-imidazol-1-yl)-propionamidomethyl)-methylene]-erythromycin A;
(11S,21R)-3-decladinosyl-11,12-dideoxy-6-O-methyl-3-oxo-12,11-[oxycarbonyl-(2-(quinoxalin-2-ylsulfanyl)-acetamidomethyl)-methylene]-erythromycin A;
(11S,21R)-3-decladinosyl-11,12-dideoxy-6-O-methyl-3-oxo-12,11-[oxycarbonyl-(3-(quinoxalin-2-ylsulfanyl)-propionamidomethyl)-methylene]-erythromycin A;
(11S,21R)-3-decladinosyl-11,12-dideoxy-6-O-methyl-3-oxo-12,11-[oxycarbonyl-[(quinolin-4-ylmethyl)-amino]-methyl)-methylene]-erythromycin A;
(11S, 21R,S)-3-decladinosyl-11,12-dideoxy-6-O-methyl-3-oxo-12, 11-[oxycarbonyl-(3-(quinolin-4-yl)-propionamido)-methylene]-erythromycin A;
(11S,21R,S)-3-dedadinosyl-11,12-dideoxy-6-O-methyl-3-oxo-12,11-[oxycarbonyl-(2-(quinoxalin-2-ylsulfanyl)-acetamido)-methylene]-erythromycin A;
(11S, 21R,S)-3-decladinosyl-11,12-dideoxy-6-O-methyl-3-oxo-12,11-[oxycarbonyl-((4-(2,3-dihydro-benzo[1,4]dioxin-6-yl)-4-oxo)-butyramido)-methylene]-erythromycin A;
(11S,21R,S)-3-decladinosyl-11,12-dideoxy-6-O-methyl-3-oxo-12,11-[oxycarbonyl-(4-(4-oxo-4,5,6,7-tetrahydro-benzo[b]thiophen-2-yl)-4-oxo-butyramido)-methylene]-erythromycin A;
(11S,21R,S)-3-decladinosyl-11,12-dideoxy-6-O-methyl-3-oxo-12, 11-[oxycarbonyl-(4-(4-methoxy-3-nitro-phenyl)-4-oxo-butyramido)-methylene]-erythromycin A;
(11S,21R,S)-3-decladinosyl-11,12-dideoxy-6-O-methyl-3-oxo-12,11-[oxycarbonyl-(4-(2-hydroxy-4,5-dimethoxy-phenyl)-4-oxo-butyramido)-methylene]-erythromycin A;
(11S,21R,S)-3-decladinosyl-11,12-dideoxy-6-O-methyl-3-oxo-12,11-[oxycarbonyl-(4-(3-hydroxy-4-methoxy-phenyl)-4-oxo-butyramido)-methylene]-erythromycin A;
(11S,21R,S)-3-decladinosyl-11,12-dideoxy-6-O-methyl-3-oxo-12,11-[oxycarbonyl-(4-(3,4-dimethoxy-phenyl)-4-oxo-butyramido)-methylene]-erythromycin A;
(11S,21R,S)-3-decladinosyl-11,12-dideoxy-6-O-methyl-3-oxo-12,11-[oxycarbonyl-(4-(4-hydroxy-3-methoxy-phenyl)-4-oxo-butyramido)-methylene]-erythromycin A;
(11S,21R,S)-3-decladinosyl-11,12-dideoxy-6-O-methyl-3-oxo-12,11-[oxycarbonyl-(2-(3-methoxy-quinoxalin-2-ylsulfanyl)-acetamido)-methylene]-erythromycin A;
(11S,21R,S)-3-dedadinosyl-11,12-dideoxy-6-O-methyl-3-oxo-12,11-[oxycarbonyl-(2-(quinoxalin-2-yloxy)-acetamido)-methylene]-erythromycin A;
(11S,21R,S)-3-decladinosyl-11,12-dideoxy-6-O-methyl-3-oxo-12,11-[oxycarbonyl-(4-(3-amino-4-methoxy-phenyl)-4-oxo-butyramide)-methylene]-erythromycin A;
(11S,21R,S)-3-decladinosyl-11,12-dideoxy-6-O-methyl-3-oxo-12,11-[oxycarbonyl-(4-hydroxymino-4-(4-methoxy-3-nitro phenyl)-butyramide)-methylene]-erythromycin A;
(11S,21R,S)-3-decladinosyl-11,12-dideoxy-6-O-methyl-3-oxo-12,11-[oxycarbonyl-(2-(quinoxaline-2-sulfonyl)-acetamide)-methylene]-erythromycin A;
(11S,21R,S)-3-decladinosyl-11,12-dideoxy-6-O-methyl-3-oxo-12,11-[oxycarbonyl-(3-(4-(pyridin-3-yl)-imidazol-1-yl)-propylamino)-methylene]-erythromycin A;
(11S,21R,S)-3-decladinosyl-11,12-dideoxy-6-O-methyl-3-oxo-12,11-[oxycarbonyl-(2-(4-(pyridin-3-yl)-imidazol-1-yl)-ethylamino)-methylene]-erythromycin A;
(11S,21R,S)-3-decladinosyl-11,12-dideoxy-6-O-methyl-3-oxo-12,11-[oxycarbonyl-(4-(quinolin-4-yl)-butylamino)-methylene]-erythromycin A;
(11S,21R,S)-3-decladinosyl-11,12-dideoxy-6-O-methyl-3-oxo-12,11-[oxycarbonyl-(2-(quinolin-4-yl)-ethylamino)-methylene]-erythromycin A;
(11S,21R,S)-3-decladinosyl-11,12-dideoxy-6-O-methyl-3-oxo-12,11-[oxycarbonyl-(3-(4-pyrimidin-4-yl-pyrazol-1-yl)-propylamino)-methylene]-erythromycin A;
(11S,21R,S)-3-decladinosyl-11,12-dideoxy-6-O-methyl-3-oxo-12,11-[oxycarbonyl-(3-[2-(methylthio)-1H-benzimidazol-1-yl]propylamino)-methylene]-erythromycin A;
(11S,21R,S)-3-decladinosyl-11,12-dideoxy-6-O-methyl-3-oxo-12,11-[oxycarbonyl-(3-[3-(4-chlorophenyl)-1H-pyrazol-5-yl]propylamino)-methylene]-erythromycin A;
(11S,21R,S)-3-decladinosyl-11,12-dideoxy-6-O-methyl-3-oxo-12,11-[oxycarbonyl-(3-(6-methoxy-2-oxo-1,3-benzoxazol-3(2H)-yl)propylamino)-methylene]-erythromycin A;
(11S,21R,S)-3-decladinosyl-11,12-dideoxy-6-O-methyl-3-oxo-12,11-[oxycarbonyl-(3-(1H-pyrrolo[2,3-b]pyridin-1-yl)propylamino)-methylene]-erythromycin A;
(11S,21R,S)-3-decladinosyl-11,12-dideoxy-6-O-methyl-3-oxo-12,11-[oxycarbonyl-(3-[3-(2,4-dimethyl-1,3-thiazol-5-yl)-1H-pyrazol-1-yl]propylamino)-methylene]-erythromycin A;
(11S, 21RS)-3-decladinosyl-11,12-dideoxy-6-O-methyl-3-oxo-12,11-[oxycarbonyl-(3-(quinoxalin-2-ylsulfanyl)-propylamino)-methylene]-erythromycin A;
(11S, 21R,S)-3-decladinosyl-11,12dideoxy-6-O-methyl-3-oxo-12,11-[oxycarbonyl-(3-(4-phenyl-1H-imidazol-1-yl)propyl)amino)-methylene]-erythromycin A;
(11S, 21R,S)-3-decladinosyl-11,12-dideoxy-6-O-methyl-3-oxo-12,11-[oxycarbonyl-(2-(4-pyridin-4-yl-1H-imidazol-1-yl)ethylamino)-methylene]-erythromycin A;
(11S, 21R,S)-3-decladinosyl-11,12-dideoxy-6-O-methyl-3-oxo-12,11-[oxycarbonyl-(2-(quinoxalin-2-ylsulfanyl)-ethylamino)-methylene] -erythromycin A;
(11S, 21S)-3-decladinosyl-11,12-dideoxy-6-O-methyl-3-oxo-12,11-[oxycarbonyl-(3-(4-(thiophen-2-yl)-imidazol-1-yl)-propylamino)-methylene]-erythromycin A;
(11S, 21R,S)-3-decladinosyl-11,12-dideoxy-6-O-methyl-3-oxo-12,11-[oxycarbonyl-((3-[3-(5-cyano-3,4-dimethylthien-2-yl)-1H-1,2,4-triazol-1-yl]propyl)amino)-methylene]-erythromycin A;
(11S, 21R,S)-3-decladinosyl-11,12-dideoxy-6-O-methyl-3-oxo-12,11-[oxycarbonyl-((3-quinolin-3-ylpropyl)amino)-methylene]-erythromycin A;
(11S, 21R,S)-3-decladinosyl-11,12-dideoxy-6-O-methyl-3-oxo-12,11-[oxycarbonyl-((3-[4-(3-nitrophenyl)-1H-imidazol-1-yl]propyl)amino)-methylene]-erythromycin A;
(11S, 21R,S)-3-decladinosyl-11,12-dideoxy-6-O-methyl-3-oxo-12,11-[oxycarbonyl-((2-[3-(1,3-thiazol-2-yl)-1H-pyrazol-1-yl]ethyl)amino)-methylene]-erythromycin A;
(11S, 21R,S)-3-decladinosyl-11,12-dideoxy-6-O-methyl-3-oxo-12,11-[oxycarbonyl-((2-(4-phenyl-1H-imidazol-1-yl)ethyl)amino)-methylene]-erythromycin A;
(11S, 21R,S)-3-decladinosyl-11,12-dideoxy-2-fluoro-6-O-methyl-3-oxo-12,11-[oxycarbonyl-(2-(quinoxalin-2-ylsulfanyl)-acetamido)-methylene]-erythromycin A;
(11S, 21R,S)-3-decladinosyl-11,12-dideoxy-2-fluoro-6-O-methyl-3-oxo-12,11-[oxycarbonyl-(3-(4-(pyridin-3-yl)-imidazol-1-yl)-propylamino)-methylene]-erythromycin A.

Further preferred compounds of the invention include:
(11S,21S)-3-decladinosyl-11,12-dideoxy-6-O-methyl-3-oxo-12,11-[oxycarbonyl-(2-(4-(pyridin-3-yl)-imidazol-1-yl)-ethylamino)-methylene]-erythromycin A;
(11S,21S)-3-decladinosyl-11,12-dideoxy-6-O-methyl-3-oxo-12,11-[oxycarbonyl-(2-(quinolin-4-yl)-ethylamino)-methylene]-erythromycin A;
(11S,21S)-3-decladinosyl-11,12-dideoxy-6-O-methyl-3-oxo-12,11-[oxycarbonyl-(3-(4-(pyridin-3-yl)-imidazol-1-yl)-propylamino)-methylene]-erythromycin A;
(11S,21S)-3-decladinosyl-11,12-dideoxy-6-O-methyl-3-oxo-12,11-[oxycarbonyl-(2-(quinoxalin-2-ylsulfanyl)-acetamido)-methylene]-erythromycin A;
(11S, 21S)-3-decladinosyl-11,12-dideoxy-6-O-methyl-3-oxo-12,11-[oxycarbonyl-((4-(2,3-dihydro-benzo[1,4]dioxin-6-yl)-4-oxo)-butyramido)-methylene]-erythromycin A;
(11S,21S)-3-dedadinosyl-11,12-dideoxy-6-O-methyl-3-oxo-12,11-[oxycarbonyl-(4-(4-methoxy-3-nitro-phenyl)-4-oxo-butyramido)-methylene]-erythromycin A;
(11S,21S)-3-decladinosyl-11,12-dideoxy-6-O-methyl-3-oxo-12, 11-[oxycarbonyl-(4-(2-hydroxy-4,5-dimethoxy-phenyl)-4-oxo-butyramido)-methylene]-erythromycin A;
(11S,21S)-3-demadinosyl-11,12-dideoxy-6-O-methyl-3-oxo-12,11-[oxycarbonyl-(4-(4-oxo-4,5,6,7-tetrahydro-benzo[b]thiophen-2-yl)-4-oxo-butyramido)-methylene]-erythromycin A;
(11S,21S)-3-decladinosyl-11,12-dideoxy-6-O-methyl-3-oxo-12,11-[oxycarbonyl-(3-(4-quinolin-2-yl-1H-pyrazol-1-yl)propylamino)-methylene]-erythromycin A;
(11S,21S)-3-decladinosyl-11,12-dideoxy-6-O-methyl-3-oxo-12,11-[oxycarbonyl-(4-(3,4-dimethoxy-phenyl)-4-oxo-butyramido)methylene]-erythromycin A;
(11S,21S)-3-decladinosyl-11,12-dideoxy-6-O-methyl-3-oxo-12,11-[oxycarbonyl-(-4-(4-hydroxy-3-methoxy-phenyl)-4-oxo-butyramido)-methylene]-erythromycin A;
(11S,21S)-3-decladinosyl-11,12-dideoxy-6-O-methyl-3-oxo-12,11-[oxycarbonyl-(3-(4-(thiophen-2-yl)-imidazol-1-yl)-propylamino)-methylene]-erythromycin A.

Compounds according to the invention also exhibit a broad spectrum of antibacterial activity against a wide range of clinical pathogenic microorganisms.
For example, using a standard microtiter broth serial dilution test, compounds of the invention have been found to exhibit useful levels of activity against a wide range of pathogenic microorganisms including Staphylococcus aureus, Streptococcus pneumoniae, Moraxella catarrhalis, Streptococcus pyogenes, Haemophilus influenzae.
Furthermore compounds of the invention are also active against intracellular pathogens such as Chlamydia pneumonia, Clamydia spp, Legionella pneumophila, Mycoplasma pneumonia, species.
The compounds of the invention may therefore be used for treating a variety of diseases caused by pathogenic bacteria in human beings and animals.

Thus, according to another aspect of the present invention, we provide a compound of formula (I) or a physiologically acceptable salt thereof for use in the therapy in a human or animal subject.

According to a further aspect of the invention we provide the use of a compound of formula (I) or a physiologically acceptable salt thereof for the manufacture of a therapeutic agent for the treatment of systemic or topical bacterial infections in a human or animal body.

According to a yet further aspect of the invention we provide a method of treatment of the human or non-human animal body to combat bacterial infections which method comprises administering to the body an effective amount of a compound of formula (I) or a physiologically acceptable salt thereof.

The term treatment is also meant to include prophylaxis.

While it is possible that, for use in therapy, a compound of the invention may be administered as the raw chemical, it is preferable to present the active ingredient as a pharmaceutical formulation.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in conventional manner with the aid of one or more suitable carriers or excipients. The compositions of the invention include those in a form especially formulated for parenteral, oral, buccal, rectal, topical, implant, ophthalmic, nasal or genito-urinary use.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories, e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, lotions, shampoos, powders, (including spray powders), pessaries, tampons, sprays, dips, aerosols, drops (e.g. eye ear or nose drops) or pour-ons.

Aerosol sprays are conveniently delivered from pressurised packs, with the use of a suitable propellant, e.g. dichlorodifluoromethane, trichlorofluoromethane, dichlorotetrafluoroethane, carbon dioxide or other suitable gas.

For topical administration by inhalation the compounds according to the invention may be delivered for use in human or veterinary medicine via a nebuliser.

The pharmaceutical compositions for topical administration may also contain other active ingredients such as corticosteroids or antifungals as appropriate.

The compositions may contain from 0.01-99% of the active material. For topical administration, for example, the composition will generally contain from 0.01-10%, more preferably 0.01-1% of the active material.

For systemic administration the daily dose as employed for adult human treatment it will range from 2-100mg/kg body weight, preferably 5-60 mg/kg body weight, which may be administered in 1 to 4 daily doses, for example, depending on the route of administration and the condition of the patient. When the composition comprises dosage units, each unit will preferably contain 200 mg to 1 g of active ingredient.
The duration of treatment will be dictated by the rate of response rather than by arbitrary numbers of days.

Compounds of general formula (I) and salts thereof may be prepared by general method outlined hereinafter. In the following description, the groups R₁, R₂, R₃, R₄, R₅, R₆, R₇, R₈, R₉, R₁₀, n, m, p, q, X, Y and A have the meaning defined for the compounds of formula (1) unless otherwise stated.

Compounds of formula (I), wherein A is -N(R₆)C(O)- or a -N(R₆)S(O₂)-, may be prepared by reaction of compounds of formula (II) 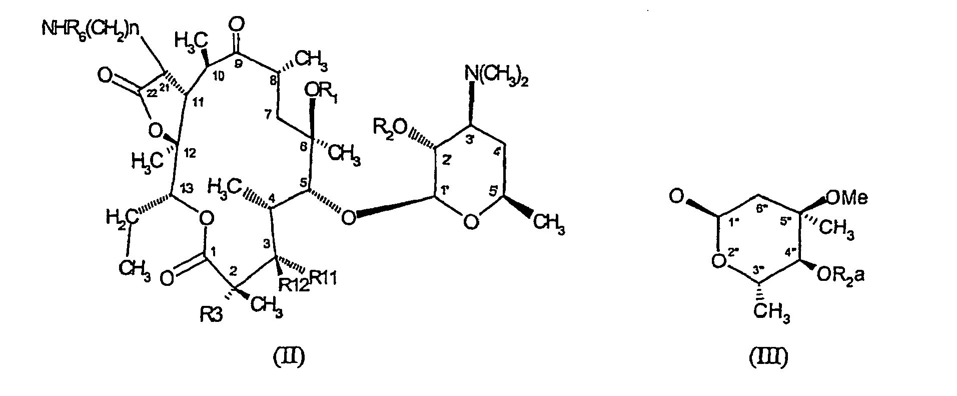 wherein R₁₁ is a cladinose derivative of formula (III), in which R₂ₐ is a hydroxy protecting group, or hydroxy, R₁₂ is hydrogen or R₁₂ together R₁₁ is an oxygen atom, with a suitable activated derivative of the acid (IV), HOC(O)XR₄ (IV) or with a suitable activated derivative of the sulfonic acid (V) HOS(O)₂XR₄ (V) respectively and, if required, subjecting the resulting compound to one or more of the following operations a) hydrolysis of the cladinose derivative (III); b) conversion of the 3-hydroxy group into the 3-oxo; c) removal of the protecting group R₂.

Suitable activated derivatives of the carboxyl group or the sulphonic acid include the corresponding acyl halide, mixed anhydride or activated ester such as a thioester or a pentafluoroester.
The reaction is preferably carried out in a suitable aprotic solvent such as halohydrocarbon (e.g. dichloromethane) or N,N-dimethylformamide optionally in the presence of a tertiary base such as pyridine, dimethylaminopyridine or triethylamine and at a temperature within the range of 0° to 120°C.

Compounds of formula (1) wherein A is -N(R₆)C(Y)N(R₇)- and R₇ is optionally substituted phenyl or C₁₋₄ alkyl, may be prepared from compounds of formula (II), wherein R₁₁ and R₁₂ have the meaning defined above, by reaction with a compound of formula R₄XNR₇C(Y)L (VI), wherein L is a suitable leaving group as above defined and R₇ is phenyl or C₁₋₄ alkyl, if required, subjecting the resulting compound to one or more of the following operations a) hydrolysis of the cladinose derivative (III); b) conversion of the 3-hydroxy group into the 3-oxo and c) removal of the protecting group R₂.

Compounds of formula (I) wherein A is -N(R₆)C(Y)NH- may be prepared from compounds of formula (II)), wherein R₁₁ and R₁₂ have the meaning defined above by reaction with a compounds of formula R₄XN=C=Y (VII), if required, subjecting the resulting compound to one or more of the following operations a) hydrolysis of the cladinose derivative (III); b) conversion of the 3-hydroxy group into the 3-oxo and c) removal of the protecting group R₂.

Compounds of formula (I) wherein A is N(R₆)- may be prepared from compounds of formula (II)), wherein R₁₁ and R₁₂ have the meaning defined above, by reaction with a compounds of formula R₄XL (VIII), wherein L is a suitable leaving group.
If required, subjecting the resulting compound to one or more of the following operations a) hydrolysis of the cladinose derivative (III); b) conversion of the 3-hydroxy group into the 3-oxo and c) removal of the protecting group R₂.
Suitable leaving groups for this reaction include halogen (e.g. chlorine, bromine or iodine) or sulfonyl (e.g. tosyl or methansulfonyl).

Compounds of formula (I) wherein R is A is a N(R₆)C(O)O group, in which R₆ is hydrogen, phenyl or C₁₋₄ alkyl, may be prepared from compounds of formula (II), wherein R₁₁ and R₁₂ have the meaning defined above, by reaction with the appropriate haloformate compound of formula R₄XOC(O)L (IX) wherein L is a suitable leaving group such as halogen (e.g. chlorine or bromine) and, if required, subjecting the resulting compound to one or more of the following operations a) hydrolysis of the cladinose derivative (III); b) conversion of the 3-hydroxy group into the 3-oxo and c) removal of the protecting group R₂.

The reactions of compounds (II) with compounds (VI), (VII), (VIII) or (IX) are conveniently carried out in a solvent such as tetrahydrofuran, acetonitrile or halohydrocarbon (e.g. dichloromethane) optionally in the presence of a base such as triethylamine and at a temperature within the range 0° to 80°C.

Compounds of formula (I) wherein A is a N=C(R₆) group, may be prepared from compounds of formula (II), wherein R₁₁ is hydroxy, R₁₂ is hydrogen or R₁₁ together R₁₂ is an oxygen atom, by reaction with a compound of formula R₄XCHO (X) and, if required, subjecting the resulting compound to one or more of the following operations a) conversion of the 3-hydroxy group into the 3-oxo and b) removal of the protecting group R₂.
The reaction is preferably carried out in a solvent such as a halohydrocarbon e.g. dichloromethane at a temperature within the range 0° to 50°C.

Compounds of formula (I), wherein A is N[C(O)R₆] may be prepared by treating a compound of formula (XI) in which R₁₁ and R₁₂ have the meaning as defined for compounds of formula (II), by acylation reaction with the activated carboxylic acid of formula(XIa) R₆COOH (XIa). 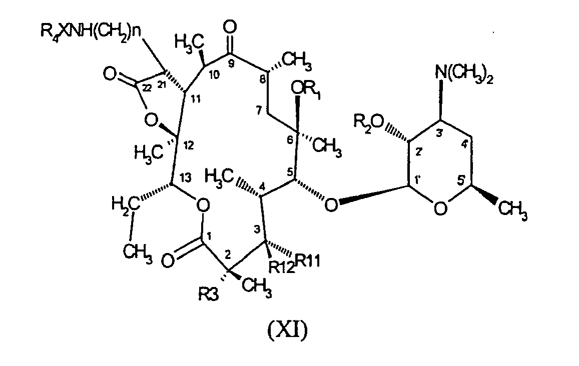 and, if required, subjecting the resulting compound to one or more of the following operations a) hydrolysis of the cladinose derivative (III); b) conversion of the 3-hydroxy group into the 3-oxo and c) removal of the protecting group R₂.
The reaction is preferably carried out in the presence of a base such as a tertiary amine e.g. triethylamine or pyridine in a solvent such as a halohydrocarbon e.g. dichloromethane at a temperature within the range 0° to 50°C.
Suitable activated derivatives of the carboxyl group or the sulphonic acid include the corresponding acyl halide, mixed anhydride or activated ester such as a thioester or a pentafluoroester.

Compounds of formula (I) in which R is hydrogen may be prepared by decarboxylation of a compound of formula (XII), wherein R₁₁ and R₁₂ have the meaning as defined for compounds of formula (II), followed, if required, subjecting the resulting compound to one or more of the following operations a) hydrolysis of the cladinose derivative (III); b) conversion of the 3-hydroxy group into the 3-oxo and c) removal of the protecting group R₂.

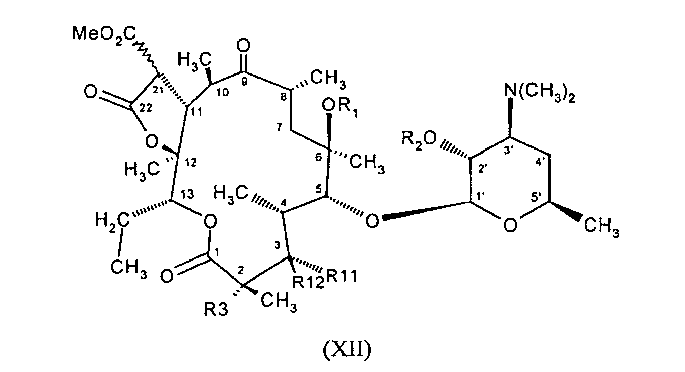

The decarboxylation may be carried out in the presence of a lithium salt such as lithium chloride, preferably in an organic solvent such as dimethylsulphoxide.

In a preferred embodiment of the invention, compounds of formula (I) in which A is -N(R₆)- and X is C₂₋₁₀alkylene interrupted by NR₈-, -N(R₈)C(Y)N(R₉)-, -N(R₈)C(O)- or-N(R₈)C(O)C(O)-, may be prepared by reaction of a compound of formula (XIV), 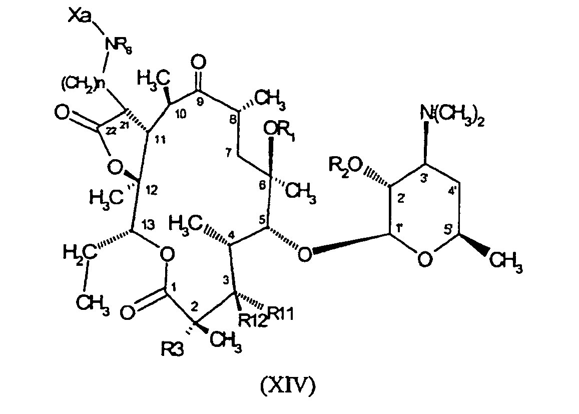 wherein Xa is C₂₋₁₀alkyl-N(R₈), R₁₁ and R₁₂ are defined as in formula (II), with compounds LXbR₄(XV), in which L is a suitable leaving group, Xb is a group selected from C(Y)N(R₉)C(Y)N(R₉)C₁₋₈alkylene, C(O), C(O)C₁₋₈alkylene, C(O)C(O) or C(O)C₁₋₈ alkylene and, if required, subjecting the resulting compound to one or more of the following operations a) hydrolysis of the cladinose derivative (III); b) conversion of the 3-hydroxy group into the 3-oxo and c) removal of the protecting group R₂.

Compounds of formula (XIV) may be prepared from compounds of formula (II) by reductive N-alkylation with a compound of formula HC(O)C₂₋₉alkyl N(R₆)(XVI). The reaction is conveniently carried in a protic solvent such as alcohol, i.e methanol, and in the presence of a suitable metal reducing agent such as sodium borohydride or sodium triacetoxyborohydride.

Compounds of formula (I) in which n is 2 or 3 and wherein X is optionally substituted and/or optionally substituted C₁₋₁₀ alkylene may be prepared from a phosphite of formula (XVII), wherein R₁₁ and R₁₂ have the meaning defined in formula (II) and R₁₃ is C₁₋₄ alkyl, 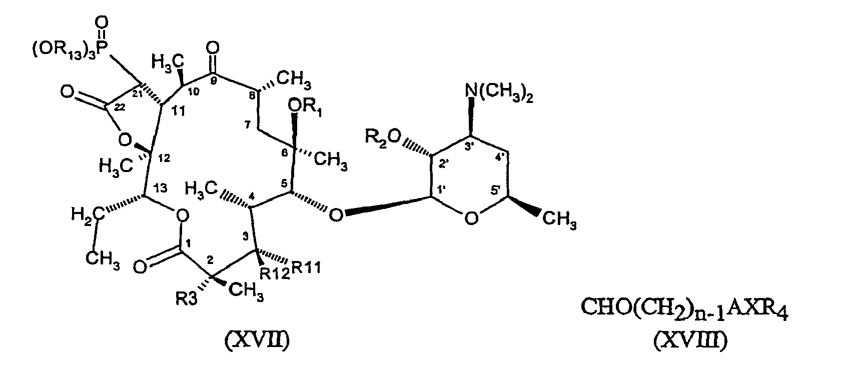 by Wittig-Horner reaction with an aldehyde of formula (XVIII), followed by reduction of the corresponding double bond using hydrogen and a metal catalyst (e.g. palladium) and, if required, subjecting the resulting compound to one or more of the following operations a) hydrolysis of the cladinose derivative (III); b) conversion of the 3-hydroxy group into the 3-oxo and c) removal of the protecting group R₂.
The Wittig-Homer reaction is carried out in the presence of a suitable organic or inorganic base such as 1,8-diazabicyclo[5.4.0]undec-7-ene or diisopropylethylamine in an aprotic solvent such as dichloromethane, preferably at a temperature ranging between -20° to +80°C.

In the reactions described above cladinose derivatives of formula (III) may be removed by treatment with an organic or inorganic acid. Example of a suitable inorganic acid is hydrochloride. The reaction is carried out in the presence of water or an organic solvent such tetrahydrofuran, dichloromethane or mixture thereof.

In the reactions described above the conversion of the 3-hydroxy group into the 3-oxo may be performed by oxidation reaction using a modified Moffatt-Pfitzner procedure.
Suitable oxidizing agent include N,N-Dimethylaminopropyl-3-ethyl carbodiimide - dimethylsulfoxide. The reaction is suitably carried out in the presence of pyridiniumtrifluoro acetate in a chlorinated solvent such as methylene chloride at -10°C to 25°C.

In a further embodiment, the oxidation may be carried out using Dess Martin periodinane reagent.

Compounds of formula (I), wherein A is NR₆ and in which R₆ is optionally substitued C₁₋₄alkyl, may be prepared by treating amino compounds of formula (II), wherein R₆ is hydrogen, with an alkylating agent L-R6 (XIX) wherein L is a suitable leaving group in the presence of a base.

Compounds of formula (II) wherein n is 0 may be prepared by intramolecular Michael reaction of compounds of formula (XX) wherein R₁₄ is a suitable nitrogen protecting group, R₁₁ and R₁₂ have the meaning defined in formula (II), in the presence of an organic base such as 1,8-diazabicyclo[5.4.0]undec-7-ene.

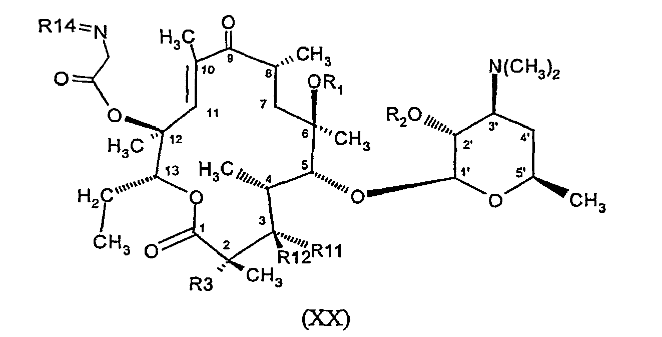

The reaction conveniently takes place in an aprotic polar solvent such as acetonitrile, dimethylformamide or an aqueous mixture thereof, followed by removal of the nitrogen protecting group R₁₄.

Suitable nitrogen protecting group R₁₄ for use in this reaction includes diarylmethylidene such as diphenylmethylidene.

Compounds of formula (II) wherein n is 1 may be prepared by reduction of a compound of formula (XXI), wherein R₁₁ and R₁₂ have the meaning defined in formula (II).

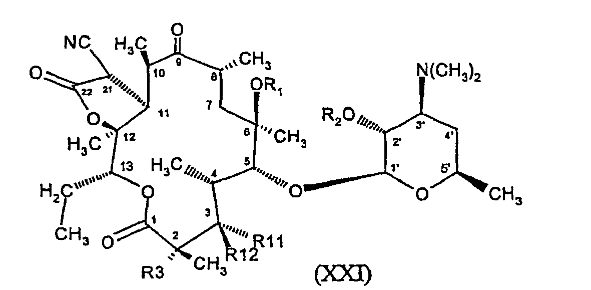

The reduction may be carried out using conventional reducing agents known in the art for converting a nitrile group into an amino group. Thus for example the reaction may be carried out using hydrogen in the presence of Raney-Nickel as catalyst.
The reaction is preferably carried out in an alcoholic solvent such as metyl, ethyl or isopropyl alcohol.

Compounds of formula (XXI) may be prepared by cyclisation of chlorine derivatives (XXII) wherein R₁₁ and R₁₂ have the meaning as defined for compounds of formula (II), 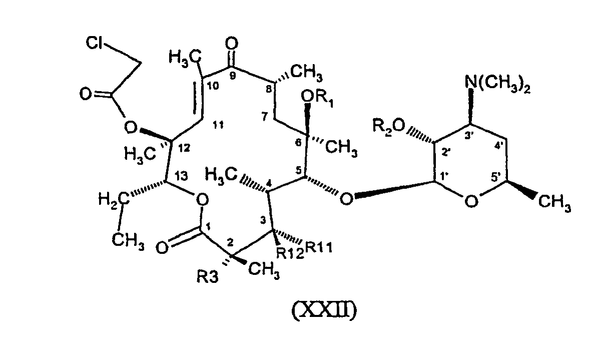 with potassium cyanide and conveniently in the presence of a solvent such as a N-N dimethylformamide, followed if required, subjecting the resulting compound to one or more of the following operations a) hydrolysis of the cladinose derivative (III); b) conversion of the 3-hydroxy group into the 3-oxo and c) removal of the protecting group R₂.

Compounds of formula (XVII) may be prepared by heating a compound of formula (XXIII) 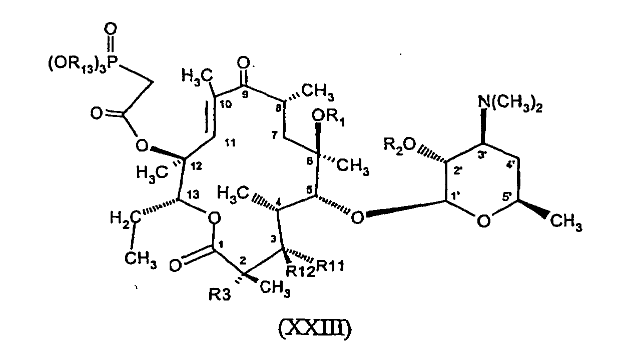 in an aprotic solvent such as N,N dimethylformamide at a temperature ranging from 60° to 120°C in the presence of a base such as 1,8-diazabicyclo[5.4.0]undec-7-ene.

Compounds of formula (XXIII) may be prepared by reaction of chlorine derivatives of formula (XXII) with (R₁₃O)₃phosphite. The reaction is carried out in a suitable aprotic solvent such as a hydrocarbon (i.e. toluene or xylene), N,N-dimethylformamide or by neat at a temperature within the range of 80° to 160°C.

Compounds of formula (XXII) may be prepared by reacting the corresponding hydroxy derivatives (XXIV), 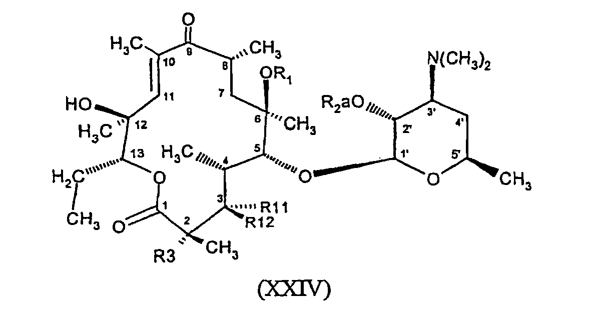 wherein R₁₁ and R₁₂ have the meaning defined in formula (II), with a suitable activated derivative of the acid HOCOCH₂Cl(XXV).
Thus for example the esterification may be carried out by reaction with anhydride (ClCH₂CO)₂O (XXVI) in a suitable aprotic solvent such as a halohydrocarbon (e.g. dichloromethane) or N,N-dimethylformamide and in the presence of a tertiary base such as pyridine, dimethylaminopyridine or triethylamine and at a temperature within the range of 0°C to 120°C.

Compounds of formula (XX) may be prepared by treating a compound of formula (XXII) with sodium azide, subjecting the resulting azido compound to the following operations: a) reduction by conventional means for reducing azido group to amino group and b) conversion of the group NH₂ into the nitrogen protecting group N=R₁₄ wherein R₁₄ has the meaning defined above and, if required, by removal of the hydroxy protecting group R₂.
The reduction to amino group may be carried out, for example, in the presence of triphenylphosphine and water.
In a further embodiment of the invention compounds of formula (XX) may be prepared by treating a compound of formula (XXII) with NH₄OH in the presence of solvent a suitable solvent for this reaction is dimethylsulphoxide and water.
Compounds of formula (XXIV), may be prepared by reacting 11,12-carbonate erythromycin A derivatives (XXVII), R₁₁ and R₁₂ have the meaning defined in formula (II), with a strong base such as 1,8 diazabicyclo [5.4.0]undec-7-ene.

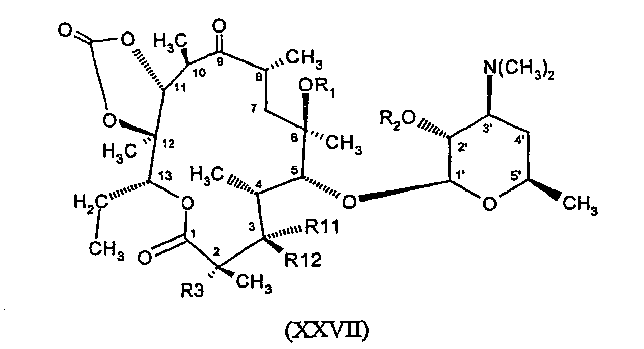

The elimination reaction may be carried out in an organic solvent such toluene, ethyl acetate, N,N dimethylformamide or a mixture thereof, conveniently with heating.
Compounds of formula (XXVII), may be prepared from erythromycin A derivatives of formula (XXVIII), 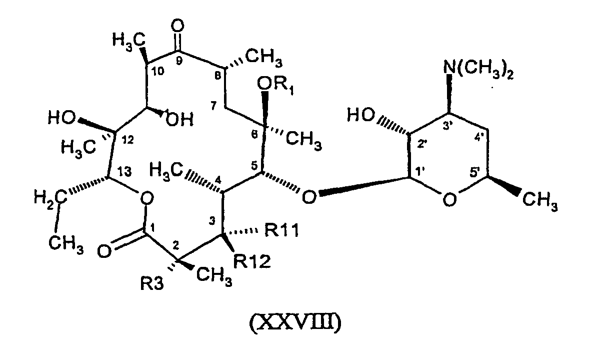 by conversion of the 2'- hydroxy group into the corresponding hydroxy protected group and by conversion of the 11, 12 hydroxy into a carbonate group using triphosgene in a suitable solvent such as dicholorometane, in the presence of pyridine.

Compounds of formula (XXVIII), may be prepared by alkylation of an oxime of formula (XXIX) 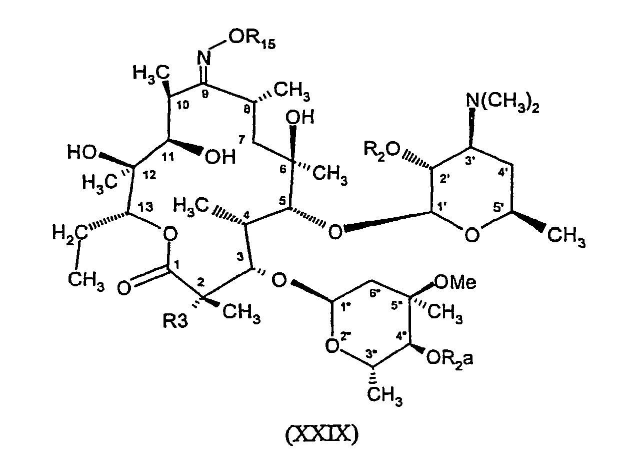 wherein R₁₅ is oxime protecting group and R₂ and R₂ₐ are a hydroxyl protecting group, with a compound of formula L-R₁ (XXX) in which L is a suitable leaving group such as a halogen (e.g. chlorine, bromine or iodine) or a sulfonyl (e.g. tosyl, methanesulfonyl), in the presence of a base, followed by hydrolysis of cladinose derivative and conversion of the 3-hydroxy group into the 3-oxo.
The reaction with compound (XXX) is preferably carried out in a solvent such as a halohydrocarbon (e.g. dichloromethane), an ether (e.g. tetrahydrofuran, dimethoxyethane), acetonitrile and the like.
Examples of the bases which may be used include potassium hydroxide, cesium hydroxide, tetraalkylammonium hydroxyde, sodium hydride, potassium hydride and the like, followed by subsequent removal of oxime protecting group.
A suitable oxime protecting goup is R₁₅, for example, 1- isopropoxycyclohex-1-yl.

Oxime compounds (XXIX) may be prepared by reaction of a compound of formula (XXXI) wherein R₂ and R₂ₐ are hydrogen, using analogous methods to those described in USP 6110965.

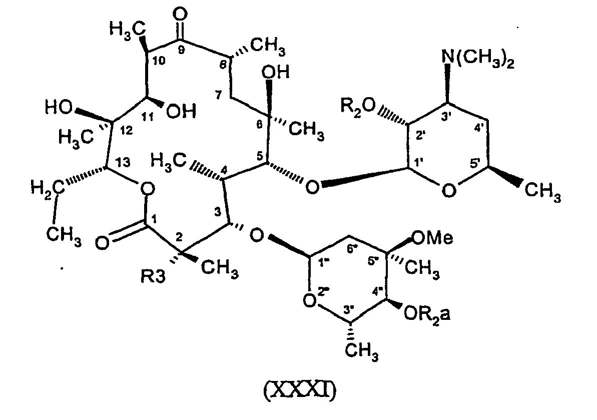

Compounds of formula (I) wherein R₃ is halogen may be prepared from compounds of formula (I) in which R₃ is hydrogen and R₂ is hydroxy protecting group by reaction with a halogenating agent in the presence of an organic or inorganic base.
Suitable halogenating agents include N-fluoro benzensulfonimide, SELECTFLUOR^{™} for fluorination, pyridinium tribromide or cyanogen bromide for bromination or hexachloroethane for chlorination.
A convenient base for the reaction is selected from sodium hydride, potassium hydride, sodium carbonate, potassium hexamethyldisilazide, lithium diisopropylamide or pyridine.
The reaction is carried out in a solvent such as N,N dimethylformamide, tetrahydrofuran or N-methylpyrrolidone or a mixture thereof, conveniently at a temperature within the range - 78° to 60°C.
Alternatively the halo group in position 2 of the macrolide ring may be introduced in an earlier step of the synthesis of compounds of formula (I). Thus, for example, it may be indroduced by treating a compound of formulas (II), (IX), (XII), (XV), (XVI), (XVII), (XVIII), (XIX), (XXII)) or (XXIII) provided that R₁₁together with R₁₂ is an oxygen atom, using the method above described for obtaining compound (I) wherein R₃ is a halo group.

Compounds of formula(I) wherein R is (CH₂)ₙR₅ may be prepared by intramolecolar cyclisation of a compound of formula (XXXII), 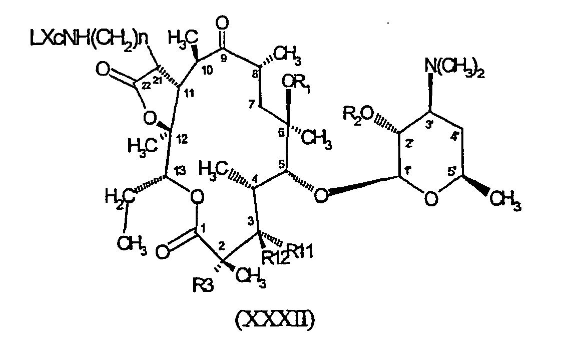 wherein L is suitable leaving group such halogen (i.e chlorine or bromine), X is C₄₋₅ alkylene chain optionally substituted by one or two groups selected from oxo 9 or 10 membered fused bicyclic heterocyclic having at least one heteroatom selected from oxygen, sulphur or nitrogen.
The reaction is suitable carried out in the presence of an inorganic base or an organic base.
Alternatively compounds of formula (I) wherein R is (CH₂)ₙR₅ may be prepared by intramolecular reductive N-alkylation of a compound of formula (XXXIII) 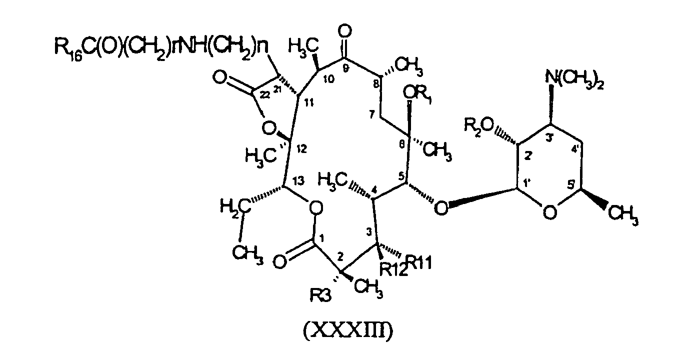 wherein R₁₆ is 9 to 10 membered fused heterocyclic groups, r is 3 or 4. This reaction was carried out conveniently carried in an aprotic solvent such as dichloroethane and in the presence of a suitable metal reducing agent such as sodium borohydride or sodium triacetoxyborohydride.

Compounds of formulas (IV), (V), (VI), (VII), (VIII), (IX), (X), (XIa), (XVI), (XVIII), (XIX), (XXI) or (XXV) are known or commercially available compounds or they may be prepared using methods known in the art.

The nitrogen protection reaction may be carried out with an appropriate imine such as benzophenone imine in an aprotic solvent e.g. dichloromethane preferably at room temperature.

Where it is desired to isolate a compound formula (I) as a salt thereof, for example a pharmaceutically acceptable salt, this may be achieved by reacting the compound of formula (I) in the form of the free base with an appropriate amount of suitable acid and in a suitable solvent such as an alcohol (e.g. ethanol or methanol), an ester (e.g. ethyl acetate) or an ether (e.g. diethyl ether or tetrahydrofuran).

Pharmaceutically acceptable salts may also be prepared from other salts, including other pharmaceutically acceptable salts, of the compound of formula (I) using conventional methods.

Suitable hydroxy protecting reagent are those described by T.W. Greene and P.G.M Wuts in Protective Groups in Organic Synthesis 2^{nd} ed., John Wiley & Son, Inc 1991, which is incorporating by reference. Examples of suitable hydroxy protecting reagents include acetic anhydride, benzoic anhydride or a trialkylsilyl chloride in a protic solvent. Examples of aprotic solvent are dichloromethane, NN-dimethylformamide, dimethylsulfoxide, tetrahydrofuran and the like.

The hydroxyl protecting groups may be removed by well known standard procedures. For example when R₂ₐ is a trialkyllsilyl group, this may be removed by treatment with tetrabutylammonium fluoride and acetic acid or by reaction with fluoride ions source such as triethyl amine tris (hydrogen fluoride) or this process is conveniently carried out in a solvent such as tetrahydrofuran or acetonitrile. When R₂ or R₂ₐ is alkanoyl (i.e acetyl or benzoyl) these may be removed by treatment with an alcohol (e.g. methanol or ethanol).

In any of the formulae (I), (II), (XI), (XIV), (XVII), (XXI), (XXXII) or (XXXIII) shown above when there is a an asymmetric carbon atom and no specific configuration is shown then the formula includes all possible configurations.
Specific stereoisomers of the compounds of formula (I) as defined in formula la and 16 essentially free of the other stereoisomers may be prepared using general processes described above starting with the appropriate stereisomer of formula (II).
The process described above for preparing the compounds of formula (II) will in general give a mixture of diastereoisomers.
The individual stereoisomers of the compounds of formula (II) may be separated each other by conventional techniques such as fractional crystallisation or more particularly by column chromatography, using for example a silica column.
In a preferred embodiment of the invention the individual stereoisomer of formula (1a) wherein R is NH₂ may be prepared by epimerisation reaction of a compound of formula(1b) or mixture of (1a) and (1b) wherein R is NH₂. The reaction is carried out in the presence of benzaldehyde and DBU, followed by hydrolysis of the imine derivative with inorganic acid such as hydrochloride. The reaction is suitable carried out in aprotic solvent such as for example toluene, N-N dimethylformamide.

The assignment of the R or S configuration at the 21-position have been made according to the rules of Cahn, Ingold and Prelog, Experientia 1956, 12, 81.

When examples are obtained as a diastereoisomeric mixture of 21R and 21S, unless otherwise stated, the ¹H-NMR spectra refers to the ¹H-NMR spectra of the predominant diastereoisomer (i.e. 21 S).
In the Intermediates and Examples unless otherwise stated:
Proton Magnetic Resonance (¹H-NMR) spectra were recorded at 500 MHz, chemical shifts are reported in ppm downfield (δ) from Me₄Si, used as internal standard, and are assigned as singlets (s), doublets (d), doublets of doublets (dd), triplets (t), quartets (q) or multiplets (m). Mass spectra were acquired with a Hewlett Packard 1100 MSD system equipped with a binary pump (Agilent Technologies), operating in positive electrospray ionisation mode. LC/MS (Liquid Chromatography/Mass Spectroscopy) data were obtained by using a HP 1100 LC system (Agilent Technologies) equipped with a Sedex Evaporative Light Scattering Detector model 75 (Sedere) coupled with a Platform LCZ Mass Spectometer (Micromass) operating in positive electrospray ionisation mode. The chromatographic analysis conditions were: column Waters XTerra MS C18 (4.6 x 30mm, 2.5µm); flow rate 0.8ml/min; mobile phase: aqueous solution of NH₄OAc (10mM, pH 6.8) (A) and acetonitrile (B).
LC (Liquid Chromatography) purifications were performed with a Waters 600 semi-preparative system equipped with a binary pumping system and a Jasco-UV detector. The chromatographic analysis conditions were: column Supelcosil ABZ+Plus (10cm x 21.2mm, 5µm); flow rate 8ml/min; mobile phase: aqueous solution of NH₄OAc (10mM, pH 6.8) (A) and acetonitrile (B).
Column chromathography was carried out over silica gel 60 (230-400 mesh ASTM - Merck AG Darmstaadt, Germany). The TLC (Thin Layer Chromatography) monitoring was performed using Merck 60 F₂₅₄ as TLC plate.
Phase separations were done by using Microfiltration Device - Filter Tube with polypropylene support (Whatman).
Resin washings were carried out on Extract-clean Tube (Alltech).
Purifications of crude products were performed by SCX-cartridges (Varian).
PS-Trisamina resin (polystyrene based) (Argonaut Technologies Inc.) was used to remove the excess of reagents.
Abbreviations which have been used in the description of the synthetic methods that follow are: Brine for aqueous saturated solution of sodium chloride, DBU 1,8-diazabicyclo[5.4.0]undec-7-ene, DCE for 1,2-dichloroethane, DCM for dichloromethane, DIPEA for N,N-diisopropylethylamine, DMAP for 4-dimethylaminopyridine, DMF for N,N-dimethylformamide, DMSO for methyl sulfoxide, EDC for 1-(3-dimethylaminopropyl)-3-ethylcarbodiimide hydrochloride, Et₂O for diethyl ether, EtOAc for ethyl acetate, HATU for O-(7-azabenzotriazol-1-yl)-N,N,N',N'-tetramethyluronium hexafluorophosphate, HOBT for 1-hydroxybenzotriazole hydrate, iPrOH for 2-propanol, MeOH for methanol, MTBE for *tert-*butyl methyl ether, TEA for triethylamine and THF for tetrahydrofuran, wt for weight.

### Intermediate 1

### 2'-O-Acetyl-11,12-carbonate-3-decladinosyl-11,12-dideoxy-6-O-methyl-3-oxo-erythromycin A

To a solution of 2'-O-acetyl-3-decladinosyl-6-O-methyl-3-oxo-erythromycin A (0.500g) in anhydrous DCM (20mL) under nitrogen atmosphere, pyridine (1.5mL) and phosgene (20% sol. in toluene, 1mL) were sequentially added. The reaction mixture was stirred overnight at room temperature then quenched with a saturated NaHCO₃ aqueous solution (50mL). The organic phase was washed with water (50mL), dried over Na₂SO₄, concentrated under reduced pressure and the crude product purified by flash chromatography (eluting with: DCM\MeOH 90\10) to give the title compound (0,360g).
TLC: DCM\MeOH 90\10 (Rf=0.6).

### Intermediate 2

### 2'-O-Acetyl-3-decladinosyl-11-deoxy-10,11-didehydro-6-O-methyl-3-oxo-erythromycin A

To a solution of intermediate **1** (0.210g) in 2\1 mixture of EtOAc\toluene (6mL), DBU (0.05mL) was added and the mixture was heated to 85°C for 6h. The reaction mixture was allowed to reach room temperature, the solvent evaporated and the crude product purified by flash chromatography (eluting with: DCM\MeOH 90\10) to give the title compound (0.150g).
TLC: DCM\MeOH 90\10 (Rf=0.7).

### Intermediate 3

### 2'-O-Acetyl-12-chloroethanoyl-3-decladinosyl-11-deoxy-10,11-didehydro-6-O-methyl-3-oxo-erythromycin A

To a solution of intermediate **2** (0.150g) in anhydrous DCM (3mL) cooled to 0°C, pyridine (0.05mL), chloroacetic anhydride (0.065g) and DMAP (5mg) were sequentially added under nitrogen atmosphere. The reaction mixture was stirred for 4h then quenched with water (10mL) and extracted with DCM (2x10mL). The organic phase was dried over Na₂SO₄, concentrated under reduced pressure and the crude material purified by flash chromatography (eluting with: DCM\MeOH 80\20) to give the title compound (0.060g).
TLC: DCM\MeOH 90\10 (Rf=0.8).

### Intermediate 4

### (11S,21R)-2'-O-Acetyl-3-decladinosyl-11,12-dideoxy-6-O-methyl-3-oxo-12,11-[oxycarbonyl-(iminomethyl)-methylene]-erythromycin A

To a solution of example **1** (0.137g) in iPrOH (20mL) Raney-Nickel (0.100g) was added. The reaction mixture was saturated with hydrogen (5atm) and stirred at room temperature for 24h. After removing the catalyst by filtration and evaporating the solvent under reduced pressure, the crude material was purified by flash chromatography (eluting with: DCM\MeOH 95\5) to give the title compound (0.012g).
¹H-NMR (CDCl₃) δ: 7.61, 7.07 (t+t, 1H), 4.94, 4.82 (dd+dd, 1H), 4.76 (m, 1H), 4.39, 4.38 (d+d, 1H), 4.24, 4.22 (d+d, 1H), 3.83 (q, 1H), 3.55 (m, 1H), 3.26 (m, 1H), 3.13 (m, 1H), 3.02, 2.90 (d+d, 1H), 2.96 (m, 1H), 2.70 (m, 1H), 2.65, 2.64 (s+s, 3H), 2.60, 2.50 (m+m, 1H), 2.26 (s, 6H), 2.07, 2.06 (s+s, 3H), 1.94 (m, 1H), 1.77-1.64 (m, 2H), 1.55 (m, 1H), 1.50, 1.46 (s+s, 3H), 1.33 (d+d, 6H), 1.32 (s, 3H), 1.21 (d, 3H), 1.17 (m, 1H), 1.14 (s, 3H), 0.97, 0.96 (d+d, 6H), 0.83, 0.80 (t+t, 3H).
TLC: DCM\MeOH 95\5 (Rf= 0.65).

### Intermediate 5

### (11S,21R)-3-Decladinosyl-11,12-dideoxy-6-O-methyl-3-oxo-12,11-[oxycarbonyl-(iminomethyl)-methylene]-erythromycin A

A solution of intermediate **4** (0.010g) in MeOH (1mL) was stirred for 48h, then concentrated under reduced pressure to give the title compound (0.009g).
¹H-NMR (CDCl₃) δ: 7.61, 7.09 (t+t, 1H), 4.94, 4.83 (dd +dd, 1H), 4.86 (bm, 1H), 4.32, 4.30 (d+d, 1H), 4.26, 4.24 (d+d, 1H), 3.86 (m, 1H), 3.56 (m, 1H), 3.13-3.10, 2.96 (m, 3H) 3.02 (m, 1H), 2.88 (bs, 1H), 2.66 (s, 3H), 2.50 (m, 1H), 2.30 (s, 6H), 1.95 (m, 1H), 1.85, 1.76 (m, 1H), 1.70-1.60 (m, 2H), 1.55 (m, 1H), 1.49, 1.46 (s+s, 3H), 1.4-1.3 (m, 6H), 1.30-1.14 (m, 2H), 1.0-0.9 (d, 3H), 0.84, 0.81 (t+t, 3H).
TLC: DCM\MeOH 95\5 (Rf= 0.45).

### Intermediate 6

### 2',4"-O-Diacetyl-6-O-methyl-erytrhromycin A

To a solution of 6-O-methyl-erytrhromycin A (50g) in anhydrous DCM (240mL), TEA (26.1mL), DMAP (0.392g) and acetic anhydride (15.2mL) were added at 0°C under nitrogen atmosphere. The resulting mixture was stirred at 0°C for 45min and overnight at room temperature. The mixture was then diluted with a saturated NH₄Cl aqueous solution (240mL) and extracted with DCM (2x200mL). The aqueous phase was neutralised with a saturated NaHCO₃ aqueous solution and extracted again with DCM (2x200mL). The combined organic layers were dried over Na₂SO₄ and concentrated under reduced pressure to give the title compound (50.7g).
m\z ([MH]⁺) = 832.

### Intermediate 7

### 11,12-Carbonate-2',4"-O-diacetyl-11,12-dideoxy-6-O-methyl-erytrhromycin A

To a solution of intermediate **6** (200g) in anhydrous DCM (1600mL) cooled to 0°C, pyridine (117mL) and a solution of triphosgene (71.2g) in anhydrous DCM (400mL) were added under nitrogen atmosphere. The resulting mixture was stirred at 0°C for 30min and then at room temperature for 15h. The mixture was then diluted with water (750mL) and extracted with DCM (2x500mL). The organic layer was washed with water (3x300mL), dried over Na₂SO₄ and concentrated under reduced pressure to give the title compound (200g).
m\z ([MH]⁺) = 858.

### Intermediate 8

### 11-Deoxy-2',4"-O-diacetyl-10,11-didehydro-6-O-methyl-erytrhromycin A

To a solution of intermediate **7** (50.5g) in a 2\1 mixture of toluene\EtOAc (675mL), DBU (12mL) was added at room temperature. The resulting mixture was stirred at 85°C for 8h and at room temperature for 5h. The reaction mixture was then diluted with brine (250mL), extracted with EtOAc (2x350mL) and dried over Na₂SO₄. The solvent was evaporated under reduced pressure and the crude material purified by crystallisation (from acetone\water) to give the title compound (46g).
m\z ([MH]⁺) = 814.

### Intermediate 9

### 12-Chloroethanoyl-11-deoxy-2',4"-O-diacetyl-10,11-didehydro-6-O-methyl-erythromycin A

To a solution of intermediate **8** (20g) in anhydrous DCM (340mL) cooled to 0°C pyridine (6mL) and chloroacetic anhydride (8.4g) were added under nitrogen atmosphere and the reaction was allowed to reach room temperature. After 16h the reaction mixture was washed with water (300mL), a saturated NH₄Cl aqueous solution (150mL) and brine (150mL) then extracted with DCM (2x300mL). The organic phase was dried over Na₂SO₄ and concentrated under reduced pressure. The crude material was dissolved in acetone (50mL) and water (100mL) was added under vigorous stirring. The precipitate was filtered and dried *in vacuo* to give the title compound (20.4g).
m\z ([MH]⁺) = 890.

### Intermediate 10

### 2'-O-Acetyl-12-chloroethanoyl-3-decladinosyl-11-deoxy-10,11-didehydro-6-O-methyl-erythromycin A

To a solution of intermediate **9** (20.2g) in THF (200mL) cooled to 0°C a 3N HCl aqueous solution (400mL) was added dropwise. The reaction mixture was allowed to reach room temperature and stirred overnight. The solution was neutralised with a saturated NaHCO₃ aqueous solution and extracted with DCM (2x200mL). The organic layer was dried over Na₂SO₄, concentrated under reduced pressure and the crude material purified by quick filtration on silica gel (eluting with: DCM\MeOH 95\5) to give the title compound (15.4g).
m\z ([MH]⁺) = 690.

### Intermediate 11

### (11S,21R)-2'-O-Acetyl-3-decladinosyl-11,12-dideoxy-6-O-methyl-12,11-[oxycarbonyl-(cyano)-methylene]-erythromycin A

To a solution of intermediate **10** (0.400g) in anhydrous DMF (30mL) potassium cyanide (0.380g) was added under nitrogen atmosphere. The mixture was stirred at room temperature for 2h, quenched with a saturated NaHCO₃ aqueous solution (50mL) and extracted with DCM (70mL). The organic phase was dried over Na₂SO₄, concentrated under reduced pressure and the crude material purified by flash chromatography (eluting with: DCM\MeOH 90\10) to give the title compound (0.430g).
TLC: DCM\MeOH 90\10 (Rf=0.50).

### Intermediate 12

### (11S-21R)-2'-O-Acetyl-3-decladinosyl-11,12-dideoxy-6-O-methyl-12,11-[oxycarbonyl-(iminomethyl)-methylene]-erythromycin A

### and

### Intermediate 13

### (11S,21R)-2'-O-Acetyl-3-decladinosyl-11,12-dideoxy-6-O-methyl-12,11-[oxycarbonyl-(aminomethyl)-methylene]-erythromycin A

To a solution of intermediate **11** (0.100g) in iPrOH (10mL) Raney-Nickel (0.100g) was added. The mixture was saturated with hydrogen (5atm) and stirred at room temperature for 24h. After removing the catalyst by filtration and evaporating the solvent, the crude product was purified by flash chromatography (eluting with: DCM\MeOH 90\10) to give the title compound **12** (0.038g) and the title compound **13** (0.027g).
TLC: DCM\MeOH 90\10, Rf (intermediate **12**) =0.43
TLC: DCM\MeOH 90\10, Rf (intermediate **13**) =0.2

### Intermediate 14

### (11S,21R)-2'-O-Acetyl-3-decladinosyl-11,12-dideoxy-6-O-methyl-12,11-[oxycarbonyl-(chloroacetamidomethyl)-methylene]-erythromycin A

To a solution of intermediate **13** (0.054g) in anhydrous DCM (2mL) pyridine (0.010mL), chloroacetic anhydride (0.016g) and DMAP (catalytic amount) were sequentially added under nitrogen atmosphere. The mixture was stirred at 0°C for 1h. The reaction was quenched with a saturated NaHCO₃ aqueous solution (5mL) and extracted with DCM (10mL). The organic phase was dried over Na₂SO₄ and concentrated under reduced pressure. The crude material was purified by flash chromatography (eluting with: DCM\MeOH 98\2) to give the title compound (0.036g).
TLC: DCM\MeOH 90\10 (Rf=0.43).

### Intermediate 15

### (11S,21R)-2'-O-Acetyl-3-decladinosyl-11,12-dideoxy-6-O-methyl-12,11-[oxycarbonyl-(chlorobutytramidomethyl)-methylene]-erythromycin A

To a solution of intermediate **13** (0.100g) in anhydrous DCM (4mL) pyridine (0.024mL), 4-chlorobutyryl chloride (0.016mL) and DMAP (catalytic amount) were sequentially added under nitrogen atmosphere. The mixture was stirred at 0°C for 2h. The reaction was quenched with a saturated NaHCO₃ aqueous solution (10mL) and extracted with DCM (20mL). The organic phase was dried over Na₂SO₄, concentrated under reduced pressure and the crude material purified by flash chromatography (eluting with: DCM\MeOH 95\5) to give the title compound (0.100g).
TLC: DCM\MeOH 90\10 (Rf=0.40).
m\z ([MH]⁺) = 789.

### Intermediate 16

### 2'-O-Acetyl-12-aminoethanoyl-3-decladinosyl-11-deoxy-10,11-didehydro-6-O-methyl-erythromycin A

To a solution of intermediate **10** (3g) in DMSO (40mL) a 32% aqueous ammonia solution (8mL) was added dropwise over 10min at room temperature. The reaction mixture was stirred for 2h at 50°C. After cooling to 0°C, water (40mL) was added and the mixture was extracted with MTBE (2x45mL). The collected organic layers were washed with brine (40mL), dried over Na₂SO₄ and concentrated under reduced pressure to give the title compound (2.5g).
TLC: DCM \MeOH 10\1 (Rf=0.28).

### Intermediate 17

### 2'-O-Acetyl-12-(benzhydrylidene)-aminoethanoyl-3-decladinosyl-11-deoxy-10,11 didehydro-6-O-methyl-erythromycin A

A solution of intermediate **16** (4.0g) and benzophenone imine (2.6mL) in anhydrous DCM (40mL) was stirred at room temperature under nitrogen atmosphere. After 36h the reaction was quenched with water (100mL) and extracted with DCM (3x300mL). The organic layer was dried over Na₂SO₄, concentrated under reduced pressure and the crude material purified by flash chromatography (eluting with: DCM\MeOH 95\5) to give the title compound (3.5g).
TLC: DCM\MeOH 10\1 (Rf=0.38).

### Intermediate 18

### (11S,21R,S)-2'-O-Acetyl-3-decladinosyl-11,12-dideoxy-6-O-methyl-12,11-[oxycarbonyl-(benzhydrylideneamino)-methylene]-erythromycin A

A solution of intermediate **17** (3.0g) and DBU (0.540mL) in acetonitrile (135mL) and water (15mL) was stirred at room temperature for 3h. After evaporating the solvent, the crude material was dissolved in DCM (300mL) and washed with water (100mL). The organic layer was dried over Na₂SO₄ and concentrated under reduced pressure to give the title compound (3.0g).
TLC: DCM\MeOH 10\1 (Rf=0.38).

### Intermediate 19

### 11-Deoxy-2',4"-O-diacetyl-10,11-didehydro-12-methoxycarbonylethanoyl-6-O-methyl-erythromycin A

To a solution of intermediate **8** (0.500g) in anhydrous toluene (100mL) under nitrogen atmosphere, pyridine (0.250mL) and methylmalonyl chloride (0.158mL) were added at 0°C. The temperature was allowed to reach room temperature. After stirring for 1h, water (50mL) was added, the organic layer was washed with brine (50mL) and dried over Na₂SO₄. The solvent was evaporated under reduced pressure and the crude material purified by quick filtration on silica gel to give the title compound (0.560g).
m\z ([MH]⁺) = 914.

### Intermediate 20

### 2'-O-Acetyl-3-decladinosyl-11-deoxy-10,11-didehydro-12-methoxycarbonylethanoyl-6-O-methyl-erythromycin A

### and

### Intermediate 21

### 2'-O-Acetyl-3-decladinosyl-11-deoxy-10,11-didehydro-12-carboxyethanoyl-6-O-methyl-erythromycin A

A solution of intermediate **19** (0.500g) in a 2N HCl aqueous solution (50mL) and THF (1mL) was stirred at room temperature for 6h. Then, the mixture was cooled to 0°C and a saturated potassium carbonate aqueous solution was added until pH= 9 was obtained. The mixture was extracted with DCM (2x50mL), the organic phase washed with brine (25mL), dried over Na₂SO₄ and concentrated under reduced pressure. The crude material was purified by flash chromatography (eluting with: DCM\MeOH = 95\5) to give the title compound **20** (0.180g), and the title compound **21** (0.180g).
m\z ([MH]⁺) (intermediate **20**) = 714. w
m\z ([MH]⁺) (intermediate **21**) = 700.

### Intermediate 22

### (10R,S,11S,21R,S)-2'-O-Acetyl-3-decladinosyl-11,12-dideoxy-6-O-methyl-12,11-[oxycarbonyl-(methoxycarbonyl)-methylene]-erythromycin A

A solution of intermediate **20** (0.150g) and DBU (0.050mL) in water (1.5mL) and acetonitrile (13.5mL) was stirred at 40°C for 6h. After evaporating the solvent under reduced pressure, the residue was dissolved in DCM (20mL), the organic phase washed with water (50mL), dried over Na₂SO₄ and concentrated under reduce pressure. The crude material was purified by flash chromatography (eluting with: DCM\MeOH 95\5) to give the title compound (0.070g).
m\z ([MH]⁺) = 714.

### Intermediate 23

### (10R,S,11R)-2'-O-Acetyl-3-decladinosyl-11,12-dideoxy-6-O-methyl-12,11-[oxycarbonylmethylene]-erythromycin A

A mixture of intermediate **22** (0.050g) and lithium chloride (0.006g) in DMF (1mL) was heated to reflux for 4h. The mixture was allowed to reach room temperature and poured into a 3% NaHCO₃ aqueous solution at 0°C, then extracted with DCM (2x15mL). The organic phase was washed with water (2x10mL), dried over Na₂SO₄ and concentrated under reduced pressure. The crude material was vpurified by flash chromatography (DCM\MeOH: 95\5) to give the title compound (0.010g).
m\z ([MH]⁺) = 656.

### Intermediate 24

### (11S,21S)-2'-O-Acetyl-3-decladinosyl-11,12-dideoxy-6-O-methyl-3-oxo-12,11-[oxycarbonyl-(2-(chloro)-ethylamino)-methylene]-erythromycin A

To a solution of example **7** (0.335g) in anhydrous acetonitrile (3mL) chloroacetaldehyde (50 wt % solution in water, 0.127mL) was added under a nitrogen atmosphere and the resulting mixture was stirred for 20h at room temperature. Sodium cyanoborohydride (1M in THF, 0.500mL) and acetic acid (0.041mL) were added and the reaction mixture was stirred for 6h at room temperature. After evaporating the solvent the residue was dissolved in DCM (25mL) and washed with a 5% NaHCO₃ aqueous solution (10mL). The organic phase was dried over Na₂SO₄ and concentrated under reduced pressure to give the title compound (0.300g).
m\z ([MH]⁺) = 731.

### Intermediate 25

### (11S,21R)-2'-O-Acetyl-3-decladinosyl-11,12-dideoxy-6-O-methyl-12,11-[oxycarbonyl-(3-quinolinyl-carbonylaminomethyl)-methylene]-erythromycin A

To a solution of intermediate **13** (0.028g) in anhydrous DMF (2.5mL) 3-quinolinecarboxylic acid (0.008g), HATU (0.017g) and DIPEA (0.017mL) were sequentially added under nitrogen atmosphere. The reaction mixture was stirred at room temperature for 4h and then the solvent evaporated under vacuum. The residue was dissolved in DCM (15mL) and washed with water (10mL). The organic phase was dried over Na₂SO₄ and concentrated under reduced pressure. The crude product was purified by flash chromatography to give the title compound (0.022g).
TLC: DCM\MeOH 90\10 (Rf=0.37)..

### Intermediate 26

### (11S,21R)-21-O-Acetyl-3-decladinosyl-11,12-dideoxy-6-O-methyl-12,11-[oxycarbonyl-(4-(4-(pyridin-3-yl)-imidazol-1-yl)-butyramidomethyl)-methylene]-erythromycin A

To a solution of intermediate **13** (0.050g) in anhydrous DMF (5mL) intermediate **49** (0.019g), HATU (0.030g) and DIPEA (0.031mL) were sequentially added under nitrogen atmosphere. The reaction mixture was stirred at room temperature for 3h, and then the solvent was removed under vacuum. The residue was dissolved in DCM (20mL), the organic phase washed with water (15mL), dried over Na₂SO₄ and concentrated under reduced pressure. The crude material was purified by flash chromatography to give the title compound (0.042g).
TLC: DCM\MeOH 90\10 (Rf=0.37).

### Intermediate 27

### (11S,21R)-2'-O-Acetyl-3-decladinosyl-11,12-dideoxy-6-O-methyl-12,11-[oxycarbonyl-(3-(4-(pyridin-3-yl)-imidazol-1-yl)-propionamidomethyl)-methylene]-erythromycin A

To a solution of intermediate **13** (0.050g) in anhydrous DMF (5mL) intermediate **48** (0.020g), HATU (0.030g) and DIPEA (0.031mL) were sequentially added under nitrogen atmosphere. The reaction mixture was stirred at room temperature for 4h and then the solvent was removed under vacuum. The residue was dissolved in DCM (15mL), the organic phase washed with water (10mL), dried over Na₂SO₄ and concentrated under reduced pressure. The crude product was purified by flash chromatography to give the title compound (0.042g).
TLC: DCM\MeOH 90\10 (Rf=0.28).

### Intermediate 28

### (11S,21R)-2'-O-Acetyl-3-decladinosyl-11,12-dideoxy-6-O-methyl-12,11-[oxycarbonyl-(2-(4-(pyridin-3-yl)-imidazol-1-yl)acetamidomethyl)-methylene]-erythromycin A

To a solution of 3-(1*H*-imidazol-4-yl)-pyridine (0.005g) in anhydrous DMF (2mL) cooled to 0°C sodium hydride (0.001g) was added under nitrogen atmosphere. The reaction mixture was stirred at 0°C for 1h then a solution of intermediate **14** (0.030g) in anhydrous DMF (1mL) was added. After 16h at room temperature the reaction was quenched with water (5mL) and the mixture extracted with DCM (10mL). The organic phase was dried over Na₂SO₄, concentrated under reduced pressure and the crude material purified by preparative TLC (eluting with: DCM\MeOH 90\10) to give the title compound (0.020g).
TLC: DCM\MeOH 90\10 (Rf=0.35).

### Intermediate 29

### (11S,21R)-2'-O-Acetyl-3-decladinosyl-11,12-dideoxy-6-O-methyl-12,11-[oxycarbonyl-(1-(pyrrolidin-2-one)-methyl)-methylene]-erythromycin A

To a solution of intermediate **15** (0.095g) in anhydrous DMF (8mL) cooled to 0°C, sodium hydride (0.004g) was added under nitrogen atmosphere. The mixture was stirred at 0°C for 1h and at room temperature for 18h. Water (5mL) was added and extracted with a mixture Et₂O\DCM 80\20 (3x10mL). The organic phase was dried over Na₂SO₄, concentrated under reduced pressure and the crude material purified by flash chromatography (eluting with: DCM\MeOH 90\10) to give the title compound (0.050g).
m\z ([MH]⁺) = 753.

### Intermediate 30

### (11S,21R)-2'-O-Acetyl-3-decladinosyl-11,12-dideoxy-6-O-methyl-12,11-[oxycarbonyl-(2-(guinoxalin-2-ylsulfanyl)-acetamidomethyl)-methylene]-erythromycin A

To a solution of intermediate **13** (0.075g) in anhydrous DMF (7mL) (quinoxalin-2-ylsulfanyl)-acetic acid (0.029g), HATU (0.046g) and DIPEA (0.030mL) were sequentially added under nitrogen atmosphere. The mixture was stirred at room temperature for 6h. The solvent was evaporated under reduced pressure and the residue dissolved in DCM (20mL). The organic phase was washed with a saturated NaHCO₃ aqueous solution (2x15mL), dried over Na₂SO₄ and concentrated under reduced pressure. The crude material was purified by flash chromatography (eluting with: DCM\MeOH 90\10) to give the title compound (0.069g).
m\z ([MH]⁺) = 887.

### Intermediate 31

### (11S,21R)-2'-O-Acetyl-3-decladinosyl-11,12-dideoxy-6-O-methyl-12,11-[oxycarbonyl-(3-(quinoxalin-2-ylsulfanyl)-propionamidomethyl)-methylene]-erythromycin A

To a solution of intermediate **13** (0.030g) in anhydrous DMF (3mL) 3-(quinoxalin-2-ylsulfanyl)-propionic acid (0.012g), HATU (0.018g) and DIPEA (0.012mL) were sequentially added under nitrogen atmosphere. The mixture was stirred at room temperature for 6h then the solvent was evaporated under reduced pressure. The residue was dissolved in DCM (15mL), the organic phase washed with a saturated NaHCO₃ aqueous solution (2x10mL), dried over Na₂SO₄ and concentrated under reduced pressure. The crude material was purified by flash chromatography (eluting with: DCM\MeOH 90\10) to give the title compound (0.035g).
m\z ([MH]⁺) = 901.

### Intermediate 32

### (11S,21R)-2'-O-Acetyl-3-decladinosyl-11,12-dideoxy-6-O-methyl-12,11-[oxycarbonyl-[(quinolin-4-ylmethylene)-amino]-methyl)-methylene]-erythromycin A

A solution of intermediate **13** (0.090g) and quinoline-4-carbaldehyde (0.023g) in anhydrous toluene (5mL) was heated at 50°C for 8h under nitrogen atmosphere. Solvent evaporation under reduced pressure gave the title compound (0.100g).
m\z ([MH]⁺) = 824.

### Intermediate 33

### 2',4"-O-Diacetyl-6-O-allyl-erytrhromycin

To a solution of 6-OAllyl erythromycin A (1g) in anhydrous DCM (5mL) cooled to 0°C, TEA (0.5mL), DMAP (0.008g) and acetic anhydride (0.31mL) were added under nitrogen atmosphere. The resulting mixture was stirred at 0°C for 1h and overnight at room temperature. Then a saturated NH₄Cl aqueous solution (30mL) was added the mixture extracted with DCM (2x50mL). The aqueous phase was neutralised with a saturated NaHCO₃ aqueous solution and extracted again with DCM (2x50mL). The combined organic layers were dried over Na₂SO₄ and evaporated under reduced pressure to give the title compound (1g).
m\z ([MH]⁺) = 858.

### Intermediate 34

### 11,12-Carbonate-2',4"-O-diacetyl-11,12-dideoxy-6-O-allyl-erytrhromycin To a solution of intermediate 33 (4.13g) in anhydrous DCM (85mL), pyridine (0.8mL) and

then phosgene (20% sol in toluene, 2.55mL) were added at 0°C under nitrogen atmosphere. The resulting mixture was stirred at 0°C for 30min and then at room temperature for 1h. The reaction mixture was then diluted with water (150mL) and extracted with DCM (2x200mL). The organic layer was washed with water (3x100mL), dried over Na₂SO₄ and evaporated under reduced pressure to give the title compound (4.02g).
m\z ([MH]⁺) = 884.

### Intermediate 35

### 11-Deoxy-2',4"-O-diacetyl-10,11-didehydro-6-O-allyl-erytrhromycin

To a solution of intermediate **34** (4.02g) in toluene (45mL) and EtOAc (23mL), DBU (0.71mL) was added at room temperature. The resulting mixture was heated to 85°C for 6h. The mixture was then diluted with brine (100mL), extracted with EtOAc (2x200mL) and dried over Na₂SO₄. Solvent evaporation under reduced pressure and purification by flash chromatography of the crude material (eluting with: DCM\MeOH\NH₄OH 95\4\0.01) gave the title compound (1.70g).
m\z ([MH]⁺) = 840.

### Intermediate 36

### 12-Chloroethanoyl-11-deoxy-2',4"-O-diacetyl-10,11-didehydro-6-O-allyl-erythromycin A

To a solution of intermediate **35** (1.7g) in anhydrous DCM (50mL) cooled at 0°C, pyridine (0.66mL), DMAP (0.012g) and chloroacetic anhydride (0.695g) were added under nitrogen atmosphere. The resulting mixture was stirred for 30min at 0°C and then at room temperature for 2.5h. The mixture was diluted with water (50mL), neutralised with a saturated aqueous solution of NaHCO₃ and extracted with DCM (2x100mL). The organic phase was washed with water (3x75mL), dried over Na₂SO₄ and evaporated under reduced pressure to give the title compound (1.8g).
m\z ([MH]⁺) = 916.

### Intermediate 37

### 2'-O-Acetyl-12-chloroethanoyl-3-decladinosyl-11-deoxy-10,11-didehydro-6-O-allyl-erythromycin A

To a solution of intermediate **36** (1.8g) in THF (35mL), a 6N HCl aqueous solution (10mL) was added at 0°C. The resulting mixture was stirred overnight at room temperature, then it was diluted with water (50mL). The pH of the solution was brought to 8-9 by addition of solid NaHCO₃ and a 1% NaOH aqueous solution, then the aqueous phase was extracted with DCM (2x100mL). Solvent evaporation under reduced pressure and treatment of the residue with Et₂O gave the title compound (1.4g).
m\z ([MH]⁺) = 716.

### Intermediate 38

### (11S,21R)-2'-O-Acetyl-3-decladinosyl-11,12-dideoxy-6-O-allyl-12,11-[oxycarbonyl-(cyano)-methylene]-erythromycin A

To a solution of intermediate **37** (1.3g) in anhydrous DMF (40mL) potassium cyanide (0.500g) was added under nitrogen atmosphere. The reaction mixture was stirred at room temperature for 1h, quenched with a 5% NaHCO₃ aqueous solution (50mL) and extracted with DCM (2x50mL). The crude material was purified by flash chromatography (eluting with: DCM\MeOH\NH₄OH 95\4\0.01) to give the title compound (0.42g).
m\z ([MH]⁺) = 707.

### Intermediate 39

### 2'-O-Acetyl-12-azidoethanoyl-3-decladinosyl-11-deoxy-10,11-didehydro-6-O-allyl-erythromycin A

To a solution of intermediate **37** (1.42g) in anhydrous DMF (110mL), sodium azide (0.211g) was added under nitrogen atmosphere. The mixture was heated to 80°C for 10min then quenched with water (100mL) and extracted with EtOAc (3x200mL). The organic layer was dried over Na₂SO₄ and concentrated under reduced pressure to give the title compound (1.36g).
m\z ([MH]⁺) = 723.

### Intermediate 40

### 2'-O-Acetyl-12-aminoethanoyl-3-decladinosyl-11-deoxy-10,11-didehydro-6-O-allyl-erythromycin A

To a solution of intermediate **39** (1.36g) in THF (25mL), triphenylphosphine (0.985g) and water (0.034mL) were added. The mixture was stirred at room temperature overnight. After evaporating the solvent, the residue was dissolved in DCM (100mL) and the solution washed with water (2x100). The organic layer was dried over Na₂SO₄ and concentrated under vacuum to give the title compound (1.30g).
m\z ([MH]⁺) = 697.

### Intermediate 41

### 2'-O-Acetyl-12-(benzhydrylidene)-aminoethanoyl-3-decladinosyl-11-deoxy-10,11-didehydro-6-O-allyl-erythromycin A

A solution of intermediate **40** (1.30g) and benzophenone imine (0.9mL) in anhydrous DCM (15mL) was stirred at room temperature under nitrogen atmosphere. After 30h the reaction was quenched with water (50mL) and extracted with DCM (3x100mL). The organic layer was dried over Na₂SO₄, filtered and concentrated under vacuum to give the title compound (1.60g).
m\z ([MH]⁺) = 861.

### Intermediate 42

### (11S,21R,S)-2'-O-Acetyl-3-decladinosyl-11,12-dideoxy-6-O-allyl-12,11-[oxycarbonyl-(benzhydrylideneamino)-methylene]-erythromycin A

A solution of intermediate **41** (1.60g) and DBU (0.3mL) in acetonitrile (90mL) and water (9mL) was stirred at room temperature for 2h. Then the solvents were evaporated and the crude material was dissolved in DCM (100mL). The mixture was washed with water (2x100mL), the organic phase dried over Na₂SO₄ and concentrated under reduced pressure. The crude material was purified by flash chromatography (eluting with: DCM\MeOH\NH₃ 9.5\0.4\0.03) to give the title compound (0.528g).
m\z ([MH]⁺) = 861.

### Intermediate 43

### Methyl (4-pyridin-1-yl-1H-imidazol-1-yl)acetate

To a stirred suspension of sodium hydride (0.769g) in anhydrous DMF (30mL) under nitrogen atmosphere, a solution of 3-(1*H*-imidazol-4-yl)-pyridine (3g) in anhydrous DMF (10mL) was added dropwise at room temperature. The mixture was stirred for 30min then methyl bromoacetate (2.4mL) was added dropwise. After stirring for 2h the solvent was evaporated under reduced pressure, the residue diluted with EtOAc (200mL) and washed with water (50mL). The aqueous phase was further extracted with EtOAc (2x40mL). The organic layer was dried over Na₂SO₄, concentrated under reduced pressure and the crude material purified by flash chromatography (eluting with: DCM\MeOH 95\5) to give the title compound (2.78g).
m\z ([MH]⁺) = 218.

### Intermediate 44

### Methyl 3-(4-pyridin-3-yl-1H-imidazol-1-yl)propionate

To a stirred suspension of sodium hydride (0.165g) in anhydrous DMF (2.5mL) cooled at 0°C a solution of 3-(1*H*-imidazol-4-yl)-pyridine (1g) in anhydrous DMF (5mL) was added dropwise under nitrogen atmosphere. The mixture was stirred for 15min at room temperature then a solution of methyl 3-bromopropanoate (0.830mL) in anhydrous DMF (5mL) was added dropwise. After stirring for 2h at 70°C the solvent was evaporated under reduced pressure, the residue diluted with EtOAc (50mL) and washed with water (15mL). The aqueous phase was further extracted with EtOAc (3x15mL). The combined organic layers were dried over Na₂SO₄, concentrated under reduced pressure and the crude product was purified by flash chromatography (eluting with: DCM\MeOH from 95\5 to 90\10) to give the title compound (0.530g).
m\z ([MH]⁺) = 232.

### Intermediate 45

### Methyl 4-(4-pyridin-3-yl-1H-imidazol-1-yl)butyrate

To a stirred suspension of sodium hydride (0.745g) in anhydrous DMF (8mL) cooled at 0°C a solution of 3-(1*H*-imidazol-4-yl)-pyridine (3g) in anhydrous DMF (16mL) was added dropwise under nitrogen atmosphere. The mixture was stirred for 15min at room temperature then a solution of methyl 4-chloro-butanoate (2.76mL) in anhydrous DMF (16mL) was added dropwise. After stirring for 2.5h at 70°C the solvent was evaporated under reduced pressure, the residue diluted with EtOAc (150mL) and washed with water (50mL). The aqueous phase was further extracted with EtOAc (3x40mL). The combined organic layers were dried over Na₂SO₄, concentrated under reduced pressure and the crude product was purified by flash chromatography (eluting with: DCM\MeOH 96\4) to give the title compound (2.2g).
m\z ([MH]⁺) = 246.

### Intermediate 46

### Methyl 5-(4-pyridin-3-yl-1H-imidazol-1-yl)pentanoate

To a stirred suspension of sodium hydride (0.745g) in anhydrous DMF (8mL) cooled at 0°C a solution of 3-(1*H*-imidazol-4-yl)-pyridine (3g) in anhydrous DMF (16mL) was added dropwise under nitrogen atmosphere. The mixture was stirred for 15min at room temperature then a solution of methyl 5-bromo-pentanoate (3.55mL) in anhydrous DMF (16mL) was added dropwise. After stirring for 2h at 70°C the solvent was evaporated under reduced pressure, the residue diluted with EtOAc (150mL) and washed with water (50mL). The aqueous phase was further extracted with EtOAc (3x40mL). The combined organic layers were dried over Na₂SO₄, concentrated under reduced pressure and the crude product was purified by flash chromatography (eluting with: DCM\MeOH 96\4) to give the title compound (4.25g).
m\z ([MH]⁺) = 260.

### Intermediate 47

### Sodium (4-Pyridin-3-yl-imidazol-1-yl)-acetate

To a solution of intermediate **43** (0.100g) in acetone (2mL) at room temperature 1M NaOH aqueous solution (0.46mL) was added. The mixture was stirred at room temperature for 20h then to reflux for 8h. Solvent evaporation under reduced pressure gave the title compound (0.100g).
m\z ([MH]⁺) = 204.

### Intermediate 48

### Sodium 3-(4-Pyridin-3-yl-imidazol-1-yl)-propionate

To a solution of intermediate **44** (0.100g) in acetone (1mL) at room temperature 1M NaOH aqueous solution (0.43mL) was added. The mixture was stirred at room temperature for 6h. Solvent evaporation under reduced pressure gave the title compound (0.099g).
m\z ([MH]⁺) = 218.

### Intermediate 49

### Sodium 4-(4-Pyridin-3-yl-imidazol-1-yl)-butyrate

To a solution of intermediate **45** (0.100g) in acetone (1mL) at room temperature 1M NaOH aqueous solution (0.40mL) was added. The mixture was stirred at room temperature for 6 h. Solvent evaporation under reduced pressure gave the title compound (0.096g).
m\z ([MH]⁺) = 232.

### Intermediate 50

### Sodium 5-(4-Pyridin-3-yl-imidazol-1-yl)-pentanoate

To a solution of intermediate **46** (0.100g) in acetone (1mL) at room temperature 1M NaOH aqueous solution (0.38mL) was added. The mixture was stirred at room temperature for 3 h. Solvent evaporation under reduced pressure gave the title compound (0.095g).
m\z ([MH]⁺) = 246.

### Intermediate 51

### 1-Methoxy-2-(4-pyridin-3-yl-1H-imidazol-1-yl)ethanol

To a stirred suspension of sodium hydride (0.166g) in anhydrous DMF (3mL) cooled to 0°C a solution of 3-(1*H*-imidazol-4-yl)-pyridine (1g) in anhydrous DMF (5mL) was added dropwise under nitrogen atmosphere. The reaction mixture was allowed to reach room temperature and after 15min a solution of bromoacetaldehyde dimethyl acetal (0.816mL) in anhydrous DMF (5mL) was added dropwise. The reaction mixture was stirred at 70°C for 4h and at room temperature overnight. After evaporating the solvent under reduced pressure the crude material was purified by flash chromatography (eluting with DCM\MeOH 90\10) to give the title compound (1.15g).
m\z ([MH]⁺) = 234
TLC: DCM\MeOH 90\10 (Rf=0.5).

### Intermediate 52

### 3-(4-Pyridin-3-yl-1H-imidazol-1-yl)propionaldehyde

To a solution of 3-(1*H*-imidazol-4-yl)-pyridine (0.350g) in anhydrous THF (15mL) cooled at 0°C acrylaldehyde (0.540mL) was added dropwise. The resulting solution was stirred at room temperature for 3 days. Solvent evaporation under reduced pressure gave the title compound (0.460g).
m\z ([MH]⁺) = 202.

### Intermediate 53

### 4-(4-Pyridin-3-yl-1H-imidazol-1-yl)butan-1-ol

To a solution of intermediate **45** (1.2g) in anhydrous THF (20mL) cooled at 0°C lithium aluminum hydride (1M in THF, 2.55mL) was added dropwise under nitrogen atmosphere. The reaction mixture was stirred for 2h at room temperature, then water (30mL) and EtOAc (75mL) were added. The solvents were evaporated under reduced pressure and the residue extracted with EtOAc (2x75mL). The combined organic layers were washed with a saturated sodium\potassium tartrate aqueous solution (80mL), dried over Na₂SO₄, concentrated under reduced pressure. The crude material was purified by flash chromatography (eluting with: DCM\MeOH 95\5) to give the title compound (0.600g)
m\z ([MH]⁺) = 218.

### Intermediate 54

### 4-(4-Pyridin-3-yl-1H-imidazol-1-yl)butyraldehyde

To a solution of oxalyl chloride (0.225mL) in anhydrous DCM (7mL) cooled to -78°C under nitrogen atmosphere a solution of DMSO (0.275mL) in anhydrous DCM (2mL) was slowly added. After 15 min at -78°C a solution of intermediate **53** (0.280g) in anhydrous DCM (5mL) was dropped within 30min. The mixture was stirred at -40°C for 4h then TEA(0.900mL) was added. The reaction was allowed to reach room temperature then water (10mL) was added. The mixture was extracted with DCM (3x20mL), the organic phase dried over Na₂SO₄, concentrated under reduced pressure and the crude material purified by flash chromatography (eluting with: DCM\MeOH 90\10) to give the title compound (0.052g)
m\z ([MH]⁺) = 216.

### Intermediate 55

### 5-(4-Pyridin-3-yl-1H-imidazol-1-yl)pentan-1-ol

To a solution of intermediate **46** (3.54g) in anhydrous THF (40mL) cooled to 0°C, lithium aluminum hydride (1M in THF, 7.8mL) was added dropwise under nitrogen atmosphere. The reaction mixture was stirred for 2.5h at room temperature then water (50mL), EtOAc (100mL) and a 28% NH₄OH aqueous solution until pH=9 were added. The solvents were evaporated under reduced pressure and the residue dissolved in EtOAc (2x100mL). The solution was washed with a saturated sodium\potassium tartrate aqueous solution, dried over Na₂SO₄, concentrated under reduced pressure and the crude material purified by flash chromatography (eluting with: DCM\MeOH from 95\5 to 90\10) to give the title compound (2.32g)
TLC: DCM\MeOH 95\5 (Rf=0.18)

### Intermediate 56

### 5-(4-Pyridin-3-yl-1H-imidazol-1-yl)pentanal

To a solution of oxalyl chloride (0.755mL) in anhydrous DCM (25mL) cooled to -78°C under nitrogen atmosphere, a solution of DMSO (1.23mL) in anhydrous DCM (8mL) was slowly added. After 15min at -78°C a solution of intermediate **55** (1g) in anhydrous DCM (18mL) was added dropwise over 30min. The mixture was stirred at -40°C for 3.5h then TEA(3.6mL) was added. The reaction mixture was allowed to reach room temperature then water (50mL) was added. The mixture was extracted with DCM (3x100mL), the organic phase dried over Na₂SO₄, concentrated under reduced pressure and the crude material purified by flash chromatography (eluting with: DCM\MeOH 92\) to give the title compound (0.635g)
m\z ([MH]⁺) = 230.

### Intermediate 57

### 4-[(E,Z)-2-Methoxyvinyl]quinoline

To a solution of (methoxymethyl)triphenylphosphonium chloride (3.27g) in anhydrous THF (60mL) cooled to -78°C sodium bis(trimethylsilyl)amide (1M in THF, 9.5mL) was added under nitrogen atmosphere and the solution was stirred for 15min. A solution of 4-quinoline carboxaldehyde (1g) in anhydrous THF (10mL) was added and the reaction mixture was stirred at -78°C for 30min, at 0°C for 1.5h, then at room temperature overnight. The reaction was quenched with water (50mL) and extracted with EtOAc (75mL). The aqueous phase was neutralised with a saturated NH₄Cl (50ml) aqueous solution and extracted again with EtOAc (2x75mL). The combined organic layers were dried over Na₂SO₄, concentrated under reduced pressure and the crude material purified by flash chromatography (eluting with: Hexane\EtOAc from 60\40 to 50\50) to give the title compound (0.905g).
m\z ([MH]⁺) =186.

### Intermediate 58

### 4-[3-(1,3-Dioxolan-2-yl)propyl]quinoline

To a solution of [2-(1,3-dioxolan-2-yl)-ethyl]-triphenylphosphonium bromide (10.6g) in anhydrous THF (150mL) cooled to -78C° a solution of sodium bis(trimethylsilyl)amide (1M in THF, 23.9mL) was added dropwise under nitrogen atmosphere. The mixture was stirred at -78°C for 30min then a solution of 4-quinoline carboxaldehyde (2.5g) in anhydrous THF (30mL) was added. The mixture was allowed to reach room temperature, DBU (1.8mL) was added and the mixture stirred for 6h at 50°C. The reaction was quenched with a saturated NH₄Cl aqueous solution of (100mL) and extracted with EtOAc (3x150mL). The organic phase was dried over Na₂SO₄, concentrated under reduced pressure and the crude material filtered on a silica pad (eluting with: cyclohexane\EtOAc 70\30). After evaporating the solvent under reduced pressure, the residue was dissolved in MeOH (20mL), palladium (10wt. % on carbon powder, 0.360g) was added and the mixture stirred under hydrogen atmosphere (1.5 atm) for 2h. The mixture was filtered over a celite pad eluting with MeOH (2x100mL) and purification by flash chromatography (eluting with: Et₂O) gave the title compound (0.700g).
m\z ([MH]⁺) = 244.

### Intermediate 59

### 4-Quinolin-4-yl-butyraldehyde

To a solution of intermediate **58** (0.500g) in acetone (5mL) a 2N HCl aqueous solution (5mL) was added and the mixture stirred at 50°C for 2h. After evaporation of the solvent under reduced pressure, NH₄OH was added to the residue until pH=9. Aqueous solution was extracted with DCM (2x35mL), the organic phase dried over Na₂SO₄ and concentrated under reduced pressure. The crude material was purified by flash chromatography (eluting with: EtOAc\cyclohexane 80\20) to give the title compound (0.315g).
m\z ([MH⁺]) = 200.

### Intermediate 60

### 4-Quinolin-4-yl-butyric acid

To a solution of intermediate **59** (0.160g) in acetone (4mL) potassium permanganate (0.063g) was added portionwise within 1h. After solvent evaporation, water (4mL) was added and the mixture refluxed for 30min. The reaction mixture was slowly cooled to 0°C and kept at 0°C overnight. The reaction mixture was filtered through a celite pad eluting with DCM (5mL) and after removing the solvent under vacuum the crude product was purified by flash chromatography (eluting with: DCM\MeOH 90\10) to give the title compound (0.050g).
m\z ([MH]⁺) = 216.

### Intermediate 61

### Ethyl 5-quinolin-4-yl-pentanoate

To a stirred suspension of [3-(ethoxycarbonyl)-propyl]-triphenylphosphonium bromide (5.6g) in anhydrous THF (100mL) cooled to -78°C a solution of sodium bis(trimethylsilyl)amide (1M in THF, 12mL) was added dropwise under nitrogen atmosphere. The mixture was stirred at -78°C for 1h then a solution of 4-quinoline carboxaldehyde (1.6g) in anhydrous THF (15mL) was dropped. The mixture was allowed to reach room temperature, stirred for 2h then heated to 50°C and stirred for 4h. The reaction was quenched with a saturated NH₄Cl aqueous solution (50mL) and extracted with EtOAc (3x100mL). The organic phase was dried over Na₂SO₄, concentrated under reduced pressure and the crude material purified by filtration on a silica gel pad (eluting with: cyclohexane\EtOAc 70\30). After evaporating the solvent under reduced pressure, the residue was dissolved in MeOH (10mL), palladium (10wt. % on carbon powder, 0.060g) was added and the mixture stirred under hydrogen atmosphere (1.5 atm) for 1h. The mixture was filtered through a celite pad eluting with MeOH (2x50mL) and subsequent solvent evaporation under reduced pressure gave the title compound (0.860g).
m\z ([MH]⁺) = 258.

### Intermediate 62

### 5-Quinolin-4-yl-pentanoic acid

To a solution of intermediate **61** (0.194g) in acetone (1mL) at room temperature 1M NaOH aqueous solution (0.78mL) was added. The mixture was refluxed for 2h. Solvent evaporation under reduced pressure gave the title compound (0.181g).
m\z ([MH]⁺) = 230.

### Intermediate 63

### 5-Quinolin-4-yl-pentanal

To a solution of intermediate **61** (0.050g) in anhydrous toluene (1mL) cooled to -78°C, diisobutylaluminium hydride (1M sol. in toluene, 0.39mL) was slowly added. The reaction mixture was stirred for 1h at-78°C, then quenched with 2mL of a mixture of water (0.25mL), acetic acid (1mL) and Et₂O (3mL) at -78°C. The temperature was allowed to reach room temperature then the crude material was filtered through a celite pad eluting with DCM (3x10mL). The organic phase was dried over Na₂SO₄ and concentrated under reduced pressure to give the title compound (0.040g).
m\z ([MH]⁺) = 214.

### Intermediate 64

### Methyl 3-(4-phenyl-1H-imidazol-1-yl)propionate

To a stirred suspension of sodium hydride (0.017g) in anhydrous DMF (0.250mL) cooled at 0°C a solution of 4-phenyl-1*H*-imidazole (0.100g) in anhydrous DMF (0.8mL) was added dropwise under nitrogen atmosphere. The mixture was stirred for 15min at room temperature then a solution of 3-bromo-propionic acid methyl ester (0.083mL) in anhydrous DMF (0.3mL) was added dropwise. After stirring for 2h at 70°C the solvent was evaporated under reduced pressure, the residue diluted with EtOAc (5mL) and washed with water (3mL). The aqueous phase was further extracted with EtOAc (3x5mL). The combined organic layers were dried over Na₂SO₄, concentrated under reduced pressure and the crude material was purified by flash chromatography (eluting with: DCM\MeOH from 100\0 to 95\5) to give the title compound (0.128g).
m\z ([MH]⁺) = 231.

### Intermediate 65

### Sodium 3-(4-Phenyl-imidazol-1-yl)-propionate

To a solution of intermediate **64** (0.034g) in acetone (0.50mL) at room temperature a 1M NaOH aqueous solution (0.15mL) was added. The mixture was stirred at room temperature for 3h. Solvent evaporation under reduced pressure gave the title compound (0.035g).
m\z ([MH]⁺) = 217.

### Intermediate 66

### Methyl (4-phenyl-1H-imidazol-1-yl)acetate

To a stirred suspension of sodium hydride (0.037g) in anhydrous DMF (0.550mL) cooled at 0°C a solution of 4-phenyl-1*H*-imidazole (0.200g) in anhydrous DMF (1.6mL) was added dropwise under nitrogen atmosphere. The mixture was stirred for 15min at room temperature then a solution of 2-chloro-acetic acid methyl ester (0.134mL) in anhydrous DMF (0.60mL) was added dropwise. After stirring for 2h at 70°C and overnight at room temperature the mixture was diluted with Et₂O (25mL) and washed with water (10mL). The aqueous phase was further extracted with Et₂O (3x15mL). The collected organic extracts were dried over Na₂SO₄, concentrated under reduced pressure and the crude product was purified by flash chromatography (eluting with: DCM\MeOH from 100\0 to 96\4) to give the title compound (0.210g).
m\z ([MH]⁺) = 217.

### Intermediate 67

### Sodium 3-(4-Phenyl-imidazol-1-yl)-propionate

To a solution of intermediate **66** (0.190g) in acetone (2.5mL) at room temperature, a 1.2M NaOH aqueous solution (0.730mL) was added. The mixture was stirred at room temperature for 2.5h and then solvent evaporation under reduced pressure gave the title compound (0.205g).
m\z ([MH]⁺) = 203

### Intermediate 68

### Methyl 3-(4-thien-2-yl-1H-imidazol-1-yl)pronionate

To a stirred suspension of sodium hydride (0.013g) in anhydrous DMF (0.250mL) cooled at 0°C a solution of 4-thiophen-2-yl-1*H*-imidazole (0.100g) in anhydrous DMF (1.6mL) was added dropwise under nitrogen atmosphere. The mixture was stirred for 15min at room temperature then a solution of methyl 3-bromopropionate (0.100nnL) in anhydrous DMF (0.3mL) was added dropwise. After stirring for 5h at 70°C and overnight at room temperature the solvent was evaporated under reduced pressure, the residue dissolved with EtOAc (5mL) and the solution washed with water (3mL). The aqueous phase was further extracted with EtOAc (3x5mL). The combined organic layers were dried over Na₂SO₄, concentrated under reduced pressure and the crude material was purified by flash chromatography (eluting with: DCM\MeOH from 100\0 to 95\5) to give the title compound (0.013g).
m\z ([MH]⁺) = 237.

### Intermediate 69

### Sodium 3-(4-thien-2-yl-1H-imidazol-1-yl)propionate

To a solution of intermediate **68** (0.013g) in acetone (0.3mL) at room temperature, a 1M NaOH aqueous solution (0.055mL) was added. The mixture was stirred at room temperature for 5h and then solvent evaporation under reduced pressure gave the title compound (0.012g).
m\z ([MH]⁺) = 223.

### Intermediate 70

### Sodium 3-(3-thiazol-2-yl-pyrazol-1-yl)-propionate

To a stirred suspension of sodium hydride (0.016g) in anhydrous DMF (0.3mL) cooled at 0°C a solution of 2-(1*H*-pyrazol-3-yl)-thiazole (0.100g) in anhydrous DMF (0.8mL) was added dropwise under nitrogen atmosphere. The mixture was stirred for 15min at room temperature then a solution of 3-bromo-propionic acid methyl ester (0.080mL) in anhydrous DMF (0.3mL) was added dropwise. After stirring for 4h at 70°C the solvent was evaporated under reduced pressure, the residue diluted with EtOAc (5mL) and washed with water (3mL). The aqueous phase was further extracted with EtOAc (3x5mL). The combined organic layers were dried over Na₂SO₄ and concentrated under reduced pressure. The crude material was dissolved in acetone (1mL) and a 1.2N NaOH aqueous solution (0.55mL) was added. The mixture was stirred at room temperature overnight. Solvent evaporation under reduced pressure gave the title compound (0.025g).
m\z ([MH]⁺) = 224.

### Intermediate 71

### Ethyl [(3-methoxyquinoxalin-2-yl)thio]acetate

To a solution of 2-chloro-3-methoxy-quinoxaline (0.100g) in anhydrous DMF (3mL) potassium carbonate (0.142g) and mercapto-acetic acid ethyl ester (0.084mL) were added and the resulting mixture was stirred at 80°C for 1.5h. The solvent was removed under reduced pressure, water (5mL) was added and the mixture was extracted with EtOAc (3x10mL). The combined organic phases were washed with brine (5mL), dried over Na₂SO₄ and evaporated under reduced pressure. The crude material was purified by flash chromatography (eluting with: cyclohexane\EtOAc 80\20) to afford the title compound (0.065g).
m\z ([MH]⁺) = 255
TLC: cyclohexane\EtOAc 80\20 (R_{f}=0.57).

### Intermediate 72

### [(3-Methoxyquinoxalin-2-yl)thio]acetic acid

To a solution of intermediate **71** (0.060g) in THF (2mL) a 3N NaOH aqueous solution (2mL) was added. The mixture was vigorously stirred at room temperature overnight, then after solvent evaporation, a 1N HCl aqueous solution was added until pH=1. The solution was extracted with DCM (3x8mL), the organic phase was washed with brine (5mL), dried over Na₂SO₄ and concentrated under reduced pressure to give the title compound (0.045g).
m\z ([MH]⁺) = 227
TLC: EtOAc\MeOH 98\2 (R_{f}=0.48)

### Intermediate 73

### Ethyl (quinoxalin-2-yloxy)acetate

To a solution of quinoxalin-2-ol (2g) in acetone (30mL) potassium carbonate (3.8g) and chloroacetic acid ethyl ester (2.2mL) were added and the resulting mixture was stirred at reflux for 6h. After evaporating the solvent under reduced pressure, water (30mL) was added and the mixture extracted with EtOAc (3x50mL). The organic phase was washed with brine (30mL) dried over Na₂SO₄ and evaporated under reduced pressure. The crude material was purified by flash chromatography (eluting with: cyclohexane\EtOAc 3\2) to afford the title compound (1.5g).
m\z ([MH]⁺) = 233.

### Intermediate 74

### (puinoxalin-2-yl-oxy)acetic acid

To a solution of intermediate **73** (0.300g) in THF (10mL) a 3N NaOH aqueous solution (10mL) was added. The mixture was vigorously stirred at room temperature for 1h, then after evaporation of the solvent, a 1N HCl aqueous solution was added until pH=1. The aqueous solution was extracted with DCM (3x20mL), the organic phase was washed with brine (10mL), dried over Na₂SO₄ and concentrated under reduced pressure to give the title compound (0.230g).
m\z ([MH]⁺) = 205

### Intermediate 75

### 3-[4-(3,5-Difluorophenyl)-1H-pyrazol-1-yl]propionaldehyde

To a solution of 4-(3,5-difluoro phenyl)-1*H*-pyrazole (0.050g) in anhydrous acetonitrile (2mL), acrylaldehyde (0.050mL) was added portionwise. The resulting solution was stirred at 55°C for 16h and then the solvent was removed under reduced pressure to give the title compound (0.52g).
m\z ([MH]⁺) = 237

### Intermediate 76

### 3-[4-(4-Chlorophenyl)-2,5-dimethyl-1H-imidazol-1-yl]propionaldehyde

To a solution of 4-(4-chloro-phenyl)-2,5-dimethyl-1*H*-imidazole (0.030g) in anhydrous acetonitrile (3mL), acrylaldehyde (0.069mL) was added portionwise. The resulting solution was stirred at 50°C for 24h and then the solvent was removed under reduced pressure to give the title compound (0.036g).
m\z ([MH]⁺) = 263

### Intermediate 77

### 3-[4-(4-Nitrophenyl)-1H-imidazol-1-yl]propionaldehyde

To a solution of 4-(4-nitro-phenyl)1*H*-imidazole (0.100g) in anhydrous acetonitrile (12mL), acrylaldehyde (0.450mL) was added portionwise. The resulting solution was stirred at 50°C for 5 days. Solvent was removed under reduced pressure to give the title compound (0.120g).
m\z ([MH]⁺) = 246

### Intermediate 78

### 3-(4-Pyridin-4-yl-imidazol-1-yl)-propionaldehyde

To a solution of 4-(1*H*-imidazol-4-yl)-pyridine (0.040g) in anhydrous acetonitrile (2mL) acrylaldehyde (0.050mL) was added portionwise. The resulting solution was stirred at 55°C for 8h and at room temperature overnight. Solvent was removed under reduced pressure to give the title compound (0.42g).
m\z ([MH]⁺) = 202

### Intermediate 79

### 3-(3-Trifluoromethyl-1H-pyrazol-4-yl)-propionaldehyde

To a solution of oxalyl chloride (0.127mL) in anhydrous DCM (4mL) cooled to -78°C under nitrogen atmosphere DMSO (0.155mL) was slowly added. After stirring for 30min at -78°C a solution of 3-(3-trifluoromethyl-1*H*-pyrazol-4-yl)-propan-1-ol (0.094mL) in anhydrous DCM (3mL) was added dropwise. The mixture was stirred at -40°C for 3h then TEA(0.507mL) was added. The reaction was allowed to reach room temperature then a saturated NaHCO₃ aqueous solution (10mL) was added and the mixture extracted with DCM (3x10mL). The organic phase was washed with brine (10mL), dried over Na₂SO₄ and concentrated under reduced pressure to give the title compound (0.100g)
m\z ([MH]⁺) = 193

### Intermediate 80

### 3-[5-Methyl-4-(4-trifluoromethyl-phenyl)-imidazol-1-yl]-propionaldehyde

To a solution of 5-methyl-4-(4-trifluoromethyl-phenyl)-1*H*-imidazole (0.030g) in anhydrous acetonitrile (2mL) acrylaldehyde (0.027mL) was added. The resulting solution was stirred at 50°C for 24h and at room temperature for 12h. Solvent was removed under reduced pressure to give the title compound (0.038g).
m\z ([MH]⁺) = 283

### Intermediate 81

### 3-Pyridin-3-yl-propionaldehyde

To a solution of oxalyl chloride (0.127mL) in anhydrous DCM (4mL) cooled to -78°C under nitrogen atmosphere DMSO (0.155mL) was slowly added. After 20min at -78°C a solution 3-pyridin-3-yl-propan-1-ol (0.100g) in anhydrous DCM (3mL) was added dropwise within 30min. The mixture was stirred at-40°C for 3h then TEA(0.507mL) was added. The reaction was allowed to reach room temperature then water (5mL) was added. The mixture was extracted with DCM (3x10mL), the organic phase dried over Na₂SO₄ and concentrated under reduced pressure. The crude material was purified by flash chromatography (eluting with: DCM\MeOH from 100\0 to 95\5) to give the title compound (0.052g)
m\z ([MH]⁺) =136

### Intermediate 82

### 3-(4-Pyridin-2-yl-pyrazol-1-yl)-propionaldehyde

To a solution of 2-(1*H*-pyrazol-4-yl)-pyridine (0.058g) in anhydrous acetonitrile (2mL) acrylaldehyde (0.022mL) was added. The resulting solution was stirred at 50°C for 3h. Solvent was removed under reduced pressure to give the title compound (0.060g).
m\z ([MH]⁺) = 202

### Intermediate 83

### 3-Pyridin-4-yl-propionaldehyde

To a solution of oxalyl chloride (0.127mL) in anhydrous DCM (3mL) cooled to -70°C under nitrogen atmosphere, a solution of DMSO (0.155mL) in anhydrous DCM (2mL) was added dropwise. The reaction mixture was stirred for 30min then a solution of 3-pyridin-4-yl-propan-1-ol (0.100g) in anhydrous DCM (2mL) was added dropwise in 30min. The mixture was stirred at -60°C for 3h then the reaction mixture was allowed to reach -10°C and TEA (0.507mL) was added. The reaction was stirred overnight reaching slowly the room temperature. A saturated NaHCO₃ aqueous solution (5mL) was added, the organic phase was dried over Na₂SO₄ and concentrated under reduced pressure to give the title compound (0.100g).
m\z ([MH]⁺) =136

### Intermediate 84

### 3-(4-Pyrimidin-4-yl-pyrazol-1-yl)-gropionaldehyde

To a solution of 4-(1*H*-pyrazol-4-yl)-pyrimidine (0.022g) in anhydrous acetonitrile (2mL) acrylaldehyde (0.020mL) was added. The reaction mixturewas stirred at 50°C for 1.5h. Solvent was removed under reduced pressure to give the title compound (0.025g).
m\z ([MH]⁺) = 203

### Intermediate 85

### 3-(4-Pyridin-4-yl-pyrazol-1-yl)-propionaldehyde

To a solution of 2-(1*H*-pyrazol-4-yl)-pyridine (0.050g) in anhydrous acetonitrile (2mL) acrylaldehyde (0.220mL) was added. The resulting solution was stirred at 50°C for 4h and at room temperature overnight. Solvent was removed under reduced pressure to give the title compound (0.050g).
m\z ([MH]⁺) = 202

### Intermediate 86

### 3-[4-(3,5-Dichloro-phenyl)-2,5-dimethyl-imidazol-1-yl]-propionaldehyde

To a solution of 4-(3,5-dichloro-phenyl)-2,5-dimethyl-1*H*-imidazole (0.035g) in anhydrous acetonitrile (3mL) acrylaldehyde (0.116mL) was added portionwise. The resulting solution was stirred at 50°C for 56h. Solvent was removed under reduced pressure to give the title compound (0.029g).
m\z ([MH]⁺) = 297

### Intermediate 87

### 3-[2,5-Dimethyl-4-(3-trifluoromethyl-phenyl)-imidazol-1-yl]-propionaldehyde

To a solution of 2,5-dimethyl-4-(3-trifluoromethyl-phenyl)-1*H*-imidazole (0.037g) in anhydrous acetonitrile (3mL) acrylaldehyde (0.120mL) was added portionwise. The resulting solution was stirred at 50°C for 48h. Solvent was removed under reduced pressure to give the title compound (0.042g).
m\z ([MH]⁺) = 297

### Intermediate 88

### 3-[4-(1,3-benzoxazol-2-yl)-1H-pyrazol-1-yl]-propionaldehyde

To a stirred suspension of 2-(1*H*-pyrazol-4-yl)-1,3-benzoxazole (0.053g) in anhydrous acetonitrile (2mL) acrylaldehyde (0.220mL) was added. The reaction mixture was stirred at 50°C for 6h. After filtration the solvent was removed under reduced pressure to give the title compound (0.034g).
m\z ([MH]⁺) = 242

### Intermediate 89

### 3-(5-Pyridin-4-yl-pyrazol-1-yl)-propionaldehyde

To a solution of 4-(1*H*-pyrazol-3-yl)-pyridine (0.030g) in anhydrous acetonitrile (1mL) acrylaldehyde (0.085mL) was added. The reaction mixture was stirred at 50°C for 48h. Solvent was removed under reduced pressure to give the title compound (0.036g).
m\z ([MH]⁺) = 202.

### Intermediate 90

### 3-(2-Pyridin-4-yl-imidazol-1-yl)-propionaldehyde

To a solution of 4-(1*H*-imidazol-2-yl)-pyridine (0.050g) in anhydrous acetonitrile (3mL) acrylaldehyde (0.080mL) was added. The reaction mixture was stirred at 50°C for 14h. Solvent was removed under reduced pressure to give the title compound (0.050g).
m\z ([MH]⁺) = 202

### Intermediate 91

### 3-(4-Quinolin-2-yl-1H-pryazol-1-yl)-propionaldehyde

To a solution of 2-(1*H*-pyrazol-4-yl)quinoline (0.050g) in anhydrous acetonitrile (5mL) acrylaldehyde (0.116mL) was added. The reaction mixture was stirred at 50°C for 3 days. Solvent was removed under reduced pressure to give the title compound (0.050g).
m\z ([MH]⁺) = 252

### Intermediate 92

### 3-(4-Ouinolin-4-yl-1H-pyrazol-1-yl)-propionaldehyde

To a solution of 4-(1*H*-pyrazol-4-yl)quinoline (0.050g) in anhydrous acetonitrile (4mL) acrylaldehyde (0.260mL) was added. The reaction mixture was stirred at 50°C for 7 days. Solvent was removed under reduced pressure to give the title compound (0.050g).
m\z ([MH]⁺) = 252

### Intermediate 93

### 3-(4-Quinoxalin-2-yl-1H-pyrazol-1-yl)-propionaldehyde

To a solution of 2-(1*H*-pyrazol-4-yl)quinoxaline (0.050g) in anhydrous acetonitrile (4mL) acrylaldehyde (0.156mL) was added. The reaction mixture was stirred at 50°C for 3 days. Solvent was removed under reduced pressure to give the title compound (0.050g).
m\z ([MH]⁺) = 253

### Intermediate 94

### 3-Thien-3-yl-propionaldehyde

To a solution of oxalyl chloride (0.190mL) in anhydrous DCM (6mL) cooled to -78°C under nitrogen atmosphere a solution of DMSO (0.230mL) in anhydrous DCM (4mL) was slowly added. After stirring for 30min at -78°C a solution of 3-thien-3-ylpropan-1-ol (0.150g) in anhydrous DCM (4mL) was added dropwise. The mixture was stirred at -40°C for 3h then TEA(0.750mL) was added. The reaction was allowed to reach room temperature then a saturated NaHCO₃ aqueous solution (10mL) was added and the mixture extracted with DCM (3x10mL). The organic phase was washed with brine (10mL), dried over Na₂SO₄ and concentrated under reduced pressure to give the title compound (0.140g)
m\z ([MH]⁺) = 141

### Intermediate 95

### 3-[5-(3-Methyl-pyrazin-2-yl)-pyrazol-1-yl]-propionaldehyde

To a solution of 2-methyl-3-(2*H*-pyrazol-5-yl)-pyrazine (0.030g) in anhydrous acetonitrile (1mL) acrylaldehyde (0.065mL) was added. The reaction mixture was stirred at 50°C for 60h. Solvent was removed under reduced pressure to give the title compound (0.038g).
m\z ([MH]⁺) = 217.

### Intermediate 96

### 3-[2-(Methylthio)-1H-benzimidazol-1-yl]-propionaldehyde

To a solution of 2-(methylthio)-1*H*-benzimidazole (0.030g) in anhydrous acetonitrile (1mL) acrylaldehyde (0.080mL) was added. The reaction mixture was stirred at 50°C for 48h. Solvent was removed under reduced pressure to give the title compound (0.039g).
m\z ([MH]⁺) = 221

### Intermediate 97

### 3-[3-(4-Chlorophenyl)-1H-pyrazol-5-yl] -propionaldehyde

To a stirred suspension of Dess-Martin periodinane (0.163g) in anhydrous DCM (4mL) 3-[3-(4-chlorophenyl)-1*H*-pyrazol-5-yl]propan-1-ol (0.050g) was added and the mixture was stirred at room temperature overnight. The reaction was quenched with a Na₂S₂O₃ solution (5% in a saturated NaHCO₃ aqueous solution, 3mL), stirred for 1h then extracted with DCM (10mL). The organic phase washed with brine (5mL), separated, dried over Na₂SO₄ and concentrated under reduced pressure to give the title compound (0.040g).
m\z ([MH]⁺) = 235

### Intermediate 98

### 3-[6-(Methylthio)-7H-purin-7-yl]-propionaldehyde

To a solution of 6-(methylthio)-7*H*-purine (0.032g) in anhydrous acetonitrile (3mL) acrylaldehyde (0.040mL) was added. The reaction mixture was stirred at 80°C for 8h and overnight at room temperature. Solvent was removed under reduced pressure to give the title compound (0.042g).
m\z ([MH]⁺) = 223

### Intermediate 99

### 3-(6-Methoxy-7H-purin-7-yl)-propionaldehyde

To a solution of 6-methoxy-7*H*-purine (0.040g) in anhydrous acetonitrile (3mL) acrylaldehyde (0.057mL) was added. The reaction mixture was stirred at 80°C for 8h. Solvent was removed under reduced pressure to give the title compound (0.055g).
m\z ([MH]⁺) = 207

### Intermediate 100

### 3-(6-Methoxy-2-oxo-1,3-benzoxazol-3(2H)-yl)-propionaldehyde

To a solution of 6-methoxy-1,3-benzoxazol-2(3H)-one (0.072g) in anhydrous acetonitrile (2mL) was added acrylaldehyde (0.060mL). The reaction mixture was stirred at 50°C for 3h. Solvent was removed under reduced pressure to give the title compound (0.080g).
m\z ([MH]⁺) = 222

### Intermediate 101

### 3-(1H-Pyrolo[2,3-b]pyridin-1-yl)-propionaldehyde

To a solution of 1*H*-pyrrolo[2,3-*b*]pyridine (0.040g) in anhydrous acetonitrile (3mL), acrylaldehyde (0.071mL) was added. The reaction mixture was stirred at 80°C for 6h and then the solvent was removed under reduced pressure to give the title compound (0.042g).
m\z ([MH]⁺) = 175

### Intermediate 102

### 3-[3-(2,4-Dimethyl-1,3-thiazol-5-yl)-1H-pyrazol-1-yl]propionaldehyde

To a solution of 2,4-dimethyl-5-(1*H*-pyrazol-3-yl)-1,3-thiazole (0.032g) in anhydrous acetonitrile (1mL) acrylaldehyde (0.040mL) was added. The reaction mixture was stirred at 50°C for 5h and then at room temperature for 3 days. Solvent evaporation under reduced pressure gave the title compound (0.042g).
m\z ([MH]⁺) = 236

### Intermediate 103

### 3-(3H-Imidazo[4,5-c]pyridin-3-yl)propionaldehyde

### and

### 3-(1H-Imidazo[4,5-c]pyridin-1-yl)propionaldehyde

To a solution of 3*H*-imidazo[4,5-*c*]pyridine (0.050g) in anhydrous acetonitrile (4mL) acrylaldehyde (0.025mL) was added. The reaction mixture was stirred at 80°C overnight and then solvent was removed under reduced pressure to give a 1\1 mixture of the title compounds (0.050g).
m\z ([MH]⁺) = 176

### Intermediate 104

### 3-(1H-Benzimidazol-1-yl) propionaldehyde

To a solution of 1*H*-benzimidazole (0.050g) in anhydrous acetonitrile (4mL) acrylaldehyde (0.160mL) was added. The reaction mixture was stirred at 80°C overnight and then the solvent was removed under reduced pressure to give the title compound (0.050g).
m\z ([MH]⁺) = 175

### Intermediate 105

### 3-(3H-Imidazo[4,5-b]pyridin-3-yl)propionaldehyde

To a solution of 3*H*-imidazo[4,5-*b*]pyridine (0.050g) in anhydrous acetonitrile (3mL) acrylaldehyde (0.250mL) was added. The reaction mixture was stirred at 80°C for 24h and then the solvent was removed under reduced pressure to give the title compound (0.050g).
m\z ([MH]⁺) = 176

### Intermediate 106

### 2-(3,3-Dimethoxy-propylsulfanyl)-quinoxaline

To a solution of 2-quinoxalinethiol (0.200g) in a mixture of anhydrous dioxane\DMF 4\1 (5mL) sodium hydride (80% mineral oil, 0.044g) was added portionwise under a nitrogen atmosphere and the reaction mixture stirred at room temperature for 15min. Then 3-bromopropionaldheyde dimethyl acetal (0.200mL) was added and the solution was heated to 80°C for 1.5h. After dilution at room temperature with EtOAc (5mL), the solution was concentrated under reduced pressure, diluted with water (10mL) and extracted with EtOAc (3x10mL). The combined organic phases were then washed with brine (10mL), dried over Na₂SO₄, and concentrated under vacuum to give a crude material that was purified by flash chromatography (eluant: cyclohexane\EtOAc 3\1) to afford the title compound (0.280g).
m\z ([MH]⁺) =265.
TLC: Cyclohehane\EtOAc 7\3 (Rf=0.47).

### Intermediate 107

### 3-(4-Phenyl-1H-imidazol-1-yl)pronionaldehyde

To a solution of 4 phenyl-1*H*-imidazole (0.050g) in anhydrous acetonitrile (4mL) acrylaldehyde (0.210mL) was added. The reaction mixture was stirred at 80°C for 24h and then the solvent was removed under reduced pressure to give the title compound (0.050g).
m\z ([MH]⁺) =201.

### Intermediate 108

### 4-[1-(2,2-Dimethoxyethyl)-1H-imidazol-4-yl]pyridine

To a stirred suspension of sodium hydride (0.009g) in anhydrous DMF (0.5mL) 4-(1*H-*imidazol-4-yl)pyridine (0.040g) was added under nitrogen atmosphere and the reaction mixture was stirred at room temperature for 30min. Then 2-bromoacetaldheyde dimethyl acetal (0.040mL) was added and the solution was heated to 70°C for 8h. After cooling to room temperature the solvent was evaporated to give the title compound (0.064g)
m\z ([MH]⁺)=234

### Intermediate 109

### 2-(2,2-Dimethoxy-ethylsulfanyl)-quinoxaline

To a solution of 2-quinoxalinethiol (0.200g) in a mixture of anhydrous dioxane\DMF 4\1 (5mL) sodium hydride (80% mineral oil, 0.044g) was added portionwise under nitrogen atmosphere and the reaction mixture stirred at room temperature for 15min. Then 2-bromoacetaldheyde dimethyl acetal (0.175mL) was added and the solution was heated to 80°C for 4.5h. After dilution at room temperature with EtOAc (5mL), the solution was concentrated under reduced pressure, diluted with water (10mL) and extracted with EtOAc (3x10mL). The combined organic phases were washed with brine (10mL), dried over Na₂SO₄, evaporated under vacuum obtaining a crude product that was purified by flash chromatography (eluting with: Cyclohexane\EtOAc 3\1) afforded the title compound (0.167g).
m\z ([MH]⁺)= 251.
TLC: Cyclohexane\EtOAc 7\3 (Rf=0.52).

### Intermediate 110

### 3-(4-(Thiophen-2-yl)-imidazol-1-yl)-propionaldehyde

To a solution of 4-thiophen-2-yl-1*H*-imidazole (0.030g) in anhydrous acetonitrile (3mL) TEA(0.022mL) was added. After stirring at room temperature for 15min acrylaldehyde (0.034mL) was added dropwise and the resulting solution was heated to 75°C for 8h and then the solvent was removed under reduced pressure to give the title compound (0.033g).
m\z ([MH]⁺) = 207.

### Intermediate 111

### 3-(6-Methyl-2-oxo-1,3-benzoxazol-3(2H)-yl)propanal

To a solution of 6-methyl-1,3-benzoxazol-2(3H)-one (0.030g) in anhydrous acetonitrile (2mL) acrylaldehyde (0.060mL) was added. The reaction mixture was stirred at 50°C for 48h and then the solvent was removed under reduced pressure to give the title compound (0.040g).
m\z ([MH]⁺) = 206.

### Intermediate 112

### 4-[1-(2,2-dimethoxyethyl)-1H-imidazol-2-yl]pyridine

To a stirred suspension of sodium hydride (0.009g) in anhydrous DMF (0.5mL) 4-(1*H-*imidazol-2-yl)pyridine (0.040g) was added under nitrogen atmosphere and the reaction mixture was stirred at room temperature for 30min. Then 2-bromoacetaldheyde dimethyl acetal (0.040mL) was added and the solution was heated to 70°C for 30h. After cooling to room temperature the solvent was evaporated under vacuum to give the title compound (0.064g)
m\z ([MH]⁺)=234

### Intermediate 113

### 3,4-Dimethyl-5-[1-(3-oxopropyl)-1H-1,2,4-triazol-3-yl]thiophene-2-carbonitrile

To a solution of 3,4-dimethyl-5-(1*H*-1,2,4-triazol-3-yl)thiophene-2-carbonitrile (0.037g) in anhydrous acetonitrile (1mL) acrylaldehyde (0.040mL) was added and the resulting solution was heated to 50°C for 5h. Solvent evaporation under vacuum gave the title compound (0.046g).
m\z ([MH]⁺)=261

### Intermediate 114

### 3-Quinolin-3-yl-propionaldehyde

To a solution of oxalyl chloride (0.290mL) in anhydrous DCM (8mL) cooled to -78°C under nitrogen atmosphere DMSO (0.340mL) was slowly added. After 1h at -78°C a solution of 3-quinolin-3-ylpropan-1-ol (0.300g) in anhydrous DCM (3mL) was added. After stirring at-40°C for 3h TEA(0.892mL) was added and the reaction mixture was allowed to reach room temperature. Water (10mL) was added and the mixture was extracted with DCM (3x10mL), the organic phase dried over Na₂SO₄ and concentrated under reduced pressure to give the title compound (0.290g)
m\z ([MH]⁺)=186.

### Intermediate 115

### 3-[4-(3-Nitrophenyl)-1H-imidazol-1-yl]propanal

To a solution of 4-(3-nitrophenyl)-1*H*-imidazole (0.040g) in anhydrous acetonitrile (5mL) acrylaldehyde (0.120mL) was added and the resulting solution was heated to 80°C and stirred for 4 days. The solvent was removed under reduced pressure to give the title compound (0.041g).
m\z ([MH]⁺) = 246.

### Intermediate 116

### 1-(2,3-Dihydro-1,4-benzodioxin-6-yl)butane-1,4-diol

To a solution of lithium aluminum hydride (1M in THF, 9.53mL) in anhydrous THF (15mL) cooled to 0°C a solution of 4-(2,3-dihydro-1,4-benzodioxin-6-yl)-4-oxobutyric acid (0.750g) in anhydrous THF (5mL) was added dropwise. The reaction mixture was heated to reflux for 24h, then it was cooled to room temperature and diluted with EtOAc (10mL). After evaporation of the solvents under reduced pressure the crude material was treated with a 1N HCl aqueous solution (20mL) and DCM (100mL). The organic phase was washed with a saturated NaHCO₃ aqueous solution (50mL), brine (50mL), dried over Na₂SO₄ and concentrated under reduced pressure. The crude material was purified by flash chromatography (EtOAc\cyclohexane from 50\50 to 100\0) to give the title compound (0.340g).
m\z ([MH]⁺)=225

### Intermediate 117

### 4-(2,3-Dihydro-1,4-benzodioxin-6-yl)-4-oxobutanal

To a solution of oxalyl chloride (0.560mL) in anhydrous DCM (5mL) cooled to -78°C under nitrogen atmosphere a solution of DMSO (0.910mL) in anhydrous DCM (5mL) was slowly added. After stirring for 30min at -78°C a solution of intermediate **116** (0.340g) in anhydrous DCM (2mL) was added dropwise. The reaction mixture was stirred at -40°C for 3h then TEA(2.5mL) was added and then the reaction mixture was allowed to reach room temperature. A saturated NaHCO₃ aqueous solution (20mL) was added and the mixture extracted with DCM (3x20mL). The organic phase was washed with brine (20mL), dried over Na₂SO₄ and concentrated under reduced pressure. The crude material was purified by flash chromatography (EtOAc\cyclohexane 40\60) to give the title compound (0.250g).
m\z ([MH]⁺)=221

### Intermediate 118

### 1-[2-(1,3-Dioxolan-2-yl)ethyl]-5-methoxy-1H-pyrrolo[3,2-b]pyridine

To a solution of 5-methoxy-1*H*-pyrrolo[3,2-b]pyridine (0.040g) in anhydrous THF (1.5mL) cooled to 0°C, sodium hydride (0.010g) was added under nitrogen atmosphere. The mixture was stirred at room temperature for 1h then 2-(2-bromoethyl)-1,3-dioxolane (0.038mL) was added and the stirring continued for an additional 6h. The reaction mixture was quenched with water (3mL) and extracted with DCM (3x5mL), the organic phase dried over Na₂SO₄ and concentrated under reduced pressure to give the title compound (0.067).
m\z ([MH]⁺)=249.

### Intermediate 119

### 2-[1-(2,2-Dimethoxyethyl)-1H-pyrazol-3-yl]-1,3-thiazole

To a stirred suspension of sodium hydride (0.005g) in anhydrous DMF (0.5mL) at 0°C, under nitrogen atmosphere, a solution of 2-(1*H*-pyrazol-3-yl)-1,3-thiazole (0.030g) in anhydrous DMF (1mL) was added. The mixture was allowed to reach room temperature and stirred for an additional 15min and then a solution of bromoacetaldehyde dimethylacetal (0.024mL) in anhydrous DMF (0.5mL) was added. After stirring the reaction mixture at room temperature for 24h, the solvent was evaporated under reduced pressure and the crude material purified by flash chromatography (eluting with: DCM) to give the title compound (0.015g).
m\z ([MH]⁺) = 240.

### Intermediate 120

### 1-(2,2-Dimethoxyethyl)-4-phenyl-1H-imidazole

To a stirred suspension of sodium hydride (0.005g) in anhydrous DMF (0.7mL) at 0°C a solution of 4-phenyl-1*H*-imidazole (0.023g) in anhydrous DMF (1mL) was added under nitrogen atmosphere. The mixture was allowed to reach room temperature and stirred for an additional 15min, then a solution of bromoacetaldehyde dimethylacetal (0.022mL) in anhydrous DMF (0.7mL) was added. After stirring the reaction mixture at room temperature for 24h the solvent was evaporated under reduced pressure and the crude material purified by flash chromatography (eluting with: DCM\MeOH from 100\0 to 90\10) to give the title compound (0.027g).
m\z ([MH]⁺) = 233.

### Intermediate 121

### 1-(2,2-Dimethoxyethyl)-4-thien-2-yl-1H-imidazole

To a stirred suspension of sodium hydride (0.019g) in anhydrous DMF (3mL) at 0°C, under nitrogen atmosphere, a solution of 4-thien-2-yl-1*H*-imidazole (0.100g) in anhydrous DMF (1mL) was added. The mixture was allowed to reach room temperature, stirred for an additional 15min and then a solution of bromoacetaldehyde dimethylacetal (0.090mL) in anhydrous DMF (1mL) was added. After stirring at room temperature for 24h the solvent was evaporated under reduced pressure and the crude material purified by flash chromatography (eluting with: DCM\MeOH from 100\0 to 99\1) to give the title compound (0.088g).
m\z ([MH]⁺) = 239.

### Example 3

### (11S,21R)-2'-O-Acetyl-3-decladinosyl-11,12-dideoxy-6-O-methyl-3-oxo-12,11-[oxycarbonyl-(aminomethyl)-methylene]-erythromycin A

To a solution of intermediate **4** (0.036g) in iPrOH (1.5mL), sodium cyanoborohydride (0.023g) and titanium(III) chloride (10 wt.% solution in 20\30 wt.% hydrochloric acid, 0.1mL) were added portionwise within 6h. The mixture was diluted with a saturated NaHCO₃ aqueous solution (3x2mL), extracted with DCM (3x2mL). The organic phase was dried over Na₂SO₄ and concentrated under reduced pressure. The crude material was purified by flash chromatography (eluting with: DCM\MeOH\NH₄OH 100\0\0, 98\2\0, 95\5\0.5) to give the title compound (0.010g).
¹H-NMR (CDCl₃) δ: 5.06 (dd, 1H), 4.76 (dd, 1H), 4.38 (d, 1H), 4.17 (d, 1H), 3.80 (q, 1H), 3.54 (bm, 1H), 3.24 (m, 1H), 3.20-3.00 (m, 4H), 2.64 (s, 3H), 2.60 (m, 1H), 2.54 (m, 1H), 2.25 (s, 1H+6H), 2.08 (s, 3H), 1.94 (m, 1H), 1.74 (m, 1H), 1.70-1.50 (m, 3H), 1.40 (d, 3H), 1.30 (m, 1H), 1.30(s, 3H), 1.26 (d, 3H), 1.17 (d, 3H), 1.16 (d, 3H), 1.12 (d, 3H), 0.86 (t, 3H).

### Example 4

### (11S,21R,S)-2'-O-Acetyl-3-decladinosyl-11,12-dideoxy-6-O-methyl-3-oxo-12,11-[oxycarbonyl-(benzhydrylideneamino)-methylene]-erythromycin A

To a solution of intermediate **18** (1.5g) and EDC (3.1g) in DCM (100mL) cooled to 0°C, DMSO (3.45mL) was added under nitrogen atmosphere. After stirring at 0°C for 10min, a solution of pyridinium trifluoroacetate (3.12g) in anhydrous DCM (15mL) was slowly added. After 10min the ice-bath was removed. The reaction mixture was stirred for 3h at room temperature then quenched with water (150mL) and extracted with DCM (3x250mL). The organic layer was dried over Na₂SO₄ and concentrated under reduced pressure. The crude material was purified by flash chromatography (eluting with: DCM\MeOH 95\5) to give the title compound (1.2g).
¹H-NMR (CDCl₃) δ: 7.80-7.20 (m, 10H), 6.40 (dd, 1H), 5.15 (s, 1H), 4.73 (m, 1H), 4.42 (d, 1H), 4.16 (d, 1H), 3.90 (q, 1H), 3.55 (m, 1H), 3.17 (m, 1H), 2.9 (m, 1H), 2.94 (d, 1H), 2.67 (m, 1H), 2.53 (s, 3H), 2.43 (m, 1H), 2.33 (s, 6H), 2.05 (s, 3H), 2.00 (m, 1H), 1.74 (m, 1H), 1.65 (m, 1H), 1.53 (s, 3H), 1.38 (d, 3H), 1.23 (s, 3H), 1.29 (d, 3H), 1.25 (m, 1H), 1.25 (d, 3H), 1.14 (d, 3H), 1.07 (d, 3H), 0.83 (t, 3H).
TLC: DCM\MeOH 10\1 (Rf=0.30).

### Example 5

### (11S,21R,S)-3-Decladinosyl-11,12-dideoxy-6-O-methyl-3-oxo-12,11-[oxycarbonyl-(benzhydrylideneamino)-methylene]-erythromycin A

A solution of example **4** (0.030g) in MeOH (1mL) was stirred at room temperature overnight. Solvent evaporation under reduced pressure gave the title compound (0.024g).
¹H-NMR (CDCl₃) δ: 7.80-7.20 (m, 10H), 6.38 (dd, 1H), 5.13 (s, 1H), 4.34 (d, 1H), 4.18 (d, 1H), 3.91 (q, 1H), 3.55 (m, 1H), 3.20 (m, 1H), 2.96 (m, 1H), 2.63 (s, 1H), 2.53 (s, 1H), 2.44 (m, 2H), 2.26 (s, 6H), 2.00 (m, 1H), 1.70-1.45 (m, 7H), 1.45 (d, 1H), 1.30 (m, 4H), 1.04 (d, 3H), 0.96 (t, 3H), 0.91 (d, 3H).
TLC: DCM\MeOH 10\1 (Rf=0.34).

### Example 6

### (11S,21R,S)-2'-O-Acetyl-3-decladinosyl-11,12-dideoxy-6-O-methyl-3-oxo-12,11-[oxycarbonyl-(amino)-methylene]-erythromycin A

A solution of example **4** (1.1g) in acetonitrile (30mL) and a 1.2N HCl aqueous solution (70mL) was stirred at room temperature for 1h. After neutralising the mixture with solid Na₂CO₃ and evaporating the solvent, the aqueous phase was extracted with DCM (3x200mL). The organic layer was dried over Na₂SO₄ and concentrated under reduced pressure to give the title compound (0.9g).
1H-NMR (CDCl₃) δ: 5.45 (dd, 1H), 4.75 (m, 1H), 4.45 (d, 1H), 4.40 (d, 1H), 4.21 (d, 1H), 3.82 (q, 1H), 3.54 (m, 1H), 3.09 (m, 1H), 2.69 (m, 1H), 2.68 (s, 3H), 2.58 (m, 1H), 2.41(m, 1H), 2.25 (s, 6H), 2.07 (m, 3H), 1.95 (m, 1H), 1.75 (m, 1H), 1.60 (m, 1H), 1.49 (s, 3H), 1.39 (d, 3H), 1.35 (m, 1H), 1.31 (s, 3H), 1.26 (d, 3H), 1.17 (d+d, 6H), 1.09 (d, 3H), 0.88 (t, 3H).
TLC: DCM\MeOH 10\1 (Rf=0.48).

### Example 7

### (11S,21S)-2'-O-Acetyl-3-decladinosyl-11,12-dideoxy-6-O-methyl-3-oxo-12,11-[oxycarbonyl-(amino)-methylene]-erythromycin A

To a solution of example **6** (15.1g) in anhydrous toluene (170mL) fresh distilled benzaldehyde (2.52mL) and pyridine (2.01mL) were added and the reaction mixture was refluxed for 4.5h. After evaporating the solvent the residue was dissolved in acetonitrile (60mL) then a 1.2N HCl aqueous solution (120mL) was added at room temperature. After stirring for 1.5h the solvent was evaporated and the aqueous acid solution extracted with EtOAc (150mL) and DCM (150mL). The aqueous phase was neutralized with solid potassium carbonate and extracted with EtOAc (2x150mL). The collected organic extracts were dried over Na₂SO₄ and concentrated under reduced pressure to give the title compound (11.5g, 96% pure by NMR analysis).
¹H-NMR (CDCl₃) δ: 5.45 (dd, 1H), 4.75 (m, 1H), 4.45 (d, 1H), 4.40 (d, 1H), 4.21 (d, 1H), 3.82 (q, 1H), 3.54 (m, 1H), 3.09 (m, 1H), 2.69 (m, 1H), 2.68 (s, 3H), 2.58 (m, 1H), 2.41(m, 1H), 2.25 (s, 6H), 2.07 (m, 3H), 1.95 (m, 1H), 1.75 (m, 1H), 1.60 (m, 1H), 1.49 (s, 3H), 1.39 (d, 3H), 1.35 (m, 1H), 1.31 (s, 3H), 1.26 (d, 3H), 1.17 (d+d, 6H), 1.09 (d, 3H), 0.88 (t, 3H).

### Example 8

### (11S,21S)-3-Decladinosyl-11,12-dideoxy-6-O-methyl-3-oxo-12,11-[oxycarbonyl-(amino)-methylene]-erythromycin A

A solution of example **7** (0.012g) in MeOH (1mL) was stirred at room temperature overnight. After evaporating the solvent under reduced pressure the crude material was purified by flash chromatography (eluting with: DCM\MeOH 100\5) to give the title compound (0.007g).
¹H-NMR (CDCl₃) δ: 5.41 (dd, 1H), 4.42 (d, 1H), 4.33 (d, 1H), 4.22 (d, 1H), 3.83 (q, 1H), 3.56 (m, 1H), 3.23 (d, 1H), 3.12 (m, 1H), 3.02 (m, 1H), 2.80 (d, 1H), 2.69 (s, 3H), 2.60 (m, 1H), 2.54 (m, 1H), 2.40 (d, 1H), 2.33 (s, 6H), 1.95 (m, 1H), 1.9-1.50 (m, 3H), 1.49 (s, 3H), 1.39 (d, 3H), 1.35 (m, 1H), 1.33 (s, 3H), 1.32 (m, 1H), 1.31 (d, 3H), 1.26 (d, 3H), 1.16(d, 3H), 1.10 (d, 3H), 0.88 (t, 3H).

### Example 9

### (10R,S,11R)-3-Decladinosyl-11,12-dideoxy-6-O-methyl-3-oxo-12,11-[oxycarbonylmethylene]-erythromycin A

To a solution of intermediate **23** (0.050g) in anhydrous DCM (25mL), EDC (0.102g) and DMSO (0.115mL) were added under nitrogen atmosphere. The mixture was cooled to 0°C and a solution of pyridinium trifluoroacetate (0.102g) in DCM (0.5mL) was added dropwise. The mixture was allowed to reach room temperature; after stirring for 5h water (10mL) was added and the mixture extracted with DCM (2x20mL). The organic phase was dried over Na₂SO₄, concentrated under reduced pressure and the crude material purified by preparative TLC (eluting with: DCM\MeOH 95\5). The recovered silica gel was stirred for 18h in MeOH (2mL), the mixture was filtered and solvent evaporation under reduced pressure gave the title compound (0.025g).
¹H-NMR (CDCl₃) δ: 4.90 (dd, 1H), 4.32 (d, 1H), 4.24 (d, 1H), 3.85 (q, 1H), 3.56 (m, 1H), 3.32 (d, 1H), 3.18 (m, 1H), 3,12 (m, 1H), 3.02 (m, 1H), 2.80 (d, 1H), 2.71 (dd, 1H), 2.63 (s, 3H), 2.55 (m, 1H), 2.47 (m, 1H), 2.27 (s, 6H), 1.87 (m, 1H), 1.70 (m, 1H), 1.62 (m, 1H), 1.58 (m, 1H), 1.50 (s, 3H), 1.38 (d, 3H), 1.30 (d, 3H), 1.31 (s, 3H), 1.30 (m, 1H), 1.25 (d, 3H), 1.22 (m, 1H), 1.14 (d, 3H), 1.07 (d, 3H), 0.86 (t, 3H).

### Example 10

### (11S,21S)-2'-O-Acetyl-3-decladinosyl-11,12-dideoxy-2-fluoro-6-O-methyl-3-oxo-12,11-[oxycarbonyl-(amino)-methylene]-erythromycin A

To a solution of example **7** (0.284g) in anhydrous THF (14mL) cooled to -10°C potassium *tert*-butoxide (1M in THF, 0.553mL) was added under nitrogen atmosphere. After 5min *N-*fluorobenzenesulfonimide (0.148g) was added at -10°C. The mixture was stirred at room temperature for 1h. The reaction mixture was diluted with DCM (15mL) and washed with water (15mL). The organic layer was dried over Na₂SO₄ and concentrated under vacuum. The crude material was purified by flash chromatography (eluting with: DCM\MeOH\NH₄OH from 100\0\0, to 94\6\0.2) to give the title compound (0.120g).
m\z ([MH]⁺) = 687.
¹H-NMR (CDCl₃) δ: 5.35 (dd, 1H), 4.74 (M, 1H), 4.48 (d, 1H), 433 (d, 1H), 3.96 (m, 1H), 3.52 (m, 1H), 2.89 (s, 3H), 2.86 (m, 1H), 2.72 (m, 1H), 2.53 (m, 1H), 2.27 (s, 6H), 2.08 (s, 3H), 2.0-1.94 (m, 2H), 1.82 (d, 3H), 1.76 (m, 1H), 1.64 (m, 1H), 1.48 (d+m, 3H+1H), 1.31 (s, 3H), 1.26 (m, 6H), 1.16 (d, 3H), 1.08 (d, 3H), 0.92 (t, 3H).

### Example 11

### (11S,21S)-3-Decladinosyl-11,12-dideoxy-2-fluoro-6-O-methyl-3-oxo-12,11-[oxycarbonyl-(amino)-methylene]-erythromycin A

A solution of example **10** (0.010g) in MeOH (1mL) was stirred at room temperature overnight. Evaporation of the solvent under reduced pressure gave the title compound (0.005g).
¹H-NMR (CDCl₃) δ: 5.33 (dd, 1H), 4.42 (d, 1H), 4.33 (d, 1H), 4.03 (dd, 1H), 3.53 (bm, 1H), 3.18 (m, 1H), 3.10 (m, 1H), 2.93 (s,3H), 2.90 (m, 1H), 2.54 (m, 2H), 2.30 (s, 6H), 2.09 (s, 1H), 2.06 (m, 1H), 1.96 (m, 1H), 1.82 (d, 1H), 1.62-1.50 (m, 6H), 1.34 (s, 3H), 1.27(m, 4H), 1.26 (s, 3H), 1.16 (d, 3H), 1.08 (d, 3H), 0.92 (t, 3H).

### Example 12

### (11S,21S)-3-Decladinosyl-11,12-dideoxy-6-O-methyl-3-oxo-12,11-[oxycarbonyl-(2-(N-t-butylcarbamate)-ethylamino)-methylene]-erythromycin A

To a solution of example **7** (1.34g) in anhydrous acetonitrile (12mL) *tert*-butyl *N-*(2-oxoethyl)carbamate (0.640g) was added under nitrogen atmosphere and the resulting mixture was stirred for 24h at room temperature. Sodium cyanoborohydride (1M in THF, 2.0mL) and acetic acid (0.114mL) were added and the reaction mixture was stirred for 12h at room temperature. After evaporating the solvent, the residue was dissolved in DCM (100mL) and washed with a saturated NaHCO₃ aqueous solution (50mL). The organic phase was dried over Na₂SO₄ and concentrated under reduced pressure. The residue was diluted in MeOH (35mL) and refluxed for 15 h. The solvent was evaporated under reduced pressure and the crude material purified by flash chromatography (eluting with: cyclohexane\acetone 85\15) to give the title compound (1.51g, % pure by NMR analysis).
m\z ([MH]⁺) = 770.

### Example 13

### (11S,21S)-2'-O-Acetyl-3-decladinosyl-11,12-dideoxy-6-O-methyl-3-oxo-12,11-[oxycarbonyl-(2-(amino)-ethylamino)-methylene]-erythromycin A

To a solution of example **11** (1.5g) in anhydrous DCM (5.4mL) trifluoroacetic acid (0.6mL) was added under nitrogen atmosphere and the resulting mixture was stirred for 2h at room temperature. The reaction mixture was diluted with EtOAc (15mL) and concentrated under reduced pressure. The residue was dissolved in DCM (100mL) and washed with a saturated NaHCO₃ aqueous solution (50mL), dried over Na₂SO₄ and concentrated under reduced pressure to give the title compound (1.05g).
m\z ([MH]⁺) = 670.
¹H-NMR (CDCl₃) δ: 5.12 (dd, 1H), 4.32 (d, 1H), 4.21 (d, 1H), 4.05 (m, 1H), 3.85 (q, 1H), 3.58 (m, 1H), 3.25 (dd, 1H), 3.20-3.0 (m, 4H), 3.05 (m, 2H), 2.67 (m, 1H), 2.68 (s, 3H), 2.61 (m, 1H), 2.40 (s, 6H), 2.32 (m, 1H), 1.91 (m, 1H), 1.78-1.68 (m, 3H), 1.60 (m, 1H), 1.47 (s, 3H), 1.38 (d, 3H), 1.32 (s, 3H), 1.30 (d, 3H),1.29 (m, 1H), 1.27 (d, 3H), 1.15 (d, 3H), 1.11 (d, 3H), 0.88 (t, 3H).

### Example 14

### (11S,21R)-2'-O-Acetyl-3-decladinosyl-11,12-dideoxy-6-O-methyl-3-oxo-12,11-[oxycarbonyl-(3-quinolinyl-carbonylaminomethyl)-methylene]-erythromycin A

To a solution of intermediate **25** (0.035g) in anhydrous DCM (1.5mL), EDC (0.041g) and DMSO (0.041mL) were added at 0°C under nitrogen atmosphere. After stirring at 0°C for 15min, pyridinium trifluoroacetate (0.042g) was added. The reaction mixture was stirred for 3h at room temperature, then quenched with water (3mL) and extracted with DCM (5mL). The organic phase was dried over Na₂SO₄ and concentrated under reduced pressure. The crude material was purified by preparative TLC (eluting with: DCM\MeOH 95\5) to give the title compound (0.016g).
m\z ([MH]⁺) = 838.

### Example 15

### (11S,21R)-3-Decladinosyl-11,12-dideoxy-6-O-methyl-3-oxo-12,11-[oxycarbonyl-(3-quinolinyl-carbonylaminomethyl)-methylene]-erythromycin A

A solution of example **14** (0.016g) in MeOH (1.5mL) was stirred for 24h then concentrated under reduced pressure to give the title compound (0.009g).
¹H-NMR (CDCl₃) δ: 9.50 (d, 1H), 8.79 (d, 1H), 8.16 (d, 1H), 7.94 (d, 1H), 7.84 (t, NH), 7.81 (t, 1H), 7.61 (t, 1H), 4.90 (dd, 1H), 4.27 (d, 1H), 4.19 (d, 1H), 4.01 (m, 2H), 3.84 (d, 1H), 3.52 (m, 3H), 3.14 (m, 3H), 2.70 (m, 1H), 2.53 (s, 3H), 2.54 (m, 1H), 2.43 (m, 1H), 2.27 (s, 6H), 1.95 (m, 1H), 1.84 (d, 1H), 1.75 (m, 1H), 1.64 (m, 1H), 1.60 (m, 1H), 1.56 (s, 3H), 1.38 (d, 3H), 1.30 (d, 3H), 1.26 (s, 3H), 1.22 (m, 1H), 1.22 (d, 3x3H), 0.88 (t, 3H).

### Example 16

### (11S,21R)-2'-O-Acetyl-3-decladinosyl-11,12-dideoxy-6-O-methyl-3-oxo-12,11-[oxycarbonyl-(4-(4-(pyridin-3-yl)-imidazol-1-yl)-butyramidomethyl)-methylene]-erythromycin A

To a solution of intermediate **26** (0.040g) in anhydrous DCM (3mL) Dess-Martin periodinane (0.030g) was added portionwise at room temperature within 5h. The reaction was quenched with a Na₂S₂O₃ solution (5% in a saturated NaHCO₃ aqueous solution, 2mL), stirred for 1h then extracted with DCM (10mL). The organic phase was dried over Na₂SO₄ and concentrated under reduced pressure. The crude material was purified by flash chromatography (eluting with: DCM\MeOH 95\5) to give the title compound (0.029g).
TLC: DCM\MeOH 90\10 (Rf=0.42).

### Example 17

### (11S,21R)-3-Decladinosyl-11,12-dideoxy-6-O-methyl-3-oxo-12,11-[oxycarbonyl]-(4-(4-(pyridin-3-yl)-imidazol-1-yl)-butyramidomethyl)-methylene]-erythromycin A

A solution of example **16** (0.029g) in MeOH (2mL) was stirred for 24h then concentrated under reduced pressure to give the title compound (0.023g).
¹H-NMR (CDCl₃) δ: 8.98 (d, 1H), 8.40 (dd, 1H), 8.10 (dd, 1H), 7.61 (d, 1H), 7.35 (d, 1H), 7.29 (m, 1H), 6.48 (t, NH), 4.89 (dd, 1H), 4.30 (d, 2H), 4.21 (d, 1H), 4.01 (m, 1H), 3.83 (q, 1H), 3.73 (m, 1H), 3.55 (m, 1H), 3.34 (m, 1H), 3.19 (m, 1H); 3.08 (m, 2H), 2.65 (s, 3H), 2.58 (m, 1H), 2.47 (m, 1H), 2.34 (m, 1H), 2.28 (s, 6H), 2.3-2.1 (m, 4H), 1.91 (m, 1H), 1.81 (m, 1H), 1.80-1.54 (m, 3H), 1.51 (s, 3H), 1.39 (d, 3H), 1.35 (m, 1H), 1.32 (s, 3H), 1.30 (d, 3H), 1.25 (d, 3H), 1.12 (d, 3H), 1.15(d, 3H), 1.12 (d, 3H), 0.87 (t, 3H).

### Example 18

### (11S,21R)-2'-O-Acetyl-3-decladinosyl-11,12-dideoxy-6-O-methyl-3-oxo-12,11-[oxycarbonyl-(3-(4-(pyridin-3-yl)-imidazol-1-yl)-propionamidomethyl)-methylene]-erythromycin A

To a solution of intermediate **27** (0.039g) in anhydrous DCM (3mL) under nitrogen atmosphere Dess Martin periodinane (0.030g) was added portionwise at room temperature within 5h. The reaction was quenched with a Na₂S₂O₃ solution (5% in a saturated NaHCO₃ aqueous solution, 7mL), stirred for 1h and then extracted with DCM (15mL). The organic phase was dried over Na₂SO₄ and concentrated under reduced pressure. The crude material was purified by flash chromatography (eluting with: DCM\MeOH 95\5) to give the title compound (0.026g).
TLC: DCM\MeOH 90\10 (Rf=0.35).

### Example 19

### (11S,21R)-3-Decladinosyl-11,12-dideoxy-6-O-methyl-3-oxo-12,11-[oxycarbonyl-(3-(4-(pyridin-3-yl)-imidazol-1-yl)-propionamidomethyl)-methylene]-erythromycin A

A solution of example **18** (0.024g) in MeOH (1mL) was stirred for 24h then concentrated under reduced pressure to give the title compound (0.020g).
¹H-NMR (CDCl₃) δ: 8.99 (d, 1H), 8.45 (dd, 1H), 8.08 (dd, 1H), 7.58 (d, 1H), 7.35 (d, 1H), 7.29 (m, 1H), 6.67 (t, NH), 4.80 (dd, 1H), 4.36 (m, 2H), 4.30 (d, 1H), 4.19 (d, 1H), 3.83 (m, 2H), 3.61 (m, 1H), 3.55 (m, 1H), 3.25 (m, 2H), 3.04 (m, 2H), 2.70 (m, 2H), 2.62 (s, 3H), 2.58 (m, 2H), 2.33 (s, 6H), 2.32 (d, 1H), 1.85 (m, 1H), 1.80 (m, 1H), 1.70 (m, 2H), 1.56 (m, 1H), 1.48 (s, 3H), 1.45 (d, 3H), 1.33 (s, 3H), 1.21 (d, 3H), 1.35 (m, 1H), 1.18 (d, 3H), 1.04 (d, 3H), 0.95 (t, 3H), 0.91 (d, 3H).

### Example 20

### (11S,21R)-3-Decladinosyl-11,12-dideoxy-6-O-methyl-3-oxo-12,11-[oxycarbonyl-(2-(4-(pyridin-3-yl)-imidazol-1-yl)-acetamidomethyl)-methylene]-erythromycin A

To a solution of intermediate **28** (0.018g) in anhydrous DCM (1.5mL) under nitrogen atmosphere Dess-Martin periodinane (0.020g) was added portionwise at room temperature within 5h. The reaction was quenched with a Na₂S₂O₃solution (5% in a saturated NaHCO₃ aqueous solution, 2mL), stirred for 1h then extracted with DCM (10mL). The organic phase was dried over Na₂SO₄ and concentrated under reduced pressure. The crude material was purified by preparative TLC (eluting with: DCM\MeOH 10\1) and the recovered silica gel was stirred overnight in MeOH (1mL). The mixture was filtered and solvent evaporation under reduced pressure gave the title compound (0.009g).
¹H-NMR (CDCl₃) δ: 9.02 (d, 1H), 8.49 (m, 1H), 8.11 (m, 1H); 7.65 (d, 1H), 7.43 (d, 1H), 7.30 (m, 1H), 6.58 (t, NH), 4.89 (dd, 1H), 4.70 (dd, 2H), 4.29 (d, 1H), 4.20 (d, 1H), 3.83 (m, 2H), 3.69 (m, 1H), 3.51 (m, 1H), 3.29 (m, 1H), 3.17 (m, 1H), 3.04 (m, 2H), 2.60 (s, 3H), 2.54 (m, 1H), 2.44 (m, 1H), 2.31 (m, 1H), 2.27 (s, 6H), 1.91 (m, 1H), 1.80 (m, 1H), 1.68 (m, 2H), 1.60 (m, 1H), 1.49 (s, 3H), 1.39 (d, 3H), 1.29 (s, 3H), 1.29 (d, 3H), 1.22 (m, 1H), 1.25 (d, 3H), 1.12 (d, 3H), 1.10 (d, 3H), 0.87 (t, 3H).

### Example 21

### (11S,21R)-3-Decladinosyl-11,12-dideoxy-6-O-methyl-3-oxo-12,11-[oxycarbonyl-(1-(pyrrolidin-2-one)-methyl)-methylene]-erythromycin A

To a solution of intermediate **29** (0.020g) in anhydrous DCM (2mL) under nitrogen atmosphere Dess-Martin periodinane (0.017g) was added portionwise at room temperature within 1h. After stirring for 5h the reaction was quenched with a solution Na₂S₂O₃ (5% in a saturated NaHCO₃ aqueous solution, 2mL), stirred for 1h then extracted with DCM (3x5mL). The organic phase was dried over Na₂SO₄ and concentrated under reduced pressure. The crude material was purified by flash chromatography (eluting with: DCM\MeOH from 100\0 to 95\5) to give a compound that was dissolved in MeOH (2mL) and stirred at room temperature overnight. Evaporation of the solvent gave the title compound (0.010g).
¹H-NMR (CDCl₃) δ: 4.39 (m, 1H), 3.76 (m, 1H), 3.52 (m, 1H), 3.39 (m, 1H), 3.15 (m, 1H), 3.04 (m, 1H), 2.41 (m, 1H), 2.36 (m, 1H), 2.32 (m, 1H), 2.10 (m, 1H), 2.02 (m, 1H), 1.12 (d, 3H).

### Example 22

### (11S,21R)-3-Decladinosyl-11,12-dideoxy-6-O-methyl-3-oxo-12,11-[oxycarbonyl-(2-(quinoxalin-2-ylsulfanyl)-acetamidomethyl)-methylene]-erythromycin A

To a solution of intermediate **30** (0.068g) in anhydrous DCM (5mL) Dess-Martin periodinane (0.064g) was added under nitrogen atmosphere. The mixture was stirred at room temperature for 6h. The reaction was quenched with a solution Na₂S₂O₃ (5% in a saturated NaHCO₃ aqueous solution, 5mL), stirred for 30min and then extracted with DCM (3x4mL). The organic phase was washed with brine (5mL), dried over Na₂SO₄ and concentrated under reduced pressure. The crude material was dissolved in MeOH (5mL) and stirred overnight. Solvent evaporation under reduced pressure gave the title compound (0.053g).
¹H-NMR (CDCl₃) δ: 8.70 (s, 1H), 8.05 (d, 1H), 8.02 (d, 1H), 7.71 (td, 1H), 7.64 (td, 1H), 7.59 (bt, 1H), 4.11 (d, 1H), 4.03 (m, 1H), 3.34 (bm, 1H), 2.98 (m, 1H), 1.01 (d, 3H).

### Example 23

### (11S,21R)-3-Decladinosyl-11,12-dideoxy-6-O-methyl-3-oxo-12,11-[oxycarbonyl-(3-(quinoxalin-2-ylsulfanyl)-propionamidomethyl)-methylene]-erythromycin A

To a solution of intermediate **31** (0.035g) in anhydrous DCM (2.5mL) Dess-Martin periodinane (0.033g) was added under nitrogen atmosphere. The mixture was stirred at room temperature for 18h. The reaction was quenched with a Na₂S₂O₃ solution (5% in a saturated NaHCO₃ aqueous solution, 2mL), stirred for 30min and then extracted with DCM (3x4mL). The organic phase was washed with brine (3mL), dried over Na₂SO₄ and concentrated under reduced pressure. The crude material was dissolved in anhydrous MeOH (2mL) and stirred overnight. The solvent was evaporated under reduced pressure and the crude material purified by flash chromatography (eluting with: DCM\MeOH from 100\0 to 95\5) to give the title compound (0.011g).
¹H-NMR (CDCl₃) δ: 8.50 (s, 1H), 8.00 (d, 1H), 7.98 (d, 1H), 7.70 (t, 1H), 7.61 (t, 1H), 6.69 (bt, 1H), 3.83 (m, 1H), 3.68 (m, 1H), 3.64 (m, 1H), 3.34 (m, 1H), 3.06 (m, 1H), 2.77 (m, 1H), 2.34 (m, 1H), 1.11 (d, 3H).

### Example 24

### (11S,21R)-3-Decladinosyl-11,12-dideoxy-6-O-methyl-3-oxo-12,11-[oxycarbonyl-[(quinolin-4-ylmethylene)-amino]-methyl)-methylene]-erythromycin A

To a solution of intermediate **32** (0.100g) in anhydrous DCM (4mL) Dess Martin periodinane (0.110g) was added portionwise within 3h under nitrogen atmosphere. The mixture was stirred at room temperature for 3h. The reaction was quenched with a Na₂S₂O₃ solution (5% in a saturated NaHCO₃ aqueous solution, 2mL), stirred for 45min and then extracted with DCM (3x4mL). The organic phase was washed with brine (3mL), dried over Na₂SO₄ and concentrated under reduced pressure. The crude material was purified by preparative TLC (eluting with: DCM\MeOH 95\5), the recovered silica gel stirred for 18h in MeOH (5mL), the mixture filtered and then solvent evaporation under reduced pressure gave the title compound (0.015g).
m\z ([MH]⁺) = 780.

### Example 25

### (11S,21R)-3-Decladinosyl-11,12-dideoxy-6-O-methyl-3-oxo-12,11-[oxycarbonyl-[(quinolin-4-ylmethyl)-amino]-methyl)-methylene]-erythromycin A

To a solution of example **24** (0.015g) in anhydrous MeOH (6mL) palladium (10wt. % on carbon powder, 0.005g) was added and the mixture stirred under hydrogen atmosphere (6 atm) for 20h. Filtration through a celite pad eluting with MeOH (20mL) and purification by flash chromatography (eluting with DCM\MeOH from 100\0 to 95\5) and by preparative TLC (eluting with: DCM\MeOH\NH₄OH 87.5\12.5\0.5) gave the title compound (0.002g).
¹H-NMR (CDCl₃) δ: 8.87 (d, 1H), 8.10 (d, 2H), 7.61 (d, 1H), 7.70 (m, 1H), 7.57 (m, 1H), 4.38 (d, 1H), 4.26 (d, 1H), 3.55 (m, 1H), 3.28-3.00 (m, 2H).

### Example 26

### (11S,21R,S)-3-Decladinosyl-11,12-dideoxy-6-O-methyl-3-oxo-12,11-[oxycarbonyl-(3-(4-(pyridin-3-yl)-imidazol-1-yl)-propionamido)-methylene]-erythromycin A

To a solution of example **6** (0.020g) in anhydrous DMF (2mL) under nitrogen atmosphere intermediate **48** (0.009g), HATU (0.013g) and DIPEA (0.013mL) were sequentially added. The reaction mixture was stirred at room temperature overnight. The solvent was evaporated under reduced pressure, the residue dissolved in DCM (15mL) and washed with water (10mL). The organic phase was dried over Na₂SO₄ and concentrated under reduced pressure. The crude material was purified by flash chromatography (eluting with: DCM\MeOH 95\5) to give a compound that was dissolved in MeOH (1mL) and stirred at room temperature overnight. Solvent evaporation under reduced pressure gave the title compound (0.009g).
¹H-NMR (CDCl₃) δ: 9.04 (d, 1H), 8.44 (dd, 1H), 8.13 (m, 1H), 7.61 (m, 1H), 7.41 (d, 1H), 6.73 (bd, 1H), 5.08 (dd, 1H), 4.94 (m, 1H), 4.45 (m, 1H), 4.26 (m, 1H), 4.25 (d, 1H), 4.08 (m, 3H), 3.80 (q, 1H), 3.51 (m, 1H), 3.15 (m, 1H), 3.04 (m, 1H), 2.98 (m, 1H), 2.65 (m, 1H), 2.60 (m, 1H), 2.52 (m, 1H), 2.44 (m, 1H), 2.39 (s, 3H), 2.34 (m, 1H), 2.26 (s, 6H), 1.90 (m, 1H), 1.75 (m, 1H), 1.65-1.55 (m, 3H), 1.48 (s, 3H), 1.37 (d, 3H), 1.27 (d, 3H), 1.22 (d, 3H), 1.25 (m, 1H), 1.17 (s, 3H), 1.16 (d, 3H), 1.12 (d, 3H), 0.87 (t, 3H).

### Example 27

### (11S,21R)-3-Decladinosyl-11,12-dideoxy-6-O-methyl-3-oxo-12,11-[oxycarbonyl-(4-(4-(pyridin-3-yl)-imidazol-1-yl)-butyramido)-methylene]-erythromycin A

### and

### Example 28

### (11S,21S)-3-Decladinosyl-11,12-dideoxy-6-O-methyl-3-oxo-12,11-[oxycarbonyl-(4-(4-(pyridin-3-yl)-imidazol-1-yl)-butyramido)-methylene]-erythromycin A

To a solution of example **6** (0.020g) in anhydrous DMF (2mL) under nitrogen atmosphere intermediate **49** (0.009g), HATU (0.013g) and DIPEA (0.013mL) were sequentially added. The reaction mixture was stirred at room temperature overnight. The solvent was evaporated under reduced pressure, the residue dissolved in DCM (15mL) and washed with water (10mL). The organic phase was dried over Na₂SO₄ and concentrated under reduced pressure. The crude material was purified by flash chromatography (eluting with: DCM\MeOH 95\5, 90\10) and the (21*R*) and (21*S*) isomers were isolated. Each isomer was dissolved in MeOH (1mL) and stirred at room temperature overnight. Evaporation of the solvent under reduced pressure gave the title compound **27** (0.003g) and the title compound **28** (0.006g).
¹H-NMR (CDCl₃) δ(example **27**): 8.98 (d, 1H), 8.89 (bd, 1H), 8.46 (d, 1M, 8.08 (d, 1H), 7.61 (d, 1H), 7.41 (d, 1H), 7.28 (m, 1H), 5.33 (dd, 1H), 5.22 (dd, 1H), 4.31 (d, 1H), 4.25 (d, 1H), 4.14 (m, 2H), 3.88 (q, 1H), 3.56 (m, 1H), 3.27 (m, 1H), 3.17 (dd, 1H), 3.06 (m, 1H), 2.99 (m, 1H), 2.66 (m, 1H), 2.57 (s, 3H), 2.46 (m, 1H), 2.40 (m, 2H), 2.27 (s, 6H), 2.21 (m, 2H), 1.86 (m, 1H), 1.84 (m, 1H), 1.70-1.50 (m, 3H), 1.54 (s, 3H), 1.36 (d, 3H), 1.33 (d, 3H), 1.29 (s, 3H), 1.25 (m, 1H), 1.24 (d, 3H), 1.17 (d, 3H), 1.08 (d, 3H), 0.90 (t, 3H).
¹H-NMR (CDCl₃) δ (example **28**): 9.02 (d, 1H), 8.46 (dd, 1H), 8.11 (m, 1H), 7.61(m, 1H), 7.37 (d, 1H), 7.31 (m, 1H), 6.47 (bd, 1H), 5.26 (dd, 1H), 4.81 (bt, 1H), 4.29 (d, 1H), 4.13 (m, 3H), 3.81 (q, 1H), 3.54 (m, 1H), 3.17 (m, 1H), 3.08-3.04 (m, 2H), 2.53 (s, 3H), 2.56 (m, 1H), 2.45 (m, 1H), 2.36 (m, 1H), 2.27 (s, 6H), 2.17-2.13 (m, 4H), 2.00 (m, 1H), 1.80 (m, 1H), 1.68 (m, 1H), 1.60 (m, 1H), 1.51 (s, 3H), 1.40 (d, 3H), 1.29 (s, 3H), 1.30 (d, 3H), 1.25 (m, 1H), 1.22 (d, 3H), 1.19 (d, 3H), 1.16 (d, 3H), 0.91 (t, 3H).

### Example 29

### (11S,21R,S)-3-Decladinosyl-11,12-dideoxy-6-O-methyl-3-oxo-12,11-[oxycarbonyl-(5-(4-(pyridin-3-yl)-imidazol-1-yl)-pentylamido)-methylene]-erythromycin A

To a solution of example **6** (0.100g) in anhydrous DMF (5mL) under nitrogen atmosphere intermediate **50** (0.048g), HATU (0.063g) and DIPEA (0.061mL) were sequentially added. The reaction mixture was stirred at room temperature overnight. The solvent was evaporated under reduced pressure, the residue dissolved in DCM (30mL) and washed with water (25mL). The organic phase was dried over Na₂SO₄ and concentrated under reduced pressure. The crude material was purified by flash chromatography (eluting with: DCM\MeOH 95\5, 90\10) to give a compound that was dissolved in MeOH (5mL) and stirred at room temperature overnight. Solvent evaporation under reduced pressure gave the title compound (0.090g).
¹H-NMR (CDCl₃) δ: 8.98 (d, 1H), 8.45 (d, 1H), 8.09 (d, 1H), 7.55 (s, 1H), 7.34 (s, 1H), 7.29 (m, 1H), 6.45 (bd, 1H), 5.19 (dd, 1H), 4.82 (m, 1H), 4.29 (d, 1H), 4.12 (d, 1H), 3.98 (m, 2H), 3.80 (q, 1H), 3.55 (m, 1H), 3.17 (m, 1H), 3.06 (m, 2H), 2.55 (s+m, 1H + 3H), 2.45 (m, 1H), 2.35 (m, 1H), 2.27 (s, 6H), 2.21 (m, 2H), 1.98 (m, 1H), 1.88 (m, 2H), 1.80 (m, 2H), 1.60-1.50 (m, 3H), 1.49 (s, 3H), 1.39 (d, 3H), 1.35 (m, 1H), 1.30 (d+s, 3H+3H), 1.23 (d, 3H), 1.16 (d, 3H+3H), 0.89 (t, 3H).

### Example 30

### (11S,21R)-2'-O-Acetyl-3-decladinosyl-11,12-dideoxy-6-O-methyl-3-oxo-12,11-[oxycarbonyl-(2-(4-(pyridin-3-yl)-imidazol-1-yl)-acetamido)-methylene]-erythromycin A

### and

### Example 31

### (11S,21S)-2'-O-Acetyl-3-decladinosyl-11,12-dideoxy-6-O-methyl-3-oxo-12,11-[oxycarbonyl-(2-(4-(pyridin-3-yl)-imidazol-1-yl)-acetamido)-methylene]-erythromycin A

To a solution of example **6** (0.100g) in anhydrous DMF (8mL) under nitrogen atmosphere a solution of intermediate **47** (0.040g) in anhydrous DMF (2mL), HATU (0.057g) and DIPEA (0.060mL) were sequentially added. The mixture was stirred at room temperature for 8h. The solvent was evaporated under reduced pressure, the residue dissolved in DCM (30mL) and washed with water (2x10mL). The aqueous phase was extracted again with DCM (20mL). The organic layers were collected, dried over Na₂SO₄ and concentrated under reduced pressure. The crude material was purified by flash chromatography (eluting with: DCM\MeOH from 95\5 to 92\8) to give the title compound **30** (0.008g) and the title compound **31** (0.021g).
TLC: DCM\MeOH 95\5 (Rf (example **30**) = 0.53)
TLC: DCM\MeOH 95\5 (Rf (example **31**) = 0.47).

### Example 32

### (11S,21R)-3-Decladinosyl-11,12-dideoxy-6-O-methyl-3-oxo-12,11-[oxycarbonyl-(2-(4-(pyridin-3-yl)-imidazol-1-yl)-acetamido)-methylene]-erythromycin A

A solution of example **30** (0.007g) in MeOH (5mL) was stirred at room temperature for 48h. After evaporating the solvent under reduced pressure, the crude material was purified by flash chromatography (eluting with: DCM\MeOH from 100\0 to 80\20) to give the title compound (0.003g).
¹H-NMR (CDCl₃) δ: 9.19 (d, 1H), 8.98 (d, 1H), 8.47 (dd, 1H), 8.06 (d, 1H), 7.70 (d, 1H), 7.42 (d, 1H), 7.29 (m, 1H), 5.24 (d, 1H), 4.80 (dd, 1H), 3.18 (m, 1H), 2.94 (d, 1H), 0.99 (d, 3H).

### Example 33

### (11S,21S)-3-Decladinosyl-11,12-dideoxy-6-O-methyl-3-oxo-12,11-[oxycarbonyl-(2-(4-(pyridin-3-yl)-imidazol-1-yl)-acetamido)-methylene]-erythromycin A

A solution of example **31** (0.021g) in MeOH (5mL) was stirred at room temperature for 48h. After evaporating the solvent under reduced pressure, the crude material was purified by flash chromatography (eluting with: DCM\MeOH from 100\0 to 80\20) to give the title compound (0.006g).
¹H-NMR (CDCl₃) δ: 8.99 (d, 1H), 8.47 (d, 1H), 8.09 (d, 1H), 7.58 (s, 1H), 7.41 (s, 1H), 7.30 (m, 1H), 6.73 (d, 1H), 4.88 (dd, 1H), 4.67 (s, 1H), 3.08 (m, 1H), 2.41 (d, 1H), 1.16 (d, 3H).

### Example 34

### (11S,21S)-3-Decladinosyl-11,12-dideoxy-6-O-methyl-3-oxo-12,11-[oxycarbonyl-((quinolin-4-yl)carbonylamino)-methylene]-erythromycin A

To a solution of example **6** (0.100g) in anhydrous DMF (8mL) under nitrogen atmosphere a solution of quinoline-4-carboxylic acid (0.026g) in anhydrous DMF (2mL), HATU (0.057g) and DIPEA (0.060mL) were added. The mixture was stirred at room temperature overnight. The solvent was evaporated under reduced pressure, the residue dissolved in DCM (30mL) and washed with a saturated NaHCO₃ aqueous solution (2x10mL). The organic phase was dried over Na₂SO₄ and concentrated under reduced pressure. The crude material was purified by flash chromatography (eluting with: DCM\MeOH 94\6). The obtained compound was dissolved in MeOH (10mL) and stirred overnight. After solvent evaporation, purification by flash chromatography (eluting with: DCM\MeOH 90\10) gave the title compound (0.032g).
¹H-NMR (CDCl₃) δ: 8.96 (d, 1H), 8.45 (d, 1H), 8.13 (d, 1H), 7.76 (t, 1H), 7.65 (t, 1H), 7.52 (d, 1H), 7.02 (d, 1H), 5.08 (dd, 1H), 3.17 (m, 1H), 2.60 (m, 1H), 1.28 (d, 3H).

### Example 35

### (11S,21S)-3-Decladinosyl-11,12-dideoxy-6-O-methyl-3-oxo-12,11-[oxycarbonyl-(3-(quinolin-4-yl)-propionamido)-methylene]-erythromycin A

To a solution of example **6** (0.100g) in anhydrous DMF (8mL) under nitrogen atmosphere 3-quinolin-4-yl-propionic acid (0.030g), HATU (0.057g) and DIPEA (0.060mL) were sequentially added. The reaction mixture was stirred at room temperature overnight. The solvent was evaporated under reduced pressure, the residue dissolved in DCM (30mL) and washed with water (25mL). The organic phase was dried over Na₂SO₄ and concentrated under reduced pressure. The crude material was purified by flash chromatography (eluting with: DCM\MeOH 94\6) to give a compound that was dissolved in MeOH (2mL) and stirred at room temperature overnight. Solvent evaporation under reduced pressure gave the title compound (0.022g).
¹H-NMR (CDCl₃) δ: 8.82 (d, 1H), 8.10 (d, 1H), 8.07 (d, 1H), 7.71 (t, 1H), 7.61 (t, 1H), 7.26 (d, 1H), 6.50 (bd, 1H), 5.14 (dd, 1H), 4.92 (dd, 1H), 4.28 (d, 1H), 4.12 (d, 1H), 3.80 (q, 1H), 3.54 (m, 1H), 3.46 (m, 2H), 3.16 (m, 1H), 3.08 (m, 1H), 3.04 (m, 1H), 2.60 (m, 2H), 2.49 (s, 3H), 2.45 (m, 1H), 2.39 (m, 1H), 2.26 (s, 6H), 2.00 (m, 1H), 1.80 (m, 2H), 1.72 (m, 1H), 1.65 (m, 1H), 1.60 (m, 1H), 1.50 (s, 3H), 1.39 (d, 3H), 1.29 (d, 3H), 1.28 (s, 3H), 1.25 (m, 1H), 1.24 (d, 3H), 1.18 (d, 3H), 1.14 (d, 3H), 0.91 (t, 3H).

### Example 36

### (11S,21S)-3-Decladinosyl-11,12-dideoxy-6-O-methyl-3-oxo-12,11-[oxycarbonyl-(4-(quinolin-4-yl)-butyramido)-methylene]-erythromycin A

To a solution of example **6** (0.075g) in anhydrous DMF (6mL) under nitrogen atmosphere intermediate **60** (0.027g), HATU (0.043g) and DIPEA (0.047mL) were sequentially added. The reaction mixture was stirred at room temperature overnight. The solvent was evaporated under reduced pressure, the residue dissolved in DCM (20mL) and washed with a saturated NaHCO₃ aqueous solution (15mL). The organic phase was dried over Na₂SO₄ and concentrated under reduced pressure. The crude material was purified by flash chromatography (eluting with: DCM\MeOH from 97\3 to 95\5) to give a compound that was dissolved in MeOH (2mL) and stirred at room temperature overnight. Solvent evaporation under reduced pressure gave the title compound (0.039g).
¹H-NMR (CDCl₃) δ: 8.81 (d, 1H), 8.11 (d, 1H), 8.09 (d, 1H), 7.69 (t, 1H), 7.58 (t, 1H), 7.26 (m, 1H), 6.47 (bd, 1H), 5.20 (dd, 1H), 4.88 (dd, 1H), 4.30 (d, 1H), 4.13 (s, 1H), 4.13 (m, 2H), 3.80 (q, 1H), 3.53 (m, 1H), 3.19-3.00 (m, 5H), 2.54 (s, 3H), 2.61-2.41 (m, 3H), 2.29 (s+m, 2H+ 6H), 2.11 (m, 2H), 2.00 (m, 1H), 1.80-1.60 (m, 7H), 1.39 (d, 3H), 1.29 (s+d, 3H+3H), 1.24 (m, 4H), 1.18 (d, 3H), 1.14 (d, 3H), 0.91 (t, 3H).

### Example 37

### (11S,21R,S)-3-Decladinosyl-11,12-dideoxy-6-O-methyl-3-oxo-12,11-|oxycarbonyl-(5-(quinolin-4-yl)-pentylamido)-methylene]-erythromycin A

To a solution of example **6** (0.100g) in anhydrous DMF (6mL) under nitrogen atmosphere intermediate **62** (0.027g), HATU (0.057g) and DIPEA (0.057mL) were sequentially added. The reaction mixture was stirred at room temperature overnight. The solvent was evaporated under reduced pressure, the residue dissolved in DCM (20mL) and washed with water (15mL). The organic phase was dried over Na₂SO₄ and concentrated under reduced pressure. The crude material was purified by flash chromatography (eluting with: DCM\MeOH 95\5) to give a compound that was dissolved in MeOH (2mL) and stirred at room temperature overnight. Solvent evaporation under reduced pressure gave the title compound (0.024g).
1H-NMR (CDCl₃) δ: 8.81 (d, 1H), 8.11 (d, 1H), 8.04 (m, 1H), 7.70 (m, 1H), 7.58 (m, 1H), 7.27 (m, 1H), 6.42 (bd, 1H), 5.18 (dd, 1H), 4.85 (dd, 1H), 4.30 (m, 1H), 4.13 (m, 1H), 3.79 (q, 1H), 3.54 (m, 1H), 3.17 (m, 1H), 3.08 (m, 4H), 2.58 (m, 1H), 2.55 (s, 3H), 2.45 (m, 1H), 2.40 (m, 1H), 2.27 (s, 6H), 2.23 (m, 2H), 1.98 (m, 1H), 1.81 (m, 4H), 1.75 (m, 1H), 1.70 (m, 1H), 1.65 (m, 1H), 1.60 (m, 1H), 1.50 (s, 3H), 1.40 (d, 3H), 1.30 (d, 3H), 1.28 (s, 3H), 1.24 (d, 3H), 1.20 (m, 1H), 1.18 (d, 3H), 1.14 (d, 3H), 0.89 (t, 3H).

### Example 38

### (11S,21R,S)-3-Decladinosyl-11,12-dideoxy-6-O-methyl-3-oxo-12,11-[oxycarbonyl-(3-(4-phenyl-imidazol-1-yl)-pronionamido)-methylene]-erythromycin A

To a solution of example **7** (0.050g) in anhydrous DMF (4mL) under nitrogen atmosphere a solution of intermediate **65** (0.018g) in anhydrous DMF (1mL), HATU (0.013g) and DIPEA (0.013mL) were sequentially added. The reaction mixture was stirred at room temperature overnight. The solvent was evaporated under reduced pressure, the residue dissolved in DCM (15mL) and washed with a saturated NaHCO₃ aqueous solution (10mL). The organic phase was dried over Na₂SO₄ and concentrated under reduced pressure. The crude material was purified by flash chromatography (eluting with: DCM\MeOH from 100\0 to 94\6) to give a compound that was dissolved in MeOH (2mL) and stirred at room temperature overnight. Evaporation of the solvent under reduced pressure gave the title compound (0.031g).
¹H-NMR (CDCl₃) δ: 7.78 (d, 2H), 7.47 (d, 1H), 7.31 (t, 2H), 7.27 (d, 1H), 7.16 (t, 1H), 6.64 (d, 1H), 5.04 (dd, 1H), 4.99 (m, 1H), 4.20 (m, 1H), 3.01 (m, 1H), 2.60-2.50 (m, 2H), 2.33 (dd, 1H), 1.12 (d, 3H).

### Example 39

### (11S,21R,S)-3-Decladinosyl-11,12-dideoxy-6-O-methyl-3-oxo-12,11-[oxycarbonyl-(2-(4-phenyl-imidazol-1-yl)-acetamido)-methylene]-erythromycin A

To a suspension of intermediate **67** (0.022g) in anhydrous DMF (1.6mL) HATU (0.037g) and DIPEA (0.015mL) were sequentially added. The mixture was stirred under nitrogen atmosphere for 30min then example **7** (0.050g) was added. After stirring at room temperature overnight the reaction mixture was diluted with DCM (3.5mL), washed with a 5% NaHCO₃ aqueous solution (3mL) while ice-cooling and the aqueous phase extracted with DCM (2.5mL). The collected organic extracts were dried over Na₂SO₄ and concentrated under reduced pressure. The residue was dissolved in MeOH (10mL) and stirred at room temperature overnight. After solvent evaporation under reduced pressure the crude material was purified by flash chromatography (eluting with: DCM\MeOH\NH₄OH from 100\0\0 to 94\6\0.5) to give the title compound (0.010g).
1H-NMR (DMSO) δ: 8.62 (d, 1H), 7.71 (d, 2H), 7.58 (d, 1H), 7.49 (d, 1H), 7.33 (t, 2H), 7.17 (t, 1H), 4.69 (d, 1H), 4.62 (d, 1H), 4.59 (d, 1H), 3.19 (m, 1H), 2.59 (m, 1H), 1.10 (d, 3H).

### Example 40

### (11S,21R,S)-3-Decladinosyl-11,12-dideoxy-6-O-methyl-3-oxo-12,11-[oxycarbonyl-(3-(4-thiophen-2-yl-imidazol-1-yl)-propionamido)-methylene]-erythromycin A

To a solution of example **7** (0.034g) in anhydrous DMF (4mL) under nitrogen atmosphere a solution of intermediate **69** (0.012g) in anhydrous DMF (1mL), HATU (0.019g) and DIPEA (0.020mL) were sequentially added. The reaction mixture was stirred at room temperature overnight. The solvent was evaporated under reduced pressure, the residue dissolved in DCM (10mL) and washed with a saturated NaHCO₃ aqueous solution (5mL). The organic phase was dried over Na₂SO₄ and concentrated under reduced pressure. The crude material was purified by flash chromatography (eluting with: DCM\MeOH from 100\0 to 94\6) to give a compound that was dissolved in MeOH (5mL) and stirred at room temperature overnight. Solvent evaporation under reduced pressure gave the title compound (0.020g).
¹H-NMR (CDCl₃) δ: 7.46 (d, 1H), 7.31 (dd, 1H), 7.20 (d, 1H), 7.14 (dd, 1H), 7.00 (dd, 1H), 6.68 (d, 1H), 5.08 (dd, 1H), 4.40 (m, 1H), 4.20 (m, 1H), 3.05 (m, 1H), 2.68 (m, 1H), 2.50 (m, 1H), 2.38 (dd, 1H), 1.16 (d, 3H).

### Example 41

### (11S,21R,S)-3-Decladinosyl-11,12-dideoxy-6-O-methyl-3-oxo-12,11-[oxycarbonyl-(3-[3-(1,3-thiazol-2-yl)-1H-pyrazol-1-yl]propionamido)-methylene]-erythromycin A

To a solution of example **7** (0.050g) in anhydrous DMF (4mL) under nitrogen atmosphere a solution of intermediate **70** (0.017g) in anhydrous DMF (1mL), HATU (0.029g) and DIPEA (0.030mL) were sequentially added. The reaction mixture was stirred at room temperature overnight. The solvent was evaporated under reduced pressure, the residue dissolved in DCM (10mL) and washed with a saturated NaHCO₃ aqueous solution (5mL). The organic phase was dried over Na₂SO₄ and concentrated under reduced pressure. The crude material was purified by flash chromatography (eluting with: DCM\MeOH from 100\0 to 95\5) to give a compound that was dissolved in MeOH (3mL) and stirred at room temperature overnight. Solvent evaporation under reduced pressure and purification by flash chromatography (eluting with: DCM\MeOH from 100\0 to 95\5) gave the title compound (0.007g).
m\z ([MH]⁺) = 832.

### Example 42

### (11S,21R,S)-2'-O-Acetyl-3-decladinosyl-11,12-dideoxy-6-O-methyl-3-oxo-12,11-[oxycarbonyl-((2S)-tert-butoxycarbonylamino-3-(1H-indol-3-yl)-propionamido)-methylene]-erythromycin A

To a solution of example **6** (0.050g) in anhydrous DMF (4mL) under nitrogen atmosphere (2*S*)-*tert*-butoxycarbonylamino-3-(1*H*-indol-3-yl)-propionic acid (0.027g), HATU (0.031g) and DIPEA (0.031mL) were sequentally added. The reaction mixture was stirred at room temperature overnight. The solvent was evaporated under reduced pressure, the residue dissolved in DCM (10mL) and washed with a saturated NaHCO₃ aqueous solution (5mL). The organic phase was dried over Na₂SO₄ and concentrated under reduced pressure. The crude material was purified by flash chromatography (eluting with: DCM\MeOH from 98\2 to 97\3) to give the title compound (0.043g).
m\z ([MH]⁺) = 955.

### Example 43

### (11S,21R,S)-3-Decladinosyl-11,12-dideoxy-6-O-methyl-3-oxo-12,11-[oxycarbonyl-((2S)-tert-butoxycarbonylamino-3-(1H-indol-3-yl)-propionamido)-methylene]-erythromycin A

A solution of example **42** (0.013g) in MeOH (2mL) was stirred at room temperature overnight. Solvent evaporation under reduced pressure gave the title compound (0.010g).
m\z ([MH]⁺) = 913.

### Example 44

### (11S,21S)-3-Decladinosyl-11,12-dideoxy-6-O-methyl-3-oxo-12,11-[oxycarbonyl-((2S)-2-amino-3-(1H-indol-3-yl)-propionamido)-methylene]-erythromycin A

To a solution of example **42** (0.025g) in anhydrous DCM (0.5mL) cooled to 0°C trifluoroacetic acid (0.1mL) was added. After removing the ice-bath, the reaction mixture was stirred at room temperature for 1h. The mixture was concentrated under reduced pressure, the residue dissolved in DCM (5mL) and washed with a saturated NaHCO₃ aqueous solution (2mL). The aqueous phase was extracted with DCM (3x3mL). The collected organic extracts were dried over Na₂SO₄ and concentrated under reduced pressure. The crude material was purified by flash chromatography (eluting with: DCM\MeOH 97\3) to give a compound that was dissolved in MeOH (1mL) and the solution was stirred at room temperature overnight. Solvent evaporation under reduced pressure gave the title compound (0.004g).
¹H-NMR (CDCl₃) δ: 8.12 (bs, 1H), 8.06 (d, 1H), 7.65 (d, 1H), 7.36 (d, 1H), 7.19 (t, 1H), 7.16 (d, 1H), 7.13 (t, 1H), 5.35 (dd, 1H), 4,75 (dd, 1H), 3.70 (dd, 1H), 3.34 (dd, 1H), 3.09-3.04 (m, 3H), 2.46-2.44 (m, 2H), 1.20 (d, 3H).
m\z ([MH]⁺) = 813.

### Example 45

### (11S,21R,S)-3-Decladinosyl-11,12-dideoxy-6-O-methyl-3-oxo-12,11-[oxycarbonyl-(2-(quinoxalin-2-ylsulfanyl)-acetamido)-methylene]-erythromycin A

To a solution of (quinoxalin-2-ylsulfanyl)-acetic acid (0.095g) in anhydrous DMF (8.3mL) under nitrogen atmosphere HATU (0.164g) and DIPEA (0.089mL) were added. The reaction mixture was stirred at room temperature for 30min then example 6 (0.262g) was added. The reaction mixture was stirred at room temperature for 20h then it was diluted with DCM (30mL) and washed with a 5% NaHCO₃ aqueous solution (20mL). The aqueous phase was extracted with DCM (25mL), the collected organic layers were washed with a 5% NaHCO₃ aqueous solution (20mL), dried over Na₂SO₄ and concentrated under reduced pressure. The residue was dissolved in MeOH (10mL) and stirred at room temperature overnight. After evaporating the solvent the crude material was purified by flash chromatography (eluting with: DCM\MeOH\NH₄OH from 100\0 to 93\7\1) to give the title compound (0.199g).
¹H-NMR (CDCl₃) δ: 8.67 (s, 1H), 8.19 (dd, 1H), 8.04 (dd, 1H), 7.90 (d, 1H), 7.74 (dt, 1H), 7.66 (dt, 1H), 5.20 (dd, 1H), 4.67 (dd, 1H), 4.30 (d, 1H), 4.06 (m+d, 1H+1H), 3.95 (d, 1H), 3.73 (q, 1H), 3.52 (m, 2H), 3.16 (dd, 1H), 3.06-3.0 (m, 2H), 2.52 (s, 3H), 2.50-2.40 (m, 2H), 2.42 (dd, 1H), 2.27 (s, 6H), 1.92 (m, 1H), 1.81-1.65 (m, 2H), 1.66 (m, 1H), 1.51 (m, 1H), 1.47 (s, 3H), 1.36 (d, 3H), 1.27 (d, 3H), 1.24 (m, 1H), 1.24 (d, 3H), 1.23 (s, 3H), 1.12 (d, 3H), 1.06 (d, 3H), 0.81 (t, 3H).

### Example 46

### (11S,21S)-3-Decladinosyl-11,12-dideoxy-6-O-methyl-3-oxo-12,11-[oxycarbonyl-(2-(quinoxalin-2-ylsulfanyl)-acetamido)-methylene]-erythromycin A

To a solution of (quinoxalin-2-ylsulfanyl)-acetic acid (0.395g) in anhydrous DMF (5mL) under nitrogen atmosphere and HATU (0.682g) and DIPEA (0.365mL) were added at room temperature. The reaction mixture was stirred at room temperature for 30min then a solution of example **7** (1.0g) in anhydrous DMF (3mL) was added. The reaction mixture was stirred at room temperature for 3h then it was poured into a 5% NaHCO₃ aqueous solution (30mL) and the solution extracted with Et₂O (2x30mL). The collected organic layers were washed with brine (30mL), dried over Na₂SO₄ and concentrated under reduced pressure. The residue was dissolved in MeOH (25mL) and stirred at room temperature overnight. After evaporating the solvent the crude material was purified by flash chromatography (eluting with: DCM\MeOH\NH₄OH from 100\0\0 to 93\7\0.2). Crystallisation from EtOAc gave the title compound (0.454g, (21*S*) isomer 99% pure by NMR analysis).
¹H-NMR (CDCl₃) δ: 8.67 (s, 1H), 8.19 (dd, 1H), 8.04 (dd, 1H), 7.90 (d, 1H), 7.74 (dt, 1H), 7.66 (dt, 1H), 5.20 (dd, 1H), 4.67 (dd, 1H), 4.30 (d, 1H), 4.06 (m+d, 1H+1H), 3.95 (d, 1H), 3.73 (q, 1H), 3.52 (m, 2H), 3.16 (dd, 1H), 3.06-3.0 (m, 2H), 2.52 (s, 3H), 2.50-2.40 (m, 2H), 2.42 (dd, 1H), 2.27 (s, 6H), 1.92 (m, 1H), 1.81-1.65 (m, 2H), 1.66 (m, 1H), 1.51 (m, 1H), 1.47 (s, 3H), 1.36 (d, 3H), 1.27 (d, 3H), 1.24 (m, 1H), 1.24 (d, 3H), 1.23 (s, 3H), 1.12 (d, 3H), 1.06 (d, 3H), 0.81 (t, 3H).

### Example 47

### (11S,21R,S)-3-Decladinosyl-11,12-dideoxy-6-O-methyl-3-oxo-12,11-[oxycarbonyl-((4-(2,3-dihydro-benzo[1,4]dioxin-6-yl)-4-oxo)-butyramido)-methylene]-erythromycin A

To a solution of 4-(2,3-dihydro-benzo[1,4]dioxin-6-yl)4-oxo-butyric acid (0.1165g) in anhydrous DMF (9.5mL) under nitrogen atmosphere HATU (0.188g) and DIPEA (0.102mL) were added. The reaction mixture was stirred at room temperature for 30min then example **7** (0.300g) was added. The reaction mixture was stirred at room temperature for 20h then it was diluted with DCM (30mL) and washed with a 5% NaHCO₃ aqueous solution (20mL). The aqueous phase was extracted with DCM (25mL), the collected organic layers were washed with a 5% NaHCO₃ aqueous solution (20mL), dried over Na₂SO₄ and concentrated under reduced pressure. The residue was dissolved in MeOH (10mL) and stirred at room temperature overnight. After evaporating the solvent the crude material was purified by flash chromatography (eluting with: DCM\MeOH\NH₄OH from 100\0\0 to 93\7\1) to give the title compound (0.170g).
¹H-NMR (CDCl₃) δ: 7.52 (d, 2H), 6.89 (d, 1H), 6.73 (d, NH), 5.20 (dd, 1H), 4.84 (dd, 1H), 4.35-4.25 (m, 5H), 4.13 (d, 1H), 3.80 (q, 1H), 3.60-3.55 (m, 2H), 3.32-3.22 (m, 2H), 3.18 (dd, 1H), 3.10-3.00 (m, 2H), 2.65-2.55 (m, 2H), 2.60 (s, 3H), 2.44 (m, 2H), 2.27 (s, 6H), 2.27 (m, 1H), 1.99 (m, 1H), 1.80 (dd, 1H), 1.75 (m, 1H), 1.68 (m, 1H), 1.60 (m, 1H), 1.50 (s, 3H), 1.40 (d, 3H), 1.32 (s, 3H), 1.30 (d, 3H), 1.25 (m, 1H), 1.24 (d, 3H), 1.19 (d, 3H), 1.17 (d, 3H), 0.93 (t, 3H).

### Example 48

### (11S,21R,S)-3-Decladinosyl-11,12-dideozy-6-O-methyl-3-oxo-12,11-[oxycarbonyl-(4-(4-oxo-4,5,6,7-tetrahydro-benzo[b]thiophen-2-yl)-4-oxo-butyramido)-methylene]-erythromycin A

To a solution of 4-(4-oxo-4,5,6,7-tetrahydro-benzo[*b*]thiophen-2-yl)-4-oxo-butyric acid (0.013g) in anhydrous DMF (0.850mL) under nitrogen atmosphere and HATU (0.019g) and DIPEA (0.010mL) were added. The reaction mixture was stirred at room temperature for 30min then example **6** (0.028g) was added. The reaction mixture was stirred at room temperature for 2.5h then it was diluted with DCM (2mL) and washed with a 5% NaHCO₃ aqueous solution (2mL). The aqueous phase was extracted with DCM (1.5mL), the collected organic layers were dried over Na₂SO₄ and concentrated under reduced pressure. The residue was dissolved in MeOH (5mL) and stirred at room temperature for 24h. After evaporating the solvent the crude material was purified by flash chromatography (eluting with: DCM\MeOH\NH₄OH from 100\0\0 to 92\8\0.2) to give the title compound (0.031g).
¹H-NMR (CDCl₃) δ: 8.02 (s, 1H), 6.68 (d, NH), 5.13 (dd, 1H), 4.88 (dd, 1H), 4.32 (d, 1H), 4.14(d, 1H), 3.81 (q, 1H), 3.55 (m, 1H), 3.28 (m, 2H), 3.21 (m, 1H), 3.09-3.03 (m, 4H), 2.62 (s, 3H), 2.63-2.57 (m, 6H), 2.46 (dd, 1H), 2.35 (s, 6H), 2.24 (m, 2H), 1.98 (m, 1H), 1.80-1.70 (m, 3H), 1.60 (m, 1H), 1.49 (s, 3H), 1.40 (d, 3H), 1.32 (s, 3H), 1.29 (d, 3H), 1.25 (m, 4H), 1.18 (d, 3H), 1.17 (d, 3H), 0.92 (t, 3H).

### Example 49

### (11S,21S)-3-Decladinosyl-11,12-dideoxy-6-O-methyl-3-oxo-12,11-[oxycarbonyl-(4-(4-oxo-4,5,6,7-tetrahydro-benzo[b]thiophen-2-yl)-4-oxo-butyramido)-methylene]-erythromycin A

To a solution of 4-(4-oxo-4,5,6,7-tetrahydro-benzo[*b*]thiophen-2-yl)-4-oxo-butyric acid (1.280g) in anhydrous DMF (12mL) under nitrogen atmosphere HATU (2.02g) and DIPEA (1.06mL) were added. The reaction mixture was stirred at room temperature for 30min then a solution of example **7** (2.90g) in anhydrous DMF (10mL) was added. The reaction mixture was stirred at room temperature overnight then it was diluted with EtOAc (100mL) and washed with a 5% NaHCO₃ aqueous solution (2x50mL) and brine (50mL). The organic layer was dried over Na₂SO₄ and concentrated under reduced pressure. The crude material was purified by flash chromatography (eluting with: DCM\MeOH from 100\0 to 98\2) to give a compound that was dissolved in MeOH (150mL) and stirred overnight After evaporating the solvent the residue was purified by flash chromatography (eluting with: DCM\MeOH from 100\0 to 97\3) to give the title compound (2.40g, (21*S*) isomer 95% pure by NMR analysis).
¹H-NMR (CDCl₃) δ: 8.02 (s, 1H), 6.68 (d, NH), 5.13 (dd, 1H), 4.88 (dd, 1H), 4.32 (d, 1H), 4.14(d, 1H), 3.81 (q, 1H), 3.55 (m, 1H), 3.28 (m, 2H), 3.21 (m, 1H), 3.09-3.03 (m, 4H), 2.62 (s, 3H), 2.63-2.57 (m, 6H), 2.46 (dd, 1H), 2.35 (s, 6H), 2.24 (m, 2H), 1.98 (m, 1H), 1.80-1.70 (m, 3H), 1.60 (m, 1H), 1.49 (s, 3H), 1.40 (d, 3H), 1.32 (s, 3H), 1.29 (d, 3H), 1.25 (m, 4H), 1.18 (d, 3H), 1.17 (d, 3H), 0.92 (t, 3H).

### Example 50

### (11S, 21R,S)-3-Decladinosyl-11,12-dideoxy-6-O-methyl-3-oxo-12,11-[oxycarbonyl-(4-(4-methoxy-3-nitro-phenyl)-4-oxo-butyramido)-methylene]-erythromycin A

To a solution of 4-(4-methoxy-3-nitro-phenyl)-4-oxo-butyric acid (0.136g) in anhydrous DMF (9mL) under nitrogen atmosphere HATU (0.205g) and DIPEA (0.110mL) were added. The reaction mixture was stirred at room temperature for 30min then example 6 (0.300g) was added. The reaction mixture was stirred at room temperature for 2.5h then it was diluted with DCM (21mL) and washed with a 5% NaHCO₃ aqueous solution (18mL). The aqueous phase was extracted with DCM (15mL), the collected organic layers were dried over Na₂SO₄ and concentrated under reduced pressure. The residue was dissolved in MeOH (30mL) and DCM (2mL) and stirred at room temperature for 48h. After evaporating the solvent the crude material was purified by LC (mobile phase: A\B 85\15 for 1min, from 85\15 to 15\85 in 20min; λ= 255nm) to give the title compound (0.149g).
¹H-NMR (CDCl₃) δ: 8.47 (d, 1H), 8.19 (dd, 1H), 7.15 (d, 1H), 6.69 (d, NH), 5.18 (dd, 1H), 4.85 (dd, 1H), 4.31 (d, 1H), 4.14 (d, 1H), 4.04 (s, 3H), 3.80 (q, 1H), 3.54 (m, 1H), 3.32 (m, 2H), 3.18 (dd, 1H), 3.06-3.0 (m, 2H), 2.66 (m, 2H), 2.60 (s, 3H), 2.46 (m, 1H), 2.42 (dd, 1H), 2.28 (s, 6H), 1.98 (m, 1H), 1.81-1.55 (m, 4H), 1.49 (s, 3H), 1.40 (d, 3H), 1.32 (s, 3H), 1.30 (d, 3H), 1.25 (d, 3H), 1.25 (m, 1H), 1.18 (d, 3H), 1.17 (d, 3H), 0.81 (t, 3H).

### Example 51

### (11S,21S)-3-Decladinosyl-11,12-dideoxy-6-O-methyl-3-oxo-12,11-[oxycarbonyl-(4-(4-methoxy-3-nitro-phenyl)4-oxo-butyramido)-methylene]-erythromycin A

To a solution of 4-(4-methoxy-3-nitro-phenyl)-4-oxo-butyric acid (2.782g) in anhydrous DMF (36mL) under nitrogen atmosphere HATU (1.230g) and DIPEA (0.656mL) were added. The reaction mixture was stirred at room temperature for 30min then example **7** (1.8g) was added. The reaction mixture was stirred at room temperature for 3h then it was diluted with Et₂O (200mL) and washed with a 5% NaHCO₃ aqueous solution (2x100mL). The aqueous phase was extracted with Et₂O (50mL) and DCM (50mL), the combined organic layers were dried over Na₂SO₄ and concentrated under reduced pressure. The crude material was purified by flash chromatography (eluting with: DCM\MeOH from 100\0 to 96\4). The obtained compound was dissolved in MeOH (100mL) and stirred at room temperature overnight. After evaporating the solvent the product was purified by flash chromatography (eluting with: DCM\MeOH from 100\0 to 96\4) to give the title compound (1.510g, (21*S*) isomer 95% pure by NMR analysis).
¹H-NMR (CDCl₃) δ: 8.47 (d, 1H), 8.19 (dd, 1H), 7.15 (d, 1H), 6.69 (d, NH), 5.18 (dd, 1H), 4.85 (dd, 1H), 4.31 (d, 1H), 4.14 (d, 1H), 4.04 (s, -OCH₃), 3.80 (q, 1H), 3.54 (m, 1H), 3.32 (m, 2H), 3.18 (dd, 1H), 3.06-3.0 (m, 2H), 2.66 (m, 2H), 2.60 (s, 3H), 2.46 (m, 1H), 2.42 (dd, 1H), 2.28 (s, 6H), 1.98 (m, 1H), 1.81-1.55 (m, 4H), 1.49 (s, 3H), 1.40 (d, 3H), 1.32 (s, 3H), 1.30 (d, 3H), 1.25 (d, 3H), 1.25 (m, 1H), 1.18 (d, 3H), 1.17 (d, 3H), 0.81 (t, 3H).

### Example 52

### (11S,21R,S)-3-Decladinosyl-11,12-dideoxy-6-O-methyl-3-oxo-12,11-[oxycarbonyl-(4-(2-hydroxy-4,5-dimethoxy-phenyl)-4-oxo-butyramido)-methylene]-erythromycin A

To a solution of 4-(2-hydroxy-4,5-dimethoxy phenyl)-4-oxo-butyric acid (0.137g) in anhydrous DMF (9mL) under nitrogen atmosphere HATU (0.205g) and DIPEA (0.110mL) were added. The reaction mixture was stirred at room temperature for 30min then example **6** (0.300g) was added. The reaction mixture was stirred at room temperature for 2.5h then it was diluted with DCM (21mL) and washed with a 5% NaHCO₃ aqueous solution (18mL). The aqueous phase was extracted with DCM (15mL), the combined organic layers were dried over Na₂SO₄ and evaporated under reduced pressure.
The residue was dissolved in MeOH (10mL) and stirred at room temperature for 24h. After evaporating the solvent the crude material was purified by LC (mobile phase: A\B from 85\15 to 15\85 in 20 min; λ= 237nm) to give the title compound (0.120g).
1H-NMR (CDCl₃) δ: 12.49 (s, 1H), 7.14 (s, 1H), 6.71 (d, NH), 6.44 (s, 1H), 5.18 (dd, 1H), 4.88 (dd, 1H), 4.31 (d, 1H), 4.14 (d, 1H), 3.91 (s, 3H), 3.88 (s, 3H), 3.80 (q, 1H), 3.55 (m, 1H), 3.41-3.30 (m, 2H), 3.20 (dd, 1H), 3.11-3.0 (m, 2H), 2.70-2.60 (m, 2H), 2.61 (s, 3H), 2.52 (m, 1H), 2.43 (dd, 1H), 2.31 (s, 6H), 1.98-1.70 (m, 3H), 1.60 (m, 1H), 1.50 (s, 3H), 1.30 (d, 3H), 1.26 (d, 3H), 1.25 (m, 1H), 1.24 (d+d, 6H), 1.19 (d, 3H), 1.17 (d, 3H), 0.91 (t, 3H).

### Example 53

### (11S,21R,S)-3-Decladinosyl-11,12-dideoxy-6-O-methyl-3-oxo-12,11-[oxycarbonyl-(4-(3-hydrozy-4-methoxy-phenyl)-4-oxo-butyramido)-methylene]-erythromycin A

To a solution of 4-(3-hydroxy-4-methoxy-phenyl)-4-oxo-butyric acid (0.121g) in anhydrous DMF (9mL) under nitrogen atmosphere HATU (0.205g) and DIPEA (0.110mL) were added. The reaction mixture was stirred at room temperature for 30min then example **6** (0.300g) was added. The reaction mixture was stirred at room temperature for 2.5h then it was diluted with DCM (21mL) and washed with a 5% NaHCO₃ aqueous solution (18mL). The aqueous phase was extracted with DCM (15mL), the combined organic layers were dried over Na₂SO₄ and concentrated under reduced pressure. The residue was dissolved in MeOH (30mL) and DCM (2mL) and stirred at room temperature for 48h. After evaporating the solvent the crude material was purified by LC (mobile phase: A\B 85\15 for 1min, from 85\15 to 15\85 in 20 min; λ= 230nm) to give the title compound (0.020g).
¹H-NMR (CDCl₃) δ: 7.57 (m, 2H), 6.89 (d, 1H), 6.71 (d, 1H), 5.88 (bs, 1H), 5.22 (dd, 1H), 4.81 (dd, 1H), 4.31 (d, 1H), 4.13 (d, 1H), 3.96 (s, 3H), 3.81 (q, 1H), 3.55 (m, 1H), 3.31 (m, 2H), 3.22 (m, 1H), 3.09-3.04 (m, 2H), 2.66-2.54 (m, 4H), 2.61 (s, 3H), 2.44 (dd, 1H), 2.34 (s, 6H), 2.00 (m, 1H), 1.81-1.70 (m, 3H), 1.6 (m, 1H), 1.50 (s, 3H), 1.41 (d, 3H), 1.31 (s, 3H), 1.29 (d, 3H), 1.25 (d, 3H), 1.25 (m, 1H), 1.19 (d, 3H), 1.17 (d, 3H), 0.93 (t, 3H).

### Example 54

### (11S,21S)-3-Decladinosyl-11,12-dideoxy-6-O-methyl-3-oxo-12,11-[oxycarbonyl-(4-(3-hydroxy-4-methoxy-phenyl)-4-oxo-butyramido)-methylene]-erythromycin A

To a solution of 4-(3-hydroxy-4-methoxy-phenyl)-4-oxo-butyric acid (0.273g) in anhydrous DMF (3.5mL), HOBT (0.165g) and EDC (0.234g) were added under nitrogen atmosphere. After stirring 5min example **7** (0.627g) was added, and the resulting mixture was stirred at room temperature for 3h. The reaction mixture was diluted with DCM (15mL) washed with a 5% NaHCO₃ aqueous solution (10mL). The organic layer was washed with brine (10ml), dried over Na₂SO₄ and concentrated under reduced pressure. The crude material was purified by flash chromatography (eluting with: DCM\MeOH from 97\3 to 95\5) to give a compound that was dissolved in MeOH (10mL) and stirred at room temperature overnight. After solvent evaporation the compound was purified by preparative LC (column Waters XTerra MS C18 (19 x 300mm, 7µm); flow rate = 12 ml\min, A\B from 70\30 to 10\90 in 25min; λ= 230mn) to give the title compound (0.165g, (21*S*) isomer 94% pure by NMR analysis).
¹H-NMR (CDCl₃) δ: 7.57 (m, 2H), 6.89 (d, 1H), 6.71 (d, 1H), 5.88 (bs, 1H), 5.22 (dd, 1H), 4.81 (dd, 1H), 4.31 (d, 1H), 4.13 (d, 1H), 3.96 (s, 3H), 3.81 (q, 1H), 3.55 (m, 1H), 3.31 (m, 2H), 3.22 (m, 1H), 3.09-3.04 (m, 2H), 2.66-2.54 (m, 4H), 2.61 (s, 3H), 2.44 (dd, 1H), 2.34 (s, 6H), 2.00 (m, 1H), 1.81-1.70 (m, 3H), 1.6 (m, 1H), 1.50 (s, 3H), 1.41 (d, 3H), 1.31 (s, 3H), 1.29 (d, 3H), 1.25 (d, 3H), 1.25 (m, 1H), 1.19 (d, 3H), 1.17 (d, 3H), 0.93 (t, 3H).

### Example 55

### (11S,21R,S)-3-Decladinosyl-11,12-dideoxy-6-O-methyl-3-oxo-12,11-[oxycarbonyl-(4-(3,4-dimethoxy-phenyl)-4-oxo-butyramido)-methylene]-erythromycin A

To a solution of 4-(3,4-dimethoxy-phenyl)-4-oxo-butyric acid (0.128g) in anhydrous DMF (9mL) under a nitrogen atmosphere HATU (0.205g) and DIPEA (0.110mL) were added. The reaction mixture was stirred at room temperature for 30min then example **6** (0.300g) was added. The reaction mixture was stirred at room temperature for 2.5h then it was diluted with DCM (21mL) and washed with a 5% NaHCO₃ aqueous solution (18mL). The aqueous phase was extracted with DCM (15mL), the combined organic layers were dried over Na₂SO₄ and concentrated under reduced pressure. The residue was dissolved in MeOH (30mL) and stirred at room temperature for 24h. After evaporating the solvent the crude material was purified by flash chromatography (eluting with: DCM\MeOH\NH₄OH from 100\0\0 to 92\8\0.2) to give the title compound (0.293g).
¹H-NMR (CDCl₃) δ: 7.95 (d, NH), 7.70 (dd, 1H), 7.55 (d, 1H), 6.99 (d, 1H), 5.30 (dd, 1H), 5.30 (dd, 1H), 4.71 (m, 1H), 4.37 (d, 1H), 4.08 (d, 1H), 3.96-3.93 (m, 4H), 3.90 (q, 1H), 3.68 (m, 1H), 3.39 (dd, 1H), 3.34 (m, 2H), 3.19 (m, 1H), 3.14 (m, 1H), 2.75 (s, 6H), 2.66-2.61 (m, 6H), 2.49 (d, 1H), 1.99-1.96 (m, 2H), 1.84-1.71 (m, 2H), 1.64 (m, 1H), 1.56 (s, 3H), 1.41 (m+d, 1H+3H), 1.32 (d, 3H), 1.31 (s, 3H), 1.26 (d, 3H), 1.23 (d, 3H), 1.21 (d, 3H), 0.95 (t, 3H).

### Example 56

### (11S,21S)-3-Decladinosyl-11,12-dideoxy-6-O-methyl-3-oxo-12,11-[oxycarbonyl-(4-(3,4-dimethoxy-phenyl)-4-oxo-butyramido)-methylene]-erythromycin A

To a solution of 4-(3,4-dimethoxy-phenyl)-4-oxo-butyric acid (0.695g) in anhydrous DMF (15mL) under nitrogen atmosphere HATU (1.08g) and DIPEA (0.586mL) were added. The reaction mixture was stirred at room temperature for 30min then example **7** (1.50g) was added. The reaction mixture was stirred at room temperature for 18h then it was diluted with DCM (35mL) and washed with a 5% NaHCO₃ aqueous solution (30mL). The aqueous phase was extracted with DCM (25mL), the combined organic layers were dried over Na₂SO₄ and concentrated under reduced pressure. The residue was dissolved in MeOH (50mL) and stirred at room temperature for 18h. After evaporating the solvent the crude material was crystallised from DCM to give the title compound (1.26g, (21*S*) isomer 98% pure by NMR analysis).
¹H-NMR (CDCl₃) δ: 7.95 (d, NH), 7.70 (dd, 1H), 7.55 (d, 1H), 6.99 (d, 1H), 5.30 (dd, 1H), 5.30 (dd, 1H), 4.71 (m, 1H), 4.37 (d, 1H), 4.08 (d, 1H), 3.96-3.93 (m, 4H), 3.90 (q, 1H), 3.68 (m, 1H), 3.39 (dd, 1H), 3.34 (m, 2H), 3.19 (m, 1H), 3.14 (m, 1H), 2.75 (s, 6H), 2.66-2.61 (m, 6H), 2.49 (d, 1H), 1.99-1.96 (m, 2H), 1.84-1.71 (m, 2H), 1.64 (m, 1H), 1.56 (s, 3H), 1.41 (m+d, 1H+3H), 1.32 (d, 3H), 1.31 (s, 3H), 1.26 (d, 3H), 1.23 (d, 3H), 1.21 (d, 3H), 0.95 (t, 3H).

### Example 57

### (11S,21R,S)-3-Decladinosyl-11,12-dideoxy-6-O-methyl-3-oxo-12,11-[oxycarbonyl-(4-(4-hydroxy-3-methoxy-phenyl)-4-oxo-butyramido)-methylene]-erythromycin A

To a solution of 4-(4-hydroxy-3-methoxy-phenyl)-4-oxo-butyric acid (0.922g) in anhydrous DMF (20mL) HOBT (0.656g) and EDC (0.933g) were added. Then example **6** (2.5g) was added, and the resulting mixture was stirred at room temperature overnight The reaction mixture was diluted with DCM (75mL) washed with a 1N HCl aqueous solution (30mL) then with a saturated NaHCO₃ aqueous solution (30mL). The organic layer was dried over Na₂SO₄ and concentrated under reduced pressure. The crude material was purified by flash chromatography (eluting with: DCM\MeOH 98\2) then dissolved in MeOH (10mL) and stirred at room temperature overnight. Solvent evaporation gave the title compound (0.600g).
1H-NMR (CDCl₃) δ: 7.57 (dd, 1H), 7.50 (d, 1H), 6.92 (d, 1H), 6.89 (d, 1H), 6.05 (bs, 1H), 5.19 (dd, 1H), 4.80 (dd, 1H), 4.28 (d, 1H), 4.10 (d, 1H), 3.93 (s, 3H), 3.77 (q, 1H), 3.58 (bs, 1H), 3.51 (m, 1H), 3.30 (m, 2H), 3.17 (dd, 1H), 3.06 (m, 1H), 3.02 (m, 1H), 2.6 (m, 3H), 2.57 (s, 3H), 2.48 (bm, 1H), 2.41 (dd, 1H), 2.28 (bs, 6H), 1.95 (m, 1H), 1.81-1.50 (m, 4H), 1.47 (s, 3H), 1.37 (d, 3H), 1.28 (s, 3H), 1.27 (d, 4H), 1.22 (d, 3H), 1.17 (d, 3H), 1.15 (d, 3H), 0.90 (t, 3H).

### Example 58

### (11S,21S)-3-Decladinosyl-11,12-dideoxy-6-O-methyl-3-oxo-12,11-[oxycarbonyl-(4-(4-hydroxy-3-methoxy-phenyl)-4-oxo-butyramido)-methylene]-erythromycin A

To a solution of 4-(4-hydroxy-3-methoxy-phenyl)-4-oxo-butyric acid (0.720g) in anhydrous DMF (12mL) HOBT (0.259g) and EDC (0.368g) were added under nitrogen atmosphere. Then example **7** (1.65g) was added, and the resulting mixture was stirred at room temperature overnight. The reaction mixture was diluted with DCM (50mL) washed with a 1N HCl aqueous solution (20mL) then with a saturated NaHCO₃ aqueous solution (20mL). The organic layer was dried over Na₂SO₄ and concentrated under reduced pressure. The crude material was purified by flash chromatography (eluting with: DCM\MeOH 98\2) then dissolved in MeOH (10mL) and stirred at room temperature overnight. Solvent evaporation gave the title compound (0.360g, (21*S*) isomer 95% pure by NMR analysis).
1H-NMR (CDCl₃) δ: 7.57 (dd, 1H), 7.50 (d, 1H), 6.92 (d, 1H), 6.89 (d, 1H), 6.05 (bs, 1H), 5.19 (dd, 1H), 4.80 (dd, 1H), 4.28 (d, 1H), 4.10 (d, 1H), 3.93 (s, 3H), 3.77 (q, 1H), 3.58 (bs, 1H), 3.51 (m, 1H), 3.30 (m, 2H), 3.17 (dd, 1H), 3.06 (m, 1H), 3.02 (m, 1H), 2.6 (m, 3H), 2.57 (s, 3H), 2.48 (bm, 1H), 2.41 (dd, 1H), 2.28 (bs, 6H), 1.95 (m, 1H), 1.81-1.50 (m, 4H), 1.47 (s, 3H), 1.37 (d, 3H), 1.28 (s, 3H), 1.27 (d, 4H), 1.22 (d, 3H), 1.17 (d, 3H), 1.15 (d, 3H), 0.90 (t, 3H).

### Example 59

### (11S,21R,S)-3-Decladinosyl-11,12-dideoxy-6-O-methyl-3-oxo-12,11-[oxycarbonyl-(2-(3-methoxy-quinoxalin-2-ylsulfanyl)-acetamido)-methylene]-erythromycin A

To a solution of intermediate **72** (0.032g) in anhydrous DMF (2mL) HATU (0.048g) and DIPEA (0.022mL) were added under nitrogen atmosphere. After stirring for 45min example 7 (0.070g) was added and the mixture stirred overnight. Water (5mL) was added and the solution extracted with DCM (2x10mL). The organic phase was washed with brine (5mL), dried over Na₂SO₄ and concentrated under reduced pressure. The residue was dissolved in MeOH (5mL) and stirred overnight. The crude material was purified by flash chromatography (eluting with: DCM\MeOH 94\6) to give the title compound (0.060g).
m\z ([MH]⁺) = 859
1H-NMR (CDCl₃) δ: 8.09 (d, 1H), 7.78 (d, 1H), 7.81 (d, 1H), 7.56 (t, 1H), 7.53 (t, 1H), 4.65 (t, 1H), 4.15 (s, 3H), 4.06 (d, 1H), 3.90 (d, 1H), 3.03 (m, 1H), 2.42 (m, 1H), 1.10 (d, 3H).

### Example 60

### (11S,21R,S)-3-Decladinosyl-11,12-dideoxy-6-O-methyl-3-oxo-12,11-[oxycarbonyl-(2-(quinoxalin-2-yloxy)-acetamido)-methylene]-erythromycin A

To a solution of intermediate **74** (0.038g) in anhydrous DMF (1.5mL) HATU (0.072g) and DIPEA (0.033mL) were added under nitrogen atmosphere. After stirring for 45min example 7 (0.090g) was added and the mixture stirred overnight. Water (8mL) was added and the solution extracted with DCM (2x15mL). Collected organic phases were washed with a saturated NaHCO₃ aqueous solution (10mL), brine (10mL), then dried over Na₂SO₄ and concentrated under reduced pressure. The residue was dissolved in MeOH (5mL) and stirred overnight. Purification of the crude material by flash chromatography (eluting with: DCM\MeOH 95\5) gave the title compound (0.047g).
m\z ([MH]⁺) = 813
¹H-NMR (CDCl₃) δ: 8.35 (s, 1H), 7.86 (d, 1H), 7.57 (td, 1H), 7.36 (m, 2H), 7.26 (d, 1H), 5.10 (dd, 1H), 5.02 (d, 1H), 4.87 (d, 1H), 3.01 (m, 1H), 2.34 (m, 1H), 1.11 (d, 3H).

### Example 61

### (11S,21R,S)-3-Decladinosyl-11,12-dideoxy-6-O-methyl-3-oxo-12,11-[oxycarbonyl-(4-(3-amino-4-methoxy-phenyl)4-oxo-butyramido)-methylene]-erythromycin A

To a solution of example **51** (0.070g) in anhydrous MeOH (5mL) palladium (10wt. % on carbon powder, 0.050g) was added and the mixture stirred under hydrogen atmosphere (1 atm) for 1hr. Filtration through a silica pad eluting with MeOH and purification by preparative TLC (eluting with: DCM\MeOH 90\10) gave the title compound (0.008g).
¹H-NMR (CDCl₃) δ: 8.47 (d, 1H), 8.19 (d, 1H), 7.15 (d, 1H), 6.68 (d, 1H), 4.82 (d, 1H), 4.04 (s, 3H), 3.32 (t, 2H), 3.07 (m, 1H), 2.70-2.60 (m, 2H), 2.39 (m, 1H), 2.08 (s, 3H), 1.11 (d, 3H)

### Example 62

### (11S,21R,S)-3-Decladinosyl-11,12-dideoxy-6-O-methyl-3-oxo-12,11-[oxycarbonyl-(4-hydroxymino-4-(4-methoxy-3-nitro-phenyl)-butyramido)-methylene]-erythromycin A

To a solution of example **51** (0.050g) in anhydrous MeOH (1mL) hydroxylamine hydrochloride (0.050g) and ammonium acetate (0.100g) were added. The reaction mixture was stirred at 60°C for 3h. After solvent evaporation, the crude material was purified by flash chromatography (eluting with: DCM\MeOH 90\10) to give the title compound (0.0 15g).
1H-NMR (CDCl₃) δ: 8.17 (d, 1H), 7.82 (dd, 1H), 7.13 (d, 1H), 6.58 (bm, 1H), 4.80 (m, 1H), 3.00 (m, 1H), 3.10 (m, 2H), 2.50 (m, 2H), 2.34 (m, 1H), 1.14 (d, 3H).

### Example 63

### (11S,21R,S)-3-Decladinosyl-11,12-dideoxy-6-O-methyl-3-oxo-12,11-[oxycarbonyl-(2-(quinoxaline-2-sulfonyl)-acetamido)-methylene]-erythromycin A

To a solution of example **46** (0.050g) in a 1\1 mixture of water\DCM (4mL) magnesium monoperoxyphtalate (0.040g) was added. After stirring for 24h the aqueous phase was extracted with DCM (3x5mL), the organic extracts washed with a 5% Na₂S₂O₅ aqueous solution (5mL), dried over Na₂SO₄ and concentrated under reduced pressure. The residue was dissolved in THF (1mL), polystyrene triphenylphosphine resin (0.050g) was added and the mixture heated to 65°C for 2h. After cooling to room temperature, the mixture was filtered and concentrated to give the title compound (0.050g).
¹H-NMR (CDCl₃) δ: 9.52 (s, 1H), 8.24 (m, 1H), 8.00-7.80 (m, 2H), 7.70 (bm, 1H), 4.83 (m, 1H), 4.49 (d, 1H), 4.04 (d, 1H), 3.06 (m, 1H), 2.50 (m, 1H), 1.15 (d, 3H).

### Example 64

### (11S,21R,S)-3-Decladinosyl-11,12-dideoxy-6-O-methyl-3-oxo-12,11-[oxycarbonyl-(3-phenyl-propylamino)-methylene]-erythromycin A

A solution of example **6** (0.045g) and 3-phenyl propionaldehyde (0.010mL) in anhydrous MeOH (1mL) was stirred at room temperature for 1h under nitrogen atmosphere. Then sodium cyanoborohydride (0.007g) and acetic acid (0.004mL) were added. The mixture was stirred at room temperature for 1h then the reaction was quenched with a saturated NaHCO₃ aqueous solution (2mL). After solvent evaporation, the aqueous phase was extracted with DCM (3x10mL), the organic layer dried over Na₂SO₄ and concentrated under reduced pressure. The residue was dissolved in MeOH (1mL) and stirred at room temperature overnight. After evaporating the solvent the crude material was purified by flash chromatography (eluting with: DCM \MeOH 95\5) to give the title compound (0.039g).
¹H-NMR (CDCl₃) δ: 7.40-7.00 (m, 5H), 5.74 (dd, 1H), 4.31 (d, 1H), 4.25 (d, 1H), 4.13 (s, 1H), 3.84 (q, 1H), 3.54 (m, 1H), 3.18 (m, 1H), 3.11 (m, 1H), 3.03 (m, 1H), 2.92-2.83 (m, 2H), 2.68 (s, 3H), 2.64 (m, 2H), 2.58 (m, 1H), 2.46 (m, 1H), 2.33 (m, 1H), 2.27 (s, 6H), 1.92 (m, 1H), 1.76 (m, 4H), 1.67 (m, 1H), 1.57 (m, 1H), 1.48 (s, 3H), 1.38 (d, 3H), 1.31 (d, 3H), 1.33 (s; 3H), 1.25 (d, 3H), 1.15 (d, 3H), 1.09 (d, 3H), 0.87 (t, 3H).
TLC: DCM\MeOH 10\1 (Rf=0.25).

### Example 65

### (11S,21R,S)-3-Decladinosyl-11,12-dideoxy-6-O-methyl-3-oxo-12,11-[oxycarbonyl-(3-(4-(pyridin-3-yl)-imidazol-1-yl)-propylamino)-methylene]-erythromycin A

A solution of example **6** (0.100g) and intermediate **52** (0.040g) in anhydrous DCM (4mL) was stirred at room temperature for 6h under nitrogen atmosphere. Then sodium cyanoborohydride (1M in THF, 0.150mL) and acetic acid (0.009mL) were added and the mixture stirred for 48h. The reaction was quenched with a saturated NaHCO₃ aqueous solution (10mL) and extracted with DCM (3x15mL). The organic layer was dried over Na₂SO₄ and concentrated under reduced pressure. The residue was dissolved in MeOH (5mL) and heated to reflux temperature for 48h. After evaporating the solvent the crude material was purified by flash chromatography (eluting with: DCM\MeOH from 100\0 to 90\10) to give the title compound (0.008g).
¹H-NMR (CDCl₃) δ: 9.00 (d, 1H), 8.45 (d, 1H), 8.11 (d, 1H), 7.59 (d, 1H), 7.39 (d, 1H), 7.29 (m, 1H), 5.55 (dd, 1H), 4.29 (d, 1H), 4.24 (d, 1H), 4.13 (s, 1H), 4.20-4.0 (m, 2H), 3.86 (q, 1H), 3.55 (m, 1H), 3.19 (m, 1H), 3.09 (m, 1H), 3.05 (m, 1H), 2.93 (m, 2H), 2.67 (s, 3H), 2.52 (m, 1H), 2.49 (m, 1H), 2.32 (d, 1H), 2.27 (s, 6H), 2.02 (m, 1H), 1.94 (m, 2H), 1.80 (m, 2H), 1.65 (m, 1H), 1.60 (m, 1H), 1.49 (s, 3H), 1.39 (d, 3H), 1.33 (s, 3H), 1.31 (d, 3H), 1.25 (m, 1H), 124 (d, 3H), 1.16 (d, 3H), 1.09 (d, 3H), 0.87 (t, 3H).

### Example 66

### (11S,21S)-3-Decladinosyl-11,12-dideoxy-6-O-methyl-3-oxo-12,11-[oxycarbonyl-(3-(4-(pyridin-3-yl)-imidazol-1-yl)-propylamino)-methylene]-erythromycin A

A solution of example **7** (0.500g) and intermediate **52** (0.180g) in anhydrous acetonitrile (7mL) was stirred at room temperature for 2h under nitrogen atmosphere. The solvent was evaporated and the residue dissolved in anhydrous MeOH (5mL). Then sodium cyanoborohydride (1M in THF, 0.375ml) and acetic acid (0.045ml) were added under nitrogen atmosphere and the mixture stirred overnight. The reaction was quenched with a saturated NaHCO₃ aqueous solution (10mL) and extracted with DCM (3x15mL). The organic layer was dried over Na₂SO₄ and concentrated under reduced pressure. The crude material was purified by flash chromatography (eluting with: DCM \MeOH 90\10) to give the title compound (0.170g, (21*S*) isomer 95% pure by NMR analysis).
¹H-NMR (CDCl₃) δ: 9.00 (d, 1H), 8.45 (d, 1H), 8.11 (d, 1H), 7.59 (d, 1H), 7.39 (d, 1H), 7.29 (m, 1H), 5.55 (dd, 1H), 4.29 (d, 1H), 4.24 (d, 1H), 4.13 (s, 1H), 4.20-4.0 (m, 2H), 3.86 (q, 1H), 3.55 (m, 1H), 3.19 (m, 1H), 3.09 (m, 1H), 3.05 (m, 1H), 2.93 (m, 2H), 2.67 (s, 3H), 2.52 (m, 1H), 2.49 (m, 1H), 2.32 (d, 1H), 2.27 (s, 6H), 2.02 (m, 1H), 1.94 (m, 2H), 1.80 (m, 2H), 1.65 (m, 1H), 1.60 (m, 1H), 1.49 (s, 3H), 1.39 (d, 3H), 1.33 (s, 3H), 1.31 (d, 3H), 1.25 (m, 1H), 1.24 (d, 3H), 1.16 (d, 3H), 1.09 (d, 3H), 0.87 (t, 3H).

### Example 67

### (11S,21R,S)-3-Decladinosyl-11,12-dideoxy-6-O-methyl-3-oxo-12,11-[oxycarbonyl-(4-(4-(pyridin-3-yl)-imidazol-1-yl)-butylamino)-methylene]-erythromycin A

A solution of example **6** (0.100g) and intermediate **54** (0.050g) in anhydrous DCM (3mL) was stirred at room temperature for 24h under nitrogen atmosphere. Then sodium cyanoborohydride (1M in THF, 0.120mL) and acetic acid (0.008mL) were added under nitrogen atmosphere and the mixture stirred for 1h. The reaction was quenched with a saturated NaHCO₃ aqueous solution (5mL) and extracted with DCM (3x10mL). The organic layer was dried over Na₂SO₄ and concentrated under reduced pressure. The crude material was purified by flash chromatography (eluting with: DCM\MeOH from 95\5 to 90\10) to give a compound that was dissolved in MeOH (2mL) and stirred at room temperature overnight. Solvent evaporation under reduced pressure gave the title compound (0.015g).
¹H-NMR (CDCl₃) δ: 8.98 (d, 1H), 8.46 (d, 1H), 8.09 (d, 1H), 7.56 (d, 1H), 7.36 (d, 1H), 7.30 (m, 1H), 5.67 (dd, 1H), 4.30 (d, 1H), 4.24 (d, 1H), 4.13 (s, 1H), 4.00 (m, 2H), 3.85 (q, 1H), 3.56 (m, 1H), 3.19 (m, 1H), 3.09 (m, 1H), 3.03 (m, 1H), 2.91 (m, 2H), 2.64 (s, 3H), 2.58 (m, 1H), 2.47 (m, 1H), 2.28 (m+s, 6H +1H), 2.00 (m, 1H), 1.89 (m, 2H), 1.79 (m, 1H), 1.70 (m, 2H), 1.60-1.50 (m, 2H), 1.48 (s, 3H), 1.39 (d, 3H), 1.31 (s, 3H), 1.31 (d, 3H), 1.25 (d, 3H), 1.25 (m, 1H), 1.15 (d, 3H), 1.09 (d, 3H), 0.83 (t, 3H).

### Example 68

### (11S,21R,S)-3-Decladinosyl-11,12-dideoxy-6-O-methyl-3-oxo-12,11-[oxycarbonyl-(5-(4-(pyridin-3-yl)-imidazol-1-yl)-pentylamino)-methylene]-erythromycin A

A solution of example **6** (0.080g) and intermediate **56** (0.042g) in anhydrous DCM (5mL) was stirred at room temperature for 1h under nitrogen atmosphere. Then sodium cyanoborohydride (1M in THF, 0.120mL) and HCl (2N in Et₂O, 0.010mL) were added under nitrogen atmosphere and the mixture stirred for 1h. The reaction was quenched with a saturated NaHCO₃ aqueous solution (10mL) and extracted with DCM (3x15mL). The organic layer was dried over Na₂SO₄ and concentrated under reduced pressure. The crude material was purified by flash chromatography (eluting with DCM\MeOH from 96\4 to 90\10) to give a compound that was dissolved in MeOH (3mL) and stirred at room temperature overnight. Solvent evaporation under reduced pressure gave the title compound (0.013g).
¹H-NMR (CDCl₃) δ: 8.99 (d, 1H), 8.47 (d, 1H), 8.10 (d, 1H), 7.55 (s, 1H), 7.31 (s, 1H), 7.29 (m, 1H), 5.67 (dd, 1H), 4.30 (d, 1H), 4.25 (d, 1H), 4.11 (s, 1H), 3.96 (m, 2H), 3.85 (q, 1H), 3.58 (m, 1H), 3.18 (dd, 1H), 3.09 (m, 1H), 3.02 (m, 1H), 2.90-2.75 (m, 2H), 2.67 (s, 3H), 2.58 (m, 1H), 2.46 (m, 1H), 2.29 (d, 1H), 2.27 (s, 6H), 1.90-1.86 (m, 3H), 1.78 (m, 1H), 1.70 (m, 2H), 1.60-1.50 (m, 3H), 1.47 (s, 3H), 1.5-1.4 (m, 4H), 1.38 (d, 3H), 1.33 (s, 3H), 1.32 (d, 3H), 1.28 (d, 3H), 1.25 (m, 1H), 1.15 (d, 3H), 1.09 (d, 3H), 0.85 (t, 3H).

### Example 69

### (11S,21R,S)-3-Decladinosyl-11,12-dideoxy-6-O-methyl-3-oxo-12,11-[oxycarbonyl-(2-(4-(pyridin-3-yl)-imidazol-1-yl)-ethylamino)-methylene]-erythromycin A

A solution of intermediate **51** (0.040g) in acetonitrile (1mL) and a 2M HCl aqueous solution (3mL) was heated to 80°C for 3h. The reaction mixture was allowed to reach room temperature, then it was added dropwise to a solution of example **6** (0.100g) dissolved in anhydrous acetonitrile (4mL) keeping the pH of the solution in the range 6-7 by addition of a saturated NaHCO₃ aqueous solution. The solution was stirred at room temperature for 2h. Acetic acid was added to the mixture to reach pH 5-6 followed by sodium cyanoborohydride (1M in THF, 0.150mL). The reaction mixture was stirred overnight at room temperature. After evaporating the solvent a saturated NaHCO₃ aqueous solution (5mL) was added and the product was extracted with DCM (3x10mL). The organic layer was dried over Na₂SO₄ and concentrated under vacuum. The crude material was purified by flash chromatography (eluting with DCM\MeOH 94\6) affording the title compound (0.027g).
¹H-NMR (CDCl₃) δ: 9.04 (d, 1H), 8.46 (dd, 1H), 8.12 (d, 1H), 7.61 (d, 1H), 7.56 (m, 1H), 7.30 (m, 1H), 5.63 (dd, 1H), 4.27 (d, 1H), 4.22 (d, 1H), 4.19 (d, 1H), 4.20-4.08 (m, 2H), 3.84 (q, 1H), 3.54 (m, 1H), 3.36-3.19 (m, 2H), 3.16 (dd, 1H), 3.05 (m, 2H), 2.55 (m, 1H), 2.44 (m, 1H), 2.43 (s, 3H), 2.28 (s, 1H), 2.27 (s, 6H), 1.85 (m, 1H), 1.75 (m, 1H), 1.70-1.65 (m, 2H), 1.55 (m, 1H), 1.48 (s, 3H), 1.40 (d, 3H), 1.30 (d, 3H), 1.24-1.20 (m, 4H), 1.23 (s, 3H), 1.14 (d, 3H), 1.09 (d, 3H), 0.82 (t, 3H).

### Example 70

### (11S,21S)-3-Decladinosyl-11,12-dideoxy-6-O-methyl-3-oxo-12,11-[oxycarbonyl-(2-(4-(pyridin-3-yl)-imidazol-1-yl)-ethylamino)-methylene]-erythromycin A

A solution of intermediate **51** (0.530g) in acetonitrile (30mL) and a 3M HCl aqueous solution (20mL) was heated to 80°C for 3h. The reaction mixture was allowed to reach room temperature, then it was added dropwise to a solution of example **7** (0.500g) dissolved in anhydrous acetonitrile (2mL) keeping the pH of the solution in the range 6-7 by addition of saturated NaHCO₃ aqueous solution. The solution was stirred at room temperature for 1h. Acetic acid was added to the mixture to reach pH 5-6 followed by sodium cyanoborohydride (1M in THF, 0.75mL). The reaction mixture was stirred overnight at room temperature. After evaporating the solvent a saturated NaHCO₃ aqueous solution (15mL) was added and the mixture was extracted with DCM (3x20mL). The organic layer was dried over Na₂SO₄ and concentrated under reduced pressure. The residue was dissolved in MeOH (10mL) and stirred overnight. After evaporating the solvent the crude material was purified by flash chromatography (eluting with DCM\MeOH from 98\2 to 90\10) to give the title compound (0.350g, (21*S*) isomer 95% pure by NMR analysis).
¹H-NMR (CDCl₃) δ: 9.04 (d, 1H), 8.46 (dd, 1H), 8.12 (d, 1H), 7.61 (d, 1H), 7.56 (m, 1H), 7.30 (m, 1H), 5.63 (dd, 1H), 4.27 (d, 1H), 4.22 (d, 1H), 4.19 (d, 1H), 4.20-4.08 (m, 2H), 3.84 (q, 1H), 3.54 (m, 1H), 3.36-3.19 (m, 2H), 3.16 (dd, 1H), 3.05 (m, 2H), 2.55 (m, 1H), 2.44 (m, 1H), 2.43 (s, 3H), 2.28 (s, 1H), 2.27 (s, 6H), 1.85 (m, 1H), 1.75 (m, 1H), 1.70-1.65 (m, 2H), 1.55 (m, 1H), 1.48 (s, 3H), 1.40 (d, 3H), 1.30 (d, 3H), 1.24-1.20 (m, 4H), 1.23 (s, 3H), 1.14 (d, 3H), 1.09 (d, 3H), 0.82 (t, 3H).

### Example 71

### (11S,21R,S)-3-Decladinosyl-11,12-dideoxy-6-O-methyl-3-oxo-12,11-[oxycarbonyl-(5-(quinolin-4-yl)-pentylamino)-methylene]-erythromycin A

A solution of example **6** (0.084g) and intermediate **63** (0.040g) in anhydrous DCM (4mL) was stirred at room temperature for 1h under nitrogen atmosphere. Then sodium cyanoborohydride (1M in THF, 0.125mL) and acetic acid (0.007mL) were added and the mixture stirred overnight. The reaction was quenched with a saturated NaHCO₃ aqueous solution (10mL) and extracted with DCM (3x10mL). The organic layer was dried over Na₂SO₄ and concentrated under reduced pressure. The crude material was purified by flash chromatography (eluting with DCM\MeOH 90\10) to give a compound that was dissolved in MeOH (3mL) and stirred at room temperature overnight. Solvent evaporation under reduced pressure gave the title compound (0.006g).
¹H-NMR (CDCl₃) δ: 8.81 (d, 1H), 8.11 (d, 1H), 8.06 (d, 1H), 7.70 (t, 1H), 7.57 (t, 1H), 7.26 (d, 1H), 5.71 (dd, 1H), 4.31 (d, 1H), 4.26 (d, 1H), 4.12 (d, 1H), 3.85 (q, 1H), 3.55 (m, 1H), 3.19 (m, 1H), 3.10 (m, 1H), 3.07 (m, 2H), 3.03 (m, 1H), 2.90 (m, 1H), 2.80 (m, 1H), 2.68 (s, 3H), 2.58 (m, 1H), 2.45 (m, 1H), 2.30 (d, 1H), 2.27 (s, 6H), 1.90 (m, 1H), 1.75 (m, 2H), 1.70 (m, 3H), 1.52 (m, 2H), 1.48 (s, 3H), 1.37 (d, 3H), 1.33 (s, 3H), 1.32 (d, 3H), 1.24 (m, 1H), 1.25 (d, 3H), 1.15 (d, 3H), 1.09 (d, 3H), 0.85 (t, 3H).

### Example 72

### (11S,21R,S)-3-Decladinosyl-11,12-dideoxy-6-O-methyl-3-oxo-12,11-[oxycarbonyl-(3-(quinolin-4-yl)-propylamino)-methylene]-erythromycin A

A solution of example **6** (0.100g) and 3-quinolin-4-yl-propionaldehyde (0.031g) in anhydrous DCM (4mL) was stirred at room temperature for 2h under nitrogen atmosphere. Then sodium cyanoborohydride (1M in THF, 0.150mL) and acetic acid (0.009mL) were added and the mixture stirred overnight. The reaction was quenched with a saturated NaHCO₃ aqueous solution (10mL) and extracted with DCM (3x10mL). The organic layer was dried over Na₂SO₄ and concentrated under reduced pressure. The crude material was purified by flash chromatography (eluting with DCM\MeOH 95\5) to give a compound that was dissolved in MeOH (3mL) and stirred at room temperature overnight. Solvent evaporation under reduced pressure gave the title compound (0.011g).
¹H-NMR (CDCl₃) δ: 8.80 (d, 1H), 8.13-8.09 (d, 2H), 7.70 (t, 1H), 7.58 (t, 1H), 7.30 (m, 1H), 5.80 (dd, 1H), 4.31 (d, 1H), 4.26 (d, 1H), 4.19 (s, 1H), 3.86 (q, 1H), 3.56 (m, 1H), 3.50 (bm, 1H), 3.26 (m, 1H), 3.19 (m, 1H), 3.12 (m, 1H), 3.06 (m, 3H), 2.90 (m, 1H), 2.69 (s, 3H), 2.58 (m, 1H), 2.46 (m, 1H), 2.36 (d, 1H), 2.27 (s, 6H), 1.92 (m, 3H), 1.80-1.70 (m, 2H), 1.65 (m, 1H), 1.60 (m, 1H), 1.50 (s, 3H), 1.40 (d, 3H), 1.33 (s+d, 3H+3H), 1.24 (m, 4H), 1.16 (d, 3H), 1.11 (d, 3H), 0.87 (t, 3H).

### Example 73

### (11S,21R,S)-3-Decladinosyl-11,12-dideoxy-6-O-methyl-3-oxo-12,11-[oxycarbonyl-(4-(quinolin-4-yl)-butylamino)-methylene]-erythromycin A

A solution of example **6** (0.130g) and intermediate **59** (0.047g) in anhydrous THF (5mL) was stirred at room temperature for 1hr under nitrogen atmosphere. Then sodium cyanoborohydride (1M in THF, 0.195mL) and acetic acid until pH=5 were added and the mixture stirred for 3h. The reaction was quenched with a saturated NaHCO₃ aqueous solution (10mL) and extracted with DCM (3x10mL). The organic layer was dried over Na₂SO₄ and concentrated under reduced pressure. The crude material was purified by flash chromatography (eluting with DCM\MeOH from 98\2 to 96\4) to give a compound that was dissolved in MeOH (5mL) and stirred at room temperature overnight. Solvent evaporation under reduced pressure gave the title compound (0.015g).
m\z ([MH]⁺) = 810.

### Example 74

### (11S,21R,S)-3-Decladinosyl-11,12-dideoxy-6-O-methyl-3-oxo-12,11-[oxycarbonyl-(2-(quinolin-4-yl)-ethylamino)-methylene]-erythromycin A

A solution of intermediate **57** (0.270g) in acetonitrile (4mL) and 3M HCl aqueous solution (4mL) was heated to 50°C for 24h. The reaction mixture was allowed to reach room temperature, then it was added dropwise to a solution of example **6** (0.500g) in anhydrous acetonitrile (4mL) keeping the pH of the solution in the range 6-7 by addition of a saturated NaHCO₃ aqueous solution. The solution was stirred at room temperature for 3h. Acetic acid was added to the mixture to reach pH 5-6 followed by sodium cyanoborohydride (1M in THF, 0.75mL). The reaction mixture was stirred overnight at room temperature. After evaporating the solvent a saturated NaHCO₃ aqueous solution (15mL) was added and the mixture was extracted with DCM (3x20mL). The organic layer was dried over Na₂SO₄ and concentrated under vacuum. The residue was dissolved in MeOH (10mL) and stirred overnight. After solvent evaporation the crude material was purified by flash chromatography (eluting with DCM\MeOH from 98\2 to 90\10) to give the title compound (0.190g).
¹H-NMR (CDCl₃) δ: 8.79 (d, 1H), 8.09 (m, 1H), 7.69 (t, 1H), 7.56 (t, 1H), 7.38 (d, 1H), 5.68 (dd, 1H), 4.29 (d, 1H), 4.22 (d, 1H), 4.21 (s, 1H), 3.83 (q, 1H), 3.54 (m, 1H), 3.41-3.1 (m, 4H), 3.17 (dd, 1H), 3.07 (m, 1H), 3.02 (m, 1H), 2.45 (m, 2H + -OCH₃), 2.30 (m, 1H), 2.27 (s, N(CH₃)₂), 1.88 (m, 1H), 1.75 (m, 2H), 1.66 (m, 1H), 1.55 (m, 1H), 1.47 (s, 3H), 1.37 (d, 3H), 1.30 (d, 3H), 1.25 (d, 3H), 1.25 (m, 4H), 1.12 (d, 3H), 1.08 (d, 3H), 0.85 (t, 3H).

### Example 75

### (11S,21S)-3-Decladinosyl-11,12-dideoxy-6-O-methyl-3-oxo-12,11-[oxycarbonyl-(2-(quinolin-4-yl)-ethylamino)-methylene]-erythromycin A

A solution of intermediate **57** (0.832g) in acetonitrile (16mL) and a 3M HCl aqueous solution (16mL) was heated to 50°C for 16h. The reaction mixture was allowed to reach room temperature, then it was added dropwise to a solution of example **7** (1.500g) in anhydrous acetonitrile (12mL) keeping the pH of the solution in the range 6-7 by addition of a saturated NaHCO₃ aqueous solution. The solution was stirred at room temperature for 1h. Acetic acid was added to the mixture to reach pH 5-6 followed by sodium cyanoborohydride (1M in THF, 3.3mL). The reaction mixture was stirred overnight at room temperature. After evaporating the solvent water (100mL) was added and the mixture extracted with EtOAc (2x100mL). The organic layer was dried over Na₂SO₄ and concentrated under vacuum. The crude material was purified by flash chromatography (DCM\MeOH 95\5). The obtained compound was dissolved in MeOH (20mL) and stirred overnight. After solvent evaporation the compound was purified by flash chromatography (eluting with DCM\MeOH 95\5) to give the title compound (0.365g, (21*S*) isomer 95% pure by NMR analysis).
¹H-NMR (CDCl₃) δ: 8.79 (d, 1H), 8.09 (m, 1H), 7.69 (t, 1H), 7.56 (t, 1H), 7.38 (d, 1H), 5.68 (dd, 1H), 4.29 (d, 1H), 4.22 (d, 1H), 4.21 (s, 1H), 3.83 (q, 1H), 3.54 (m, 1H), 3.41-3.1 (m, 4H), 3.17 (dd, 1H), 3.07 (m, 1H), 3.02 (m, 1H), 2.45 (m, 2H + 3H), 2.30 (m, 1H), 2.27 (s, 6H), 1.88 (m, 1H), 1.75 (m, 2H), 1.66 (m, 1H), 1.55 (m, 1H), 1.47 (s, 3H), 1.37 (d, 3H), 1.30 (d, 3H), 1.25 (d, 3H), 1.25 (m, 4H), 1.12 (d, 3H), 1.08 (d, 3H), 0.85 (t, 3H).

### Example 76

### (11S,21S)-3-Decladinosyl-11,12-dideoxy-6-O-methyl-3-oxo-12,11-[oxycarbonyl-((quinolin-4-yl)-methylamino)-methylene]-erythromycin A

A solution of example **7** (0.050g) and quinoline-4-carbaldehyde (0.020g) in anhydrous toluene (3mL) was stirred at 100°C for 16h under nitrogen atmosphere. After evaporation of the solvent the crude material was dissolved in anhydrous MeOH (5mL) palladium (10wt. % on carbon powder, 0.006g) was added and the mixture stirred under hydrogen atmosphere (1 atm) for 5h. Filtration through a celite pad eluting with MeOH and purification by flash chromatography (eluting with: DCM\MeOH 93\7) gave the title compound (0.007g).
m\z ([MH]⁺) = 768
¹H-NMR (CDCl₃) δ: 8.89 (d, 1H), 8.11 (d, 1H), 8.09 (d, 1H), 7.69 (t, 1H), 7.60 (t, 1H), 7.56 (t, 1H), 4.65 (m, 1H), 4.48 (m, 1H), 4.38 (m, 1H), 3.09 (m, 1H), 2.43 (m, 1H), 1.14 (d, 3H).

### Example 77

### (11S,21R,S)-3-Decladinosyl-11,12-dideoxy-6-O-methyl-3-oxo-12,11-[oxycarbonyl-(3-(5-methyl-furan-2-yl)-butylamino)-methylene]-erythromycin A

A solution of 3-(S-methyl-2-furyl)butanal (0.030g) and example **6** (0.067g) in anhydrous acetonitrile (1mL) was stirred at room temperature for 5h under nitrogen atmosphere. Then sodium cyanoborohydride (1M in THF, 0.250mL) and acetic acid (0.025mL) were added and the mixture was stirred at room temperature for 24h. MeOH (1mL) was added and the reaction mixture heated to 60°C for 24h. After evaporation under reduced pressure the residue was dissolved in DCM (10mL) and washed with a saturated NaHCO₃ aqueous solution (2x3mL). The organic phase was dried over Na₂SO₄ and concentrated under vacuum. The crude material was purified by flash chromatography (eluting with: DCM\MeOH\NH₄OH 95\5\0.5) to give the title compound (0.029g).
¹H-NMR (CDCl₃) δ: 5.82 (m, 2H), 4.10 (m, 1H), 3.02 (m, 1H), 2.86 (s, 2H), 2.77 (m, 1H), 2.40-2.20 (m, 3H+1H), 1.80-1.60 (m, 2H), 1.08 (d, 3H).

### Example 78

### (11S,2R,S)-3-Decladinosyl-11,12-dideoxy-6-O-methyl-3-oxo-12,11-[oxycarbonyl-(3-pyridin-4-yl-prop-2-enylamino)-methylene]-erythromycin A

A solution of 3-(5-methyl-2-furyl)butanal oxalate (0.045g), example **6** (0.067g) and DIPEA (0.051mL) in anhydrous acetonitrile (1mL) was stirred at room temperature for 1.5h under nitrogen atmosphere. Then sodium cyanoborohydride (1M in THF, 0.250mL) and acetic acid (0.025mL) were added and the mixture was stirred at room temperature for 15h. MeOH (1mL) was added and the reaction mixture heated to 60°C for 48h. After evaporation under reduced pressure the residue was dissolved in DCM (10mL) and washed with a saturated NaHCO₃ aqueous solution (2x3mL). The organic phase was dried over Na₂SO₄ and concentrated under reduced pressure. The crude material was purified by flash chromatography (eluting with: DCM, DCM\MeOH\NH₄OH from 96\4\0.1 to 92\8\0.2) to give the title compound (0.016g).
m\z ([MH]⁺) = 744.
¹H-NMR (CDCl₃) δ: 8.52 (d, 2H), 7.28 (d, 2H), 6.59 (dd, 2H), 4.24 (m, 1H), 3.65 (m, 2H), 3.05 (m, 1H), 2.38 (m, 1H), 1.10 (d, 3H).

### Example 79

### (11S,21R,S)-3-Decladinosyl-11,12-dideoxy-6-O-methyl-3-oxo-12,11-[oxycarbonyl-(3-(4-(3,5-difluoro-phenyl)-pyrazol-1-yl)-propylamino)-methylene]-erythromycin A

To a solution of example **7** (0.030g) in anhydrous THF (1mL) intermediate **75** (0.050g) was added and the mixture was stirred at room temperature for 3h under nitrogen atmosphere. Then sodium cyanoborohydride (1M in THF, 0.150mL) and acetic acid (0.010mL) were added and the mixture was allowed to react for 4h.The solvent was evaporated under reduced pressure, the residue dissolved in DCM (5mL) and washed with a saturated NaHCO₃ aqueous solution (2x3mL). The organic phase was dried over Na₂SO₄ and concentrated under reduced pressure. The residue was dissolved in MeOH (1mL) and the mixture stirred overnight. After evaporation of the solvent the crude material was purified by flash chromatography (eluting with: DCM\MeOH 90\10) to give the title compound ((0.014g).
¹H-NMR (CDCl₃) δ: 7.83 (s, 1H), 7.73 (s, 1H), 7.03 (dd, 1H), 6.62 (tt, 1H), 4.28 (m, 2H), 4.12 (s, 1H), 3.04 (m, 1H), 2.91 (m, 1H), 2.82 (m, 1H), 2.32 (bs, 2H), 2.07 (m, 1H), 2.00 (m, 1H), 1.09 (d, 3H).

### Example 80

### (11S,21R,S)-3-Decladinosyl-11,12-dideoxy-6-O-methyl-3-oxo-12,11-[oxycarbonyl-(3-(4-(4-chloro-phenyl)-2,5-dimethyl-imidazol-1-yl)-propylamino)-methylene]-erythromycin A

A solution of example **7** (0.050g) and intermediate **76** (0.025g) in anhydrous acetonitrile (1.5mL) was stirred at room temperature for 5h under nitrogen atmosphere. The solvent was evaporated under reduced pressure, the residue dissolved in anhydrous MeOH (1.5mL) then sodium cyanoborohydride (1M in THF, 0.037mL) and acetic acid (0.005mL) were added. After 18h the solvent was evaporated under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 95\5) to give the title compound (0.028g).
¹H-NMR (CDCl₃) δ: 7.55 (d, 2H), 7.33 (d, 2H), 4.15 (m, 1H), 4.00 (m, 1H), 3.85 (m, 1H), 3.05 (m, 1H), 3.02-2.90 (m, 2H), 2.42 (s, 3H), 2.35 (s, 3H), 2.33 (m, 1H), 1.90 (m, 1H), 1.72 (m, 1H), 1.09 (d, 3H).

### Example 81

### (11S,21R,S)-3-Decladinosyl-11,12-dideoxy-6-O-methyl-3-oxo-12,11-[oxycarbonyl-(3-(4-(4-nitro-phenyl)-imidazol-1-yl)-propylamino)-methylene]-erythromycin A

A solution of example **7** (0.050g) and intermediate **77** (0.023g) in anhydrous acetonitrile (1.5mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.005mL) were added. After 24h the solvent was evaporated under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 95\5) to give the title compound (0.007g).
¹H-NMR (CDCl₃) δ: 8.24 (d, 2H), 7.96 (d, 2H), 7.61 (d, 1H), 7.54 (d, 1H), 4.14 (m, 1H), 4.20÷4.06 (m, 2H), 3.00÷2.88 (m, 2H), 2.32 (m, 1H), 2.03 (m, 1H), 1.92 (m, 1H).

### Example 82

### (11S,21R,S)-3-Decladinosyl-11,12-dideoxy-6-O-methyl-3-oxo-12,11-[oxycarbonyl-(3-(4-pyridin-4-yl-imidazol-1-yl)-propylamino)-methylene]-erythromycin A

A solution of example **7** (0.050g) and intermediate **78** (0.025g) in anhydrous acetonitrile (2mL) was stirred at room temperature for 16h under nitrogen atmosphere then heated to 50°C for 4h. After evaporating the solvent under reduced pressure, the crude was dissolved in anhydrous MeOH (2mL) then sodium cyanoborohydride (1M in THF, 0.100mL) and acetic acid (0.006mL) were added. After 24h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 90\10) to give the title compound (0.014g).
¹H-NMR (CDCl₃) δ: 8.57 (d, 1H), 7.70 (d, 1H), 7.62 (s, 1H), 7.52 (s, 1H), 4.20-4.16 (m, 2H), 3.05 (m, 1H), 2.96-2.88 (m, 2H), 2.32 (m, 1H), 2.02 -1.92 (m+m, 2H), 1.10 (d, 3H).

### Example 83

### (11S,21R,S)-3-Decladinosyl-11,12-dideoxy-6-O-methyl-3-oxo-12,11-[oxycarbonyl-(3-(3-trifluoromethyl-1H-pyrazol-4-yl)-propylamine)-methylene]-erythromycin A

A solution of example **7** (0.020g) and intermediate **79** (0.015g) in anhydrous acetonitrile (2mL) was stirred at room temperature for 3h under nitrogen atmosphere then heated to 40°C for 4h. After evaporating the solvent under reduced pressure, the crude material was dissolved in anhydrous MeOH (2mL) then sodium cyanoborohydride (1M in THF, 0.015mL) and acetic acid (0.003mL) were added. After 24h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 98\2) to give the title compound (0.004g).
¹H-NMR (CDCl₃) δ: 7.53 (s, 1H), 4.12 (d, 1H), 3.03 (m, 1H), 2.92 (m, 1H), 2.84 (m, 1H), 2.70-2.60 (m, 2H), 2.11 (m, 1H), 1.70 (m, 2H), 1.09 (d, 3H).

### Example 84

### (11S,21R,S)-3-Decladinosyl-11,12-dideoxy-6-O-methyl-3-oxo-12,11-[oxycarbonyl-(3-(5-methyl-4-(4-trifluoromethyl-phenyl)-imidazol-1-yl)-propylamino)-methylene]-erythromycin A

A solution of example **7** (0.050g) and intermediate **80** (0.038g) in anhydrous acetonitrile (1mL) was stirred at room temperature overnight under nitrogen atmosphere. The solvent was evaporated under reduced pressure, the crude dissolved in anhydrous MeOH (1mL) then sodium cyanoborohydride (1M in THF, 0.037mL) and acetic acid (0.004mL) were added. After 24h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH\NH₄OH from 98\2\0 to 95\5\0.5) and by preparative TLC (DCM\MeOH\NH₄OH 90\9\0.5) to give the title compound (0.017g).
¹H-NMR (CDCl₃) δ: 7,78 (d, 2H), 7.64 (d, 2H), 7.59 (s, 1H), 5.60 (dd, 1H), 4.30 (d, 1H), 4.25 (d, 1H), 4.15 (s, 1H), 4.10 (m, 1H), 3.96 (m, 1H), 3.86 (q, 1H), 3.56 (m, 1H), 3.19 (dd, 1H), 3.09 (m, 1H), 3.04 (m, 1H), 2.96 (m, 2H), 2.68 (s, 3H), 2.58 (m, 1H), 2.45 (m, 1H), 2.42 (s, 3H), 2.33 (s, 1H), 2.27 (s, 6H), 2.00÷1.88 (2H), 1.90 (m, 1H), 1.81 (dd, 1H), 1.73 (bd, 1H), 1.67 (m, 1H), 1.60 (m, 1H), 1.49 (s, 3H), 1.38 (d, 3H), 1.34 (s, 3H), 1.32 (d, 3H), 1.25 (m, 1H), 1.25 (d, 3H), 1.16 (d, 3H), 1.10 (d, 3H), 0.88 (t, 3H).

### Example 85

### (11S,21R,S)-3-Decladinosyl-11,12-dideoxy-6-O-methyl-3-oxo-12,11-[oxycarbonyl-(3-(pyridin-3-yl)-propylamino)-methylene]-erythromycin A

A solution of example **7** (0.050g) and intermediate **81** (0.020g) in anhydrous acetonitrile (0.7mL) was stirred at room temperature for 3h under nitrogen atmosphere. The solvent was evaporated under reduced pressure, the crude dissolved in anhydrous MeOH (1mL) then sodium cyanoborohydride (1M in THF, 0.035mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 95\5) to give the title compound (0.009g).
m\z ([MH]⁺) = 746.
¹H-NMR (CDCl₃) δ: 8.47-8.40 (m, 2H), 7.51 (m, 1H), 7.19 (m, 1H), 4.14 (m, 1H), 3.03 (m, 1H), 2.98-2.85 (m, 2H), 2.75-2.60 (m, 2H), 2.31 (bm, 1H), 1.82-1.76 (m, 2H), 1.09 (d, 3H).

### Example 86

### (11S,21R,S)-3-Decladinosyl-11,12-dideoxy-6-O-methyl-3-oxo-12,11-[oxycarbonyl-(3-(4-pyridin-2-yl-pyrazol-1-yl)-propylamino)-methylene]-erythromycin A

A solution of example **7** (0.050g) and intermediate **82** (0.025g) in anhydrous acetonitrile (3mL) was stirred at 50°C for 3h and at room temperature overnight under nitrogen atmosphere. The solvent was evaporated under reduced pressure, the crude dissolved in anhydrous MeOH (2.2mL) then sodium cyanoborohydride (1M in THF, 0.040mL) and acetic acid (0.020mL) were added. After 10h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 95\5) to give the title compound (0.026g).
¹H-NMR (CDCl₃) δ: 8.54 (d, 1H), 8.01 (s, 1H), 7.98 (s, 1H), 7.65 (t, 1H), 7.50 (t, 1H), 7.07 (m, 1H), 4.27 (d, 1H), 4.14 (m, 2H), 3.04 (m, 1H), 2.89 (m, 2H), 2.34 (m, 1H), 2.12 (m, 1H), 2.03 (m, 1H), 1.09 (d, 3H).

### Example 87

### (11S,21R,S)-3-Decladinosyl-11,12-dideoxy-6-O-methyl-3-oxo-12,11-[oxycarbonyl-(3-(pyridin-4-yl)-propylamino)-methylene]-erythromycin A

A solution of example **7** (0.050g) and intermediate **83** (0.050g) in anhydrous acetonitrile (1mL) was stirred at room temperature overnight under nitrogen atmosphere. The solvent was evaporated under reduced pressure, the crude dissolved in anhydrous MeOH (1mL) then sodium cyanoborohydride (1M in THF, 0.037mL) and acetic acid (0.004mL) were added. After 24h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 90\10) and by preparative TLC (eluting with: DCM\MeOH\NH₄OH 90\9\0.5) to give the title compound (0.014g).
¹H-NMR (CDCl₃) δ: 8.47 (d, 2H), 7.13 (d, 2H), 5.70 (dd, 1H), 4.31 (d, 1H), 4.25 (d, 1H), 4.14 (s, 1H), 3.85 (q, 1H), 3.56 (m, 1H), 3.18 (dd, 1H), 3.10 (m, 1H), 3.03 (q, 1H), 2.95÷2.83 (m, 2H), 2.65÷2.55 (m, 2H), 2.67 (s, 3H), 2.59 (m, 1H), 2.46 (m, 1H), 2.31(s, 1H), 2.27 (s, 6H), 1.91 (m, 1H), 1.82÷1.76 (m, 3H), 1.73 (m, 1H), 1.68 (m, 1H), 1.58 (m, 1H), 1.48 (s, 3H), 1.39 (d, 3H), 1.34 (s, 3H), 1.32 (d, 3H), 1.25 (m, 1H), 1.25 (d, 3H), 1.15 (d, 3H), 1.09 (d, 3H), 0.87 (t, 3H).

### Example 88

### (11S,21R,S)-3-Decladinosyl-11,12-dideoxy-6-O-methyl-3-oxo-12,11-[oxycarbonyl-(3-(4-pyrimidin-4-yl-pyrazol-1-yl)-propylamino)-methylene]-erythromycin A

A solution of example **7** (0.028g) and intermediate **84** (0.018g) in anhydrous acetonitrile (1.5mL) was stirred at room temperature for 30min then heated to 50°C for 6h under nitrogen atmosphere. The solvent was evaporated under reduced pressure, the crude dissolved in anhydrous MeOH (2mL) then sodium cyanoborohydride (1M in THF, 0.025mL) and acetic acid (0.004mL) were added. After 24h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.005g).
¹H-NMR (CDCl₃) δ: 9.09 (d, 1H), 8.63 (d, 1H), 8.18 (s, 1H), 8.07 (s, 1H), 7.47 (d, 1H), 4.30 (m, 2H), 4.13 (s, 1H), 3.04 (m, 1H), 2.92-2.80 (m, 2H), 2.33 (m, 1H), 2.11 (m, 1H), 2.00 (m, 1H), 1.09 (d, 3H).

### Example 89

### (11S,21R,S)-3-Decladinosyl-11,12-dideoxy-6-O-methyl-3-oxo-12,11-[oxycarbonyl-(3-(4-pyridin-4-yl-pyrazol-1-yl)-propylamino)-methylene]-erythromycin A

A solution of example **7** (0.050g) and intermediate **85** (0.018g) in anhydrous acetonitrile (2.2mL) was stirred at 50°C for 16h under nitrogen atmosphere. The solvent was evaporated under reduced pressure, the crude dissolved in anhydrous MeOH (3mL) then sodium cyanoborohydride (1M in THF, 0.040mL) and acetic acid (0.020mL) were added. After 18h the solvent was removed under reduced pressure, the residue dissolved in DCM (5mL) and washed with a NaHCO₃ saturated aqueous solution (2x3mL). The organic phase was dried over Na₂SO₄ and concentrated under reduced pressure. The crude material was purified by flash chromatography (eluting with: DCM\MeOH 95\5) to give the title compound (0.014g).
¹H-NMR (CDCl₃) δ: 8.55 (d, 1H), 7.96 (s, 1H), 7.85 (s, 1H), 7.42 (d, 1H), 4.13 (d, 1H), 3.04 (m, 1H), 2.89 (m, 1H), 2.84 (m, 1H), 2.33 (m, 1H), 2.10 (m, 1H), 1.95 (m, 1H), 1.10 (d, 3H).

### Example 90

### (11S,21S)-3-Decladinosyl-11,12-dideoxy-6-O-methyl-3-oxo-12,11-[oxycarbonyl-(3-(4-(3,5-dichloro-phenyl)-2,5-dimethyl-imidazol-1-yl)-propylamino)-methylene]-erythromycin A

A solution of example **7** (0.030g) and intermediate **86** (0.029g) in anhydrous acetonitrile (1.5mL) was stirred at room temperature for 5h under nitrogen atmosphere. The solvent was evaporated under reduced pressure, the crude dissolved in anhydrous MeOH (1.5mL) then sodium cyanoborohydride (1M in THF, 0.022mL) and acetic acid (0.003mL) were added. After 12h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.022g).
¹H-NMR (CDCl₃) δ: 7.47 (d, 2H), 7.15 (t, 1H), 4.11 (m, 1H), 3.98 (m, 1H), 3.82 (m, 1H), 2.93 (m, 2H), 2.38 (s, 3H), 2.33 (s, 3H), 2.29 (m, 1H), 1.6÷1.8 (m, 2H).

### Example 91

### (11S,21R,S)-3-Decladinosyl-11,12-dideoxy-6-O-methyl-3-oxo-12,11-[oxycarbonyl-(3-(2,5-dimethyl-4-(3-trifluoromethyl-phenyl)-imidazol-1-yl)-propylamino)-methylene]-erythromycin A

A solution of example **7** (0.040g) and intermediate **87** (0.042g) in anhydrous acetonitrile (1.5mL) was stirred at room temperature for 4h under nitrogen atmosphere. The solvent was evaporated under reduced pressure, the crude dissolved in anhydrous MeOH (1.5mL) then sodium cyanoborohydride (1M in THF, 0.030mL) and acetic acid (0.004mL) were added. After 12h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.026g).
¹H-NMR (CDCl₃) δ: 7.84 (bs, 1H), 7.74 (m, 1H), 7.43 (m, 2H), 4.12 (s, 1H), 3.99 (m, 1H), 3.82 (m, 1H), 2.9÷2.88 (m, 2H), 2.40 (s, 3H), 2.33 (s, 3H), 2.30 (s, 1H), 1.85÷1.75 (m, 2H).

### Example 92

### (11S,21R,S)-3-Decladinosyl-11,12-dideoxy-6-O-methyl-3-oxo-12,11-[oxycarbonyl-(3-[4-(1,3-benzoxazol-2-yl)-1H-pyrazol-1-yl]-propylamino)-methylene]-erythromycin A

A solution of example **7** (0.037g) and intermediate **88** (0.022g) in anhydrous acetonitrile (1.5mL) was stirred at 50°C for 6h under nitrogen atmosphere. The solvent was evaporated under reduced pressure, the crude dissolved in anhydrous MeOH (2mL) then sodium cyanoborohydride (1M in THF, 0.030mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.007g).
¹H-NMR (CDCl₃) δ: 8.17 (s, 1H), 8.14 (s, 1H), 7.68 (dd, 1H), 7.51 (dd, 1H), 7.29 (m, 2H), 4.34-4.29 (m, 2H), 4.14 (s, 1H), 3.03 (m, 1H), 2.90 (m, 2H), 2.33 (bm, 1H), 2.15 (m, 1H), 2.00 (m, 1H), 1.09 (d, 3H)

### Example 93

### (11S,21R,S)-3-Decladinosyl-11,12-dideoxy-6-O-methyl-3-oxo-12,11-[oxycarbonyl-(3-(5-Pyridin-4-yl-pyrazol-1-yl)-propylamino)-methylene]-erythromycin A

A solution of example **7** (0.050g) and intermediate **89** (0.036g) in anhydrous acetonitrile (1.5mL) was stirred at room temperature for 20h under nitrogen atmosphere. The solvent was evaporated under reduced pressure, the crude dissolved in anhydrous MeOH (2mL) then sodium cyanoborohydride (1M in THF, 0.075mL) and acetic acid (0.010mL) were added. After 20h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.011g).
¹H-NMR (CDCl₃) δ: 8.60 (d, 1H), 7.69 (d, 1H), 7.52 (d, 1H), 6.62 (d, 1H), 4.29 (m, 2H), 4.14 (m, 1H), 3.04 (m, 1H), 2.88 (m, 2H), 2.33 (m, 1H), 2.20 (m, 1H), 1.98 (m, 1H), 1.10 (d, 3H).

### Example 94

### (11S,21R,S)-3-Decladinosyl-11,12-dideoxy-6-O-methyl-3-oxo-12,11-[oxycarbonyl-(3-(2-pyridin-4-yl-1H-imidazol-1-yl)-propylamino)-methylene]-erythromycin A

A solution of example **7** (0.050g) and intermediate **90** (0.025g) in anhydrous acetonitrile (2mL) was stirred at 50°C for 6h then at room temperature overnight under nitrogen atmosphere. The solvent was evaporated under reduced pressure, the crude dissolved in anhydrous MeOH (2mL) then sodium cyanoborohydride (1M in THF, 0.150mL) and acetic acid (0.006mL) were added. After 72h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH 90\10) to give the title compound (0.014g).
¹H-NMR (CDCl₃) δ: 8. 27 (d, 1H), 7.59 (d, 1H), 7.19 (m, 1H), 7.16 (m, 1H), 4.30-4.10 (m, 3H), 3.15 (m, 2H), 2.96 (m, 1H), 2.31 (m, 1H), 2.00-1.82 (m, 2H), 1.09 (d, 3H).

### Example 95

### (11S,21R,S)-3-Decladinosyl-11,12-dideoxy-6-O-methyl-3-oxo-12,11-[oxycarbonyl-(3-(4-quinolin-2-yl-1H-pyrazol-1-yl)-propylamino)-methylene]-erythromycin A

A solution of example **7** (0.050g) and intermediate **91** (0.032g) in anhydrous acetonitrile (1mL) was stirred at room temperature for 18h under nitrogen atmosphere. The solvent was evaporated under reduced pressure, the crude dissolved in anhydrous MeOH (1mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 98\2 to 94\6) and by preparative TLC (DCM\MeOH\NH₄OH 90\9\0.5) to give the title compound (0.018g).
¹H-NMR (CDCl₃) δ: 8.20 (s, 1H), 8.14 (s, 1H), 8.12 (d, 1H), 8.04 (d, 1H), 7.76 (d, 1H), 7.67 (t, 1H), 7.66 (d, 1H), 7.45 (t, 1H), 5.68 (dd, 1H), 4. 31 (m, 3H), 4.26 (d, 1H), 4.15 (s, 1H), 3.86 (q, 1H), 3.56 (m, 1H), 3.18 (dd, 1H), 3.11 (m, 1H), 3.05 (m, 1H), 2.95+2.80 (m, 2H), 2.70 (s, 3H), 2. 58 (m, 1H), 2.46 (m, 1H), 2.35 (s, 1H), 2.27 (s, 6H), 2.14 (m, 1H), 2.06 (m, 1H), 1.96 (m, 1H), 1.80 (m, 1H), 1.74 (m, 1H), 1.70 (m, 1H), 1.65 (m, 1H), 1.50 (s, 3H), 1.40 (d, 3H), 1.34 (s, 3H), 1.32 (d, 3H), 1.25 (m, 1H), 1.24 (d, 3H), 1.16 (d, 3H), 1.10 (d, 3H), 0.90 (t, 3H).

### Example 96

### (11S,21R,S)-3-Decladinosyl-11,12-dideoxy-6-O-methyl-3-oxo-12,11-[oxycarbonyl-(3-(4-quinolin-4-yl-1H-pyrazol-1-yl)-propylamino)-methylene]-erythromycin A

A solution of example **7** (0.050g) and intermediate **92** (0.034g) in anhydrous acetonitrile (1mL) was stirred at room temperature for 18h under nitrogen atmosphere. The solvent was evaporated under reduced pressure, the crude dissolved in anhydrous MeOH (1mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 98\2 to 94\6) to give the title compound (0.020g).
¹H-NMR (CDCl₃) δ: 8.89 (d, 1H), 8.25 (d, 1H), 8.14 (d, 1H), 7.87 (s, 1H), 7.84 (s, 1H), 7.73 (m, 1H), 7.59 (m, 1H), 7.42 (d, 1H), 5.63 (dd, 1H), 4.37 (m, 2H), 4.30 (d, 1H), 4.24 (d, 1H), 4.16 (s, 1H), 3.85 (q, 1H), 3.55 (m, 1H), 3.18 (dd, 1H), 3.10 (m, 1H), 3.04 (m, 1H), 2.94 (m, 2H), 2.70 (s, 3H), 2.58 (m, 1H), 2.45 (m, 1H), 2.34 (s, 1H), 2.27 (s, 6H), 2.19 (m, 1H), 2.05 (m, 1H), 1.88 (m, 1H), 1.80 (dd, 1H), 1.73 (m, 1H), 1.67 (m, 1H), 1.56 (m, 1H), 1.49 (s, 3H), 1.38 (d, 3H), 1.33 (s, 3H), 1.32 (d, 3H), 1.25 (m, 1H), 1.24 (d, 3H), 1.15(d, 3H), 1.10 (d, 3H), 0.83 (t, 3H).

### Example 97

### (11S,21R,S)-3-Decladinosyl-11,12-dideoxy-6-O-methyl-3-oxo-12,11-[oxycarbonyl-(3-(4-quinoxalin-2-yl-1H-pyrazol-1-yl)-propylamino)-methylene]-erythromycin A

A solution of example **7** (0.050g) and intermediate **93** (0.030g) in anhydrous acetonitrile (1mL) was stirred at room temperature for 18h under nitrogen atmosphere. The solvent was evaporated under reduced pressure, the crude dissolved in anhydrous MeOH (1mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 98\2 to 94\6) and by preparative TLC (DCM\MeOH\NH₄OH 90\9\0.5) to give the title compound (0.006g).
¹H-NMR (CDCl₃) δ: 9.10 (s, 1H), 8.28 (s, 1H), 8.22 (s, 1H), 8.03 (m, 2H), 7.73 (t, 1H), 7.66 (t, 1H), 5.63 (dd, 1H), 4.35 (m, 2H), 4.30 (d, 1H), 4.26 (d, 1H), 4.15 (m, 1H), 3.87 (q, 1H), 3.55 (m, 1H), 3.17 (m, 1H), 3.11 (m, 1H), 3.05 (m, 1H), 3.00÷2.80 (m, 2H), 2.71 (s, 3H), 2.60 (m, 1H), 2.46 (m, 1H), 2.35 (m, 1H), 2.27 (s, 6H), 2.15 (m, 1H), 2.06 (m, 1H), 1.96 (m, 1H), 1.80 (m, 1H), 1.74 (m, 1H), 1.68 (m, 1H), 1.60 (m, 1H), 1.50 (s, 3H), 1.40 (d, 3H), 1.34 (s, 3H), 1.32 (d, 3H), 130 (m, 1H), 1.24 (d, 3H), 1.16 (d, 3H), 1.10 (d, 3H), 0.90 (t, 3H).

### Example 98

### (11S,21R,S)-3-Decladinosyl-11,12-dideoxy-6-O-methyl-3-oxo-12,11-[oxycarbonyl-(3-thien-3-ylpropylamino)-methylene]-erythromycin A

A solution of example **7** (0.042g) and intermediate **94** (0.025g) in anhydrous acetonitrile (2mL) was stirred at 50°C for 6h under nitrogen atmosphere. The solvent was evaporated under reduced pressure, the crude dissolved in anhydrous MeOH (2mL) then sodium cyanoborohydride (1M in THF, 0.035mL) and acetic acid (0.004mL) were added. After 18h at room temperature the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 98\2) to give the title compound (0.012g).
¹H-NMR (CDCl₃) δ: 7.21 (m, 1H), 6.94 (m, 2H), 4.13 (m, 1H), 3.03 (m, 1H), 2.91 (m, 1H), 2.83 (m, 1H), 2.80-2.60 (m, 2H), 2.31 (m, 1H), 1.80 (m, 2H), 1.09 (d, 3H).

### Example 99

### (11S,21R,S)-3-Decladinosyl-11,12-dideoxy-6-O-methyl-3-oxo-12,11-[oxycarbonyl-(3-[5-(3-methyl-pyrazin-2-yl)-pyrazol-1-yl]-propylamino)-methylene]-erythromycin A

A solution of example **7** (0.050g) and intermediate **95** (0.037g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.075mL) and acetic acid (0.009mL) were added. After 20h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) to give the title compound (0.018g).
¹H-NMR (CDCl₃) δ: 8.44 (d, 1H), 8.36 (d, 1H), 7.54 (d, 1H), 6.82 (d, 1H), 4.40-4.20 (m, 2H), 4.14 (m, 1H), 3.04 (m, 1H), 2.90 (s, 3H), 2.89 (m, 2H), 2.33 (m, 1H), 2.15 (m, 1H), 2.05 (m, 1H), 1.10 (d, 3H).

### Example 100

### (11S,21R,S)-3-Decladinosyl-11,12-dideoxy-6-O-methyl-3-oxo-12,11-[oxycarbonyl-(3-[2-(methylthio)-1H-benzimidazol-1-yl]-propylamino)-methylene]-erythromycin A

A solution of example **7** (0.050g) and intermediate **96** (0.037g) in anhydrous acetonitrile (1mL) was stirred at room temperature for 3h under nitrogen atmosphere. The solvent was evaporated under reduced pressure, the crude dissolved in anhydrous MeOH (2mL) then sodium cyanoborohydride (1M in THF, 0.075mL) and acetic acid (0.009mL) were added. After 20h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 98\2) to give the title compound (0.020g).
¹H-NMR (CDCl₃) δ: 7.66 (m, 1H), 7.40 (m, 1H), 7.35 (m, 1H), 7.20 (m, 1H), 4.15 (m, 1H), 4.25 (m, 1H), 4.13 (m, 1H), 3.05 (m, 1H), 2.95 (m, 2H), 2.79 (m, 2H), 2.36 (m, 1H), 2.00 (m, 1H), 1.86 (m, 1H), 1.10 (d, 3H).

### Example 101

### (11S,21R,S)-3-Decladinosyl-11,12-dideoxy-6-O-methyl-3-oxo-12,11-[oxycarbonyl-(3-[3-(4-chlorophenyl)-1H-pyrazol-5-yl]-propylamino)-methylene]-erythromycin A

A solution of example **7** (0.040g) and intermediate **97** (0.035g) in anhydrous acetonitrile (1.5mL) was stirred at room temperature for 12h then heated to 50°C for 6h under nitrogen atmosphere. The solvent was evaporated under reduced pressure, the crude dissolved in anhydrous MeOH (1.5mL) then sodium cyanoborohydride (1M in THF, 0.030mL) and acetic acid (0.004mL) were added. After 12h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.017g).
¹H-NMR (CDCl₃) δ: 7.73 (d, 2H), 7.35 (d, 2H), 6.35 (s, 1H), 4.15 (m, 1H), 2.93 (m, 2H), 2.82 (m, 1H), 2.34 (m, 1H), 1.88 (m, 1H).

### Example 102

### (11S,21R,S)-3-Decladinosyl-11,12-dideoxy-6-O-methyl-3-ozo-12,11-[oxycarbonyl-(3-[6-(methylthio)-7H-purin-7-yl]-propylamino)-methylene]-erythromycin A

A solution of example **7** (0.040g) and intermediate 98 (0.042g) in anhydrous acetonitrile (1.5mL) was stirred at room temperature for 3h then heated to 50°C for 3h under nitrogen atmosphere. The solvent was evaporated under reduced pressure, the crude dissolved in anhydrous MeOH (1.5mL) then sodium cyanoborohydride (1M in THF, 0.060mL) and acetic acid (0.008mL) were added. After 6h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 98\2) to give the title compound (0.005g).
¹H-NMR (CDCl₃) δ: 8.85 (m, 1H), 8.25 (s, 1H), 4.63 (m, 1H), 4.45 (m, 1H), 4.15 (m, 1H), 2.97 (m, 2H), 2.76 (s, 3H), 2.33 (m, 1H), 2.0÷2.2 (m, 2H).

### Example 103

### (11S,21R,S)-3-Decladinosyl-11,12-dideoxy-6-O-methyl-3-oxo-12,11-[oxycarbonyl-(3-(6-methoxy-7H-purin-7-yl)-propylamino)-methylene]-erythromycin A

A solution of example **7** (0.050g) and intermediate **99** (0.031g) in anhydrous acetonitrile (1.5mL) was stirred at room temperature overnight under nitrogen atmosphere. The solvent was evaporated under reduced pressure, the crude dissolved in anhydrous MeOH (1.5mL) then sodium cyanoborohydride (1M in THF, 0.074mL) and acetic acid (0.010mL) were added. After 4h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.015g).
¹H-NMR (CDCl₃) δ: 8.54 (s, 1H), 8.07 (s, 1H), 5.58 (dd, 1H), 4.37 (t, 2H), 4.31 (d, 1H), 4.25 (d, 1H), 4.19 (s, 3H), 4.13 (bs, 1H), 3.86 (q, 1H), 3.55 (m, 1H), 3.20 (m, 1H), 3.10 (m, 1H), 3.04 (m, 1H), 2.89 (bm, 2H), 2.70 (s, 3H), 2.59 (m, 1H), 2.48 (bm, 1H), 2.34 (s, 1H), 2.29 (s, 6H), 2.12 (m, 1H), 2.04 (m, 1H), 1.95 (m, 1H), 1.85-1.48 (m, 5H), 1.49 (s, 3H), 1.39 (d, 3H), 1.34 (s, 3H), 1.32 (d, 3H), 1.25 (d+m, 3H+1H), 1.16 (d, 3H), 1.10 (d, 3H), 0.89 (t, 3H).

### Example 104

### (11S,21R,S)-3-Decladinosyl-11,12-dideoxy-6-O-methyl-3-oxo-12,11-[oxycarbonyl-(3-(6-methoxy-2-oxo-1,3-benzoxazol-3(2H)-yl)-propylamino)-methylene]-erythromycin A

A solution of example **7** (0.043g) and intermediate **100** (0.025g) in anhydrous acetonitrile (2mL) was stirred at room temperature for 30min then heated to 50°C for 6h under nitrogen atmosphere. The solvent was evaporated under reduced pressure, the crude dissolved in anhydrous MeOH (2mL) then sodium cyanoborohydride (1M in THF, 0.030mL) and acetic acid (0.005mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 97\3) to give the title compound (0.007g).
¹H-NMR (CDCl₃) δ: 7.05 (d, 1H), 6.82 (d, 1H), 6.47 (dd, 1H), 4.13 (s, 1H), 3.87 (m, 2H), 3.81 (s, 3H), 3.04 (m, 1H), 2.97-2.92 (m, 2H), 2.34 (m, 1H), 1.96 (m, 1H), 1.80 (m, 1H), 1.09 (d, 3H).

### Example 105

### (11S,21R,S)-3-Decladinosyl-11,12-dideoxy-6-O-methyl-3-oxo-12,11-[oxycarbonyl-(3-(1H pyrrolo[2,3-b]pyridin-1-yl)-propylamino)-methylene]-erythromycin A

A solution of example **7** (0.050g) and intermediate **101** (0.042g) in anhydrous acetonitrile (1.5mL) was stirred at room temperature for 6h under nitrogen atmosphere. The solvent was evaporated under reduced pressure, the crude dissolved in anhydrous MeOH (1.5mL) then sodium cyanoborohydride (1M in THF, 0.037mL) and acetic acid (0.005mL) were added. After 8h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH\NH₄OH 95\5\0.5) to give the title compound (0.006g).
¹H-NMR (CDCl₃) δ: 8.30 (dd, 1H), 7.87 (dd, 1H), 7.30 (d, 1H), 7.03 (dd, 1H), 6.43 (d, 1H), 5.71 (dd, 1H), 4.44÷4.36 (m, 2H), 4.32 (d, 1H), 4.25 (d, 1H), 4.13 (s, 1H), 3.85 (q, 1H), 3.55 (m, 1H), 3.22 (dd, 1H), 3.11 (m, 1H), 3.04 (m, 1H), 2.90÷2.78 (m, 2H), 2.70 (s, 3H), 2.62÷2.50 (m, 2H), 2.35 (s, 1H), 2.32 (s, 6H), 2.06 (m, 1H), 2.00 (m,1H), 1.95 (m, 1H), 1.80÷1.68 (m, 3H), 1.58 (m, 1H), 1.49 (s, 3H), 1.39 (d, 3H), 1.33 (s, 3H), 1.32 (d, 3H), 1.25 (m, 1H), 1.25 (d, 3H), 1.15 (d, 3H), 1.10 (d, 3H), 0.89 (t, 3H).

### Example 106

### (11S,21R,S)-3-Decladinosyl-11,12-dideoxy-6-O-methyl-3-oxo-12,11-[oxycarbonyl-(2-(1,3-dioxo-1,3-dihydro-2H-isoindol-2-yl)-ethylamino)-methylene]-erythromycin A

To a solution of example **13** (0.232g) in anhydrous DMF (5mL) 2-[(1,3-thiazol-2-ylamino)carbonyl]benzoic acid (0.097g), (benzotriazol-1-yloxy)tripyrrolidinophosphonium hexafluorophosphate (0.186g) and DIPEA (0.120mL) were added and the resulting mixture was stirred overnight at room temperature. It was diluted with DCM (10mL) and washed with a 5% NaHCO₃ aqueous solution (2x5mL). The organic phase was dried over Na₂SO₄ and concentrated under reduced pressure. The residue was dissolved in DCM (5mL) loaded on SCX-cartridge (5g, loading 0.75mmol\g, previously washed with 50mL of MeOH), washed with MeOH (50mL), then the product eluted with NH₃ (0.25M solution in MeOH, 60mL), followed by MeOH (10mL). Solvent evaporation gave the title compound (0.170g).
¹H-NMR (CDCl₃) δ: 7.80 (m, 2H), 7.67 (m, 2H), 5.53 (dd, 1H), 4.30 (d, 1H), 4.24 (d, 1H), 4.17 (s, 1H), 3.84 (m, 2H), 3.74 (m, 1H), 3.55 (m, 1H), 3.20-3.15 (m, 2H), 3.12-2.88 (m, 3H), 2.63 (s, 3H), 2.54 (m, 1H), 2.47 (m, 1H), 2.30 (m, 1H), 2.27 (s, 6H), 1.86 (m, 1H), 1.75-1.65 (m, 3H), 1.48 (m, 1H), 1.43 (s, 3H), 1.29 (s, 3H), 1.28-1.24 (m, 2*3H+1H), 1.19 (d, 3H), 1.12 (d, 3H), 1.06 (d, 3H), 0.73 (t, 3H).

### Example 107

### (11S,21R,S)-3-Decladinosyl-11,12-dideoxy-6-O-methyl-3-oxo-12,11-[oxycarbonyl-(3-[3-(2,4-dimethyl-1,3-thiazol-5-yl)-1H-pyrazol-1-yl]-propylamino)-methylene]-erythromycin A

A solution of example **7** (0.050g) and intermediate **102** (0.042g) in anhydrous acetonitrile (1mL) was stirred at room temperature for 6h under nitrogen atmosphere. The solvent was evaporated under reduced pressure, the crude dissolved in anhydrous MeOH (2mL) then sodium cyanoborohydride (1M in THF, 0.070mL) and acetic acid (0.009mL) were added. After 20h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 98\2) to give the title compound (0.016g).
¹H-NMR (CDCl₃) δ: 7.49 (d, 1H), 6.36 (d, 1H), 5.62 (dd, 1H), 4.31 (d, 1H), 4.25 (d, 1H), 4.23 (m, 2H), 4.13 (s, 1H), 3.85 (q, 1H), 3.56 (m, 1H), 3.19 (m, 1H), 3.10 (m, 1H), 3.03 (m, 1H), 2.92+2.82 (m, 2H), 2.69 (s, 3H), 2.66 (s, 3H), 2.58 (m, 1H), 2.55 (s, 3H), 2.47 (m, 1H), 2.33 (s, 1H), 2.28 (s, 6H), 2.08 (m, 1H), 1.98 (m, 1H), 1.94 (m, 1H), 1.80 (m, 1H), 1.76=1.66 (m, 2H), 1.62÷1.50 (m, 1H), 1.49 (s, 3H), 1.39 (d, 3H), 1.34 (s, 3H), 1.32 (d, 3H), 1.25 (m, 1H), 1.25 (d, 3H), 1.15 (d, 3H), 1.10 (d, 3H), 0.88 (t, 3H).

### Example 108

### (11S,21R,S)-3-Decladinosyl-11,12-dideoxy-6-O-methyl-3-oxo-12,11-[oxycarbonyl-(3-(1H-imidazo[4,5-c]pyridin-1-yl)-propylamino)-methylene]-erythromycin A

### and

### Example 109

### (11S,21R,S)-3-Decladinosyl-11,12-dideoxy-6-O-methyl-3-oxo-12,11-[oxycarbonyl-(3-(3H-imidazo[4,5-c]pyridin-3-yl)-propylamino)-methylene]-erythromycin A

A solution of example **7** (0.050g) and intermediate **103** (0.034g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by preparative TLC (DCM\MeOH\NH₄OH 90\9\0.5) to give the title compound 108 (0.008g) and the title compound 109 (0.004g).
¹H-NMR (CDCl₃) δ (example 108): 9.11 (s, 1H), 8.44 (d, 1H), 8.08 (s, 1H), 7.47 (d, 1H), 4.41 (m, 1H), 4.28 (m, 1H), 4.14 (m, 1H), 3.05 (m, 1H), 3.00-2.90 (m, 2H), 2.31 (m, 1H), 2.08 (m, 1H), 1.95 (m, 1H), 1.09 (d, 3H).
¹H-NMR (CDCl₃) δ (example 109): 8.90 (s, 1H), 8.45 (d, 1H), 8.16 (s, 1H), 7.70 (d, 1H), 4.48 (m, 1H), 4.37 (m, 1H), 4.15 (bs, 1H), 3.08 (m, 1H), 2.95 (m, 1H), 2.33 (m, 1H), 2.14 (m, 1H), 2.04 (m, 1H), 1.09 (d, 3H).

### Example 110

### (11S,21R,S)-3-Decladinosyl-11,12-dideoxy-6-O-methyl-3-oxo-12,11-[oxycarbonyl-(3-(1H-benzimidazol-1-yl)-propylamino)-methylene]-erythromycin A

A solution of example **7** (0.050g) and intermediate **104** (0.025g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) to give the title compound (0.021g).
¹H-NMR (CDCl₃) δ: 8.00 (s, 1H), 7.80 (d, 1H), 7.48 (d, 1H), 7.40-7.20 (m, 2H), 4.38 (m, 1H), 4.24 (m, 1H), 4.10 (m, 1H), 3.05 (m, 1H), 3.03 (m, 1H), 2.98 (m, 1H), 2.32 (m, 1H), 2.02 (m, 1H), 1.93 (m, 1H), 1.09 (d, 3H).

### Example 111

### (11S,21R,S)-3-Decladinosyl-11,12-dideoxy-6-O-methyl-3-oxo-12,11-[oxycarbonyl-(3-(3H imidazo[4,5-b]pyridin-3-yl)-propylamino)-methylene]-erythromycin A

A solution of example **7** (0.050g) and intermediate **105** (0.034g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) and by preparative TLC (eluting with: DCM\MeOH\NH₄OH 90\9\0.5) to give the title compound (0.004g).
¹H-NMR (CDCl₃) δ: 8.39 (dd, 1H), 8.21 (s, 1H), 8.05 (dd, 1H), 7.22 (dd, 1H), 4.43 (t, 2H), 4.15 (m, 1H), 3.04 (m, 1H), 2.32 (m, 1H), 1.10 (d, 3H).

### Example 112

### (11S,21R,S)-3-Decladinosyl-11,12-dideoxy-6-O-methyl-3-oxo-12,11-[oxycarbonyl-(3-(quinoxalin-2-ylsulfanyl)-propylamino)-methylene]-erythromycin A

A solution of intermediate **106** (0.080g) in acetonitrile (3mL) and a 2N HCl aqueous solution (2mL) was heated to 40°C for 1h. The reaction mixture was cooled down to room temperature, then it was added dropwise to a solution of example **7** (0.090g) dissolved in anhydrous acetonitrile (3mL) keeping the pH of the solution in the range 6-7 by addition of saturated NaHCO₃ aqueous solution. The solution was stirred at room temperature overnight. Acetic acid was added to the mixture to reach pH 5-6 followed by sodium cyanoborohydride (1M in THF, 0.200mL). The reaction mixture was stirred for 5h at room temperature. After evaporating the solvent a saturated NaHCO₃ aqueous solution (10mL) was added and the product was extracted with DCM (4x15mL). The organic layer was dried over Na₂SO₄ and concentrated under vacuum. The residue was dissolved in MeOH (4mL) and stirred overnight. After evaporating the solvent the crude material was purified by flash chromatography (eluting with DCM\MeOH from 98\2 to 96\4) affording the title compound (0.050g).
m\z ([MH]⁺) = 829.
¹H-NNM (CDCl₃) δ: 8.57 (s, 1H), 7.99 (d, 1H), 7.96 (d, 1H), 7.69 (t, 1H), 7.60 (t, 1H), 5.71 (dd, 1H), 4.32 (d, 1H), 4.25 (m, 1H), 4.17 (m, 1H), 3.84 (q, 1H), 3.57 (m, 1H), 3.25 (m, 1H), 3.50-2.90 (m, 4H), 3.20-2.90 (m, 4H), 2.69 (s, 3H), 2.58 (m, 1H), 2.37 (s, 6H), 2.34 (m, 1H), 2.10-1.85 (m, 3H), 1.80-1.40 (m, 4H), 1.49 (s, 3H), 1.37 (d, 3H), 1.31 (d, 3H), 1.31 (s, 3H), 1.28 (m, 1H), 1.25 (d, 3H), 1.15 (d, 3H), 1.10 (d, 3H), 0.85 (t, 3H).

### Example 113

### (11S,21R,S)-3-Decladinosyl-11,12-dideoxy-6-O-methyl-3-oxo-12,11-[oxycarbonyl-(3-(4-phenyl-1H-imidazol-1-yl)-propylamino)-methylene]-erythromycin A

A solution of example **7** (0.050g) and intermediate **107** (0.027g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by preparative TLC (eluting with: DCM\MeOH\NH₄OH 90\9\0.5) to give the title compound (0.012g).
¹H-NMR (CDCl₃) δ: 7.79 (d, 2H), 7.56 (m, 1H), 7.36 (m, 2H), 7.29 (d, 1H), 7.25 (m, 1H), 4.32 (m, 1H), 4.15 (m, 1H), 4.04 (m, 1H), 3.05 (m, 1H), 3.05-2.95 (m, 2H), 2.33 (m, 1H), 1.98 (m, 1H), 1.80 (m, 1H), 1.09 (d, 3H).

### Example 114

### (11S,21R,S)-3-Decladinosyl-11,12-dideoxy-6-O-methyl-3-oxo-12,11-[oxycarbonyl-(2-(4-pyridin-4-yl-1H-imidazol-1-yl)-ethylamino)-methylene]-erythromycin A

A solution of intermediate **108** (0.060g) in acetonitrile (1.5mL) and a 3M HCl aqueous solution (1.5mL) was stirred at 70°C for 14h. The reaction mixture was allowed to reach room temperature, then it was added dropwise to a solution of example **7** (0.030g) dissolved in anhydrous acetonitrile (1mL) keeping the pH of the solution in the range 6-7 by addition of a saturated NaHCO₃ aqueous solution. The solution was stirred at room temperature for 4h. Acetic acid was added to the mixture to reach pH 5-6 followed by sodium cyanoborohydride (1M in THF, 0.150mL). The reaction mixture was stirred for 72h at room temperature. After evaporating the solvent a saturated NaHCO₃ aqueous solution (3mL) was added and the mixture was extracted with DCM (3x5mL). The organic layer was dried over Na₂SO₄ and concentrated under vacuum. The crude material was purified by flash chromatography (eluting with DCM\MeOH from 100\0 to 97\3) and by preparative TLC (eluting with: DCM\MeOH 90\10) to give the title compound (0.004g).
¹H-NMR (CDCl₃) δ: 8.57 (d, 2H), 7.72 (d, 2H), 7.71 (m, 1H), 7.60 (m, 1H), 5.63 (dd, 1H), 4.27 (d, 1H), 4.19 (s, 1H), 4.18 (d, 1H), 4.12 (m, 2H), 3.85 (q, 1H), 3.55 (m, 1H), 3.32 (m, 1H), 3.24 (m, 1H), 3.16 (dd, 1H), 3.05 (m, 1H), 3.03 (m, 1H), 2.56 (m, 1H), 2.44 (m, 1H), 2.41 (s, 3H), 2.27 (s, 1H), 2.26 (s, 6H), 1.85 (m, 1H), 1.78 (m, 1H), 1.70÷1.63 (m, 2H), 1.55 (m, 1H), 1.48 (s, 3H), 1.41 (d, 3H), 1.31 (d, 3H), 1.24 (m, 1H), 1.24 (d, 3H), 1.23 (s, 3H), 1.14 (d, 3H), 1.09 (d, 3H), 0.81 (t, 3H).

### Example 115

### (11S,21R,S)-3-Decladinosyl-11,12-dideoxy-6-O-methyl-3-oxo-12,11-[oxycarbonyl-(2-(quinoxalin-2-ylsulfanyl)-ethylamino)-methylene]-erythromycin A

A solution of intermediate **109** (0.067g) in acetonitrile (3mL) and a 2N HC1 aqueous solution (2mL) was stirred overnight. Then, the reaction mixture was added dropwise to a solution of example **7** (0.090g) dissolved in anhydrous acetonitrile (3mL) keeping the pH of the solution in the range 6-7 by addition of saturated NaHCO₃ aqueous solution. The reaction mixture was stirred at room temperature for 3h. Acetic acid was added to the mixture to reach pH 5-6 followed by sodium cyanoborohydride (1M in THF, 0.200mL). The reaction mixture was stirred for 5h at room temperature. After evaporating the solvent a saturated NaHCO₃ aqueous solution (10mL) was added and the mixture was extracted with DCM (4x15mL). The organic layer was dried over Na₂SO₄ and concentrated under vacuum. The residue was dissolved in MeOH (4mL) and stirred overnight. After evaporating the solvent the crude material was purified by flash chromatography (eluting with DCM\MeOH from 98\2 to 96\4) to give the title compound (0.076g).
m\z ([MH]⁺) = 815.
¹H-NMR (CDCl₃) δ: 8.55 (s, 1H), 7.99 (d, 1H), 7.93 (d, 1H), 7.68 (t, 1H), 7.60 (t, 1H), 5.68 (dd, 1H), 4.32 (d, 1H), 4.26 (d, 1H), 4.21 (m, 1H), 3.83 (q, 1H), 3.62-3.48 (m, 3H), 3.23 (m, 2H, 3.19 (dd, 1H), 3.10 (m, 1H), 3.04 (m, 1H), 2.74 (s, 3H), 2.58 (m, 1H), 2.48 (m, 1H), 2.35 (s, 1H), 2.29 (s, 6H), 1.91 (m, 1H), 1.80-1.67 (m, 3H), 1.55 (m, 1H), 1.49 (s, 3H), 1.35 (d, 3H), 1.30 (d, 3H), 1.34 (s, 3H), 1.26 (m, 1H), 1.25 (d, 3H), 1.14 (d, 3H), 1.09 (d, 3H), 0.86 (t, 3H).

### Example 116

### (11S,21R,S)-3-Decladinosyl-11,12-dideoxy-6-O-methyl-3-oxo-12,11-[oxycarbonyl-(3-(4-(thiophen-2-yl)-imidazol-1-yl)-propylamino)-methylene]-erythromycin A

A solution of example **6** (0.040g) and intermediate **110** (0.033g) in anhydrous acetonitrile (1.5mL) was stirred at room temperature for 12h. After evaporating the solvent the residue was dissolved in anhydrous MeOH (1.5mL) and sodium cyanoborohydride (1M in THF, 0.030mL) and acetic acid (0.004mL) were added. The reaction mixture was stirred for 12h. The solvent was evaporated under vacuum, the residue dissolved in DCM (5mL) and washed with a saturated NaHCO₃ aqueous solution (2x2mL). The organic layer was dried over Na₂SO₄ and concentrated under vacuum. The crude material was purified by flash chromatography (eluting with: DCM \MeOH 100\0, 98\2, 97\3) affording the title compound (0.025g).
¹H-NMR (CDCl₃) δ: 7.52 (d, 1H), 7.18 (d, 1H), 7.27 (m, 1H), 7.16 (d, 1H), 7.02 (dd, 1H), 5.58 (dd, 1H), 4.31 (d, 1H), 4.25 (d, 1H), 4.14 (m, 1H), 4.12 (m, 1H), 4.02 (m, 1H), 3.86 (q, 1H), 3.56 (m, 1H), 3.20 (m, 1H), 3.10 (m, 1H), 3.04 (m, 1H), 2.93 (m, 2H), 2.68 (s, 3H), 2.60 (m, 1H), 2.50 (m, 1H), 2.32 (m, 1H), 2.30 (s; 6H), 2.05-1.85 (m, 3H), 1.80 (m, 1H), 1.70 (m, 2H), 1.60 (m, 1H), 1.49 (s, 3H), 1.39 (d, 3H), 1.34 (s, 3H), 1.32 (d, 3H), 1.25 (d+m, 3H+1H), 1.16 (d, 3H), 1.10 (d, 3H), 0.88 (t, 3H).

### Example 117

### (11S,21S)-3-Decladinosyl-11,12-dideoxy-6-O-methyl-3-oxo-12,11-[oxycarbonyl-(3-(4-(thiophen-2-yl)-imidazol-1-yl)-propylamino)-methylene]-erythromycin A

A solution of example **7** (0.750g) and intermediate **110** (0.300g) in anhydrous acetonitrile (15mL) was stirred at room temperature for 12h. After evaporating the solvent the residue was dissolved in anhydrous MeOH (15mL) and sodium cyanoborohydride (1M in THF, 0.728mL) and acetic acid (0.084mL) were added. The mixture was stirred overnight at room temperature. The solvent was evaporated under vacuum, the crude material dissolved in DCM (50mL) and washed with a saturated NaHCO₃ aqueous solution (2x20mL). The organic layer was dried over Na₂SO₄ and concentrated under vacuum. The crude material was purified by flash chromatography (eluting with: DCM \MeOH from 95\5 to 90\10) to give the title compound (0.872g, (21*S*) isomer 95% pure by NMR analysis).
¹H-NMR (CDCl₃) δ: 7.52 (d, 1H), 7.18 (d, 1H), 7.27 (m, 1H), 7.16 (d, 1H), 7.02 (dd, 1H), 5.58 (dd, 1H), 4.31 (d, 1H), 4.25 (d, 1H), 4.14 (m, 1H), 4.12 (m, 1H), 4.02 (m, 1H), 3.86 (q, 1H), 3.56 (m, 1H), 3.20 (m, 1H), 3.10 (m, 1H), 3.04 (m, 1H), 2.93 (m, 2H), 2.68 (s, -OCH₃), 2.60 (m, 1H), 2.50 (m, 1H), 2.32 (m, 1H), 2.30 (s, N(CH₃)₂), 2.05-1.85 (m, 3H), 1.80 (m, 1H), 1.70 (m, 2H), 1.60 (m, 1H), 1.49 (s, 3H), 1.39 (d, 3H), 1.34 (s, 3H), 1.32 (d, 3H), 1.25 (d+m, 3H+1H), 1.16 (d, 3H), 1.10 (d, 3H), 0.88 (t, 3H).

### Example 118

### (11S,21R,S)-3-Decladinosyl-11,12-dideoxy-6-O-methyl-3-oxo-12,11-[oxycarbonyl-(3-(6-methyl-2-oxo-1,3-benzoxazol-3(2H)-yl)-propylamino)-methylene]-erythromycin A

A solution of example **7** (0.050g) and intermediate **111** (0.035g) in anhydrous acetonitrile (2mL) was stirred at 50°C for 6h. After cooling to room temperature solvent was evaporated, the residue dissolved in anhydrous MeOH (2mL) and sodium cyanoborohydride (1M in THF, 0.140mL) and acetic acid (0.010mL) were added. The mixture was stirred overnight at room temperature. The solvent was evaporated under vacuum, the residue dissolved in DCM (50mL) and washed with a saturated NaHCO₃ aqueous solution (2x20mL). The organic layer was dried over Na₂SO₄ and concentrated under vacuum. The crude material was purified by flash chromatography (eluting with: DCM \MeOH from 100\0 to 95\5) affording the title compound (0.020g).
¹H-NMR (CDCl₃) δ: 7.20-6.80 (m, 3H), 5.66 (dd, 1H), 4.38 (d, 1H), 4.26 (d, 1H), 4.11 (s, 1H), 3.88 (m, 3H), 3.67 (m, 1H), 3.41 (m, 1H), 3.20-2.85 (m, 5H), 2.71 (s, 3H), 2.70 (bs, 6H), 2.59 (m, 1H), 2.41-2.35 (m, 1H+ 3H), 2.00-1.55 (m, 7H), 1.50 (s, 3H), 1.39 (d, 1H+3H), 1.32-1.27 (m, 3*3H), 1.18 (d, 3H), 1.10 (d, 3H), 0.88 (t, 3H).

### Example 119

### (11S,21R,S)1-3-Decladinosyl-11,12-dideoxy-6-O-methyl-3-oxo-12,11-[oxycarbonyl-(2-(2-pyridin-4-yl-1H-imidazol-1-yl)-ethylamino)-methylene]-erythromycin A

A solution of intermediate **112** (0.060g) in acetonitrile (3mL) and a 3M HCl aqueous solution (3mL) was stirred at 80°C for 3 days. The reaction mixture was allowed to reach room temperature, then it was added dropwise to a solution of example **7** (0.050g) dissolved in anhydrous acetonitrile (2mL) keeping the pH of the solution in the range 6-7 by addition of a saturated NaHCO₃ aqueous solution. The solution was stirred at room temperature for 24h. Acetic acid was added to the mixture to reach pH 5-6 followed by sodium cyanoborohydride (1M in THF, 0.070mL). The reaction mixture was stirred for 16h at room temperature. After evaporating the solvent a saturated NaHCO₃ aqueous solution (3mL) was added and the product was extracted with DCM (3x5mL). The organic layer was dried over Na₂SO₄ and concentrated under vacuum. The crude material was dissolved in MeOH (1mL) and stirred overnight. After evaporating the solvent the compound was purified by flash chromatography (eluting with DCM\MeOH from 100\0 to 97\3) to give the title compound (0.004g).
m\z ([MH]⁺) = 798

### Example 120

### (11S,21R,S)-3-Decladinosyl-11,12-dideoxy-6-O-methyl-3-oxo-12,11-[oxycarbonyl-(3-[3-(5-cyano-3,4-dimethylthien-2-yl)-1H-1,2,4-triazol-1-yl]-propylamino)-methylene]-erythromycin A

A solution of example **7** (0.050g) and intermediate **113** (0.046g) in anhydrous acetonitrile (2mL) was stirred at room temperature for 18h under nitrogen atmosphere. The solvent was evaporated under reduced pressure, the residue dissolved in anhydrous MeOH (2mL) then sodium cyanoborohydride (1M in THF, 0.070mL) and acetic acid (0.009mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) to give the title compound (0.001 g).
¹H-NMR (CDCl₃) δ: 8.23 (s, 1H), 5.51 (dd, 1H), 4.38÷4.20 (m, 4H), 4.12(s, 1H), 3.86 (m, 1H), 3.55 (m, 1H), 3.18 (dd, 1H), 3.12÷3.00 (m, 2H), 2.98÷2.84 (m, 2H), 2.68 (s, 3H), 2.58 (m, 1H), 2.52 (s, 3H), 2.44 (m, 1H), 2,37 (s, 3H), 2.32 (s, 1H), 2.27 (s, 6H), 2.12 (m, 1H), 2.02 (m, 1H), 1.92 (m, 1H), 1.81 (m, 1H), 1.74÷1.63 (m, 2H), 1.55 (m, 1H), 1.49 (s, 3H), 1.38 (d, 3H), 1.33 (s, 3H), 1.32 (d, 3H), 1.25(m, 1H), 1.25 (d, 3H), 1.15(d, 3H), 1.09 (d, 3H), 0.88 (t, 3H).

### Example 121

### (11S,21R,S)-3-Decladinosyl-11,12-dideoxy-6-O-methyl-3-oxo-12,11-[oxycarbonyl-(3-(quinolin-3-yl)-propylamino)-methylene]-erythromycin A

A solution of example **7** (0.050g) and intermediate **114** (0.041g) in anhydrous acetonitrile (2mL) was stirred at room temperature for 18h under nitrogen atmosphere. The solvent was evaporated under reduced pressure, the residue dissolved in anhydrous MeOH (2mL) then sodium cyanoborohydride (1M in THF, 0.070mL) and acetic acid (0.009mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) to give the title compound (0.009g).
¹H-NMR (CDCl₃) δ: 8.79 (d, 1H), 8.07 (d, 1H), 7.97 (d, 1H), 7.79 (d, 1H), 7.65 (t, 1H), 7.52 (t, 1H), 5.74 (dd, 1H), 4.32 (d, 1H), 4.25 (d, 1H), 4.17 (s, 1H), 3.85 (q, 1H), 3.55 (m, 1H), 3.22 (m, 1H), 3.11 (m, 1H), 3.04 (m, 1H), 3.02÷2.78 (m, 4H), 2.69 (s, 3H), 2.99 (m, 1H), 2.52 (m, 1H), 2.34 (s, 1H), 2.32 (s, 6H), 1.94 (m, 1H), 1.89 (m, 2H), 1.88÷1.66 (m, 3H), 1.62÷1.50 (m, 1H), 1.49 (s, 3H), 1.39 (d, 3H), 1.32 (m, 6H), 1.25 (m, 1H), 1.25 (d, 3H), 1.16 (d, 3H), 1.10 (d, 3H), 0.88 (t, 3H).

### Example 122

### (11S,21R,S)-3-Decladinosyl-11,12-dideoxy-6-O-methyl-3-oxo-12,11-[oxycarbonyl-(3-[4-(3-nitrophenyl)-1H-imidazol-1-yl]-propylamino)-methylene]-erythromycin A

A solution of example **7** (0.050g) and intermediate **115** (0.026g) in anhydrous acetonitrile (1mL) was stirred at room temperature for 18h under nitrogen atmosphere. The solvent was evaporated under reduced pressure, the crude dissolved in anhydrous MeOH (2mL) then sodium cyanoborohydride (1M in THF, 0.037mL) and acetic acid (0.004mL) were added. After 18h the solvent was removed under reduced pressure, the residue dissolved in DCM (5mL) and washed with a saturated NaHCO₃ aqueous solution (2x3mL). The organic phase was dried over Na₂SO₄ and concentrated under reduced pressure. The crude material was purified by flash chromatography (eluting with: DCM\MeOH from 100\0 to 94\6) to give the title compound (0.007g).
¹H-NMR (CDCl₃) δ: 8.62 (t, 1H), 8.17 (d, 1H), 8.05 (d, 1H), 7.60 (d, 1H), 4.15 (m, 2H), 4.09 (m, 1H), 4.05 (m, 1H), 3.02 (m, 1H), 3.04-2.92 (m, 2H), 2.33 (m, 1H), 2.05 (m, 1H), 1.98 (m, 1H), 1.12 (d, 3H).

### Example 123

### (11S,21R,S)-3-Decladinosyl-11,12-dideoxy-6-O-methyl-3-oxo-12,11-[oxycarbonyl-([2-(2,3-dihydro-1,4-benzodioxin-6-yl)pyrrolidin-1-yl])-methylene]-erythromycin A

To a solution of example **7** (0.100g) in anhydrous acetonitrile intermediate **117** (0.066g) was added portionwise at room temperature under nitrogen atmosphere. After stirring overnight the solvent was removed under reduced pressure, the residue dissolved in MeOH (2.5mL) and sodium cyanoborohydride (1M in THF, 0.022mL) and acetic acid (0.013mL) added. The mixture was stirred overnight. The solvent was evaporated under vacuum, the residue dissolved in EtOAc (5mL) and washed with a saturated NaHCO₃ aqueous solution (2x2mL) and brine (2mL). The organic layer was dried over Na₂SO₄ and concentrated under vacuum. The crude material was purified by flash chromatography (eluting with: DCM \MeOH from 100\0 to 97\3) affording the title compound (0.070g).
m\z ([MH]⁺) = 798

### Example 124

### (11S,21R,S)-3-Decladinosyl-11,12-dideoxy-6-O-methyl-3-oxo-12,11-[oxycarbonyl-(3-(5-methoxy-1H-pyrrolo[3,2-b]pyridin-1-yl)-propylamino)-methylene]-erythromycin A

A solution of intermediate **118** (0.065g) in acetonitrile (2.5mL) and a 3M HCl aqueous solution (2.5mL) was stirred at 60°C for 8h. The reaction mixture was allowed to reach room temperature, then it was added dropwise to a solution of example **7** (0.040g) dissolved in anhydrous acetonitrile (1mL) keeping the pH of the solution in the range 6-7 by addition of a saturated NaHCO₃ aqueous solution. The mixture was stirred at room temperature for 24h. Acetic acid was added to reach pH 5-6 followed by sodium cyanoborohydride (1M in THF, 0.060mL). The reaction mixture was stirred overnight at room temperature. After evaporating the solvent a saturated NaHCO₃ aqueous solution (3mL) was added and the mixture was extracted with DCM (3x5mL). The organic layer was dried over Na₂SO₄ and concentrated under vacuum. The crude material purified by flash chromatography (eluting with DCM\MeOH from 100\0 to 95\5) to give the title compound (0.014g).
¹H-NMR (CDCl₃) δ: 7.66 (d, 1H), 7.25 (d, 1H), 6.62 (m, 1H), 6.51 (d, 1H), 4.40 (m, 1H), 4.30 (m, 1H), 4.15 (m, 1H), 4.00 (s, 3H), 3.03 (m, 1H), 3.05-2.88 (m, 2H), 2.30 (m, 1H), 2.06 (m, 1H), 1.96 (m, 1H), 1.10 (d, 3H).

### Example 125

### (11S,21R,S)-3-Decladinosyl-11,12-dideoxy-6-O-methyl-3-oxo-12,11-[oxycarbonyl-(2-[3-(1,3-thiazol-2-yl)-1H-pyrazol-1-yl]-ethylamino)-methylene]-erythromycin A

A solution of intermediate **119** (0.015g) in acetonitrile (1mL) and a 3M HCl aqueous solution (1mL) was stirred at room temperature for 16h and heated to 50°C for 8h. The reaction mixture was allowed to reach room temperature, then it was added dropwise to a solution of example **7** (0.033g) dissolved in anhydrous acetonitrile (1mL) keeping the pH of the solution in the range 6-7 by addition of saturated NaHCO₃ aqueous solution. The solution was stirred at room temperature overnight. Acetic acid was added to reach pH 5-6 followed by sodium cyanoborohydride (1M in THF, 0.0.25mL). The reaction mixture was stirred overnight at room temperature. After evaporating the solvent a saturated NaHCO₃ aqueous solution (3mL) was added and the mixture was extracted with DCM (3x5mL). The organic layer was dried over Na₂SO₄ and concentrated under vacuum. The residue was dissolved in MeOH (1mL) and stirred overnight. After evaporating the solvent the crude material was purified by flash chromatography (eluting with DCM\MeOH from 100\0 to 97\3) and by preparative LC (A\B from 80\20 to 10\90 in 20min) to give the title compound (0.014g).
¹H-NMR (CDCl₃) δ: 7.80 (d, 1H), 7.69 (d, 1H), 7.27 (d, 1H), 6.76 (d, 1H), 5.63 (dd, 1H), 4.33 (m, 3H), 4.23 (m, 2H), 3.84 (m, 1H), 3.57 (m, 1H), 3.35 (m, 2H), 3.22 (m, 1H), 3.10-3.00 (m, 2H), 2.64-2.50 (m, 2H), 2.50 (s, 3H), 2.33 (s, 6H), 2.30 (s, 1H), 1.90 (m, 1H), 1.80-1.66 (m, 3H), 1.60-1.40 (m+s, 1H+3H), 1.40-1.20 (m, 4*3H+1H), 1.14 (d, 3H), 1.09 (d, 3H), 0.86 (t, 3H).

### Example 126

### (11S,21R,S)-3-Decladinosyl-11,12-dideoxy-6-O-methyl-3-oxo-12,11-[oxycarbonyl-(2-(4-phenyl-1H-imidazol-1-yl)-ethylamino)-methylene]-erythromycin A

A solution of intermediate **120** (0.023g) in acetonitrile (0.5mL) and a 3M HCl aqueous solution (0.7mL) was heated to 70°C for 24h. The reaction mixture was allowed to reach room temperature, then it was added dropwise to a solution of example **7** (0.039g) dissolved in anhydrous acetonitrile (0.5mL) keeping the pH of the solution in the range 6-7 by addition of saturated NaHCO₃ aqueous solution. The solution was stirred at room temperature for 6h. Acetic acid was added to reach pH 5-6 followed by sodium cyanoborohydride (1M in THF, 0.0.58mL). The reaction mixture was stirred overnight at room temperature. After evaporating the organic solvent the aqueous phase was extracted with EtOAc (3x5mL). The organic layer was washed with brine (3mL), dried over Na₂SO₄ and concentrated under vacuum. The crude material was purified by flash chromatography (eluting with DCM\MeOH from 97\3 to 95\5) affording the title compound (0.026g).
¹H-NMR (CDCl₃) δ: 7.80 (d, 2H), 7.58 (d, 1H), 7.45 (d, 1H), 7.36 (t, 2H), 7.21 (m, 1H), 4.21 (m, 1H), 4.14-4.07 (m, 2H), 3.30-3.20 (m, 2H), 3.07 (m, 1H), 2.29 (m, 1H), 1.10 (d, 3H).

### Example 127

### (11S,21R,S)-3-Decladinosyl-11,12-dideoxy-6-O-methyl-3-oxo-12,11-[oxycarbonyl-(2-(4-thien-2-yl-1H-imidazol-1-yl)-ethylamino)-methylene]-erythromycin A

A solution of intermediate **121** (0.088g) in acetonitrile (1.5mL) and a 3M HCl aqueous solution (1mL) was heated to 70°C for 24h. The reaction mixture was allowed to reach room temperature, then it was added dropwise to a solution of example **7** (0.100g) dissolved in anhydrous acetonitrile (1mL) keeping the pH of the solution in the range 6-7 by addition of saturated NaHCO₃ aqueous solution. The solution was stirred at room temperature for 6h. Acetic acid was added to reach pH 5-6 followed by sodium cyanoborohydride (1M in THF, 0.150mL). The reaction mixture was stirred overnight at room temperature. After evaporating the organic solvent the aqueous phase was extracted with EtOAc (3x5mL). The organic layer was washed with brine (3mL), dried over Na₂SO₄ and concentrated under vacuum. The crude material was purified by flash chromatography (eluting with DCM\MeOH from 97\3 to 95\5). The obtained compound was dissolved in MeOH (5mL) stirred overnight at room temperature. After evaporating the solvent, the residue was purified by flash chromatography (eluting with DCM\MeOH from 100\0 to 95\5) to give the title compound (0.034g).
¹H-NMR (CDCl₃) δ: 7.54 (d, 1H), 7.32 (d, 1H), 7.28 (dd, 1H), 7.15 (dd, 1H), 7.01 (dd, 1H), 4.20 (m, 1H), 4.11-4.07 (m, 2H), 3.30-3.20 (m, 2H), 3.06 (m, 1H), 2.30 (m, 1H), 1.10 (d, 3H).

### Example 128

### (11S,21R,S)-3-Decladinosyl-11,12-dideoxy-6-O-methyl-3-oxo-12,11-[oxycarbonyl-(quinolin-2-ylmethylamino)-methylene]-erythromycin A

A solution of example **6** (0.050g) and quinoline-2-carbaldehyde (0.015g) in anhydrous toluene (3mL) was heated to 110°C for 3h. The solution was allowed to reach room temperature and concentrated under reduced pressure. The residue was dissolved in MeOH (5mL) and palladium palladium (10wt. % on carbon powder, 0.020g) was added and the mixture stirred under hydrogen atmosphere (1 atm) for 1h. Filtration through a celite pad eluting with MeOH (10mL) and purification by flash chromatography (eluting with DCM\MeOH 90\10) gave the title compound (0.009g).
¹H-NMR (CDCl₃) δ: 8.74 (s, 1H), 8.30 (d, 1H), 8.21 (d, 1H), 8.13 (td, 1H), 7.83 (t, 1H), 7.57 (t, 1H), 7.30 (t, 1H), 5.16 (bs, 1H), 3.12 (m, 1H), 2.93 (bs, 1H), 1.29 (d, 3H).

### Example 129

### (11S,21R,S)-3-Decladinosyl-11,12-dideoxy-6-O-methyl-3-oxo-12,11-[oxycarbonyl-(quinolin-3-ylmethylamino)-methylene]-erythromycin A

A solution of example **6** (0.050g) and quinoline-3-carbaldehyde (0.016g) in anhydrous toluene (3mL) was stirred at 100°C for 15h. After evaporation of the solvent under reduced pressure, the residue was dissolved in MeOH (1mL) and sodium cyanoborohydride (1M in THF, 0.060mL) and acetic acid (0.004mL) were added. The reaction mixture was stirred overnight at room temperature. After evaporating the solvent a saturated NaHCO₃ aqueous solution (3mL) was added and the mixture was extracted with DCM (3x5mL). The organic layer was dried over Na₂SO₄ and concentrated under vacuum. The crude material was purified by flash chromatography (eluting with DCM\MeOH 94\6) to give the title compound (0.007g).
¹H-NMR (CDCl₃) δ: 8.94 (d, 1H), 8.23 (d, 1H), 8.09 (d, 1H), 7.82 (d, 1H), 7.67 (t, 1H), 7.53 (t, 1H), 4.29 (d, 1H), 4.28 (m, 2H), 4.18 (m, 1H), 3.07 (m, 1H), 2.48 (m, 1H), 1.10 (d, 3H).

### Example 130

### (11S,21R,S)-3-Decladinosyl-11,12-dideoxy-6-O-methyl-3-oxo-12,11-[oxycarbonyl-(N-[3-(4-pyridin-3-yl-1H-imidazol-1-yl]-propylacetamido)-methylene]-erythromycin A

To a solution of example **66** (0.010g) in anhydrous DCM (0.4mL) DMAP (0.050g) and acetyl chloride (0.030mL) were added portionwise over 4 days. Water (2mL) was added and the mixture was extracted with DCM (3x5mL). The organic phase was dried over Na₂SO₄ and concentrated under vacuum. The crude material was purified by flash chromatography (eluting with DCM\MeOH from 97\3 to 95\5). The compound was dissolved in MeOH (1mL) and stirred overnight. Solvent evaporation gave the title compound (0.002g).
¹H-NMR (CDCl₃) δ: 8.97(s, 1H), 8.48 (d, 1H), 8.07 (d, 1H), 7.62 (s, 1H), 7.39 (s, 1H), 7.31 (m, 1H), 5.53 (bm, 1H), 4.35 (d, 1H), 4.29 (m, 1H), 4.15 (m, 1H), 4.01 (d, 1H), 3.83 (m, 1H), 3.72 (q, 1H), 3.65 (bs, 1H), 3.57-3.52 (m, 3H), 3.21 (m, 1H), 3.10 (m, 1H), 2.73 (m, 1H), 2.65-2.50 (m, 2H+3H), 2.39-2.22 (m, 2H+6H), 2.00 (s, 3H), 1.99 (m, 1H), 1.70-1.50 (m, 4H), 1.46 (s, 3H), 1.40 (d, 3H), 1.29-1.23 (m, 7H), 1.26 (s, 3H), 1.10 (d, 3H+3H), 0.95 (t, 3H).

### Example 131

### (11S,21R,S)-3-Decladinosyl-11,12-dideoxy-6-O-methyl-3-oxo-12,11-[oxycarbonyl-(benzyl-carbamate)-methylene]-erythromycin A

To a solution of example **7** (0.070g) in anhydrous DCM (2mL) cooled to 0°C TEA(0.090mL), DMAP (catalytic amount) and benzyl chloroformate (0.070g) were added under nitrogen atmosphere. The mixture was stirred at 0°C for 3h. After reaching room temperature a saturated NaHCO₃ aqueous solution (3mL) was added and the mixture was extracted with DCM (2x5mL). The organic phase was dried over Na2SO4 and concentrated under reduced pressure. The crude material was purified by flash chromatography (eluting with: DCM\MeOH from 100\0 to 98\2). The obtained compound was dissolved in MeOH (1mL) and stirred at room temperature overnight. Solvent evaporation gave the title compound (0.002g).
¹H-NMR (CDCl₃) δ: 7.40-7.20 (m, 5H), 5.81 (d, 1H), 5.16 (m, 1H), 5.08 (m, 1H), 4.80 (bm, 1H), 3.07 (m, 1H), 2.44 (m, 1H), 1.18 (d, 3H).

### Example 132

### (11S,21R,S)-3-Decladinosyl-11,12-dideoxy-6-O-methyl-3-oxo-12,11-[oxycarbonyl-((quinolin-2-ylmethylene)-amino)-methylene]-erythromycin A

A solution of example **6** (0.050g) and quinoline-2-carbaldehyde (0.015g) in anhydrous toluene (3mL) was heated to 110°C for 3h. The solution was allowed to reach room temperature and concentrated under reduced pressure. The residue was dissolved in MeOH (5mL) and stirred for 16h. Solvent evaporation gave the title compound (0.050g).
¹H-NMR (CDCl₃) δ: 8.99 (d, 1H), 8.47 (d, 1H), 8.09 (d, 1H), 7.58 (s, 1H), 7.41 (s, 1H), 7.30 (m, 1H), 6.73 (d, 1H), 4.88 (dd, 1H), 4.67 (s, 1H), 3.08 (m, 1H), 2.41 (d, 1H), 1.16 (d, 3H).

### Example 133

### (11S,21R,S)-3-Decladinosyl-11,12-dideoxy-2-fluoro-6-O-methyl-3-oxo-12,11-[oxycarbonyl-(2-(quinoxalin-2-ylsulfanyl)-acetamido)-methylene]-erythromycin A

To a solution of (quinoxalin-2-ylsulfanyl)-acetic acid (0.056g) in anhydrous DMF (4mL) under a nitrogen atmosphere HATU (0.097g) and DIPEA (0.053mL) were added. The reaction mixture was stirred at room temperature for 20 min then example **11** (0.160g) was added. The reaction mixture was stirred at room temperature for 6h then it was diluted with DCM (10mL) and washed with a 5% NaHCO₃ aqueous solution (10mL). The aqueous phase was extracted with DCM (3x10mL), the combined organic layers washed with a 5% NaHCO₃ aqueous solution (10mL), dried over Na₂SO₄ and evaporated under reduced pressure. The residue was dissolved in MeOH (5mL) and stirred at room temperature overnight. After evaporating the solvent the crude material was purified by flash chromatography (DCM\MeOH 95\5) to give the title compound (0.126g).
m\z ([MH]⁺) = 847
¹H-NMR (CDCl₃) δ: 8.63 (s, 1H), 8.20 (d, 1H), 7.99 (m, 2H), 7.72 (t, 1H), 7.65 (t, 1H), 5.47 (dd, 1H), 4.36 (d, 1H), 3.98 (s, 1H), 3.92 (d, 1H), 3.51 (m, 1H), 3.16 (m, 1H), 3.02 (m, 1H), 2.88 (m, 1H), 2.64 (s, 3H), 2.58 (m, 1H), 2.36 (m, 1H), 2.34 (s, 6H₂), 2.10 (m, 1H), 2.03 (m, 1H), 1.97 (m, 1H), 1.73 (m, 4H), 1.60-1.40 (m, 8H), 1.23 (m, 4H), 1.15-1.01 (m, 9H), 0.90 (t, 3H).

### Example 134

### (11S,21R,S)-3-Decladinosyl-11,12-dideoxy-2-fluoro-6-O-methyl-3-oxo-12,11-[oxycarbonyl-(4-(2-hydroxy-4,5-dimethoxy-phenyl)-4-oxo-butyramido)-methylene]-erythromycin A

To a solution of 4-(2-hydroxy-4,5-dimethoxy-phenyl)-4-oxo-butyric acid (0.031g) in anhydrous DMF (2mL) HATU (0.047g) and DIPEA (0.025mL) were added under nitrogen atmosphere. After stirring for 30min example **10** (0.070g) was added and the mixture stirred overnight. A 5% NaHCO₃ aqueous solution (3mL) was added and the mixture extracted with DCM (2x3mL). The organic phase was dried over Na₂SO₄ and evaporated under reduced pressure. The residue was dissolved in MeOH (3mL) and stirred overnight. After solvent evaporation the crude material was purified by flash chromatography (eluting with: DCM\MeOH\NH40H from 100\0\0 to 85\15\0.2) to give the title compound (0.027g).
¹H-NMR (CDCl₃) δ:12.46 (s, 1H), 7.15 (s, 1H), 6.72 (d, 1H), 6.44 (s, 1H), 4.44 (m, 1H), 3.91 (s, 3H), 3.34 (s, 3H), 3.34 (m, 2H), 3.04 (m, 1H), 2.70-2.52 (m, 2H), 2.34 (m, 1H), 1.80 (d, 3H), 1.17 (d, 3H).

### Example 135

### (11S,21R,S)-3-Decladinosyl-11,12-dideoxy-2-fluoro-6-O-methyl-3-oxo-12,11-[oxycarbonyl-(4-(3,4-dimethoxy-phenyl)-4-oxo-butyramido)-methylene]-erythromycin A

To a solution of 4-(3,4-dimethoxy-phenyl)-4-oxo-butyric acid (0.029g) in anhydrous DMF (2mL) HATU (0.047g) and DIPEA (0.025mL) were added under nitrogen atmosphere. After stirring for 30min example **10** (0.070g) was added and the mixture stirred overnight. A 5% NaHCO₃ aqueous solution (3mL) was added and the mixture extracted with DCM (2x3mL). The organic phase was dried over Na₂SO₄ and evaporated under reduced pressure. The residue was dissolved in MeOH (3mL) and stirred overnight. After solvent evaporation the crude material was purified by flash chromatography (eluting with: DCM\MeOH\NH4OH from 100\0\0 to 85\15\0.2) to give the title compound (0.024g).
¹H-NMR (CDCl₃) δ: 7.64 (d, 1H), 7.53 (m, 1H), 6.89 (d, 1H), 6.77 (d, 1H), 4.40 (t, 1H), 3.96 (s, 3H), 3.94 (s, 3H), 3.35 (m, 2H), 3.04 (m, 1H), 2.70-2.55 (m, 2H), 2.34 (m, 1H), 1.81 (d, 3H), 1.17 (d, 3H).

### Example 136

### (11S,21R,S)-3-Decladinosyl-11,12-dideoxy-fluoro-6-O-methyl-3-oxo-12,11-[oxycarbonyl-(4-(4-methoxy-3-nitro-phenyl)4-oxo-butyramido)-methylene]-erythromycin A

To a solution of 4-(4-methoxy-3-nitro-phenyl)-4-oxo-butyric acid (0.031g) in anhydrous DMF (2mL) HATU (0.047g) and DIPEA (0.025mL) were added under nitrogen atmosphere. After stirring for 30min example **10** (0.070g) was added and the mixture stirred overnight. A 5% NaHCO₃ aqueous solution (3mL) was added and the mixture extracted with DCM (2x3mL). The organic phase was dried over Na₂SO₄ and evaporated under reduced pressure. The residue was dissolved in MeOH (3mL) and stirred overnight. After solvent evaporation the crude material was purified by flash chromatography (eluting with: DCM\MeOH\NH40H from 100\0\0 to 85\15\0.2) to give the title compound (0.030g).
¹H-NMR (CDCl₃) δ: 8.49 (d, 1H), 8.20 (dd, 1H), 7.15 (d, 1H), 6.66 (d, 1H), 4.40 (t, 1H), 4.04 (s, 3H), 3.31 (m, 2H), 2.99 (m, 1H), 2.73 (m, 1H), 2.61 (m, 1H), 2.18 (m, 1H), 1.18 (d, 3H).

### Example 137

### (11S,21R,S)-3-Decladinosyl-11,12-dideoxy-2-fluoro-6-O-methyl-3-oxo-12,11-[oxycarbonyl-(2-(4-(pyridin-3-yl)-1H-imidazol-1-yl)-ethylamino)-methylene]-erythromycin A

A solution of intermediate **51** (0.070g) in acetonitrile (2.5mL) and a 3M HCl aqueous solution (2.5mL) was heated to 80°C for 24h. The reaction mixture was allowed to reach room temperature, then it was added dropwise to a solution of example **10** (0.070g) dissolved in anhydrous acetonitrile (2mL) keeping the pH of the solution in the range 6-7 by addition of a saturated NaHCO₃ aqueous solution. The mixture was stirred at room temperature overnight. Acetic acid was added to reach pH 5-6 followed by sodium cyanoborohydride (1M in THF, 0.150mL). The reaction mixture was stirred overnight at room temperature. After evaporating the solvent a saturated NaHCO₃ aqueous solution (3mL) was added and the mixture was extracted with DCM (2x5mL). The organic layer was dried over Na₂SO₄ and concentrated under vacuum. The crude material was purified by flash chromatography (eluting with DCM\MeOH\NH₄OH from 100\0\0 to 80\20\0.2) to give the title compound (0.006g).
¹H-NMR (CDCl₃) δ: 9.05 (d, 1H), 8.44 (d, 1H), 8.13 (m, 1H), 7.65 (d, 1H), 7.57 (d, 1H), 7.27 (m, 2H), 4.20-4.10 (m, 2H), 4.07 (bs, 1H), 3.40-3.25 (m, 2H), 2.87 (m, 1H), 2.32 (m, 1H), 1.81 (d, 3H), 1.08 (d, 3H).

### Example 138

### (11S,21R,S)-3-Decladinosyl-11,12-dideoxy-2-fluoro-6-O-methyl-3-oxo-12,11-[oxycarbonyl-(3-(4-(pyridin-3-yl)-imidazol-1-yl)-propylamino)-methylene]-erythromycin A

A solution of example **10** (0.070g) and intermediate **52** (0.035g) in anhydrous acetonitrile (3.5mL) was stirred at room temperature for 6h. After evaporating the solvent the residue was dissolved in anhydrous MeOH (3mL) and sodium cyanoborohydride (1M in THF, 0.051mL) and acetic acid (0.008mL) were added. The mixture was stirred overnight. The solvent was evaporated under vacuum, the residue dissolved in DCM (10mL) and washed with a saturated NaHCO₃ aqueous solution (2x10mL). The organic layer was dried over Na₂SO₄ and concentrated under reduced pressure. The crude material was purified by flash chromatography (eluting with: DCM\MeOH\NH₄OH from 100\0\0 to 80\20\0.2) to give the title compound (0.012g).
m\z ([MH]⁺) = 830.
¹H-NMR (CDCl₃) δ: 8.99 (d, 1H), 8.46 (dd, 1H), 8.11 (dd, 1H), 7.63 (s, 1H), 7.40 (s, 1H), 7.30 (m, 1H), 5.45 (dd, 1H), 4.42 (d, 1H), 4.18 (m, 1H), 4.08 (m, 1H), 4.04 (m, 1H), 3.55 (m, 1H), 3.18 (dd, 1H), 3.11 (m, 1H), 3.00 (m, 1H), 2.92 (m, 1H), 2.95 (s, 3H), 2.95 (m, 1H), 2.58 (m, 1H), 2.52 (m, 1H), 2.30 (s, 6H), 1.95 (m, 2H), 1.83 (d, 3H), 1.57 (d, 3H), 1.7 (m, 1H), 1.68 (m, 1H), 1.60 (m, 1H), 1.33 (s, 3H), 1.26 (s, 3H), 1.25 (m, 1H),), 1.25 (d, 3H), 1.16 (d, 3H), 1.09 (d, 3H), 0.92 (t, 3H).

### Example 139

### (11S,21R,S)-3-Decladinosyl-11,12-dideoxy-6-O-methyl-3-oxo-12,11-[oxycarbonyl-((2-methyl-6-nitrophenyl)-ureido)-2-ethylamino)-methylene]-erythromycin A

To a solution of example **13** (0.039g) in anhydrous DCM (2mL) cooled to -10°C a solution of 6-methyl-2-nitroisocyanate (0.010g) in anhydrous DCM (2mL) was added and the mixture was stirred at -10°C for 2h. The reaction mixture was quenched with a saturated NaHCO₃ aqueous solution (2mL), the aqueous phase was extracted with DCM (2mL). The organic phase was dried over Na₂SO₄ and concentrated under reduced pressure. The crude material was purified by flash chromatography (eluting with: DCM\MeOH from 97\3 to 95\5) to give the title compound (0.010g).
¹H-NMR (CDCl₃) δ: 7.97 (bs, 1H), 7.78 (d, 1H), 7.44 (d, 1H), 7.15 (t, 1H), 6.23 (bs, 1H), 4.22 (s, 1H), 3.45 (m, 1H), 3.26-3.19 (m, 2H), 3.08 (m, 1H), 2.91 (m, 1H), 2.32 (s, 1H), 1.12 (d, 3H).

### Example 142

### (11S,21R,S)-2'-O-Acetyl-3-decladinosyl-11,12-dideoxy-6-O-allyl-3-oxo-12,11-[oxycarbonyl-(benzhydrylideneamino)-methylene]-erythromycin A

To a solution of intermediate **42** (0.528g) and EDC (0.35g) in DCM anhydrous (40mL) cooled to 0°C, DMSO (0.4mL) was added. After 10 min at 0°C, a solution of pyridinium trifluoroacetate (0.36g) in DCM (2mL) was slowly added. After 10min the ice bath was removed. Two further additions of EDC (0.35g each time), DMSO (0.4mL each time) and pyridinium trifluoroacetate (0.36g each time) were performed. The reaction mixture was quenched with water (50mL) and extracted with DCM (3x50mL). The organic layer was dried over Na₂SO₄ and concentrated under vacuum to give the title compound (0.520g).
TLC: DCM\MeOH\NH₃ 20\2\0.2 (Rf=0.39).

### Example 143

### (11S,21R,S)-2'-O-Acetyl-3-decladinosyl-11,12-dideoxy-6-O-allyl-3-oxo-12,11-[oxycarbonyl-(amino)-methylene]-erythromycin A

A solution of example **142** (0.52g) in acetonitrile (66mL) and 1.2N HCl aqueous solution (154mL) was stirred at room temperature for 1h. After neutralising the mixture with solid Na₂CO₃ and evaporating the solvent under vacuum, the mixture was extracted with DCM (3x50mL). The organic layer was dried over Na₂SO₄ and concentrated under reduced pressure to give the title compound (0.47g).
¹H-NMR (CDCl₃) δ: 5.88 (dd, 1H), 5.70 (m, 1H), 5.13 (d, 1H), 4.75 (m, 1H), 4.57 (s, 1H), 4.44 (d, 1H), 4.38 (d, 1H), 3.91 (q, 1H), 3.75 (q, dd), 3.61 (m, 1H), 3.50 (m, 1H), 3.24 (m, 1H), 3.08 (m, 1H), 2.70 (m, 1H), 2.64 (m, 1H), 2.46 (bs, 1H), 2.26 (s, 6H), 2.18 (m, 6H), 1.60-1.40 (m, 5H), 1.40-1.20 (m, 13H), 1.15 (d, 3H), 1.08 (d, 3H), 0.88 (t, 3H).

### Example 144

### (11S,21R,S)-3-Decladinosyl-11,12-dideoxy-6-O-allyl-3-oxo-12,11-[oxycarbonyl-(amino)-methylene]-erythromycin A

A solution of example **143** (0.002g) in MeOH (0.3mL) was stirred at room temperature overnight. Solvent evaporation under reduced pressure gave the title compound (0.002g).
¹H-NMR (CDCl₃) δ: 5.82 (dd, 1H), 5.73 (m, 1H), 5.14 (d, 1H), 5.02 (d, 1H), 4.54 (s, 1H), 4.40 (d, 1H), 4.38 (d, 1H), 3.93 (q, 1H), 3.76 (m, 1H), 3.62 (m, 1H), 3.52 (m, 1H), 3.24 (m, 1H), 3.21 (m, 1H), 3.09 (m, 1H), 2.66 (m, 1H), 2.50 (m, 1H), 2.47 (m, 1H), 2.28 (s, 6H), 1.95 (m, 1H), 1.85 (m, 2H), 1.80-0.80 (several m, 27H).
m\z ([MH]⁺) = 653.

### Example 145

### (11S,21R,S)-3-Decladinosyl-11,12-dideoxy-6-O-methyl-3-oxo-12,11-[oxycarbonyl-(2-(quinolin-2-ylsulfanyl)-ethylamino)-methylene]-erythromycin A

To a solution of quinolin-2-thiol (0.008g) in anhydrous DMF (0.500mL) sodium hydride (1.2mg) was added and the mixture stirred at room temperature for 15min then intermediate **24** (0.025g) was added and the reaction mixture stirred at 60°C for 5h. The reaction mixture was diluted with DCM (3mL) and washed with a saturated NaHCO₃ aqueous solution (1mL). The organic phase was dried over Na₂SO₄ and evaporated under reduced pressure. The crude material was purified by flash chromatography (eluting with: DCM\MeOH 98\2) to give a compound that was dissolved in MeOH (1mL) and stirred at room temperature overnight. Solvent evaporation under reduced pressure gave the title compound (0.010g).
LC\MS analysis (mobile phase: A\B from 90\10 to 10\90 in 10 min, 10\90 for 2 min, mass range 150-1300 amu): retention time: 8.7 min, m\z ([MH]⁺) =815.

### Example 146

### (11S,21R,S)-3-Decladinosyl-11,12-dideoxy-6-O-methyl-3-oxo-12,11-[oxycarbonyl-(2-(benzothiazol-2-ylsulfanyl)-ethylamino)-methylene]-erythromycin A

To a solution of benzothiazole-2-thiol (0.009g) in anhydrous DMF (0.500mL) sodium hydride (1.2mg) was added and the mixture stirred at room temperature for 15min then intermediate **24** (0.025g) was added and the reaction mixture stirred at 60°C for 5h. The reaction mixture was diluted with DCM (3mL), washed with a saturated NaHCO₃ aqueous solution (1mL). The organic phase was dried over Na₂SO₄ and evaporated under reduced pressure. The crude material was purified by flash chromatography (eluting with: DCM\MeOH 98\2) to give a compound that was dissolved in MeOH (1mL) and stirred at room temperature overnight. Solvent evaporation under reduced pressure gave the title compound (0.012g).
LC\MS analysis (mobile phase: A\B from 90\10 to 10\90 in 10 min, 10\90 for 2 min, mass range 150-1300 amu): retention time: 8.5 min, m\z ([MH]⁺) =821.

### Example 147

### 3-Pyridin-3-yl-acrylamide of (11S,21R,S)-3-decladinosyl-11,12-dideoxy-6-O-methyl-3-oxo-12,11-[oxycarbonyl-(amino)-methylene]-erythromycin A

To 3-pyridin-3-yl-acrylic acid (1.3mg) a solution of HATU (0.003g) in anhydrous DMF (0.050mL) and DIPEA (0.002mL) in anhydrous DMF (0.050mL) were added, followed by a solution of example **6** (0.005g) in anhydrous DMF (0.050mL). The reaction mixture was stirred at room temperature for 18 h, then it was diluted with DCM (0.350mL), washed with a 5% NaHCO₃ aqueous solution (0.300mL), then passed through a phase-separation syringe. The aqueous phase was extracted with DCM (0.250mL) and the collected organic extracts evaporated under vacuum. The crude material was dissolved in DCM (0.700mL), loaded on SCX-cartridge (250mg, loading 0.28mmol/g, previously washed with 4mL of MeOH), washed with MeOH (3.5mL), then the product eluted with NH₃ (0.25M solution in MeOH, 1mL), followed by MeOH (0.7mL). The collected fractions were left in the NH₃/MeOH solution overnight. After evaporating the solvent the title compound (0.002g) was obtained. LC/MS analysis (mobile phase: AB from 90/10 to 10/90 in 10 min, 10/90 for 5 min; mass range 150-1200 amu): retention time: 5 min, m\z ([MH]⁺) = 758.

### Examples 148-312:

### Example 148

### 3-Benzo[1,3]dioxol-5-yl-acrylamide of (11S,21R,S)-3-decladinosyl-11,12-dideoxy-6-O-methyl-3-oxo-12,11-[oxycarbonyl-(amino)-methylene]-erythromycin A

### Example 149

### (4-Methyl-2-oxo-2H-chromen-7-yloxy)acetamide of (11S,21R,S)-3-decladinosyl-11,12-dideoxy-6-O-methyl-3-oxo-12,11-[oxycarbonyl-(amino)-methylene]-erythromycin A

### Example 150

### [(Furan-2-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 151

### 3-(Thiophen-2-ylsulfanyl)-propionamide of (11S,21R,S)-3-decladinosyl-11,12-dideoxy-6-O-methyl-3-oxo-12,11-[oxycarbonyl-(amino)-methylene]-erythromycin A

### Example 152

### (Benzo[1,3]dioxol-5-ylamino)-phenyl-acetamide of (11S,21R,S)-3-decladinosyl-11,12-dideoxy-6-O-methyl-3-oxo-12,11-[oxycarbonyl-(amino)-methylene]-erythromycin A

### Example 153

### {[3-(2-Chloro-phenyl)-5-methyl-isoxazole-4-carbonyl]-amino}-acetamide of (11S,21R,S)-3-decladinosyl-11,12-dideoxy-6-O-methyl-3-oxo-12,11-[oxycarbonyl-(amino)-methylene]-erythromycin A

### Example 154

### 2-Acetylamino-3-(6-methyl-1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 155

### 2-Acetylamino-3-(5-methyl-1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 156

### (E)-3-(2,3-Dimethoxy-pyrimidin-5-yl)-acrylamide of (11S,21R,S)-3-decladinosyl-11,12-dideoxy-6-O-methyl-3-oxo-12,11-[oxycarbonyl-(amino)-methylene]-erythromycin A

### Example 157

### [5-(2-Methoxy-phenyl)-4-phenyl-4H-[1,2,4]triazol-3-ylsulfanyl]-acetamide of (11S,21R,S)-3-decladinosyl-11,12-dideoxy-6-O-methyl-3-oxo-12,11-[oxycarbonyl-(amino)-methylene]-erythromycin A

### Example 158

### 2-(4-Methyl-[1,2,3]thiadiazol-5-ylsulfanyl)-propionamide of (11S,21R,S)-3-decladinosyl-11,12-dideoxy-6-O-methyl-3-oxo-12,11-[oxycarbonyl-(amino)-methylene]-erythromycin A

### Example 159

### (7-Methyl-thieno[3,2-d]pyrimidin-4-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 160

### [5-(2-Chloro-phenyl)-Dyrimidin-4-ylsulfanyl]-acetamide of (11S,21R,S)-3-decladinosyl-11,12-dideoxy-6-O-methyl-3-oxo-12,11-[oxycarbonyl-(amino)-methylene]-erythromycin A

### Example 161

### (4-Methyl-5-quinolin-6-yl-4H-[1,2,4]triazol-3-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 162

### [(5-Bromo-furan-2-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 163

### [(Thiophene-2-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 164

### (4-Hydroxy-2-methyl-pyridin-3-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 165

### 3-(1H-Indol-3-yl)-acrylamide of (11S,21R,S)-3-decladinosyl-11,12-dideoxy-6-O-methyl-3-oxo-12,11-[oxycarbonyl-(amino)-methylene]-erythromycin A

### Example 166

### 4-Oxo-4-thiphen-2-yl-butyramide of (11S,21R,S)-3-decladinosyl-11,12-dideoxy-6-O-methyl-3-oxo-12,11-[oxycarbonyl-(amino)-methylene]-erythromycin A

### Example 167

### 4-(4,5-Dimethoxy-2-nitro-phenyl)-4-oxo-butyramide of (11S,21R,S)-3-decladinosyl-11,12-dideoxy-6-O-methyl-3-oxo-12,11-[oxycarbonyl-(amino)-methylene]-erythromycin A

### Example 168

### 4-(2-Methoxy-phenyl)-4-oxo-butyramide of (11S,21R,S)-3-decladinosyl-11,12-dideoxy-6-O-methyl-3-oxo-12,11-[oxycarbonyl-(amino)-methylene]-erythromycin A

### Example 169

### 4-Oxo-4-pyridin-3-yl-butyramide of (11S,21R,S)-3-decladinosyl-11,12-dideoxy-6-O-methyl-3-oxo-12,11-[oxycarbonyl-(amino)-methylene]-erythromycin A

### Example 170

### 4-(4-Methylsulfanyl-phenyl)4-oxo-butyramide of (11S,21R,S)-3-decladinosyl-11,12-dideoxy-6-O-methyl-3-oxo-12,11-[oxycarbonyl-(amino)-methylene]-erythromycin A

### Example 171

### 3-(1H-Imidazol-4-yl)-acrylamide of (11S,21R,S)-3-decladinosyl-11,12-dideoxy-6-O-methyl-3-oxo-12,11-[oxycarbonyl-(amino)-methylene]-erythromycin A

### Example 172

### 4-Thien-2-yl-butyramide of (11S,21R,S)-3-decladinosyl-11,12-dideoxy-6-O-methyl-3-oxo-12,11-[oxycarbonyl-(amino)-methylene]-erythromycin A

### Example 173

### 3-(1H-Indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 174

### (Pyridin-4-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 175

### (Pyrimidin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 176

### [(Pyridine-3-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 177

### (Z)-3-Pyridin-4-yl-acrylamide of (11S,21R,S)-3-decladinosyl-11,12-dideoxy-6-O-methyl-3-oxo-12,11-[oxycarbonyl-(amino)-methylene]-erythromycin A

### Example 178

### (E)-3-Pyridin-4-yl-acrylamide of (11S,21R,S)-3-decladinosyl-11,12-dideoxy-6-O-methyl-3-oxo-12,11-[oxycarbonyl-(amino)-methylene]-erythromycin A

### Example 179

### [2-(6-Methyl-pyridin-2-yl)-1-phenyl-ethylsulfanyl]-acetamide of (11S,21R,S)-3-decladinosyl-11,12-dideoxy-6-O-methyl-3-oxo-12,11-[oxycarbonyl-(amino)-methylene]-erythromycin A

### Example 180

### [(4-Oxo-4H-chromene-2-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 181

### 3-(1,4-Dioxo-3,4-dihydrophthalazin-2(1H)-yl-propionamide of (11S,21R,S)-3-decladinosyl-11,12-dideoxy-6-O-methyl-3-oxo-12,11-[oxycarbonyl-(amino)-methylene]-erythromycin A

### Example 182

### (4-Methyl-[1,2,3]thiadizol-5-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 183

### (Benxothiazol-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 184

### 3-(1H-Imidazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 185

### 3-Pyridin-3-yl-propionamide of (11S,21R,S)-3-decladinosyl-11,12-dideoxy-6-O-methyl-3-oxo-12,11-[oxycarbonyl-(amino)-methylene]-erythromycin A

### Example 186

### [(4-Methoxy-quinoline-2-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 187

### (3-Phenyl-[1,2,4]oxadiazol-5-ylamino)-acetamide of (11S,21R,S)-3-decladinosyl-11,12-dideoxy-6-O-methyl-3-oxo-12,11-[oxycarbonyl-(amino)-methylene]-erythromycin A

### Example 188

### [4-(6-Oxo-1,4,5,6-tetrahydro-pyridazin-3-yl)-phenoxy]-acetamide of (11S,21R,S)-3-decladinosyl-11,12-dideoxy-6-O-methyl-3-oxo-12,11-[oxycarbonyl-(amino)-methylene]-erythromycin A

### Example 189

### 3-(5-Methyl-1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 190

### 4-(2,3-dioxo-2,3-dihydro-1H-indol-5-yl)-butyramide of (11S,21R,S)-3-decladinosyl-11,12-dideoxy-6-O-methyl-3-oxo-12,11-[oxycarbonyl-(amino)-methylene]-erythromycin A

### Example 191

### 3-(1,3,8-Trimethyl-2,4,7-trioxo-1,2,3,4,7,8-hexahydropteridin-6-yl)-propionamide of (11S,21R,S)-3-decladinosyl-11,12-dideoxy-6-O-methyl-3-oxo-12,11-[oxycarbonyl-(amino)-methylene]-erythromycin A

### Example 192

### 3-(4-Oxo-4,7-dihydro-3H-pyrrolo[2,3-d]pyrimidin-5-yl)-propionamide of (11S,21R,S)-3-decladinosyl-11,12-dideoxy-6-O-methyl-3-oxo-12,11-[oxycarbonyl-(amino)-methylene]-erythromycin A

### Example 193

### [4-(1,3-Dimethyl-6-oxo-2-thioxo-2,3,6,9-tetrahydro-1H-purin-8-yl)-phenoxy]-acetamide of (11S,21R,S)-3-decladinosyl-11,12-dideoxy-6-O-methyl-3-oxo-12,11-[oxycarbonyl-(amino)-methylene]-erythromycin A

### Example 194

### 2-Benzoylamino-3-(1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 195

### 3-Phenyl-4-(pyridin-2-ylcarbamoyl)-butyramide of (11S,21R,S)-3-decladinosyl-11,12-dideoxy-6-O-methyl-3-oxo-12,11-[oxycarbonyl-(amino)-methylene]-erythromycin A

### Example 196

### 3-Benzo[1,3]dioxol-5-yl-2-benzoylamino-acrylamide of (11S,21R,S)-3-decladinosyl-11,12-dideoxy-6-O-methyl-3-oxo-12,11-[oxycarbonyl-(amino)-methylene]-erythromycin A

### Example 197

### 3-(3-Phenyl-ureido)-3-thiophen-3yl-propionamide of (11S,21R,S)-3-decladinosyl-11,12-dideoxy-6-O-methyl-3-oxo-12,11-[oxycarbonyl-(amino)-methylene]-erythromycin A

### Example 198

### 3-(Furan-2-yl)-propionamide of (11S,21R,S)-3-decladinosyl-11,12-dideoxy-6-O-methyl-3-oxo-12,11-[oxycarbonyl-(amino)-methylene]-erythromycin A

### Example 199

### 2-Hydroxy-3-(1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 200

### 3-(5-Phenyl-1H-pyrrol-2-yl)-propionamide of (11S,21R,S)-3-decladinosyl-11,12-dideoxy-6-O-methyl-3-oxo-12,11-[oxycarbonyl-(amino)-methylene]-erythromycin A

### Example 201

### 4-Oxo-4-thiophen-2-yl-butyramide of (11S,21R,S)-3-decladinosyl-11,12-dideoxy-6-O-methyl-3-oxo-12,11-[oxycarbonyl-(amino)-methylene]-erythromycin A

### Example 202

### (4-Methyl-pyrimidin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 203

### 4-[2-(1,3-Dioxo-1,3-dihydro-2H-isoindol-2-yl)-phenyl]-butyramide of (11S,21R,S)-3-decladinosyl-11,12-dideoxy-6-O-methyl-3-oxo-12,11-[oxycarbonyl-(amino)-methylene]-erythromycin A

### Example 204

### 4-(2-Methyl-1-oxo-1,2-dihydroisoquinolin-3-yl)-butyramide of (11S,21R,S)-3-decladinosyl-11,12-dideoxy-6-O-methyl-3-oxo-12,11-[oxycarbonyl-(amino)-methylene]-erythromycin A

### Example 205

### 3-[3-(4-Methoxyphenyl)-1,2,4-oxadiazol-5-yl]-propionamide of (11S,21R,S)-3-decladinosyl-11,12-dideoxy-6-O-methyl-3-oxo-12,11-[oxycarbonyl-(amino)-methylene]-erythromycin A

### Example 206

### (4,6-Dimethyl-pyrimidin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 207

### 3-(2-Oxo-1,3-benzoxazol-3(2H)-yl)-propionamide of (11S,21R,S)-3-decladinosyl-11,12-dideoxy-6-O-methyl-3-oxo-12,11-[oxycarbonyl-(amino)-methylene]-erythromycin A

### Example 208

### 4-(1,3-Dimethyl-2,6-dioxo-2,3,6,7-tetrahydro-1H-purin-8-yl)-butyramide of (11S,21R,S)-3-decladinosyl-11,12-dideoxy-6-O-methyl-3-oxo-12,11-[oxycarbonyl-(amino)-methylene]-erythromycin A

### Example 209

### 2-Formylamino-2-(1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 210

### 4-Methyl-2-[(2-methylsulfanyl-pyridine-3-carbonyl)-amino]-pentanamide of (11S,21R,S)-3-decladinosyl-11,12-dideoxy-6-O-methyl-3-oxo-12,11-[oxycarbonyl-(amino)-methylene]-erythromycin A

### Example 211

### (3,5,6-Trichloro-pyridin-2-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 212

### (5-Phenyl-pyrimidin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 213

### 3-(6-Bromo-benzo[1,3]dioxol-5-yl)-2-cyano-acrylamide of (11S,21R,S)-3-decladinosyl-11,12-dideoxy-6-O-methyl-3-oxo-12,11-[oxycarbonyl-(amino)-methylene]-erythromycin A

### Example 214

### 3-[3-(4-Nitrophenyl)-1,2,4-oxadiazol-5-yl]-propionamide of (11S,21R,S)-3-decladinosyl-11,12-dideoxy-6-O-methyl-3-oxo-12,11-[oxycarbonyl-(amino)-methylene]-erythromycin A

### Example 215

### 3-(1,3-Benzothiazol-2-yl)-propionamide of (11S,21R,S)-3-decladinosyl-11,12-dideoxy-6-O-methyl-3-oxo-12,11-[oxycarbonyl-(amino)-methylene]-erythromycin A

### Example 216

### 3-(3-Pyridin-2-yl-1,2,4-oxadiazol-5-yl)-propionamide of (11S,21R,S)-3-decladinosyl-11,12-dideoxy-6-O-methyl-3-oxo-12,11-[oxycarbonyl-(amino)-methylene]-erythromycin A

### Example 217

### 4-(3-Pyridin-4-yl-1,2,4-oxadiazol-5-yl)-butyramide of (11S,21R,S)-3-decladinosyl-11,12-dideoxy-6-O-methyl-3-oxo-12,11-[oxycarbonyl-(amino)-methylene]-erythromycin A

### Example 218

### 4-(3-Pyridin-2-yl-1,2,4-oxadiazol-5-yl)-butyramide of (11S,21R,S)-3-decladinosyl-11,12-dideoxy-6-O-methyl-3-oxo-12,11-[oxycarbonyl-(amino)-methylene]-erythromycin A

### Example 219

### 3-[3-(4-Chlorophenyl)-1,2,4-oxadiazol-5-yl]-pronionamide of (11S,21R,S)-3-decladinosyl-11,12-dideoxy-6-O-methyl-3-oxo-12,11-[oxycarbonyl-(amino)-methylene]-erythromycin A

### Example 220

### 4-[3-(4-Chlorophenyl)-1,2,4-oxadiazol-5-yl]-butyramide of (11S,21R,S)-3-decladinosyl-11,12-dideoxy-6-O-methyl-3-oxo-12,11-[oxycarbonyl-(amino)-methylene]-erythromycin A

### Example 221

### 4-(1,3-Benzodioxol-5-yl)-butyramide of (11S,21R,S)-3-decladinosyl-11,12-dideoxy-6-O-methyl-3-oxo-12,11-[oxycarbonyl-(amino)-methylene]-erythromycin A

### Example 222

### 4-[3-(5-Oxo-2,3-dihydro-5H-[1,3]thiazolo[3,2-a]pyrimidin-6-yl)-1,2,4-oxadiazol-5-yl]-butyramide of (11S,21R,S)-3-decladinosyl-11,12-dideoxy-6-O-methyl-3-oxo-12,11-[oxycarbonyl-(amino)-methylene]-erythromycin A

### Example 223

### 4-[3-(3-Nitrophenyl)-1,2,4-oxadiazol-5-yl]-butyramide of (11S,21R,S)-3-decladinosyl-11,12-dideoxy-6-O-methyl-3-oxo-12,11-[oxycarbonyl-(amino)-methylene]-erythromycin A

### Example 224

### 3-Pyrimidin-2-yl-propionamide of (11S,21R,S)-3-decladinosyl-11,12-dideoxy-6-O-methyl-3-oxo-12,11-[oxycarbonyl-(amino)-methylene]-erythromycin A

### Example 225

### 4-(2,3-Dihydro-1,4-benzodioxin-6-yl)-butyramide of (11S,21R,S)-3-decladinosyl-11,12-dideoxy-6-O-methyl-3-oxo-12,11-[oxycarbonyl-(amino)-methylene]-erythromycin A

### Example 226

### 3-[3-Chloro-5-(trifluoromethyl)pyridin-2-yl]-propionamide of (11S,21R,S)-3-decladinosyl-11,12-dideoxy-6-O-methyl-3-oxo-12,11-[oxycarbonyl-(amino)-methylene]-erythromycin A

### Example 227

### 4-(1H-Indol-3-yl)-butyramide of (11S,21R,S)-3-decladinosyl-11,12-dideoxy-6-O-methyl-3-oxo-12,11-[oxycarbonyl-(amino)-methylene]-erythromycin A

### Example 228

### (5-Trifluoromethyl-pyridin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 229

### (Quinolin-8-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 230

### 3-(Quinoxalin-2-ylsulfanyl)-propionamide of (11S,21R,S)-3-decladinosyl-11,12-dideoxy-6-O-methyl-3-oxo-12,11-[oxycarbonyl-(amino)-methylene]-erythromycin A

### Example 231

### (2-Pyridin-2-yl-6-trifluoromethyl-pyrimidin-4-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 232

### 3-(3-Chloro-5-trifluoromethyl-pyridin2-yl)-acrylamide of (11S,21R,S)-3-decladinosyl-11,12-dideoxy-6-O-methyl-3-oxo-12,11-[oxycarbonyl-(amino)-methylene]-erythromycin A

### Example 233

### 3-Pyridin-2-yl-acrylamide of (11S,21R,S)-3-decladinosyl-11,12-dideoxy-6-O-methyl-3-oxo-12,11-[oxycarbonyl-(amino)-methylene]-erythromycin A

### Example 234

### (2,2-Dimethyl-4-oxo-chroman-7-yloxy)-acetamide of (11S,21R,S)-3-decladinosyl-11,12-dideoxy-6-4-methyl-3-oxo-12,11-[oxycarbonyl-(amino)-methylene]-erythromycin A

### Example 235

### [3-(5-Oxo-2,3-dihydro-5H-thiazolo[3,2-a]pyrimidin-6-yl)-[1,2,4]oxadiazol-5-ylmethylsulfanyl]-acetamide of (11S,21R,S)-3-decladinosyl-11,12-dideoxy-6-O-methyl-3-oxo-12,11-[oxycarbonyl-(amino)-methylene]-erythromycin A

### Example 236

### (5,6,7,8-Tetrahydro-quinazolin-4-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 237

### Benzo[1,3]dioxol-5-yl-propionamide of (11S,21R,S)-3-decladinosyl-11,12-dideoxy-6-O-methyl-3-oxo-12,11-[oxycarbonyl-(amino)-methylene]-erythromycin A

### Example 238

### [5-(5-Nitro-furan-2-yl)-[1,3,4]oxadiazol-2-ylsulfanyl]-acetamide of (11S,21R,S)-3-decladinosyl-11,12-dideoxy-6-O-methyl-3-oxo-12,11-[oxycarbonyl-(amino)-methylene]-erythromycin A

### Example 239

### (Pyridin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 240

### [(2-Phenoxy-pyridine-3-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 241

### 3-Benzo[1,3]dioxol-5-yl-2-cyano-acrylamide of (11S,21R,S)-3-decladinosyl-11,12-dideoxy-6-O-methyl-3-oxo-12,11-[oxycarbonyl-(amino)-methylene]-erythromycin A

### Example 242

### (Benzenesulfonyl-pyridin-2-yl-amino)-acetamide of (11S,21R,S)-3-decladinosyl-11,12-dideoxy-6-O-methyl-3-oxo-12,11-[oxycarbonyl-(amino)-methylene]-erythromycin A

### Example 243

### (3-Chloro-4-methyl-2-oxo-2H-chromen-7-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 244

### (5-Bromo-4-hydroxy-2-methyl-pyridin-3-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 245

### (4-Methyl-4H-[1,2,4]triazol-3-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 246

### (2,2,5-Trimethyl-4-oxo-chroman-7-yloxy)-acetamide of (11S,21R,S)-3-decladinosyl-11,12-dideoxy-6-O-methyl-3-oxo-12,11-[oxycarbonyl-(amino)-methylene]-erythromycin A

### Example 247

### 3-(1-tert-Butyl-3,5-dimethyl-1H-pyrazol-4-yl)-acrylamide of (11S,21R,S)-3-decladinosyl-11,12-dideoxy-6-O-methyl-3-oxo-12,11-[oxycarbonyl-(amino)-methylene]-erythromycin A

### Example 248

### (1H-Indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 249

### Thien-3-yl-acetamide of (11S,21R,S)-3-decladinosyl-11,12-dideoxy-6-O-methyl-3-oxo-12,11-[oxycarbonyl-(amino)-methylene]-erythromycin A

### Example 250

### (2-Phenyl-1,3-thiazol-4-yl)-acetamide of (11S,21R,S)-3-decladinosyl-11,12-dideoxy-6-O-methyl-3-Oxo-12,11-[oxycarbonyl-(amino)-methylene]-erythromycin A

### Example 251

### (1H-Indol-3-yl)-oxo-acetamide of (11S,21R,S)-3-decladinosyl-11,12-dideoxy-6-O-methyl-3-oxo-12,11-[oxycarbonyl-(amino)-methylene]-erythromycin A

### Example 252

### Thien-2-yl-acetamide of (11S,21R,S)-3-decladinosyl-11,12-dideoxy-6-O-methyl-3-oxo-12,11-[oxycarbonyl-(amino)-methylene]-erythromycin A

### Example 253

### 1H-Imidazol-4-yl-acetamide of (11S,21R,S)-3-decladinosyl-11,12-dideoxy-6-O-methyl-3-oxo-12,11-[oxycarbonyl-(amino)-methylene]-erythromycin A

### Example 254

### 1,3-Benzodioxol-5-y-acetamide of (11S,21R,S)-3-decladinosyl-11,12-dideoxy-6-O-methyl-3-oxo-12,11-[oxycarbonyl-(amino)-methylene]-erythromycin A

### Example 255

### (2-Pyrazin-2-yl-1,3-thiazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 256

### (5-Bromo-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 257

### 1-Benzothien-3-yl-acetamide of (11S,21R,S)-3-decladinosyl-11,12-dideoxy-6-O-methyl-3-oxo-12,11-[oxycarbonyl-(amino)-methylene]-erythromycin A

### Example 258

### Pyridin-2-yl-acetamide of (11S,21R,S)-3-decladinosyl-11,12-dideoxy-6-O-methyl-3-oxo-12,11-[oxycarbonyl-(amino)-methylene]-erythromycin A

### Example 259

### (1-Methyl-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 260

### (5-Fluoro-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 261

### (5-Methoxy-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 262

### (4-Oxo-3,4-dihydrophthalazin-1-yl)-acetamide of (11S,21R,S)-3-decladinosyl-11,12-dideoxy-6-O-methyl-3-oxo-12,11-[oxycarbonyl-(amino)-methylene]-erythromycin A

### Example 263

### (5-Methyl-2,4-dioxo-3,4-dihydropyrimidin-1(2H)-yl)-acetamide of (11S,21R,S)-3-decladinosyl-11,12-dideoxy-6-O-methyl-3-oxo-12,11-[oxycarbonyl-(amino)-methylene]-erythromycin A

### Example 264

### (1,5-Dimethyl-3-oxo-2-phenyl-2,3-dihydro-1H-pyrazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 265

### (5-Methoxy-2-methyl-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 266

### (2-methyl-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 267

### (5-Methyl-2-phenyl-1,3-oxazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 268

### (6-Hydroxy-pyridazin-3-yl)-(4-methoxy-phenyl)-acetamide of (11S,21R,S)-3-decladinosyl-11,12-dideoxy-6-O-methyl-3-oxo-12,11-[oxycarbonyl-(amino)-methylene]-erythromycin A

### Example 269

### (5-Methyl-1-phenyl-1H-pyrazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 270

### (5-Methyl-2-phenyl-1,3-thiazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 271

### Pyridin-3-yl-acetamide of (11S,21R,S)-3-decladinosyl-11,12-dideoxy-6-O-methyl-3-oxo-12,11-[oxycarbonyl-(amino)-methylene]-erythromycin A

### Example 272

### (5-Hydroxy-1H-indol-3-yl)-acetamide of (11S,21R,S)-3-decladinosyl-11,12-dideoxy-6-O-methyl-3-oxo-12,11-[oxycarbonyl-(amino)-methylene]-erythromycin A

### Example 273

### 1-Benzothien-4-yl-acetamide of (11S,21R,S)-3-decladinosyl-11,12-dideoxy-6-O-methyl-3-oxo-12,11-[oxycarbonyl-(amino)-methylene]-erythromycin A

### Example 274

### 2-Furyl-acetamide of (11S,21R,S)-3-decladinosyl-11,12-dideoxy-6-O-methyl-3-oxo-12,11-[oxycarbonyl-(amino)methylene]-erythromycin A

### Example 275

### (2,3-Dimethyl-1H-indol-5-yl)-acetamide of (11S,21R,S)-3-decladinosyl-11,12-dideoxy-6-O-methyl-3-oxo-12,11-[oxycarbonyl-(amino)-methylene]-erythromycin A

### Example 276

### 4-(1,3-Benzothiazol-2-yl)-butyramide of (11S,21R,S)-3-decladinosyl-11,12-dideoxy-6-O-methyl-3-oxo-12,11-[oxycarbonyl-(amino)-methylene]-erythromycin A

### Example 277

### 3-(2-Methyl-1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 278

### 4-[3-(5-Nitrothien-3-yl)-1,2,4-oxadiazol-5-yl]-butyramide of (11S,21R,S)-3-decladinosyl-11,12-dideoxy-6-O-methyl-3-oxo-12,11-[oxycarbonyl-(amino)-methylene]-erythromycin A

### Example 279

### 3-(1-Methyl-1H-benzimidazol-2-yl)-propionamide of (11S,21R,S)-3-decladinosyl-11,12-dideoxy-6-O-methyl-3-oxo-12,11-[oxycarbonyl-(amino)-methylene]-erythromycin A

### Example 280

### 3-(4,6-Dimethoxypyrimidin-2-yl)-propionamide of (11S,21R,S)-3-decladinosyl-11,12-dideoxy-6-O-methyl-3-oxo-12,11-[oxycarbonyl-(amino)-methylene]-erythromycin A

### Example 281

### (6,7-Dmethoxy-isoquinolin-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 282

### [3-(2-Chlorophenyl)-5-methyl-isoxazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 283

### [4-(4-Oxo-1,2,3-benzotriazin-3(4H)-yl)phenyl]-acetamide of (11S,21R,S)-3-decladinosyl-11,12-dideoxy-6-O-methyl-3-oxo-12,11-[oxycarbonyl-(amino)-methylene]-erythromycin A

### Example 284

### [2-(2,4-Difluorophenyl)-1,3-thiazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 285

### [2-({[(5-Methyl-1,3,4-thiadiazol-2-yl)thio]acetyl}amino)-1,3-thiazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 286

### (2-{[(Pyridin-2-ylthio)acetyl]amino}-1,3-thiazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 287

### (2-{[(Phenylthio)acetyl]amino}-1,3-thiazol-4-yl)-acetamide of (11S,21R,S)-3-decladinosyl-11,12-dideoxy-6-O-methyl-3-oxo-12,11-[oxycarbonyl-(amino)-methylene]-erythromycin A

### Example 288

### {2-[(4-Bromobenzoyl)amino]-1,3-thiazol-4-yl}acetamide of (11S,21R,S)-3-decladinosyl-11,12-dideoxy-6-O-methyl-3-oxo-12,11-[oxycarbonyl-(amino)-methylene]-erythromycin A

### Example 289

### {2-[(3-Chlorobenzoyl)amino]-1,3-thiazol-4-yl}-acetamide of (11S,21R,S)-3-decladinosyl-11,12-dideoxy-6-O-methyl-3-oxo-12,11-[oxycarbonyl-(amino)-methylene]-erythromycin A

### Example 290

### {2-[(2-Chlorobenzoyl)amino]-1,3-thiazol-4-yl}-acetamide of (11S,21R,S)-3-decladinosyl-11,12-dideoxy-6-O-methyl-3-oxo-12,11-[oxycarbonyl-(amino)-methylene]-erythromycin A

### Example 291

### [1-(6-Chloropyridazin-3-yl)-1H-indol-3-yl]-acetamide of (11S,21R,S)-3-decladinosyl-11,12-dideoxy-6-O-methyl-3-oxo-12,11-[oxycarbonyl-(amino)-methylene]-erythromycin A

### Example 292

### [2-(4-Chlorophenyl)-1,3-thiazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 293

### [4-(4-Bromophenyl)-5-oxo-4,5-dihydro-1H-1,2,4-triazol-1-yl]-acetamide of (11S,21R,S)-3-decladinosyl-11,12-dideoxy-6-O-methyl-3-oxo-12,11-[oxycarbonyl-(amino)-methylene]-erythromycin A

### Example 294

### (2-Oxo-1,3-benzoxazol-3(2H)-yl)-acetamide of (11S,21R,S)-3-decladinosyl-11,12-dideoxy-6-O-methyl-3-oxo-12,11-[oxycarbonyl-(amino)-methylene]-erythromycin A

### Example 295

### [2-(4-Methoxyphenyl)-1,3-thiazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 296

### 3-Furyl-acetamide of (11S,21R,S)-3-decladinosyl-11,12-dideoxy-6-O-methyl-3-oxo-12,11-[oxycarbonyl-(amino)-methylene]-erythromycin A

### Example 297

### (4-Methyl-2-thioxo-1,3-thiazol-3(2H)-yl)-acetamide of (11S,21R,S)-3-decladinosyl-11,12-dideoxy-6-O-methyl-3-oxo-12,11-[oxycarbonyl-(amino)-methylene]-erythromycin A

### Example 298

### [2-(Benzoylamino)-1,3-thiazol-4-yl]-acetamide of (11S,21R,S)-3-decladinosyl-11,12-dideoxy-6-O-methyl-3-oxo-12,11-[oxycarbonyl-(amino)-methylene]-erythromycin A

### Example 299

### [4-(3,5-Dimethyl-1H-pyrazol-1-yl)phenyl]-acetamide of (11S,21R,S)-3-decladinosyl-11,12-dideoxy-6-O-methyl-3-oxo-12,11-[oxycarbonyl-(amino)-methylene]-erythromycin A

### Example 300

### [4-(1H-Pyrazol-1-yl)phenyl]-acetamide of (11S,21R,S)-3-decladinosyl-11,12-dideoxy-6-O-methyl-3-oxo-12,11-[oxycarbonyl-(amino)-methylene]-erythromycin A

### Example 301

### 1-Benzofuran-4-yl-acetamide of (11S,21R,S)-3-decladinosyl-11,12-dideoxy-6-O-methyl-3-oxo-12,11-[oxycarbonyl-(amino)-methylene]-erythromycin A

### Example 302

### 2-Acetylamino-3-(1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

### Example 303

### 3-(1,3-Benzodioxol-5-yl)-propionamide of (11S,21R,S)-3-decladinosyl-11,12-dideoxy-6-O-methyl-3-oxo-12,11-[oxycarbonyl-(amino)-methylene]-erythromycin A

### Example 304

### 3-(1H-Benzimidazol-2-yl)-pronionamide of (11S,21R,S)-3-decladinosyl-11,12-dideoxy-6-O-methyl-3-oxo-12,11-[oxycarbonyl-(amino)-methylene]-erythromycin A

### Example 305

### 3-(6-Ethylsulfanyl-pyridin-3-yl)-acrylamide of (11S,21R,S)-3-decladinosyl-11,12-dideoxy-6-O-methyl-3-oxo-12,11-[oxycarbonyl-(amino)-methylene]-erythromycin A

### Example 306

### [4-Oxo-2-(1H-tetrazol-5-yl)-4H-chromen-7-yloxy]-acetamide of (11S,21R,S)-3-decladinosyl-11,12-dideoxy-6-O-methyl-3-oxo-12,11-[oxycarbonyl-(amino)-methylene]-erythromycin A

### Example 307

### 3-(1-Oxoisoquinolin-2(1H)-yl)-propionamide of (11S,21R,S)-3-decladinosyl-11,12-dideoxy-6-O-methyl-3-oxo-12,11-[oxycarbonyl-(amino)-methylene]-erythromycin A

### Example 308

### 2-Benzoylamino-3-(1H-imidazol-4-yl)-propionamide of (11S,21R,S)-3-decladinosyl-11,12-dideox-y-6-O-methyl-3-oxo-12,11-[oxycarbonyl-(amino)-methylene]-erythromycin A

### Example 309

### 3-Thien-2-yl-propionamide of (11S,21R,S)-3-decladinosyl-11,12-dideoxy-6-O-methyl-3-oxo-12,11-[oxycarbonyl-(amino)-methylene]-erythromycin A

### Example 310

### (3-Methyl-6-trifluoromethyl-3H-imidazo[4,5-b]pyridin-2-ylsulfanyl)-acetamide of (11S,21R,S)-3-decladinosyl-11,12-dideoxy-6-O-methyl-3-oxo-12,11-[oxycarbonyl-(amino)-methylene]-erythromycin A

### Example 311

### [(2-p-Tolysulfanyl-pyridine-3-carbonyl)-amino]-acetamide of (11S,21R,S)-3-decladinosyl-11,12-dideoxy-6-O-methyl-3-oxo-12,11-[oxycarbonyl-(amino)-methylene]-erythromycin A

### Example 312

### 3-[3-(3-nitrophenyl)-1,2,4-oxadiazol-5-yl]-propionamide of (11S,21R,S)-3-decladinosyl-11,12-dideoxy-6-O-methyl-3-oxo-12,11-[oxycarbonyl-(amino)-methylene]-erythromycin A

Examples **148-312** were obtained starting from example **6** (5 mg) by following the same procedure as reported for Example **147**.
The name and amount of starting material (i.e carboxylic acid) and LC/MS analysis (retention time and m/z) of examples **148-312** are reported in the Table 1.

**Table 1**

| **Ex. N** | **Carboxylic acid** | **Amount (mg) (min)** | **Ret. time** | **Mass analysis m/z [MH]**^{**+**} |
|---|---|---|---|---|
| **148** | 3-Benzo[1,3]dioxol-5-yl-acrylic acid | 1.7 | 6.1 | 801 |
| | | | 6.8 | |
| **149** | (4-Methyl-2-oxo-2*H*-chromen-7-yloxy)-acetic acid | 2.1 | 6,7 | 843 |
| **150** | [(Furan-2-carbonyl)-amino]-acetic acid | 1.5 | 4,9 | 778 |
| **151** | 3-(Thiophen-2-ylsulfanyl)-propionic acid | 1.7 | 6,5 | 797 |
| | | | 7,3 | |
| **152** | (Benzo[1,3]dioxol-5-ylamino)-phenyl-acetic acid | 2.4 | 7,0 | 880 |
| | | | 7,4 | |
| **153** | {[3-(2-Chloro-phenyl)-5-methyl-isoxazole-4-carbonyl]-amino}-acetic acid | 2.7 | 6,3 | 903 |
| **154** | 2-Acetylamino-3-(6-methyl-1*H*- indol-3-yl)-propionic acid | 2.3 | 5,9 | 869 |
| **155** | 2-Acetylamino-3-(5-methyl-1*H*-indol-3-yl)-propionic acid | 2.3 | 5,8 | 869 |
| **156** | (*E*)-3-(2,3-Dimethoxy-pyrimidin-5-yl)-acrylic acid | 1.9 | 6,7 | 819 |
| | | | 7,4 | |
| **157** | [5-(2-Methoxy-phenyl)-4-phenyl-4*H*-[1,2,4]triazol-3-ylsulfanyl]-acetic acid | 3.0 | 7,6 | 950 |
| **158** | 2-(4-Methyl-[1,2,3]thiadiazol-5-ylsulfanyl)-propionic acid | 1.8 | 7,3 | 813 |
| | | | 8,0 | |
| **159** | (7-Methyl-thieno[3,2-*d*]pyrimidin-4-ylsulfanyl)-acetic acid | 2.2 | 7,5 | 849 |
| | | | 8,1 | |
| **160** | [5-(2-Chloro-phenyl)-pyrimidin-4-ylsulfanyl]-acetic acid | 2.5 | 7,9 | 889 |
| | | | 8,4 | |
| **161** | (4-Methyl-5-quinolin-6-yl-4*H*-[1,2,4]triazol-3-ylsulfanyl)-acetic acid | 2.6 | 6,3 | 909 |
| **162** | [(5-Bromo-furan-2-carbonyl)-amino]-acetic acid | 2.2 | 6,6 | 856 |
| **163** | [(Thiophene-2-carbonyl)-amino]-acetic acid | 1.6 | 6,3 | 794 |
| **164** | (4-Hydroxy-2-methyl-pyridin-3-yloxy)-acetic acid | 1.6 | 5,2 | 792 |
| **165** | 3-(1*H*-Indol-3-yl)-acrylic acid | 1.7 | 7,3 | 796 |
| | | | 8,1 | |
| **166** | 4-Oxo-4-thiphen-2-yl-butyric acid | 1.7 | 6,6 | 793 |
| | | | 6,6 | |
| **167** | 4-(4,5-Dimethoxy-2-nitro-phenyl)-4-oxo-butyric acid | 2.5 | 6,3 | 892 |
| **168** | 4-(2-Methoxy-phenyl)-4-oxo-butyric acid | 1.9 | 6,3 | 817 |
| **169** | 4-Oxo-4-pyridin-3-yl-butyric acid | 1.6 | 5,0 | 788 |
| | | | 5,4 | |
| **170** | 4-(4-Methylsulfanyl-phenyl)-4-oxo-butyric acid | 2.0 | 6,6 | 833 |
| **171** | 3-(1*H*-Imidazol-4-yl)-acrylic acid | 1,1 | 4,54 | 747 |
| | | | 4,87 | |
| **172** | 4-Thien-2-yl- butyric acid | 1,4 | 6,47 | 779 |
| | | | 7,17 | |
| **173** | 3-(1*H*-Indol-3-yl)-propionic acid | 1,6 | 6,17 | 798 |
| | | | 6,66 | |
| **174** | (Pyridin-4-ylsulfanyl)-acetic acid | 1,4 | 5,02 | 778 |
| | | | 5,50 | |
| **175** | (Pyrimidin-2-ylsulfanyl)-acetic acid | 1,4 | 5,59 | 779 |
| **176** | [(Pyridine-3-carbonyl)-amino]-acetic acid | 1,5 | 4,51 | 789 |
| **177** | (*Z*)-3-Pyridin-4-yl-acrylic acid | 1,2 | 5,05 | 758 |
| | | | 5,64 | |
| **178** | (*E*)-3-Pyridin-4-yl-acrylic acid | 1,2 | 5,02 | 758 |
| | | | 5,62 | |
| **179** | [2-(6-Methyl-pyridin-2-yl)-1-phenyl-ethylsulfanyl]-acetic acid | 2,4 | 6,95 | 896 |
| | | | 7,45 | |
| **180** | [(4-Oxo-4*H*-chromene-2-carbonyl)-amino]-acetic acid | 2,0 | 5,32 | 856 |
| **181** | 3-(1,4-Dioxo-3,4-dihydrophthalazin-2(1*H*)-yl)-propionic acid | 1,9 | 4,54 | 843 |
| **182** | (4-Methyl-[1,2,3]thiadiazol-5-ylsulfanyl)-acetic acid | 1,6 | 5,88 | 799 |
| | | | 6,39 | |
| **183** | (Benxothiazol-2-ylsulfanyl)-acetic acid | 1,8 | 6,81 | 834 |
| | | | 7,17 | |
| **184** | 3-(1*H*-Imidazol-4-yl)-propionic acid | 1,2 | 4,39 | 749 |
| | | | 4,59 | |
| **185** | 3-Pyridin-3-yl-propionic acid | 1,2 | 4,96 | 760 |
| | | | 5,42 | |
| **186** | [(4-Methoxy-quinoline-2-carbonyl)-amino]-acetic acid | 2,1 | 6,4 | 869 |
| **187** | (3-Phenyl-[1,2,4]oxadiazol-5-ylamino)-acetic acid | 1,8 | 6,27 | 827 |
| **188** | [4-(6-Oxo-1,4,5,6-tetrahydro-pyridazin-3-yl)-phenoxy]-acetic acid | 2,0 | 5,32 | 857 |
| **189** | 3-(5-Methyl-1*H*-indol-3-yl)-propionic acid | 1,7 | 6,51 | 812 |
| | | | 7,00 | |
| **190** | 4-(2,3-(dioxo-2,3-dihydro-1*H*-indol-5-yl)-butyric acid | 1,9 | 5,38 | 842 |
| | | | 5,80 | |
| **191** | 3-(1,3,8-Trimethyl-2,4,7-trioxo-1,2,3,4,7,8-hexahydropteridin-6-yl)-propionic acid | 2,4 | 4.60 | 903 |
| | | | 4,92 | |
| **192** | 3-(4-Oxo-4,7-dihydro-3*H*-pyrrolo[2,3-d]pyrimidin-5-yl)-propionic acid | 1,7 | 4,47 | 816 |
| **193** | [4-(1,3-Dimethyl-6-oxo-2-thioxo-2,3,6,9-tetrahydro-1*H*-purin-8-yl)-phenoxy]-acetic acid | 2,8 | 6,56 | 955 |
| | | | 6,88 | |
| **194** | 2-Benzoylamino-3-(1*H*-indol-3-yl)-propionic acid | 2,5 | 6,5 | 917 |
| **195** | 3-Phenyl-4-(pyridin-2-ylcarbamoyl)-butyric acid | 2,3 | 6,31 | 893 |
| | | | 6,62 | |
| **196** | 3-Benzo[1,3]dioxol-5-yl-2-benzoylamino-acrylic acid | 2,6 | 6,53 | 920 |
| **197** | 3-(3-Phenyl-ureido)-3-thiophen-3yl-propionic acid | 2,4 | 6,64 | 899 |
| **198** | 3-(Furan-2-yl)-propionic acid | 1,2 | 5,91 | 749 |
| | | | 6,45 | |
| **199** | 2-Hydroxy-3-(1*H*-indol-3-yl)-propionic acid | 1,7 | 5,96 | 814 |
| **200** | 3-(5-Phenyl-1*H*-pyrrol-2-yl)-propionic acid | 1,8 | 7,13 | 824 |
| | | | 7,48 | |
| **201** | 4-Oxo-4-thiophen-2-yl-butyric acid | 1,5 | 5,86 | 793 |
| | | | 6,42 | |
| **202** | (4-Methyl-pyrimidin-2-ylsulfanyl)-acetic acid | 1,5 | 5,53 | 793 |
| | | | 5,85 | |
| **203** | 4-[2-(1,3-Dioxo-1,3-dihydro-2*H*-isoindol-2-yl)-phenyl]-butyric acid | 2,6 | 6,64 | 918 |
| | | | 7,16 | |
| **204** | 4-(2-Methyl-1-oxo-1,2-dihydroisoquinolin-3-yl)-butyric acid | 2,0 | 5,76 | 854 |
| **205** | 3-[3-(4-Methoxyphenyl)-1,2,4-oxadiazol-5-yl]-propionic acid | 2,0 | 6,51 | 857 |
| | | | 7,10 | |
| **206** | (4,6-Dimethyl-pyrimidin-2-ylsulfanyl)-acetic acid | 1,6 | 5,74 | 807 |
| | | | 6,10 | |
| **207** | 3-(2-Oxo-1,3-benzoxazol-3(2*H*)-yl)-propionic acid | 1,7 | 5,95 | 816 |
| | | | 6,36 | |
| **208** | 4-(1,3-Dimethyl-2,6-dioxo-2,3,6,7-tetrahydro-1*H*-purin-8-yl)-butyric acid | 2,2 | 4,60 | 875 |
| | | | 4,92 | |
| **209** | 2-Formylamino-2-(1*H*-indol-3-yl)-propionic acid | 1,9 | 5,6 | 841 |
| **210** | 4-Methyl-2-[(2-methylsulfanyl-pyridine-3-carbonyl)-amino]-pentanoic acid | 2,3 | 6,60 | 891 |
| | | | 6,92 | |
| **211** | (3,5,6-Trichloro-pyridin-2-yloxy)-acetic acid | 2,1 | 7,41 | 864 |
| | | | 7,81 | |
| **212** | (5-Phenyl-pyrimidin-2-ylsulfanyl)-acetic acid | 2,0 | 6,76 | 855 |
| | | | 7,07 | |
| **213** | 3-(6-Bromo-benzo[1,3]dioxol-5-yl)-2-cyano-acrylic acid | 2,4 | 7,19 | 904 |
| | | | 7,84 | |
| **214** | 3-[3-(4 Nitrophenyl)-1,2,4-oxadiazol-5-yl]-propionic acid | 2,2 | 6,75 | 872 |
| | | | 7,29 | |
| **215** | 3-(1,3-Benzothiazol-2-yl)-propionic acid | 1,7 | 6,09 | 816 |
| | | | 6,56 | |
| **216** | 3-(3-Pyridin-2-yl-1,2,4-oxadiazol-5-yl)-propionic acid | 1,80 | 6,27 | 828 |
| **217** | 4-(3-Pyridin-4-yl-1,2,4-oxadiazol-5-yl)-butyric acid | 19 | 6,57 | 842 |
| | | | 7,23 | |
| **218** | 4-(3-Pyridin-2-yl-1,2,4-oxadiazol-5-yl)-butyric acid | 1,9 | 6,45 | 842 |
| | | | 7,05 | |
| **219** | 3-[3-(4-Chlorophenyl)-1,2,4-oxadiazol-5-yl]-propionic acid | 2,1 | 8,55 | 861 |
| | | | 9,15 | |
| **220** | 4-[3-(4-Chlorophenyl)-1,2,4-oxadiazol-5-yl]-butyric acid | 2,2 | 8,68 | 875 |
| | | | 9,34 | |
| **221** | 4-(1,3-Benzodioxol-5-yl)-butyric acid | 1,7 | 7,71 | 817 |
| | | | 8,43 | |
| **222** | 4-[3-(5-Oxo-2,3-dihydro-5*H*-[1,3]thiazolo[3,2-a]pyrimidin-6-yl)-1,2,4-oxadiazol-5-yl]-butyric acid | 2,5 | 5,97 | 917 |
| | | | 6,51 | |
| **223** | 4-[3-(3-Nitrophenyl)-1,2,4-oxadiazol-5-yl]-butyric acid | 2,3 | 8,07 | 886 |
| | | | 8,73 | |
| **224** | 3-Pyrimidin-2-yl-propionic acid | 1,3 | 5,55 | 761 |
| **225** | 4-(2,3-Dihydro-1,4-benzodioxin-6-yl)-butyric acid | 1,8 | 7,59 | 831 |
| | | | 8,31 | |
| **226** | 3-[3-Chloro-5-(trifluoromethyl)pyridin-2-yl]-propionic acid | 2,1 | 8,13 | 862 |
| | | | 8,78 | |
| **227** | 4-(1*H*-Indol-3-yl)-butyric acid | 1,7 | 7,77 | 812 |
| | | | 8,43 | |
| **228** | (5-Trifluoromethyl-pyridin-2-ylsulfanyl)-acetic acid | 1,9 | 8,06 | 846 |
| | | | 8,43 | |
| **229** | (Quinolin-8-yloxy)-acetic acid | 2,0 | 6,93 | 812 |
| **230** | 3-(Quinoxalin-2-ylsulfanyl)-propionic acid | 1,9 | 7,82 | 843 |
| **231** | (2-Pyridin-2-yl-6-trifluoromethyl-pyrimidin-4-ylsulfanyl)-acetic acid | 2,6 | 7,95 | 924 |
| | | | 8,25 | |
| **232** | 3-(3-Chloro-5-trifluoromethyl-pyridin2-yl)-acrylic acid | 2,1 | 8,31 | 860 |
| | | | 9,27 | |
| **233** | 3-Pyridin-2-yl-acrylic acid | 1,2 | 6,27 | 758 |
| | | | 7,05 | |
| **234** | (2,2-Dimethyl-4-oxo-chroman-7-yloxy)-acetic acid | 2,1 | 7,48 | 859 |
| | | | 7,90 | |
| **235** | [3-(5-Oxo-2,3-dihydro-5*H-*thiazolo[3,2-a]pyrimidin-6-yl)-[1,2,4]oxadiazol-5-ylmethylsulfanyl]-acetic acid | 2,7 | 6,27 | 935 |
| | | | 6,74 | |
| **236** | (5,6,7,8-Tetrahydro-quinazolin-4-ylsulfanyl)-acetic acid | 1,8 | 7,23 | 833 |
| | | | 7,71 | |
| **237** | Benzo[1,3]dioxol-5-yl-propionic acid | 1,6 | 7,65 | 799 |
| | | | 8,37 | |
| **238** | [5-(5-Nitro-furan-2-yl)-[1,3,4]oxadiazol-2-ylsulfanyl]-acetic acid | 2,2 | 7,34 | 880 |
| | | | 7,70 | |
| **239** | (Pyridin-2-ylsulfanyl)-acetic acid | 1,4 | 7,05 | 778 |
| **240** | [(2-Phenoxy-pyridine-3-carbonyl)-amino]-acetic acid | 2,2 | 7,11 | 881 |
| **241** | 3-Benzo[1,3]dioxol-5-yl-2-cyano-acrylic acid | 1,8 | 7,82 | 826 |
| **242** | (Benzenesulfonyl-pyridin-2-yl-amino)-acetic acid | 2,4 | 7,05 | 901 |
| **243** | (3-Chloro-4-methyl-2-oxo-2*H*-chromen-7-yloxy)-acetic acid | 2,2 | 7,95 | 877 |
| | | | 8,36 | |
| **244** | (5-Bromo-4-hydroxy-2-methyl-pyridin-3-yloxy)-acetic acid | 2,2 | 5,67 | 870 |
| **245** | (4-Methyl-4*H*-[1,2,4]triazol-3-ylsulfanyl)-acetic acid | 1,4 | 5,37 | 782 |
| | | | 5,61 | |
| **246** | (2,2,5-Trimethyl-4-oxo-chroman-7-yloxy)-acetic acid | 2,2 | 7,95 | 873 |
| **247** | 3-(1-*tert*-Butyl-3,5-dimethyl-1*H*-pyrazol-4-yl)-acrylic acid | 1,8 | 7,36 | 831 |
| | | | 8,20 | |
| **248** | (1*H*-Indol-3-yl)-acetic acid | 1,4 | 7,17 | 784 |
| **249** | Thien-3-yl-acetic acid | 1,2 | 5,7 | 751 |
| | | | 6,4 | |
| **250** | (2-Phenyl-1,3-thiazol-4-yl)-acetic acid | 1,8 | 6,8 | 828 |
| | | | 7,2 | |
| **251** | (1*H*-Indol-3-yl)-oxo-acetic acid | 1,6 | 6,5 | 798 |
| **252** | Thien-2-yl-acetic acid | 1,2 | 6,0 | 751 |
| | | | 6,7 | |
| **253** | 1*H*-Imidazol-4-yl-acetic acid | 1,3 | 4,3 | 735 |
| **254** | 1,3-Benzodioxol-5-ylacetic acid | 1,5 | 5,9 | 789 |
| **255** | (2-Pyrazin-2-yl-1,3-thiazol-4-yl)-acetic acid | 1,8 | 5,5 | 830 |
| **256** | (5-Bromo-1*H*-indol-3-yl)-acetic acid | 2,1 | 6,5 | 862 |
| | | | 7,0 | |
| **257** | 1-Benzothien-3-yl-acetic acid | 1,6 | 6,8 | 801 |
| **258** | Pyridin-2-yl-acetic acid | 1,4 | 5,1 | 746 |
| **259** | (1-Methyl-1*H*-indol-3-yl)-acetic acid | 1,6 | 6,6 | 798 |
| **260** | (5-Fluoro-1*H*-indol-3-yl)-acetic acid | 1,6 | 6,1 | 802 |
| | | | 6,5 | |
| **261** | (5-Methoxy-1*H*-indol-3-yl)-acetic acid | 1,7 | 5,8 | 814 |
| **262** | (4-Oxo-3,4-dihydrophthalazin-1-yl)-acetic acid | 1,7 | 5 | 813 |
| **263** | (5-Methyl-2,4-dioxo-3,4-dihydropyrimidin-1(2*H*)-yl)-acetic acid | 1,5 | 4,6 | 793 |
| **264** | (1,5-Dimethyl-3-oxo-2-phenyl-2,3-dihydro-1*H*-pyrazol-4-yl)-acetic acid | 2,0 | 5,4 | 855 |
| **265** | (5-Methoxy-2-methyl-1*H*-indol-3-yl)-acetic acid | 1,8 | 6 | 828 |
| **266** | (2-Methyl-1*H*-indol-3-yl)-acetic acid | 1,6 | 6,2 | 798 |
| | | | 6,6 | |
| **267** | (5-Methyl-2-phenyl-1,3-oxazol-4-yl)-acetic acid | 1,8 | 6,7 | 826 |
| **268** | (6-Hydroxy-pyridazin-3-yl)-(4-methoxy-phenyl)-acetic acid | 2,1 | 5,5 | 869 |
| **269** | (5-Methyl-1-phenyl-1*H*-pyrazol-4-yl)-acetic acid | 1,8 | 6,0 | 825 |
| | | | 6,3 | |
| **270** | (5-Methyl-2-phenyl-1,3-thiazol-4-yl)-acetic acid | 1,9 | 7,1 | 842 |
| **271** | Pyridin-3-yl-acetic acid | 1,2 | 4,8 | 746 |
| **272** | (5-Hydroxy-1*H*-indol-3-yl)-acetic acid | 1,6 | 5 | 800 |
| **273** | 1-Benzothien-4-yl-acetic acid | 1,6 | 6,7 | 801 |
| **274** | 2-Furyl-acetic acid | 1,1 | 5,7 | 732 |
| **275** | (2,3-Dimethyl-1*H*-indol-5-yl)-acetic acid | 1,7 | 6,4 | 812 |
| | | | 7,0 | |
| **276** | 4-(1,3-Benzothiazol-2-yl)-butyric acid | 1,8 | 6,4 | 830 |
| | | | 7,0 | |
| **277** | 3-(2-Methyl-1*H*-indol-3-yl)-propionic acid | 1,7 | 6,5 | 812 |
| | | | 7,0 | |
| **278** | 4-[3-(5-Nitrothien-3-yl)-1,2,4-oxadiazol-5-yl] butyric acid | 2,3 | 6,8 | 892 |
| | | | 7,4 | |
| **279** | 3-(1-Methyl-1*H*-benzimidazol-2-yl)-propionic acid | 1,7 | 5,7 | 813 |
| **280** | 3-(4,6-Dimethoxypyrimidin-2-yl)-propionic acid | 1,7 | 5,9 | 821 |
| **281** | (6,7-Dmethoxy-isoquinolin-4-yl)-acetic acid | 2,0 | 5,4 | 856 |
| **282** | [3-(2-Chlorophenyl)-5-methyl-isoxazol-4-yl]-acetic acid | 2,1 | 6,8 | 860 |
| | | | 7,3 | |
| **283** | [4-(4-Oxo-1,2,3-benzotriazin-3(4*H*)-yl)phenyl]-acetic acid | 2,3 | 6,4 | 890 |
| | | | 6,9 | |
| **284** | [2-(2,4-Difluorophenyl)-1,3-thiazol-4-yl]-acetic acid | 2,1 | 7,2 | 864 |
| **285** | [2-({[(5-Methyl-1,3,4-thiadiazol-2-yl)thio]acetyl}amino)-1,3-thiazol-4-yl]-acetic acid | 2,7 | 5,5 | 939 |
| **286** | (2-{[(Pyridin-2-ylthio)acetyl]amino}-1,3-thiazol-4-yl)-acetic acid | 2,5 | 6,1 | 918 |
| **287** | (2-{[(Phenylthio)acetyl]amino}-1,3-thiazol-4-yl)-acetic acid | 2,5 | 6,6 | 917 |
| **288** | {2-[(4-Bromobenzoyl)amino]-1,3-thiazol-4-yl}acetic acid | 2,8 | 6,9 | 949 |
| **289** | {2-[(3-Chlorobenzoyl)amino]-1,3-thiazol-4-yl}-acetic acid | 2,4 | 6,8 | 905 |
| **290** | {2-[(2-Chlorobenzoyl)amino]-1,3-thiazol-4-yl}-acetic acid | 2,4 | 6,3 | 905 |
| **291** | [1-(6-Chloropyridazin-3-yl)-1*H*-indol-3-yl]-acetic acid | 2,4 | 6,9 | 896 |
| **292** | [2-(4-Chlorophenyl)-1,3-thiazol-4-yl]-acetic acid | 2,1 | 7,5 | 862 |
| **293** | [4-(4-Bromophenyl)-5-oxo-4,5-dihydro-1*H*-1,2,4-triazol-1-yl]-acetic acid | 2,4 | 6,3 | 906 |
| **294** | (2-Oxo-1,3-benzoxazol-3(2*H*)-yl)-acetic acid | 1,7 | 6,1 | 802 |
| | | | 6,4 | |
| **295** | [2-(4-Methoxyphenyl)-1,3-thiazol-4-yl]-acetic acid | 2,0 | 6,8 | 858 |
| **296** | 3-Furyl-acetic acid | 1,0 | 5,6 | 735 |
| **297** | (4-Methyl-2-thioxo-1,3-thiazol-3(2*H*)-yl)-acetic acid | 1,6 | 5,8 | 798 |
| **298** | [2-(Benzoylamino)-1,3-thiazol-4-yl]-acetic acid | 2,2 | 6,2 | 871 |
| **299** | [4-(3,5-Dimethyl-1*H*-pyrazol-1-yl)phenyl]-acetic acid | 1,9 | 6,2 | 839 |
| | | | 6,7 | |
| **300** | [4-(1*H*-Pyrazol-1-yl)phenyl]-acetic acid | 1,7 | 5,9 | 811 |
| **301** | 1-Benzofuran-4-yl-acetic acid | 1,4 | 6,5 | 785 |
| **302** | 2-Acetylamino-3-(1*H*-indol-3-yl)-propionic acid | 2,0 | 5,63 | 855 |
| | | | 5,71 | |
| **303** | 3-(1,3-Benzodioxol-5-yl)-propionic acid | 1,6 | 6,28 | 803 |
| | | | 6,76 | |
| **304** | 3-(1*H*-Benzimidazol-2-yl)-propionic acid | 1,6 | 5,55 | 799 |
| **305** | 3-(6-Ethylsulfanyl-pyridin-3-yl)-acrylic acid | 1,7 | 6,55 | 818 |
| | | | 7,34 | |
| **306** | [4-Oxo-2-(1*H*-tetrazol-5-yl)-4*H*-chromen-7-yloxy]-acetic acid | 2,4 | 4,88 | 897 |
| **307** | 3-(1-Oxoisoquinolin-2(1*H*)-yl)-propionic acid | 1,8 | 6,72 | 826 |
| | | | 6,92 | |
| **308** | 2-Benzoylamino-3-(1*H*-imidazol-4-yl)-propionic acid | 2,1 | 5,07 | 868 |
| **309** | 3-Thien-2-yl-propionic acid | 1,3 | 6,30 | 765 |
| | | | 6,89 | |
| **310** | (3-Methyl-6-trifluoromethyl-3*H*-imidazo[4,5-b]pyridin-2-ylsulfanyl)-acetic acid | 2,4 | 7,96 | 900 |
| **311** | [(2-*p*-Tolysulfanyl-pyridine-3-carbonyl)-amino]-acetic acid | 2,5 | 7,57 | 911 |
| | | | 7,84 | |
| **312** | 3-[3-(3-nitophenyl)-1,2,4-oxadiazol-5-yl]-propionic acid | 2,2 | 7,92 | 872 |
| | | | 8,53 | |

### Example 313

### (2R)-2-Amino-3-(1H-indol-3-yl)-propionamide of (11S,21R,S)-3-decladinosyl-11,12-dideoxy-6-O-methyl-3-oxo-12,11-[oxycarbonyl-(amino)-methylene]-erythromycin A

To (2*R*)-*tert*-butoxycarbonylamino-3-(1*H*-indol-3-yl)-propionic acid (0.0025g) a solution of HATU (0.003g) in anhydrous DMF (0.050mL) and DIPEA (0.002mL) in anhydrous DMF (0.050mL) were added, followed by a solution of example **6** (0.005g) in anhydrous DMF (0.050mL). The reaction mixture was stirred at room temperature for 18h, then it was diluted with DCM (0.350mL), washed with a 5% NaHCO₃ aqueous solution (0.300mL), then passed through a phase-separation syringe. The aqueous phase was extracted with DCM (0.250mL) and the collected organic extracts evaporated under vacuum. The residue was dissolved in a 10% TFA solution in anhydrous DCM (0.300mL) and the mixture stirred for 1.5h. The solution was diluted with EtOAc (0.400mL) then solvents evaporated under reduced pressure. The crude material was dissolved in DCM (0.700mL), loaded on SCX-cartridge (100mg, loading 0.75mmol/g, previously washed with 4mL of MeOH), washed with MeOH (4mL), then the product eluted with NH₃ (0.25M solution in MeOH, 1.5mL), followed by MeOH (2mL) and solvents evaporating under vacuum. The residue was dissolved in MeOH (1.7mL) and stirred overnight at room temperature. After evaporating the solvent the title compound (0.001g) was obtained.
LC/MS analysis (mobile phase: A/B from 90/10 to 10/90 in 10 min, 10/90 for 3 min; mass range 150-1000 amu): retention time: 4.95 min, m\z ([MH]⁺) = 813.

### Examples 314-320:

### Example 314

### (2S)-2-Amino-3-(1-methyl-1H-imidazol-5-yl)-propionamide of (11S,21R,S)-3-decladinosyl-11,12-dideoxy-6-O-methyl-3-oxo-12,11-[oxycarbonyl-(amino)-methylene]-erythromycin A

### Example 315

### (2R)-2-Amino-3-(1,3-thiazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 316

### (2S)-2-Amino-3-(1,3-thiazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 317

### (2R)-2-Amino-3-(1H-imidazol-5-yl)-propionamide of (11S,21R,S)-3-decladinosyl-11,12-dideoxy-6-O-methyl-3-oxo-12,11-[oxycarbonyl-(amino)-methylene]-erythromycin A

### Example 318

### (2R)-2-Amino-3-pyridin-3-yl-propionamide of (11S,21R,S)-3-decladinosyl-11,12-dideoxy-6-O-methyl-3-oxo-12,11-[oxycarbonyl-(amino)-methylene]-erythromycin A

### Example 319

### (2S)-2-Amino-3-(1-methyl-1H-imidazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 320

### (2S)-2-Amino-3-pyridin-2-yl-propionamide of (11S,21R,S)-3-decladinosyl-11,12-dideoxy-6-O-methyl-3-oxo-12,11-[oxycarbonyl-(amino)-methylene]-erythromycin A

Examples **314-320** were obtained starting from example **6** (5 mg) by following the same procedure as reported for Example **313**.
The name and amount of starting material (i.e carboxylic acid) and LC/MS analysis (retention time and m/z) of examples **314-320** are reported in the table 2.

**Table 2**

| **Ex. N** | **Carboxylic acid** | **Amount (mg)** | **Ret. time (min)** | **Mass analysis m/z [MH]**^{**+**} |
|---|---|---|---|---|
| **314** | (2*S*)-2-[(*tert*-butoxycarbonyl)amino]-3-(1-methyl-1*H*-imidazol-5-yl)-propionic acid | 2,2 | 5,55 | 778 |
| **315** | (2*R*)-2-[(*tert*-butoxycarbonyl)amino]-3-(1,3-thiazol-4-yl)-propionic acid | 2,2 | 4,05 | 781 |
| **316** | (2*S*)-2-[(*tert*-butoxycarbonyl)amino]-3-(1,3-thiazol-4-yl)-propionic acid | 2,2 | 4,22 | 781 |
| | | | 4,83 | |
| **317** | (2*R*)-2-[(*tert*-butoxycarbonyl)amino]-3-(1*H*-imidazol-5-yl)-propionic acid | 2,1 | 3,51 | 764 |
| | | | 4,16 | |
| **318** | (2*R*)-2-[(*tert*-butoxycarbonyl)amino]-3-pyridin-3-yl-propionic acid | 22 | 3,98 | 775 |
| | | | 4,70 | |
| **319** | (2*S*)-2-[(*tert*-butoxycarbonyl)amino]-3-(1-methyl-1*H*-imidazol-4-yl)-propionic acid | 2,2 | 4,05 | 778 |
| | | | 4,41 | |
| **320** | (2*S*)-2-[(*tert*-butoxycarbonyl)amino]-3-pyridin-2-yl-propionic acid | 2,2 | 4,11 | 775 |
| | | | 4,77 | |

### Example 321

### (2S)-2-Amino-3-pyridin-4-yl-propionamide of (11S,21R,S)-3-decladinosyl-11,12-dideoxy-6-O-methyl-3-oxo-12,11-[oxycarbonyl-(amino)-methylene]-erythromycin A

To (2*S*)-2-{[(9*H*-fluoren-9-ylmethoxy)carbonyl]amino}-3-pyridin-4-yl-propionic acid (0.0032g) a solution of HATU (0.003g) in anhydrous DMF (0.050mL) and DIPEA (0.002mL) in anhydrous DMF (0.050mL) were added, followed by a solution of example **6** (0.005g) in anhydrous DMF (0.050mL). The reaction mixture was stirred at room temperature for 18h, then it was diluted with DCM (0.350mL), washed with a 5% NaHCO₃ aqueous solution (0.300mL), then passed through a phase-separation syringe. The aqueous phase was extracted with DCM (0.250mL) and the collected organic extracts evaporated under vacuum. The residue was dissolved in anhydrous DMF (0.350mL) then piperazinomethyl polystyrene resin (0.030g, loading 1.39 mmol/g) was added and the mixture stirred for 2.5 days. The mixture was filtered and the resin rinsed with DCM (0.400mL), DMF (0.400mL) and DCM (0.200mL) and the filtrates evaporated under reduced pressure.
The crude material was dissolved in DCM (0.700mL), loaded on SCX-cattridge (100mg, loading 0.75mmol/g, previously washed with 4mL of MeOH), washed with MeOH (4mL), then the product eluted with NH₃ (0.25M solution in MeOH, 1.5mL), followed by MeOH (2mL) and solvents evaporating under vacuum. The residue was dissolved in MeOH (1.7mL) and stirred overnight at room temperature. After evaporating the solvent the title compound (0.001g) was obtained.
LC/MS analysis (mobile phase: A/B from 90/10 to 10/90 in 10 min, 10/90 for 3 min; mass range 150-1000 amu): retention time: 3.98/4.70 min, m\z ([MH]⁺)= 775.

### Examples 322-327:

### Example 322

### (2S)-2-Amino-3-{1-[(benzyloxy)methyl]-1H-imidazol-5-yl}-propionamide of (11S,21R,S)-3-decladinosyl-11,12-dideoxy-6-O-methyl-3-oxo-12,11-[oxycarbonyl-(amino)-methylene]-erythromycin A

### Example 323

### (2S)-2-Amino-3-(1-benzyl-1H-imidazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 324

### (2S)-2-Amino-3-(1H-imidazol-4-yl)-propionamide of (11S,21R,S)-3-decladinosyl-11,12-dideoxy-6-O-methyl-3-oxo-12,11-[oxycarbonyl-(amino)-methylene]-erythromycin A

### Example 325

### (2S)-2-Amino-3-{1-[(benzyloxy)methyl]-1H-imidazol-4-yl}-propionamide of (11S,21R,S)-3-decladinosyl-11,12-dideoxy-6-O-methyl-3-oxo-12,11-[oxycarbonyl-(amino)-methylene]-erythromycin A

### Example 326

### (3S)-3-Amino-4-(1H-indol-3-yl)-butyramide of (11S,21R,S)-3-decladinosyl-11,12-dideoxy-6-O-methyl-3-oxo-12,11-[oxycarbonyl-(amino)-methylene]-erythromycin A

### Example 327

### (2R)-2-Amino-3-pyridin-4-yl-propionamide of (11S,21R,S)-3-decladinosyl-11,12-dideoxy-6-O-methyl-3-oxo-12,11-[oxycarbonyl-(amino)-methylene]-erythromycin A

Examples **322-327** were obtained starting from example **6** (5 mg) by following the same procedure as reported for Example **321**.
The name and amount of starting material (i.e carboxylic acid) and LC/MS analysis (retention time and m/z) of examples **322-327** are reported in the table 3.

**Table 3**

| **Ex.N** | **Carboxylic acid** | **Amount (mg)** | **Ret time (min)** | **Mass analysis m/z [MH]**^{**+**} |
|---|---|---|---|---|
| **322** | (2*S*)-2-{[(9*H*-fluoren-9-ylmethoxy)carbonyl]amino}-3-{1-[(benzyloxy)methyl]-1*H*-imidazol-5-yl}-propionic acid | 4,1 | 5,66 | 884 |
| **323** | (2*S*)-2-{[(9*H*-fluoren-9-ylmethoxy)carbonyl]amino}-3-(1-benzyl-1*H*-imidazol-4-yl)-propionic acid | 3,8 | 5,01 | 854 |
| | | | 5,61 | |
| **324** | (2*S*)-2-{[(9*H*-fluoren-9-ylmethoxy)carbonyl]amino}-3-(1*H*-imidazol-4-yl)-propionic acid | 3,1 | 3,92 | 764 |
| | | | 4,28 | |
| **325** | (2*S*)-2-{[(9*H*-fluoren-9-ylmethoxy)carbonyl]amino}-3-{1-[(benzyloxy)methyl]-1*H*-imidazol-4-yl}-propionic acid | 4,1 | 5,67 | 884 |
| **326** | (3*S*)-3-{[(9*H*-fluoren-9-ylmethoxy)carbonyl]amino}-4-(1*H*-indol-3-yl)-butyric acid | 3,6 | 5,07 | 827 |
| | | | 5,43 | |
| **327** | (2*R*)-2-{[(9*H*-fluoren-9-ylmethoxy)carbonyl]amino}-3-pyridin-4-yl-propionic acid | 3,2 | 3,87 | 775 |
| | | | 4,70 | |

### Example 328

### (11S,21R,S)-3-Decladinosyl-11,12-dideoxy-6-O-methyl-3-oxo-12,11-[oxycarbonyl-(benzoyl-ureido)-methylene]-erythromycin A

To a solution of example **6** (0.005g) in anhydrous THF (0.100mL) a solution of benzoyl isocyanate (2.2mg) in anhydrous THF (0.300mL) was added. The reaction mixture was heated at 60°C for 24h. After cooling to room temperature PS-Trisamine resin (loading 3.62mmol/g, 0.030g) was added and reacted at 60°C for 24h. After cooling to room temperature the mixture was filtered and the resin rinsed with THF (2x0240mL), DCM (2x0.240mL), THF (4x0.170mL) and the filtrate evaporated. The residue was dissolved in MeOH (1mL) and reacted at room temperature overnight. After evaporating the solvent the title compound (0.003g) was obtained.
LC/MS analysis (mobile phase: A/B from 90/10 to 10/90 in 10 min, 10/90 for 2 min; retention time: 6.08/6.51 min, m\z ([MH]⁺) = 774.

### Examples 329-398:

### Example 329

### (11S,21R,S)-3-Decladinosyl-11,12-dideoxy-6-O-methyl-3-oxo-12,11-[oxycarbonyl-(phenyl-ureido)-methylene]-erythromycin A

### Example 330

### (11S,21R,S)-3-Decladinosyl-11,12-dideoxy-6-O-methyl-3-oxo-12,11-[oxycarbonyl-((2,6-dichloropyridin-4-yl)-ureido)-methylene]-erythromycin A

### Example 331

### (11S,21R,S)-3-Decladinosyl-11,12-dideoxy-6-O-methyl-3-oxo-12,11-[oxycarbonyl-((3,5-dimethylisoxazol-4-yl)-ureido)-methylene]-erythromycin A

### Example 332

### (11S,21R,S)-3-Decladinosyl-11,12-dideoxy-6-O-methyl-3-oxo-12,11-[oxycarbonyl-((pyridin-3-yl)-ureido)-methylene]-erythromycin A

### Example 333

### (11S,21R,S)-3-Decladinosyl-11,12-dideoxy-6-O-methyl-3-oxo-12,11-[oxycarbonyl-((2,2,4,4-tetrafluoro-4H-1,3-benzodioxin-6-yl)-ureido)-methylene]-erythromycin A

### Example 334

### (11S,21R,S)-3-Decladinosyl-11,12-dideoxy-6-O-methyl-3-oxo-12,11-[oxycarbonyl-((4-(N,N-dimethylamino)phenyl)-ureido)-methylene]-erythromycin A

### Example 335

### (11S,21R,S)-3-Decladinosyl-11,12-dideoxy-6-O-methyl-3-oxo-12,11-[oxycarbonyl-((4-nitrophenyl)-ureido)methylene]-erythromycin A

### Example 336

### (11S,21R,S)-3-Decladinosyl-11,12-dideoxy-6-O-methyl-3-oxo-12,11-[oxycarbonyl-((3-nitrophenyl)-ureido)-methylene]-erythromycin A

### Example 337

### (11S,21R,S)-3-Decladinosyl-11,12-dideoxy-6-O-methyl-3-oxo-12,11-[oxycarbonyl-((2-nitrophenyl)-ureido)-methylene]-erythromycin A

### Example 338

### (11S,21R,S)-3-Decladinosyl-11,12-dideoxy-6-O-methyl-3-oxo-12,11-[oxycarbonyl-((3,5-dinitrophenyl)-ureido)-methylene]-erythromycin A

### Example 339

### (11S,21R,S)-3-Decladinosyl-11,12-dideoxy-6-O-methyl-3-oxo-12,11-[oxycarbonyl-((4-methyl-2-nitrophenyl)-ureido)-methylene]-erythromycin A

### Example 340

### (11S,21R,S)-3-Decladinosyl-11,12-dideoxy-6-O-methyl-3-oxo-12,11-[oxycarbonyl-((2-methyl-4-nitrolphenyl)-ureido)-methylene]-erythromycin A

### Example 341

### (11S,21R,S)-3-Decladinosyl-11,12-dideoxy-6-O-methyl-3-oxo-12,11-[oxycarbonyl-((2-methyl-5-nitrophenyl)-ureido)-methylene]-erythromycin A

### Example 342

### (11S,21R,S)-3-Decladinosyl-11,12-dideoxy-6-O-methyl-3-oxo-12,11-[oxycarbonyl-((4-methyl-3-nitrophenyl)-ureido)-methylene]-erythromycin A

### Example 343

### (11S,21R,S)-3-Decladinosyl-11,12-dideoxy-6-O-methyl-3-oxo-12,11-[oxycarbonyl-((2-methyl-3-nitrophenyl)-ureido)-methylene]-erythromycin A

### Example 344

### (11S,21R,S)-3-Decladinosyl-11,12-dideoxy-6-O-methyl-3-oxo-12,11-[oxycarbonyl-((2-methyl-6-nitrophenyl)-ureido)-methylene]-erythromycin A

### Example 345

### (11S,21R,S)-3-Decladinosyl-11,12-dideoxy-6-O-methyl-3-oxo-12,11-[oxycarbonyl-((5-chloro-2-nitrophenyl)-ureido)-methylene]-erythromycin A

### Example 346

### (11S,21R,S)-3-Decladinosyl-11,12-dideoxy-6-O-methyl-3-oxo-12,11-[oxycarbonyl-((2-chloro-4-nitrophenyl)-ureido)-methylene]-erythromycin A

### Example 347

### (11S,21R,S)-3-Decladinosyl-11,12-dideoxy-6-O-methyl-3-oxo-12,11-[oxycarbonyl-((4-chloro-3-nitrophenyl)-ureido)-methylene]-erythromycin A

### Example 348

### (11S,21R,S)-3-Decladinosyl-11,12-dideoxy-6-O-methyl-3-oxo-12,11-[oxycarbonyl-((4-chloro-2-nitrophenyl)-ureido)-methylene]-erythromycin A

### Example 349

### (11S,21R,S)-3-Decladinosyl-11,12-dideoxy-6-O-methyl-3-oxo-12,11-[oxycarbonyl-((4-fluoro-3-nitrophenyl)-ureido)-methylene]-erythromycin A

### Example 350

### (11S,21R,S)-3-Decladinosyl-11,12-dideoxy-6-O-methyl-3-oxo-12,11-[oxycarbonyl-((2-fluoro-5-nitrophenyl)-ureido)-methylene]-erythromycin A

### Example 351

### (11S,21R,S)-3-Decladinosyl-11,12-dideoxy-6-O-methyl-3-oxo-12,11-[oxycarbonyl-((2-methoxy-5-nitrophenyl)-ureido)-methylene]-erythromycin A

### Example 352

### (11S,21R,S)3-Decladinosyl-11,12-dideoxy-6-O-methyl-3-oxo-12,11-[oxycarbonyl-((2-methoxy-4-nitrophenyl)-ureido)-methylene]-erythromycin A

### Example 353

### (11S,21R,S)-3-Decladinosyl-11,12-dideoxy-6-O-methyl-3-oxo-12,11-[oxycarbonyl-((4-methoxy-2-nitrophenyl)-ureido)-methylene]-erythromycin A

### Example 354

### (11S,21R,S)-3-Decladinosyl-11,12-dideoxy-6-O-methyl-3-oxo-12,11-[oxycarbonyl-((4-methoxyphenyl)-ureido)-methylene]-erythromycin A

### Example 355

### (11S,21R,S)-3-Decladinosyl-11,12-dideoxy-6-O-methyl-3-oxo-12,11-[oxycarbonyl-((3-methoxyphenyl)-ureido)-methylene]-erythromycin A

### Example 356

### (11S,21R,S)-3-Decladinosyl-11,12-dideoxy-6-O-methyl-3-oxo-12,11-[oxycarbonyl-((2-methoxyphenyl)-ureido)-methylene]-erythromycin A

### Example 357

### (11S,21R,S)-3-Decladinosyl-11,12-dideoxy-6-O-methyl-3-oxo-12,11-[oxycarbonyl-((3,4-dimethoxyphenyl)-ureido)-methylene]-erythromycin A

### Example 358

### (11S,21R,S)-3-Decladinosyl-11,12-dideoxy-6-O-methyl-3-oxo-12,11-[oxycarbonyl-((2,4-dimethoxyphenyl)-ureido)-methylene]-erythromycin A

### Example 359

### (11S,21R,S)-3-Decladinosyl-11,12-dideoxy-6-O-methyl-3-oxo-12,11-[oxycarbonyl-((2,5-dimethoxyphenyl)-ureido)-methylene]-erythromycin A

### Example 360

### (11S,21R,S)-3-Decladinosyl-11,12-dideoxy-6-O-methyl-3-oxo-12,11-[oxycarbonyl-((3,5-dimethoxyphenyl)-ureido)-methylene]-erythromycin A

### Example 361

### (11S,21R,S)-3-Decladinosyl-11,12-dideoxy-6-O-methyl-3-oxo-12,11-[oxycarbonyl-((2,6-dimethoxyphenyl)-ureido)-methylene]-erythromycin A

### Example 362

### (11S,21R,S)-3-Decladinosyl-11,12-dideoxy-6-O-methyl-3-oxo-12,11-[oxycarbonyl-((1,3-benzodioxol-5-yl)-ureido)-methylene]-erythromycin A

### Example 363

### (11S,21R,S)-3-Decladinosyl-11,12-dideoxy-6-O-methyl-3-oxo-12,11-[oxycarbonyl-((2-methoxv-5-methylphenyl)-ureido)-methylene]-erythromycin A

### Example 364

### (11S,21R,S)-3-Decladinosyl-11,12-dideoxy-6-O-methyl-3-oxo-12,11-[oxycarbonyl-((2-ethoxyphenyl)-ureido)-methylene]-erythromycin A

### Example 365

### (11S,21R,S)-3-Decladinosyl-11,12-dideoxy-6-O-methyl-3-oxo-12,11-[oxycarbonyl-((4-ethoxvohenyl)-ureido)-methylene]-erythromycin A

### Example 366

### (11S,21R,S)-3-Decladinosyl-11,12-dideoxy-6-O-methyl-3-oxo-12,11-[oxycarbonyl-((4-phenoxyphenyl)-ureido)-methylene]-erythromycin A

### Example 367

### (11S,21R,S)-3-Decladinosyl-11,12-dideoxy-6-O-methyl-3-oxo-12,11-[oxycarbonyl-((2-phenoxyphenyl)-ureido)-methylene]-erythromycin A

### Example 368

### (11S,21R,S)-3-Decladinosyl-11,12-dideoxy-6-O-methyl-3-oxo-12,11-[oxycarbonyl-((3-phenoxyphenyl)-ureido)-methylene]-erythromycin A

### Example 369

### (11S,21R,S)-3-Decladinosyl-11,12-dideoxy-6-O-methyl-3-oxo-12,11-[oxycarbonyl-((3-(cyclopentyloxy)-4-methoxyphenyl)-ureido)-methylene]-erythromycin A

### Example 370

### (11S,21R,S)-3-Decladinosyl-11,12-dideoxy-6-O-methyl-3-oxo-12,11-[oxycarbonyl-((5-chloro-2-methoxyphenyl)-ureido)-methylene]-erythromycin A

### Example 371

### (11S,21-R,S)-3-Decladinosyl-11,12-dideoxy-6-O-methyl-3-oxo-12,11-[oxycarbonyl-((3-chloro-4-methoxyphenyl-ureido)-methylene]-erythromycin A

### Example 372

### (11S,21R,S)-3-Decladinosyl-11,12-dideoxy-6-O-methyl-3-oxo-12,11-[oxycarbonyl-((5-chloro-2,4-dimethoxyphenyl)-ureido)-methylene]-erythromycin A

### Example 373

### (11S,21R,S)-3-Decladinosyl-11,12-dideoxy-6-O-methyl-3-oxo-12,11-[oxycarbonyl-((4-(trifluoromethoxy)phenyl)-ureido)-methylene]-erythromycin A

### Example 374

### (11S,21R,S)-3-Decladinosyl-11,12-dideoxy-6-O-methyl-3-oxo-12,11-for-[oxycarbonyl-((2-(trifluoromethoxy)phenyl)-ureido)-methylene]-erythromycin A

### Example 375

### (11S,21R,S)-3-Decladinosyl-11,12-dideoxy-6-O-methyl-3-oxo-12,11-[oxycarbonyl-((4-(difluoromethoxy)phenyl)-ureido)-methylene]-erythromycin A

### Example 376

### (11S,21R,S)-3-Decladinosyl-11,12-dideoxy-6-O-methyl-3-oxo-12,11-[oxycarbonyl-((2-(difluoromethoxy)phenyl)-ureido)-methylene]-erythromycin A

### Example 377

### (11S,21R,S)-3-Decladinosyl-11,12-dideoxy-6-O-methyl-3-oxo-12,11-[oxycarbonyl-((4-(methylthio)phenyl)-ureido)-methylene]-erythromycin A

### Example 378

### (11S,21R,S)-3-Decladinosyl-11,12-dideoxy-6-O-methyl-3-oxo-12,11-[oxycarbonyl-((3-(methylthio)phenyl)-ureido)-methylene]-erythromycin A

### Example 379

### (11S,21R,S)-3-Decladinosyl-11,12-dideoxy-6-O-methyl-3-oxo-12,11-[oxycarbonyl-((2-(methylthio)phenyl)-ureido)-methylene]-erythromycin A

### Example 380

### (11S,21R,S)-3-Decladinosyl-11,12-dideoxy-6-O-methyl-3-oxo-12,11-[oxycarbonyl-((4-[(trifluoromethyl)thio]phenyl)-ureido)-methylene]-erythromycin A

### Example 381

### (11S,21R,S)-3-Decladinosyl-11,12-dideoxy-6-O-methyl-3-oxo-12,11-[oxycarbonyl-((4-acetylphenyl)-ureido)-methylene]-erythromycin A

### Example 382

### (11S,21R,S)-3-Decladinosyl-11,12-dideoxy-6-O-methyl-3-oxo-12,11-[oxycarbonyl-((3-acetylphenyl)-ureido)-methylene]-erythromycin A

### Example 383

### (11S,21R,S)-3-Decladinosyl-11,12-dideoxy-6-O-methyl-3-oxo-12,11-[oxycarbonyl-((4-cyanophenyl)-ureido)-methylene]-erythromycin A

### Example 384

### (11S,21R,S)-3-Decladinosyl-11,12-dideoxy-6-O-methyl-3-oxo-12,11-[oxycarbonyl-((3-cyanophenyl)-ureido)-methylene]-erythromycin A

### Example 385

### (11S,21R,S)-3-Decladinosyl-11,12-dideoxy-6-O-methyl-3-oxo-12,11-[oxycarbonyl-((3-(trifluoromethyl)-phenyl)-ureido)-methylene]-erythromycin A

### Example 386

### (11S,21R,S)-3-Decladinosyl-11,12-dideoxy-6-O-methyl-3-oxo-12,11-[oxycarbonyl-((4-chlorophenyl)-ureido)-methylene]-erythromycin A

### Example 387

### (11S,21R,S)-3-Decladinosyl-11,12-dideoxy-6-O-methyl-3-oxo-12,11-[oxycarbonyl-((3-chlorophenyl)-ureido)-methylene]-erythromycin A

### Example 388

### (11S,21R,S)-3-Decladinosyl-11,12-dideoxy-6-O-methyl-3-oxo-12,11-[oxycarbonyl-((2-chlorophenyl)-ureido)-methylene]-erythromycin A

### Example 389

### (11S,21R,S)-3-Decladinosyl-11,12-dideoxy-6-O-methyl-3-oxo-12,11-[oxycarbonyl-((3,4-dichlorophenyl)-ureido)-methylene]-erythromycin A

### Example 390

### (11S,21R,S)-3-Decladinosyl-11,12-dideoxy-6-O-methyl-3-oxo-12,11-[oxycarbonyl-((4-fluorophenyl)-ureido)-methylene]-erythromycin A

### Example 391

### (11S,21R,S)-3-Decladinosyl-11,12-dideoxy-6-O-methyl-3-oxo-12,11-[oxycarbonyl-((benzyl)-ureido)-methylene]-erythromycin A

### Example 392

### (11S,21R,S)-3-Decladinosyl-11,12-dideoxy-6-O-methyl-3-oxo-12,11-[oxycarbonyl-((4-methoxybenzyl)-ureido)-methylene]-erythromycin

### Example 393

### (11S,21R,S)-3-Decladinosyl-11,12-dideoxy-6-O-methyl-3-oxo-12,11-[oxycarbonyl-((2-chlorobenzyl)-ureido)-methylene]-erythromycin A

### Example 394

### (11S,21R,S)-3-Decladinosyl-11,12-dideoxy-6-O-methyl-3-oxo-12,11-[oxycarbonyl-((3,4-dichlorobenzyl)-ureido)-methylene]-erythromycin A

### Example 395

### (11S,21R,S)-3-Decladinosyl-11,12-dideoxy-6-O-methyl-3-oxo-12,11-[oxycarbonyl-((4-fluorobenzyl)-ureido)-methylene]-erythromycin A

### Example 396

### (11S,21R,S)-3-Decladinosyl-11,12-dideoxy-6-O-methyl-3-oxo-12,11-[oxycarbonyl-((4-bromobenzyl)-ureido)-methylene]-erythromycin A

### Example 397

### (11S,21R,S)-3-Decladinosyl-11,12-dideoxy-6-O-methyl-3-oxo-12,11-[oxycarbonyl-((2-phenylethyl)-ureido)-methylene]-erythromycin A

### Example 398

### (11S,21R,S)-3-Decladinosyl-11,12-dideoxy-6-O-methyl-3-oxo-12,11-[oxycarbonyl-((2-thien-2-ylethyl)-ureido)-methylene]-erythromycin A

Examples **329-398** were obtained starting from example **6** (5 mg) by following the same procedure as reported for Example **328**.
The name and amount of starting material (i.e isocyanate) and LC/MS analysis (retention time and m/z) of examples **329-398** are reported in the table 4.

**Table 4**

| **Ex. N** | **Isocyanate** | **Amount (mg)** | **Ret. time (min)** | **Mass analysis m/z [MH]**^{**+**} |
|---|---|---|---|---|
| **329** | Isocyanatobenzene | 1,78 | 5.50 | 746 |
| | | | 6.10 | |
| **330** | 2,6-Dichloro-4-isocyanatopyridine | 2,83 | 6.51 | 815 |
| | | | 7.16 | |
| **331** | 4-Isocyanato-3,5-dimethylisoxazole | 2,06 | 5.11 | 765 |
| **332** | 3-Isocyanatopyridine | 1,80 | 5.00 | 747 |
| | | | 5.43 | |
| **333** | 2,2,4,4-Tetrafluoro-6-isocyanato-4*H*-1,3-benzodioxine | 3,72 | 7.59 | 876 |
| | | | 8.20 | |
| **334** | N-(4-isocyanatophenyl)-*N,N*-dimethylamine | 2,42 | 5.90 | 789 |
| **335** | 1-Isocyanato-4-nitrobenzene | 2,45 | 6.40 | 791 |
| | | | 7.00 | |
| **336** | 1-Isocyanato-3-nitrobenzene | 2,45 | 6.33 | 791 |
| | | | 6.87 | |
| **337** | 1-Isocyanato-2-nitrobenzene | 2,45 | 6.93 | 791 |
| **338** | 1-Isocyanato-3,5-dinitrobenzene | 3,13 | 6.85 | 836 |
| | | | 7.39 | |
| **339** | 1-Isocyanato-4-methyl-2-nitrobenzene | 2,66 | 6.81 | 805 |
| | | | 7.57 | |
| **340** | 1-Isocyanato-2-methyl-4-nitobenzene | 2,66 | 6.62 | 805 |
| | | | 7.22 | |
| **341** | 2-Isocyanato-1-methyl-4-nitrobenzene | 2,66 | 6.47 | 805 |
| | | | 6.90 | |
| **342** | 4-Isocyanato-1-methyl-2-nitrobenzene | 2,66 | 6.70 | 805 |
| | | | 7.20 | |
| **343** | 1-Isocyanato-2-methyl-3-nitrobenzene | 2,66 | 6.40 | 805 |
| | | | 6.80 | |
| **344** | 2-Isocyanato-1-methyl-3-nitrobenzene | 2,66 | 6.18 | 805 |
| | | | 6.57 | |
| **345** | 4-Chloro-2-isocyanato-1-nitrobenzene | 2,97 | 7.11 | 825 |
| | | | 7.83 | |
| **346** | 2-Chloro-1-isocyanato-4-nitrobenzene | 2,97 | 6.86 | 825 |
| | | | 7.57 | |
| **347** | 1-Chloro-4-isocyanato-2-nitrobenzene | 2,97 | 6.91 | 825 |
| | | | 7.51 | |
| **348** | 4-Chloro-1-isocyanato-2-nitrobenzene | 2,97 | 7.05 | 825 |
| | | | 7.84 | |
| **349** | 1-Fluoro-4-isocyanato-2-nitrobenzene | 2,72 | 6.48 | 809 |
| | | | 7.00 | |
| **350** | 1-Fluoro-2-isocyanato-4-nitrobenzene | 2,72 | 6.50 | 809 |
| | | | 7.00 | |
| **351** | 2-Isocyanato-1-methoxy-4-nitrobenzene | 2,90 | 6.40 | 821 |
| | | | 7.00 | |
| **352** | 1-Isocyanato-2-methoxy-4-nitrobenzene | 2,90 | 6.56 | 821 |
| | | | 7.26 | |
| **353** | 1-Isocyanato-4-methoxy-2-nitrobenzene | 2,90 | 6.48 | 821 |
| | | | 7.13 | |
| **354** | 1-Isocyanato-4-methoxybenzene | 2,23 | 5.60 | 776 |
| | | | 5.90 | |
| **355** | 1-Isocyanato-3-methoxybenzene | 2,23 | 5.70 | 776 |
| | | | 6.10 | |
| **356** | 1-Isocyanato-2-methoxybenzene | 2,23 | 5.40 | 776 |
| | | | 6.10 | |
| **357** | 4-Isocyanato-1,2-dimethoxybenzene | 2,68 | 5.30 | 806 |
| | | | 5.60 | |
| **358** | 1-Isocyanato-2,4-dimethoxybenzene | 2,68 | 5.60 | 806 |
| | | | 6.10 | |
| **359** | 2-Isocyanato-1,4-dimethoxybenzene | 2,68 | 6.00 | 806 |
| | | | 6.80 | |
| **360** | 1-Isocyanato-3,5-dimethoxybenzene | 2,68 | 6.17 | 806 |
| | | | 6.64 | |
| **361** | 2-Isocyanato-1,3-dimethoxybenzene | 2,68 | 5.50 | 806 |
| | | | 5.94 | |
| **362** | 5-Isocyanato-1,3-benzodioxole | 2,44 | 5.60 | 790 |
| | | | 5.90 | |
| **363** | 2-Isocyanato-1-methoxy-4-methylbenzene | 2,44 | 5.90 | 790 |
| | | | 6.60 | |
| **364** | 1-Ethoxy-2-isocyanatobenzene | 2,44 | 5.80 | 790 |
| | | | 6.60 | |
| **365** | 1-Ethoxy-4-isocyanatobenzene | 2,44 | 5.80 | 790 |
| | | | 6.10 | |
| **366** | 1-Isocyanato-4-phenoxybenzene | 3,16 | 7.03 | 838 |
| | | | 7.30 | |
| **367** | 1-Isocyanato-2-phenoxybenzene | 3,16 | 6.70 | 838 |
| | | | 7.30 | |
| **368** | 1-Isocyanato-3-phenoxybenzene | 3,16 | 7.10 | 838 |
| | | | 7.40 | |
| **369** | 2-(Cyclopentyloxy)-4-isocyanato-1-methoxybenzene | 3,49 | 6.60 | 860 |
| | | | 6.80 | |
| **370** | 4-chloro-2-isocyanato-1-methoxybenzene | 2,74 | 6.89 | 810 |
| | | | 7.63 | |
| **371** | 2-Chloro-4-isocyanato-1-methoxybenzene | 2,74 | 6.10 | 810 |
| | | | 6.40 | |
| **372** | 1-Chloro-5-isocyanato-2,4-dimethoxybenzene | 3,19 | 6.10 | 810 |
| | | | 6.50 | |
| **373** | 1-Isocyanato-4-(trifluoromethoxy)benzene | 3,04 | 7.13 | 830 |
| | | | 7.74 | |
| **374** | 1-Isocyanato-2-(trifluoromethoxy)benzene | 3,04 | 6.97 | 830 |
| | | | 7.63 | |
| **375** | 1-(Difluoromethoxy)-4-isocyanatobenzene | 2,77 | 6.20 | 812 |
| | | | 6.60 | |
| **376** | 1-(Difluoromethoxy)-2-isocyanatobenzene | 2,77 | 6.61 | 812 |
| | | | 7.22 | |
| **377** | 1-Isocyanato-4-(methylthio)benzene | 2,47 | 6.20 | 792 |
| | | | 6.50 | |
| **378** | 1-Isocyanato-3-(methylthio)benzene | 2,47 | 6.20 | 792 |
| | | | 6.70 | |
| **379** | 1-Isocyanato-2-(methylthio)benzene | 2,47 | 5.90 | 792 |
| | | | 6.50 | |
| **380** | 1-Isocyanato-4-[(trifluoromethyl)thio]-benzene | 3,28 | 7.60 | 846 |
| | | | 8.20 | |
| **381** | 1-(4-Isocyanatophenyl)ethanone | 2,41 | 5.73 | 788 |
| | | | 6.26 | |
| **382** | 1-(3-Isocyanatophenyl)ethanone | 2,41 | 5.83 | 788 |
| | | | 6.34 | |
| **383** | 4-Isocyanatobenzonitrile | 2,15 | 6.05 | 771 |
| | | | 6.59 | |
| **384** | 3-Isocyanatobenzonitrile | 2,15 | 6.10 | 771 |
| | | | 6.60 | |
| **385** | 1-Isocyanato-3-(triffuoromethyl)benzene | 2,80 | 7.10 | 814 |
| | | | 7.60 | |
| **386** | 1-Chloro-4-isocyanatobenzene | 2,30 | 6.70 | 780 |
| | | | 7.30 | |
| **387** | 1-Chloro-3-isocyanatobenzene | 2,30 | 6.69 | 780 |
| | | | 7.32 | |
| **388** | 1-Chloro-2-isocyanatobenzene | 2,30 | 6.69 | 780 |
| | | | 7.32 | |
| **389** | 1,2-Dichloro-4-isocyanatobenzene | 2,81 | 7.23 | 814 |
| | | | 7.92 | |
| **390** | 1-Fluoro-4-isocyanatobenzene | 2,05 | 5.80 | 764 |
| | | | 6.20 | |
| **391** | (Isocyanatomethyl)benzene | 1,99 | 6.00 | 760 |
| | | | 6.40 | |
| **392** | 1-(Isocyanatomethyl)-4-methoxybenzene | 2,44 | 5.92 | 790 |
| | | | 6.28 | |
| **393** | 1-Chloro-2-(isocyanatomethyl)benzene | 2,51 | 6.38 | 794 |
| | | | 6.84 | |
| **394** | 1,2-Dichloro-4-(isocyanatomethyl)benzene | 3,02 | 6.99 | 828 |
| **395** | 1-Fluoro-4-(isocyanatomethyl)benzene | 2,26 | 6.09 | 778 |
| | | | 6.40 | |
| **396** | 1-Bromo-4-(isocyanatomethyl)benzene | 3,17 | 6.70 | 838 |
| | | | 7.00 | |
| **397** | (2-Isocyanatoethyl)benzene | 2,20 | 6.30 | 774 |
| | | | 6.70 | |
| **398** | 2-(2-Isocyanatoethyl)thiophene | 2,29 | 6.10 | 780 |
| | | | 6.50 | |

### Example 399

### (11S,21R,S)-3-Decladinosyl-11,12-dideoxy-6-O-methyl-3-oxo-12,11-[oxycarbonyl-(benzoyl-thioureido)-methylene]-erythromycin A

A solution of example **6** (0.005g) in MeOH (0.350mL) was reacted overnight at room temperature. After evaporating the solvent a solution of benzoyl isothiocyanate (2.4mg) in DCE (0.400mL) was added and the reaction mixture was heated at 60°C for 26h. After cooling to room temperature PS-Trisamine resin (loading 3.62mmol/g, 0.030g) was added and reacted at room temperature for 15h. The mixture was filtered and the resin rinsed with DCE (2x0.230mL), DCM (3x0.160mL, 2x0.120mL). The filtrate was evaporated to give the title compound (0.003g).
LC/MS analysis (mobile phase: A/B from 90/10 to 10/90 in 16 min, 10/90 for 4 min; retention time: 9.1/10.0min, m\z ([MH]⁺) = 790.

### Examples 400-425:

### Example 400

### (11S,21R,S)-3-Decladinosyl-11,12-dideoxy-6-O-methyl-3-oxo-12,11-[oxycarbonyl-((4-(N,N-dimethylamino)-phenyl)-thioureido)-methylene]-erythromycin A

### Example 401

### (11S,21R,S)-3-Decladinosyl-11,12-dideoxy-6-O-methyl-3-oxo-12,11-[oxycarbonyl-((4-(N,N-diethylamino)phenyl)-thioureido)-methylene]-erythromycin A

### Example 402

### (11S,21R,S)-3-Decladinosyl-11,12-dideoxy-6-O-methyl-3-oxo-12,11-[oxycarbonyl-((4-(N,N-dimethylamino)-1-naphthyl)-thioureido)-methylene]-erythromycin A

### Example 403

### (11S,21R,S)-3-Decladinosyl-11,12-dideoxy-6-O-methyl-3-oxo-12,11-[oxycarbonyl-((4-nitrophenyl)-thioureido)-methylene]-erythromycin A

### Example 404

### (11S,21R,S)-3-Decladinosyl-11,12-dideoxy-6-O-methyl-3-oxo-12,11-[oxycarbonyl-((2-methyl-5-nitrophenyl)-thioureido)-methylene]-erythromycin A

### Example 405

### (11S,21R,S)-3-Decladinosyl-11,12-dideoxy-6-O-methyl-3-oxo-12,11-[oxycarbonyl-((2-chloro-4-nitrophenyl)-thioureido)-methylene]-erythromycin A

### Example 406

### (11S,21R,S)-3-Decladinosyl-11,12-dideoxy-6-O-methyl-3-oxo-12,11-[oxycarbonyl-((2-methoxy-4-nitrophenyl)-thioureido)-methylene]-erythromycin A

### Example 407

### (11S,21R,S)-3-Decladinosyl-11,12-dideoxy-6-O-methyl-3-oxo-12,11-[oxycarbonyl-((4-methoxy-2-nitrophenyl)-thioureido)-methylene]-erythromycin A

### Example 408

### (11S,21R,S)-3-Decladinosyl-11,12-dideoxy-6-O-methyl-3-oxo-12,11-[oxycarbonyl-((2-methoxyphenyl)-thioureido)-methylene]-erythromycin A

### Example 409

### (11S,21R,S)-3-Decladinosyl-11,12-dideoxy-6-O-methyl-3-oxo-12,11-[oxycarbonyl-((2,5-dimethoxyphenyl)-thioureido)-methylene]-erythromycin A

### Example 410

### (11S,21R,S)-3-Decladinosyl-11,12-dideoxy-6-O-methyl-3-oxo-12,11-[oxycarbonyl-((2-methoxy-5-methylphenyl)-thioureido)-methylene]-erythromycin A

### Example 411

### (11S,21R,S)-3-Decladinosyl-11,12-dideoxy-6-O-methyl-3-oxo-12,11-[oxycarbonyl-((4-methoxy-1,1'-biphenyl-3-yl)-thioureido)-methylene]-erythromycin A

### Example 412

### (11S,21R,S)-3-Decladinosyl-11,12-dideoxy-6-O-methyl-3-oxo-12,11-[oxycarbonyl-((4-ethoxyphenyl)-thioureido)-methylene]-erythromycin A

### Example 413

### (11S,21R,S)-3-Decladinosyl-11,12-dideoxy-6-O-methyl-3-oxo-12,11-[oxycarbonyl-((4-(benzyloxy)phenyl)-thioureido)-methylene]-erythromycin A

### Example 414

### (11S,21R,S)-3-Decladinosyl-11,12-dideoxy-6-O-methyl-3-oxo-12,11-[oxycarbonyl((5-chloro-2-methoxyphenyl)-thioureido)-methylene]-erythromycin A

### Example 415

### (11S,21R,S)-3-Decladinosyl-11,12-dideoxy-6-O-methyl-3-oxo-12,11-[oxycarbonyl-((4-(methylthio)-phenyl)-thioureido)-methylene]-erythromycin A

### Example 416

### (11S,21R,S)-3-Decladinosyl-11,12-dideoxy-6-O-methyl-3-oxo-12,11-[oxycarbonyl-((benzyl)-thioureido)-methylene]-erythromycin A

### Example 417

### (11S,21R,S)-3-Decladinosyl-11,12-dideoxy-6-O-methyl-3-oxo-12,11-[oxycarbonyl-((2-furylmethyl)-thioureido)-methylene]-erythromycin A

### Example 418

### (11S,21R,S)-3-Decladinosyl-11,12-dideoxy-6-O-methyl-3-oxo-12,11-[oxycarbonyl-((4-methoxybenzyl)-thioureido)-methylene]-erythromycin A

### Example 419

### (11S,21R,S)-3-Decladinosyl-11,12-dideoxy-6-O-methyl-3-oxo-12,11-[oxycarbonyl-((2-methoxybenzyl)-thioureido)-methylene]-erythromycin A

### Example 420

### (11S,21R,S)-3-Decladinosyl-11,12-dideoxy-6-O-methyl-3-oxo-12,11-[oxycarbonyl-((2-chlorobenzyl)-thioureido)-methylene]-erythromycin A

### Example 421

### (11S,21R,S)-3-Decladinosyl-11,12-dideoxy-6-O-methyl-3-oxo-12,11-[oxycarbonyl-((3,4-dichlorobenzyl)-thioureido)-methylene]-erythromycin A

### Example 422

### (11S,21R,S)-3-Decladinosyl-11,12-dideoxy-6-O-methyl-3-oxo-12,11-[oxycarbonyl-((2-phenylethyl)-thioureido)-methylene]-erythromycin A

### Example 423

### (11S,21R,S)-3-Decladinosyl-11,12-dideoxy-6-O-methyl-3-oxo-12,11-[oxycarbonyl-((2-morpholin-4-ylethyl)-thioureido)-methylene]-erythromycin A

### Example 424

### (11S,21R,S)-3-Decladinosyl-11,12-dideoxy-6-O-methyl-3-oxo-12,11-[oxycarbonyl-((2-(3,4-dimethoxyphenyl)ethyl)-thioureido)-methylene]-erythromycin A

### Example 425

### (11S,21R,S)-3-Decladinosyl-11,12-dideoxy-6-O-methyl-3-oxo-12,11-[oxycarbonyl-((2-(4-chlorophenyl)-ethyl)-thioureido)-methylene]-erythromycin A

Examples **400-425** were obtained starting from example **6** (5 mg) by following the same procedure as reported for Example **399**.

The name and amount of starting material (i.e isocyanate) and LC/MS analysis (retention time) of examples **400-425** are reported in the table 5.

**Table 5**

| **Ex. N** | **Isothiocyanate** | **Amount (mg)** | **Ret. time (min)** | **Mass analysis m/z [MH]**^{**+**} |
|---|---|---|---|---|
| **400** | *N*-(4-Isothiocyanatophenyl)-*N,N*-dimethylamine | 2,66 | 8.7 | 805 |
| | | | 9.0 | |
| **401** | *N,N-*Diethyl-*N*-(4-isothiocyanatophenyl)amine | 3,08 | 10.3 | 833 |
| **402** | *N*-(4-Isothiocyanato-1-naphthyl)-*N,N*-dimethylamine | 3,41 | 10.2 | 855 |
| | | | 10.4 | |
| **403** | 1-Isothiocyanato-4-nitrobenzene | 2,69 | 9.5 | 807 |
| | | | 10.2 | |
| **404** | 2-Isothiocyanato-1-methyl-4-nitrobenzene | 2,90 | 9.3 | 821 |
| | | | 9.8 | |
| **405** | 2-Chloro-1-isothiocyanato-4-nitrobenzene | 3,21 | 9.9 | 841 |
| | | | 10.5 | |
| **406** | 1-Isothiocyanato-2-methoxy-4-nitrobenzene | 3,14 | 9.7 | 837 |
| | | | 10.5 | |
| **407** | 1-Isothiocyanato-4-methoxy-2-nitrobenzene | 3,14 | 9.3 | 837 |
| | | | 9.8 | |
| **408** | 1-Isothiocyanato-2-methoxybenzene | 2,47 | 8.4 | 792 |
| | | | 9.6 | |
| **409** | 2-Isothiocyanato-1,4-dimethoxybenzene | 2,92 | 8.5 | 822 |
| | | | 9.6 | |
| **410** | 2-Isothiocyanato-1-methoxy-4-methylbenzene | 2,68 | 9.0 | 806 |
| **411** | 3-Isothiocyanato-4-methoxy-1,1'-biphenyl | 3,61 | 10.4 | 868 |
| | | | 11.3 | |
| **412** | 1-Ethoxy-4-isothiocyanatobenzene | 2,68 | 9.2 | 806 |
| | | | 9.9 | |
| **413** | 1-(Benzyloxy)-4-isothiocyanatobenzene | 3,61 | 10.7 | 868 |
| | | | 11.3 | |
| **414** | 4-Chloro-2-isothiocyanato-1-methoxybenzene | 2,98 | 9.4 | 826 |
| | | | 10.5 | |
| **415** | 1-Isothiocyanato-4-(methylthio)benzene | 2,71 | 9.4 | 808 |
| | | | 10.2 | |
| **416** | (Isothiocyanatomethyl)benzene | 2,23 | 9.5 | 776 |
| **417** | 2-(Isothiocyanatomethyl)furan | 2,08 | 8.5 | 765 |
| **418** | 1-(Isothiocyanatomethyl)-4-methoxybenzene | 2,68 | 9.5 | 806 |
| **419** | 1-(Isothiocyanatomethyl)-2-methoxybenzene | 2,68 | 9.6 | 806 |
| **420** | 1-Chloro-2-(isothiocyanatomethyl)benzene | 2,75 | 10.2 | 810 |
| **421** | 1,2-Dichloro-4-(isothiocyanatomethyl)benzene | 3,26 | 10.8 | 844 |
| | | | 11.1 | |
| **422** | (2-Isothiocyanatoethyl)benzene | 2,44 | 10.1 | 790 |
| **423** | 4-(2-Isothiocyanatoethyl)morpholine | 2,58 | 6.6 | 799 |
| | | | 6.9 | |
| **424** | 4-(2-Isothiocyanatoethyl)-1,2-dimethoxybenzene | 3,34 | 9.1 | 850 |
| **425** | 1-Chloro-4-(2-isothiocyanatoethyl)benzene | 2,96 | 11.0 | 824 |

### Example 426

### 3-Amino-isonicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

To 3-amino-isonicotinic acid (0.001g) a solution of benzotriazole-1-yl-oxy-tris-pyrrolidino-phosphonium hexafluorophosphate (0.004g) in anhydrous DMF (0.150mL) and a solution of DIPEA (0.003mL) in anhydrous DMF (0.150mL) were added followed by the addition of a solution of example **13** (0.005g) in anhydrous DMF (0.100mL). The reaction mixture was stirred at room temperature for 48h, then it was diluted with DCM (0.600mL), washed with a 5% NaHCO₃ aqueous solution (0.500mL), then passed through a phase-separation syringe. The aqueous phase was extracted with DCM (0.400mL) and the collected organic extracts evaporated under vacuum. The crude material was dissolved in DCM (0.500mL), loaded on a SCX-cartridge (250mg, loading 0.75mmol/g), washed with MeOH (4mL), then the product eluted with NH₃ (0.25M solution in MeOH, 1.5mL). After evaporating the solvent the title compound (0.003g) was obtained.
LC/MS analysis (mobile phase: A/B from 90/10 to 10/90 in 10 min, 10/90 for 2 min, mass range 150-1300 amu): retention time: 4.99 min, m\z ([MH]⁺) = 790.

### Examples 427-601:

### Example 427

### 5-Methyl-3-phenyl-isoxazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 428

### 1,5-Dimethyl-3-oxo-2-phenyl-2,3-dihydro-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 429

### 2-Trifluoromethyl-[1,8]naphthyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 430

### 6-Nitro-2-oxo-2H-chromene-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 431

### 4-Amino-2-methylsulfanyl-pyrimidine-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 432

### 5-Chloro-1-methyl-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 433

### 2-Pyrazin-2-yl-thiazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 434

### 3-Methyl-2-oxo-1,2-dihydro-quinoline-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 435

### 2-Methyl-imidazo[1,2,-a]pyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 436

### 4-Methoxy-1,3-dimethyl-1H-pyrazolo[3,4-b]pyridine-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 437

### 3-Methyl-5-(4-methyl-[1,2,3]thiadiazol-5-yl)-isoxazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 438

### 4-Acetyl-1-methyl-1H-pyrrole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 439

### 6-[1,2,4]Triazol-1-yl-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 440

### Isonicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 441

### 5-Oxo-2,3-dihydro-5H-thiazolo[3,2-a]pyrimidine-6-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 442

### 5-Nitro-1H-pyrazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 443

### 2-Methylsulfanyl-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 444

### 7-Hydroxy-2-oxo-2H-chromene-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 445

### Cinnoline-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 446

### 6-Amino-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 447

### 1-Methyl-5-nitro-1H-pyrazole-4-carboxamide of (11S,21R,S)-3-decladinosyl-11,12-dideoxy-6-O-methyl-3-oxo-12,11-(oxycarbonyl-(2-(amino)-ethylamino)-methylene]-erythromycin A

### Example 448

### 4,7-Dimethyl-pyrazolo[5,1-c][1,2,4]triazine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 449

### 2-Methoxy-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 450

### 3,5-Dimethyl-isoxazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 451

### 4-Oxo-4,5,6,7-tetrahydro-benzofuran-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 452

### 3-Amino-pyrazine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 453

### Pyrazine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 454

### 4-Phenyl-[1,2,3]thiadiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 455

### 5-Methyl-3-methylsulfanyl-isothiazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 456

### 2,6-Dimethyl-4-oxo-4H-pyran-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 457

### 1-Oxo-1,2-dihydro-isoquinoline-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 458

### 4-Acetyl-3-cyano-5-methyl-1H-pyrrole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 459

### 4-Methyl-3-oxo-3,4-dihydro-2H-benzo[1,4]thiazine-6-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 460

### 6-Methyl-imidazo[2,1-b]thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 461

### 2-Phenoxy-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 462

### 5-Nitro-1H-indole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 463

### 4,8-Dihydroxy-quinoline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 464

### 2-Hydroxy-quinoline-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 465

### (1H-Indol-3-yl)-oxo-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 466

### Furan-2-yl-oxo-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 467

### 2-Amino-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 468

### 1H-Benzotriazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 469

### 3-Methyl-furan-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 470

### 5-Chloro-1,3-dimethyl-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 471

### 4-Nitro-1H-pyrazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 472

### 4,6-Dimethyl-2-oxo-2H-pyran-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 473

### 2-Amino-5-chloropyrimidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 474

### 5-Methyl-1H-pyrazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 475

### 1-Methyl-5-oxopyrrolidine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 476

### 2-Chloro-6-methyl-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 477

### (4R)-2-Thioxo-1,3-thiazolidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 478

### 2,2-Dimethyl-5-oxotetrahydrofuran-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 479

### 2,4-Dioxo-1,2,3,4-tetrahydropyrimidine-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 480

### 2-(Methoxycarbonyl-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 481

### 2-Methyl-1,8-naphthyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 482

### 2-(Trifluoromethyl)-1,6-naphthyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 483

### 1-Benzyl-5-oxopyrrolidine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 484

### 3-(Aminocarbonyl)pyrazine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 485

### 4-Amino-2-methylpyrimidine-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 486

### Isoxazole-5-carboxamide of (11S,2R,S)-3-decladinosyl-11,12-dideoxy-6-O-methyl-3-oxo-12,11-[oxycarbonyl-(2-(amino)-ethylamino)-methylene]-erythromycin A

### Example 487

### 5-Methylisoxazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 488

### 6-Cyanonicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 489

### 1-Ethyl-3-methyl-1H-pyrazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 490

### 5-Methylisoxazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 491

### 5-Oxo-1-(thien-2-ylmethyl)-pyrrolidine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 492

### 2-(Pyridin-2-ylcarbonyl)-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 493

### 1-(2-Furylmethyl)-5-oxopyrrolidine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 494

### 5-(Methoxycarbonyl)-pyridine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 495

### 2-(4-Methyl-1,2,3-thiadiazol-5-yl)-1,3-thiazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 496

### 3-Oxo-2,3-dihydro-1H-indazole-4-carboxamide of (11S,21R,S)-3-decladinosyl-11,12-dideoxy-6-O-methyl-3-oxo-12 11-[oxycarbonyl-(2-(amino)-ethylamino)-methylene]-erythromycin A

### Example 497

### 2-Methyl-1,6-naphthyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 498

### 4-Methyl-2-pyridin-4-yl-1,3-thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 499

### 4-Methyl-2-pyridin-3-yl-1,3-thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 500

### 4-(Morpholin-4-ylmethyl)-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 501

### 6-(1H-Imidazol-1-yl)-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 502

### 5-Methoxy-2-(1,3,5-trimethyl-1H-pyrazol-4-yl)-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 503

### 4-Methyl-2-pyrazin-2-yl-1,3-thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 504

### 2,5-Dimethyl-1H-pyrrole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 505

### 4-{[(4,6-Dimethylpyrimidin-2-yl)amino]carbonyl}-5-methylisoxazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 506

### 1-Methy-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 507

### 5-Chloro-1-methyl-3-(trifluoromethyl)-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 508

### 1-Pyrimidin-2-ylpiperidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 509

### 1-Methyl-3-(trifluoromethyl)-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 510

### (4R)-2-Oxo-1,3-thiazolidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 511

### (2S)-1-Acetylpyrrolidine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 512

### 5-Nitro-furan-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 513

### 1-Methylpyrrolidine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 514

### 4-Acetyl-3,5-dimethyl-1H-pyrrole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 515

### 6-Methylnicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 516

### 6-Methyl-3,4-dihydro-2H-pyran-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 517

### 2,7-Dimethylpyrazolo[1,5-a]pyrimidine-6-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 518

### 4-Methyl-1,2,3-thiadiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 519

### 4-(Trifluoromethyl)-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 520

### 4-Oxo-4H-chromene-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 521

### 5-Amino-1H-pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 522

### 2-Chloronicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 523

### 2-Oxo-2H-pyran-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 524.

### Furan-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 525

### 1,2,3-Thiadiazole-4-carboxamide of (11S,21R,S)-3-decladinosyl-11,12-dideoxy-6-O-methyl-3-oxo-12,11-[oxycarbonyl-(2-(amino)ethylamino)-methylene]-erythromycin A

### Example 526

### 1,5-Dimethyl-1H-pyrazole-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 527

### 1H-Pyrazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 528

### 8-Methyl-O-oxo-4H-pyrido[1,2-a]pyrimidine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 529

### 2,6-Dimethoxynicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 530

### 4-Chloro-1,3-dimethyl-1H-pyrazolo[3,4-b]pyridine-5-carboxamide of (11S,21R,S)-3-decladinosyl-11,12-dideoxy-6-O-methyl-3-oxo-12,11-[oxycarbonyl-(2-(amino)-ethylamino)-methylenel-erythromycin A

### Example 531

### 2-Methylnicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 532

### 5-Amino-2,6-dioxo-1,2,3,6-tetrahydropyrimidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 533

### 4-Hydroxy-3-(morpholin-4-ylmethyl)-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 534

### 5-Methylpyrazine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 535

### 6-Oxo-1,4,5,6-tetrahydropyridazine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 536

### 1H-pyrrole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 537

### 2,2-Dimethyl-4-oxo-3,4-dihydro-2H-pyran-6-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 538

### 1-Methyl-1H-pyrrole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 539

### 2,3-Dihydro-1,4-benzodioxine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 540

### 2,4-Dimethyl-1,3-thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 541

### 1-Methyl-1,2,5,6-tetrahydropyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 542

### 1-Oxy-pyridine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 543

### 1-Acetylpiperidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 544

### Tetrahydrofuran-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 545

### (2S)-5-Oxopyrrolidine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 546

### 6-Hydroxynicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 547

### 3,6-Dichloropyridazine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 548

### 1-Ethyl-7-methyl-4-oxo-1,4-dihydro-1,8-naphthyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 549

### Furan-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 550

### Pyridine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 551

### Nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 552

### Tetrahydrofuran-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 553

### 2-Methyl-furan-3-carboxamide of (11S,21R,S)-3-decladinosyl-11,12-dideoxy-6-O-methyl-3-oxo-12,11-oxycarbonyl-(2-(amino)-ethylamino)-methylene]-erythromycin A

### Example 554

### (2R)-Tetrahydrofuran-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 555

### 2-(5-Oxopyrrolidin-2-ylsulfanyl)-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 556

### 1-Allyl-2-oxo-1,2-dihydropyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 557

### 2-Methyl-4-(trifluoromethyl)-1,3-thiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 558

### 6-Hydroxy-1-methyl-2-oxo-1,2-dihydropyrimidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 559

### 3-Methylisoxazole-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 560

### 5-Methoxy-1,3-oxazole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 561

### 2-[6-(Acetylamino)-pyridin-3-ylsulfanyl]-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 562

### 4-(4-Methylpiperazin-1-yl)-3-nitro-benzamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 563

### 4-Isopropyl-1,2,3-thiadiazole-5-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 564

### 3-Oxo-2-phenyl-2,3-dihydropyridazine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 565

### 5-Bromo-2-oxo-1,2-dihydropyridine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 566

### 1-(Methoxycarbonyl)piperidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 567

### 1-(6-Chloropyridazin-3-yl)piperidine-4-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 568

### 4-Chloro-6-oxo-1-phenyl-1,6-dihydropyridazine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 569

### 1-(3-Chlorophenyl)-4-methoxy-6-oxo-1,6-dihydropyridazine-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 570

### (1-Methyl-4-nitro-1H-pyrazol-5-yl)-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 571

### (1,3-Dimethyl-2,6-dioxo-2,3,6,7-tetrahydro-1H-purin-8-yl)-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 572

### 5-Oxo-4,5-dihydrofuran-3-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 573

### 2R,4S)-4-(Acetyloxy)-1,3-oxathiolane-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 574

### 3-Hydroxyisonicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 575

### 3-Hydroxypyridine-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 576

### 2-Hydroxynicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 577

### 7-Hydroxy-4-oxo-4H-chromene-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 578

### 1-Oxy-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 579

### 1H-Indole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 580

### 5-Methoxy-1H-indole-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 581

### 6-chloro-4-oxo-4H-chromene-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 582

### Quinoline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 583

### 4-Hydroxy-6-methoxyquinoline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 584

### 4-Methyl-3-oxo-3,4-dihydroquinoxaline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 585

### 4-(2-Hydroxyethyl)-3-oxo-3,4-dihydroquinoxaline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 586

### 3-Methoxyquinoxaline-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 587

### 1-Benzofuran-2-carboxamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 588

### 6-Ethyl-5-oxothiomorpholine-3-carboxamide of (11S,21R,S)-3-decladinosyl-11,12-dideoxy-6-O-methyl-3-ozo-12,11-oxycarbonyl-(2-(amino)-ethylamino)-methylene]-erythromycin A

### Example 589

### 2-Chloro-1-oxy-nicotinamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 590

### Oxo-(thien-2-yl)-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 591

### Oxo-(phenyl)-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 592

### 3-(2-Nitrophenyl)-2-oxo-propionamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 593

### 3-(1H-Indol-3-yl)-2-oxo-propionamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 594

### 2-Oxo-3-phenyl-propionamide of (11S,21R,S)-3-decladinosyl-11,12-dideoxy-6-O-methyl-3-oxo-12,11-[oxycarbonyl-(2-(amino)-ethylamino)-methylene]-erythromycin

### Example 595

### (2E)-3-(1H-Imidazol-4-yl)-acrylamide of (11S,21R,S)-3-decladinosyl-11,12-dideoxy-6-O-methyl-3-oxo-12,11-[oxycarbonyl-(2-amino)-ethylamino)-methylene]-erythromycin A

### Example 596

### (2E)-3-(1H-Indol-3-yl)-acrylamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 597

### 3-(1,3-Benzodioxol-5-yl)prop-2-ynamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 598

### (2E)-3-Quinolin-3-yl-acrylamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 599

### (2E)-3-Quinolin-2-yl-acrylamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 600

### (Quinoxalin-2-ylsulfanyl)-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

### Example 601

### [5-(5-Nitro-furan-2-yl)-[1,3,4]oxadiazol-2-ylsulfanyl]-acetamide of (11S,21R,S)-3-decladinosyl-11,12-dideoxy-6-O-methyl-3-oxo-12,11-[oxycarbonyl-(2-(amino)-ethylamino)-methylene]-erythromycin A

Example **427-601** were obtained starting from example **13** (5 mg) by following the same procedure as reported for Example **426**.
The name and amount of starting material (i.e carboxylic acid) and LC/MS analysis (retention time and m/z) of examples **427-601** are reported in table 6.

**Table 6**

| | | | | |
|---|---|---|---|---|
| **Ex.N** | **Carboxylic acid** | **Amount (mg)** | **Ret. time (min)** | **Mass analysis m/z [MH]**^{**+**} |
| **427** | 5-Methyl-3-phenyl-isoxazole-4-carboxylic acid | 1,5 | 6,75 | 855 |
| **428** | 1,5-Dimethyl-3-oxo-2-phenyl-2,3-dihydro-1*H*-pyrazole-4-carboxylic acid | 1,7 | 5,7 | 884 |
| **429** | 2-Trifluoromethyl-[1,8]naphthyridine-3-carboxylic acid | 1,8 | 5,82 | 894 |
| **430** | 6-Nitro-2-oxo-2*H*-chromene-3-carboxylic acid | 1,8 | 6,88 | 887 |
| **431** | 4-Amino-2-methylsulfanyl-pyrimidine-5-carboxylic acid | 1,4 | 5,94 | 837 |
| **432** | 5-Chloro-1-methyl-1*H*-pyrazole-4-carboxylic acid | 1,2 | 5,47 | 812 |
| **433** | 2-Pyrazin-2-yl-thiazole-4-carboxylic acid | 1,5 | 5,92 | 859 |
| **434** | 3-Methyl-2-oxo-1,2-dihydro-quinoline-4-carboxylic acid | 1,5 | 5,45 | 855 |
| **435** | 2-Methyl-imidazo[1,2,-*a*]pyridine-3-carboxylic acid | 1,3 | 5,64 | 828 |
| **436** | 4-Methoxy-1,3-dimethyl-1*H*-pyrazolo[3,4-*b*]pyridine-5-carboxylic acid | 1,7 | 5,7 | 873 |
| **437** | 3-Methyl-5-(4-methyl-[1,2,3]thiadiazol-5-yl)-isoxazole-4-carboxylic acid | 1,7 | 6,68 | 877 |
| **438** | 4-Acetyl-1-methyl-1*H*-pyrrole-2-carboxylic acid | 1,2 | 5,56 | 819 |
| **439** | 6-[1,2,4]Triazol-1-yl-nicotinic acid | 1,4 | 5,52 | 842 |
| **440** | Isonicotinic acid | 0,9 | 5,22 | 775 |
| **441** | 5-Oxo-2,3-dihydro-5*H*-thiazolo[3,2-*a*]pyrimidine-6-carboxylic acid | 1,5 | 5,35 | 850 |
| **442** | 5-Nitro-1*H*-pyrazole-3-carboxylic acid | 1,2 | 5,44 | 809 |
| **443** | 2-Methylsulfanyl-nicotinic acid | 1,3 | 6,07 | 821 |
| **444** | 7-Hydroxy-2-oxo-2*H*-chromene-3-carboxylic acid | 15 | 5,7 | 856 |
| **445** | Cinnoline-4-carboxylic acid | 1,3 | 5,7 | 826 |
| **446** | 6-Amino-nicotinic acid | 1,0 | 5,1 | 790 |
| **447** | 1-Methyl-5-nitro-1*H*-pyrazole-4-carboxylic acid | 1,3 | 6 | 823 |
| **448** | 4,7-Dimethyl-pyrazolo[5,1-*c*][1,2,4]triazine-3-carboxylic acid | 1,4 | 6,4 | 844 |
| **449** | 2-Methoxy-nicotinic acid | 1,1 | 6,2 | 805 |
| **450** | 3,5-Dimethyl-isoxazole-4-carboxylic acid | 1,1 | 6 | 793 |
| **451** | 4-Oxo-4,5,6,7-tetrahydro-benzofuran-3-carboxylic acid | 1,3 | 6,2 | 832 |
| **452** | 3-Amino-pyrazine-2-carboxylic acid | 1,0 | 5,9 | 791 |
| **453** | Pyrazine-2-carboxylic acid | 0,9 | 5,6 | 776 |
| **454** | 4-Phenyl-[1,2,3]thiadiazole-5-carboxylic acid | 1,5 | 7,1 | 858 |
| **455** | 5-Methyl-3-methylsulfanyl-isothiazole-4-carboxylic acid | 1,4 | 6,7 | 841 |
| **456** | 2,6-Dimethyl-4-oxo-4*H*-pyran-3-carboxylic acid | 1,3 | 5,5 | 820 |
| **457** | 1-Oxo-1,2-dihydro-isoquinoline-4-carboxylic acid | 1,4 | 5,5 | 841 |
| **458** | 4-Acetyl-3-cyano-5-methyl-1*H*-pyrrole-2-carboxylic acid | 1,4 | 5,8 | 844 |
| **459** | 4-Methyl-3-oxo-3,4-dihydro-2*H*-benzo[1,4]thiazine-6-carboxylic acid | 1,7 | 6,2 | 875 |
| **460** | 6-Methyl-imidazo[2,1-*b*]thiazole-5-carboxylic acid | 1,4 | 5,8 | 834 |
| **461** | 2-Phenoxy-nicotinic acid | 1,6 | 6,9 | 867 |
| **462** | 5-Nitro-1*H*-indole-2-carboxylic acid | 1,5 | 6,9 | 858 |
| **463** | 4,8-Dihydroxy-quinoline-2-carboxylic acid | 1,5 | 5,6 | 857 |
| **464** | 2-Hydroxy-quinoline-4-carboxylic acid | 1,4 | 5,5 | 841 |
| **465** | (1*H*-Indol-3-yl)-oxo-acetic acid | 1,4 | 6,7 | 841 |
| **466** | Furan-2-yl-oxo-acetic acid | 1,0 | 6,3 | 792 |
| **467** | 2-Amino-nicotinic acid | 1,0 | 9.1 | 790 |
| **468** | 1*H*-Benzotriazole-5-carboxylic acid | 1.2 | 6.9 | 815 |
| **469** | 3-Methyl-furan-2-carboxylic acid | 1.0 | 11 | 778 |
| **470** | 5-Chloro-1,3-dimethyl-1*H*-pyrazole-4-carboxylic acid | 1.3 | 5.7 | 826 |
| **471** | 4-Nitro-1*H*-pyrazole-3-carboxylic acid | 1,2 | 5,40 | 809 |
| **472** | 4,6-Dimethyl-2-oxo-2*H*-pyran-5-carboxylic acid | 1,3 | 5,48 | 820 |
| | | | 5,89 | |
| **473** | 2-Amino-5-chloropyrimidine-4-carboxylic acid | 1,3 | 5,49 | 825 |
| **474** | 5-Methyl-1*H*-pyrazole-3-carboxylic acid | 0,9 | 5,16 | 778 |
| **475** | 1-Methyl-5-oxopyrrolidine-3-carboxylic acid | 1,1 | 4,67 | 795 |
| **476** | 2-Chloro-6-methylnicotinic acid | 1,3 | 5,75 | 823 |
| **477** | (4R)-2-Thioxo-1,3-thiazolidine-4-carboxylic acid | 1,2 | 5,72 | 815 |
| **478** | 2,2-Dimethyl-5-oxotetrahydrofuran-3-carboxylic acid | 1,2 | 5,66 | 810 |
| **479** | 2,4-Dioxa-1,2,3,4-tetrahydropyrimidine-5-carboxylic acid | 1,3 | 4,70 | 808 |
| **480** | 2-(Methoxycarbonyl-nicotinic acid | 1,4 | 5,79 | 833 |
| **481** | 2-Methyl-1,8-naphthyridine-3-carboxylic acid | 1,4 | 5,05 | 840 |
| **482** | 2-(Trifluoromethyl)-1,6-naphthyridine-3-carboxylic acid | 1,8 | 6,15 | 894 |
| **483** | 1-Benzyl-5-oxopyrrolidine-3-carboxylic acid | 1,6 | 5,93 | 871 |
| **484** | 3-(Aminocarbonyl)pyrazine-2-carboxylic acid | 1,2 | 4,66 | 819 |
| **485** | 4-Amino-2-methylpyrimidine-5-carboxylic acid | 1,1 | 4,98 | 805 |
| **486** | Isoxazole-5-carboxylic acid | 0,8 | 4,46 | 765 |
| | | | 5,55 | |
| **487** | 5-Methylisoxazole-3-carboxylic acid | 0,9 | 5,98 | 779 |
| **488** | 6-Cyanonicotinicacid | 1,1 | 5,90 | 800 |
| **489** | 1-Ethyl-3 inethyl-1*H*-pyrazole-5-carboxylic acid | 1,2 | 6,05 | 806 |
| **490** | 5-Methylisoxazole-4-carboxylic acid | 0,9 | 4,35 | 779 |
| **491** | 5-Oxo-1-(thien-2-ylmethyl)-pyrrolidule-3-carboxylic acid | 1,7 | 5,79 | 877 |
| **492** | 2-(Pyridin-2-ylcarbonyl)-benzoic acid | 1,7 | 6,18 | 879 |
| | | | 6,44 | |
| **493** | 1-(2-Furylmethyl)-5-oxopyrrolidine-3-carboxylic acid | 1,6 | 5,55 | 861 |
| **494** | 5-(Methoxycarbonyl)-pyridine-2- carboxylic acid | 1,4 | 6,24 | 833 |
| **495** | 2-(4-Methyl-1,2,3-thiadiazol-5-yl)-1,3-thiazole-4-carboxylic acid | 1,7 | 6,35 | 879 |
| **496** | 3-Oxo-2,3-dihydro-1*H*-indazole-4-carboxylic acid | 1,3 | 5,63 | 830 |
| **497** | 2-Methyl-1,6-naphthyridine-3-carboxylic acid | 1,4 | 5,19 | 840 |
| **498** | 4-Methyl-2-pyridin-4-yl-1,3-thiazole-5-carboxylic acid | 1,6 | 5,94 | 872 |
| **499** | 4-Methyl-2-pyridin-3-yl-1,3-thiazole-5-carboxylic acid | 1,6 | 5,94 | 872 |
| **500** | 4-(morpholin-4-ylmethyl)-benzoic acid hydrochloride | 1,9 | 5,68 | 873 |
| **501** | 6-(1*H*-Imidazol-1-yl)-nicotinic acid | 1,4 | 5,40 | 841 |
| **502** | 5-Methoxy-2-(1,3,5-trimethyl-1*H*-pyrazol-4-yl)-benzoic acid | 1,9 | 6,13 | 912 |
| **503** | 4-Methyl-2-pyrazin-2-yl-1,3-thiazole-5-carboxylic acid | 1,7 | 6,18 | 873 |
| **504** | 2,5-Dimethyl-1*H*-pyrrole-3-carboxylic acid | 1,0 | 5,64 | 791 |
| **505** | 4-{[(4,6-Dimethylpyrimidin-2-yl)amino]carbonyl}-5-methylisoxazole-3-carboxylic acid | 2,1 | 6,49 | 928 |
| **506** | 1-Methyl-1*H*-pyrazole-4-carboxylic acid | 0,9 | 5,03 | 778 |
| **507** | 5-Chloro-1-methyl-3-(trifluoromethyl)-1*H*-pyrazole-4-carboxylic acid | 1,7 | 6,57 | 880 |
| **508** | 1-Pyrimidin-2-ylpiperidine-4-carboxylic acid | 1,5 | 5,89 | 859 |
| **509** | 1-Methyl-3-(trifluoromethyl)-1*H*-pyrazole-4-carboxylic acid | 1,4 | 6,02 | 846 |
| **510** | (4R)-2-Oxo-1,3-thiazolidine-4-carboxylic acid | 1,1 | 5,11 | 799 |
| **511** | (2S)-1-Acetylpyrrolidine-2-carboxylic acid | 1,2 | 4,95 | 809 |
| **512** | 5-Nitro-furan-2-carboxylic acid | 1,2 | 6,18 | 809 |
| **513** | 1-Methylpyrrolidine-2-carboxylic acid | 1,1 | 5,28 | 781 |
| **514** | 4-Acetyl-3,5-dimethyl-1*H*-pyrrole-2-carboxylic acid | 1,4 | 5,63 | 833 |
| **515** | 6-Methylnicotinic acid | 1,0 | 5,31 | 789 |
| **516** | 6-Methyl-3,4-dihydro-2*H*-pyran-5-carboxylic acid | 1,1 | 5,95 | 794 |
| **517** | 2,7-Dimethylpyrazolo[1,5-a]pyrimidine-6-carboxylic acid | 1,4 | 5,63 | 843 |
| **518** | 4-Methyl-1,2,3-thiadiazole-5-carboxylic acid | 1,1 | 6,01 | 796 |
| **519** | 4-(Trifluoromethyl)-nicotinic acid | 1,4 | 5,98 | 843 |
| **520** | 4-Oxo-4*H*-chromene-2-carboxylic acid | 1,4 | 6,2 | 842 |
| **521** | 5-Amino-1*H*-pyrazole-4-carboxylic acid | 0,9 | 5,1 | 779 |
| **522** | 2-Chloronicotinic acid | 1,2 | 5,7 | 809 |
| **523** | 2-Oxo-2*H*-pyran-5-carboxylic acid | 1,0 | 5,4 | 792 |
| | | | 5,8 | |
| **524** | Furan-3-carboxylic acid | 0,8 | 5,9 | 764 |
| **525** | 1,2,3-Thiadiazole-4-carboxylic acid | 1,0 | 5,8 | 782 |
| **526** | 1,5-Dimethyl-1*H*-pyrazole-3-carboxylic acid | 1,0 | 5,6 | 792 |
| **527** | 1*H*-Pyrazole-4-carboxylic acid | 0,8 | 4,5 | 764 |
| **528** | 8-Methyl-4-oxo-4*H*-pyrido[1,2-a]pyrimidine-3-carboxylic acid | 1,5 | 5,7 | 856 |
| **529** | 2,6-Dimethoxynicotinic acid | 1,4 | 6,9 | 835 |
| **530** | 4-Chloro-1,3-dimethyl-1*H*-pyrazolo[3,4-b]pyridine-5-carboxylic acid | 1,7 | 6,2 | 877 |
| **531** | 2-Methylnicotinic acid | 1,0 | 5,4 | 781 |
| **532** | 5-Amino-2,6-dioxo-1,2,3,6-tetrahydropyrimidine-4-carboxylic acid | 1,3 | 4,8 | 823 |
| **533** | 4-Hydroxy-3-(morpholin-4-ylmethyl)-benzoic acid | 1,9 | 6,0 | 889 |
| **534** | 5-Methylpyrazine-2-carboxylic acid | 1,0 | 5,8 | 790 |
| **535** | 6-Oxo-1,4,5,6-tetrahydropyridazine-3-carboxylic acid | 1,1 | 5,1 | 794 |
| **536** | 1*H*-pyrrole-2-carboxylic acid | 0,8 | 5,9 | 763 |
| **537** | 2,2-Dimethyl-4-oxo-3,4-dihydro-2*H*-pyran-6-carboxylic acid | 1,3 | 6,0 | 822 |
| **538** | 1-Methyl-1*H*-pyrrole-2-carboxylic acid | 0,9 | 6,3 | 777 |
| **539** | 2,3-Dihydro-1,4-bendioxine-2-carboxylic acid | 1,3 | 6,9 | 832 |
| **540** | 2,4-Dimethyl-1,3-thiazole-5-carboxylic acid | 1,2 | 5,8 | 809 |
| **541** | 1-Methyl-1,2,5,6-tetrahydropyridine-3-carboxylic acid | 1,3 | 4,9 | 793 |
| **542** | 1-Oxy-pyridine-2-carboxylic acid | 1,0 | 5,3 | 791 |
| **543** | 1-Acetylpiperidine-4-carboxylic acid | 1,3 | 5,1 | 823 |
| **544** | Tetrahydrofuran-3-carboxylic acid | 0,9 | 5,6 | 768 |
| **545** | (2S)-5-Oxopyrrolidine-2-carboxylic acid | 1,0 | 4,7 | 781 |
| **546** | 6-Hydroxynicotinic acid | 1,0 | 4,6/4,8 | 791 |
| **547** | 3,6-Dichloropyridazine-4-carboxylic acid | 1,4 | 6,4 | 844 |
| **548** | 1-Ethyl-7-methyl-4-oxo-1,4-dihydro-1,8-naphthyridine-3-carboxylic acid | 1,7 | 6,7 | 884 |
| **549** | Furan-2-carboxylic acid | 0,8 | 5,8 | 764 |
| **550** | Pyridine-2-carboxylic acid | 0,9 | 6,0 | 775 |
| **551** | Nicotinic acid | 0,9 | 5,3 | 775 |
| **552** | Tetrahydrofuran-2-carboxylic acid | 0,9 | 5,6 | 768 |
| **553** | 2-Methyl-furan-3-carboxylic acid | 0,9 | 6,3 | 778 |
| **554** | (2R)-Tetrahydrofuran-2-carboxylic acid | 0,9 | 5,6 | 768 |
| **555** | 2-(5-Oxopyrrolidin-2-ylsulfanyl)-benzoic acid | 1,8 | 5,7 | 889 |
| | | | 6,1 | |
| **556** | 1-Allyl-2-oxo-1,2-dilxydropyridine-3-carboxylic acid | 1,3 | 6,0 | 831 |
| **557** | 2-Methyl-4-(trifluoromethyl)-1,3-thiazole-5-carboxylic acid | 1,6 | 6,7 | 863 |
| **558** | 6-Hydroxy-1-methyl-2-oxo-1,2-dihydropyrimidine-4-carboxylic acid | 1,3 | 5,1 | 822 |
| **559** | 3-Methylisoxazole-4-carboxylic acid | 0,9 | 5,9 | 779 |
| **560** | 5-Methoxy-1,3-oxazole-2-carboxylic acid | 1,1 | 5,4 | 795 |
| | | | 5,8 | |
| **561** | 2-[6-(Acetylamino)-pyridin-3-ylsulfanyl]-nicotinic acid | 2,2 | 5,9 | 941 |
| **562** | 4-(4-Methylpiperazin-1-yl)-3-nitro-benzoic acid | 2,0 | 6,0 | 917 |
| **563** | 4-Isopropyl-1,2,3-thiadiazole-5-carboxylic acid | 1,3 | 6,9 | 824 |
| **564** | 3-Oxo-2-phenyl-2,3-dihydropyridazine-4-carboxylic acid | 1,6 | 6,7 | 868 |
| **565** | 5-Bromo-2-oxo-1,2-dihydropyridine-3-carboxylic acid | 1,6 | 5,6 | 869 |
| **566** | 1-(Methoxycarbonyl)piperidine-4-carboxylic acid | 1,4 | 5,6 | 839 |
| **567** | 1-(6-Chloropyridazin-3-yl)piperidine-4-carboxylic acid | 1,8 | 6,0 | 893 |
| **568** | 4-Chloro-6-oxo-1-phenyl-1,6-dihydropyridazine-3-carboxylic acid | 1,9 | 6,2 | 902 |
| | | | 6,6 | |
| **569** | 1-(3-Chlorophenyl)-4-methoxy-6-oxo-1,6-dihydropyridazine-3-carboxylic acid | 2,1 | 6,2 | 932 |
| | | | 6,5 | |
| **570** | (1-Methyl-4-nitro-1*H*-pyrazol-5-yl)-acetic acid | 1,4 | 5,9 | 837 |
| **571** | (1,3-Dimethyl-2,6-dioxo-2,3,6,7-tetrahydro-1*H*-purin-8-yl)-acetic acid | 1,8 | 5,2 | 890 |
| **572** | 5-Oxo-4,5-dihydrofuran-3-carboxylic acid | 1,0 | 7,2 | 780 |
| **573** | (2R,4S)-4-(Acetyloxy)-1,3-oxathiolane-2-carboxylic acid | 1,4 | 6,0 | 844 |
| **574** | 3-Hydroxyisonicotinic acid | 1,0 | 5,2 | 791 |
| **575** | 3-Hydroxypyridine-2-carboxylic acid, | 1,0 | 6,7 | 791 |
| | | | 7,1 | |
| **576** | 2-Hydroxynicotinic acid | 1,0 | 5,0 | 791 |
| **577** | 7-Hydroxy-4-oxo-4*H*-chromene-2-carboxylic acid | 1,5 | 5,4 | 858 |
| **578** | 1-Oxy-nicotinic acid | 1,0 | 4,8 | 791 |
| **579** | 1*H*-Indole-2-carboxylic acid | 1,2 | 6,8 | 813 |
| **580** | 5-Methoxy-1*H*-indole-2-carboxylic acid | 1,4 | 6,7 | 843 |
| **581** | 6-chloro-4-oxo-4*H*-chromene-2-carboxylic acid | 1,7 | 7,0 | 876 |
| **582** | Quinoline-2-carboxylic acid | 1,3 | 7,1 | 825 |
| **583** | 4-Hydroxy-6-methoxyquinoline-2-carboxylic acid | 1,6 | 5,7 | 871 |
| **584** | 4-Methyl-3-oxo-3,4-dihydroquinoxaline-2-carboxylic acid | 1,5 | 5,8 | 856 |
| **585** | 4-(2-Hydroxyethyl)-3-oxo-3,4-dihydroquinoxaline-2-carboxylic acid | 1,7 | 5,3 | 886 |
| **586** | 3-Methoxyquinoxaline-2-carboxylic acid | 1,5 | 6,6 | 856 |
| **587** | 1-Benzofuran-2-carboxylic acid | 1,2 | 6,8 | 814 |
| **588** | 6-Ethyl-5-oxothiomorpholine-3-carboxylic acid | 1,4 | 5,8 | 841 |
| **589** | 2-Chloro-1-oxy-nicotinic acid | 1,3 | 4,5 | 825 |
| **590** | Oxo(thien-2-yl)-acetic acid | 1,2 | 6,9 | 808 |
| **591** | Oxo-(phenyl)-acetic acid | 1,1 | 6,9 | 802 |
| **592** | 3-(2-Nitrophenyl)-2-oxo-propionic acid | 1,6 | 6,7 | 861 |
| | | | 7,1 | |
| **593** | 3-(1*H*-Indol-3-yl)-2-oxo-propionic acid | 1,5 | 8,1 | 855 |
| **594** | 2-Oxo-3-phenyl-propionic acid | 1,2 | 6,7 | 816 |
| | | | 7,3 | |
| **595** | (2E)-3-(1*H*-Imidazol-4-yl)-acrylic acid | 1,0 | 4,9 | 790 |
| **596** | (2E)-3-(1*H*-Indol-3-yl)-acrylic acid | 1,4 | 6,4 | 839 |
| | | | 6,7 | |
| **597** | 3-(1,3-Benzodioxol-5-yl)prop-2-ynoic acid | 1,4 | 6,8 | 842 |
| **598** | (2E)-3-Quinolin-3-yl-acrylic acid | 1,5 | 6,2 | 851 |
| **599** | (2E)-3-Quinolin-2-yl-acrylic acid | 1,5 | 6,2 | 851 |
| **600** | (Quinoxalin-2-ylsulfanyl)-acetic acid | 1,6 | 6,7 | 872 |
| **601** | [5-(5 Nitro-furan-2-yl)-[1,3,4]oxadiazol-2 ylsulfanyl]-acetic acid | 2,0 | 6,3 | 923 |
| | | | 6,3 | |

### Pharmacy examples

**Tablets**

| | mg/tab |
|---|---|
| Active ingredient | 320 |
| Lactose | 150 |
| Ethyl cellulose | 20 |
| Sodium lauryl sulphate | 7 |
| Magnesium stearate | 3 |
| Tablet core | 500 |

The active ingredient and the lactose are blended together and then granulated using water as granulating fluid. The dried granules are blended with ethyl cellulose, sodium lauryl sulphate and magnesium stearate and the tablet core formed using an appropriate punch. The tablet may be coated using conventional technique and coatings.

### Injection

The sterile vials were filled with the sterile active ingredient (500 mg). Purge the vial head space with sterile nitrogen; close the vials using rubber and metal overseals. The product may be constituted by dissolving in water for injection(10 ml) or other suitable sterile vehicle for injection shortly before administration.

### Activity data

The value of MIC (microbial inhibition concentration), obtained according to NCCLS (National Committee for Clinical Laboratory Standards), of the preferred compounds of the invention against erythromycin susceptible Streptococcus pneumoniae and Streptococcus pyogenes are less then or equal to 1 ug/ml.

In particular Examples 70, 56, 54, 49, 51, 58, 75, 66, 47, 52, 117 showed MIC<=0.1 ug/ml against erythromycin susceptible Streptococcus, pneumoniae strains. Furthermore Examples 70, 56, 54, 49, 51, 58, 75, 66, 47, 52, 117 showed MIC in the range <= 8 - 0.06ug/ml against erythromycin resistant Streptococcus pneumoniae strains.

## Claims

1. A compound of formula (I) 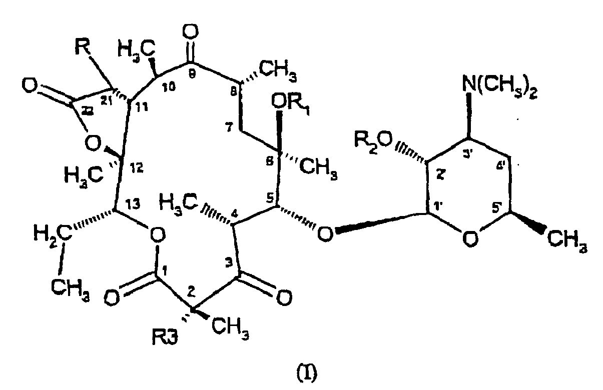 wherein
R is (CH₂)ₙA-X-R₄ or (CH₂)ₙR₅;
A is a group selected from -N(R₆)-, -N[C(O)R₆]-, -N(R₆)C(O)-, -N(R₆)S(O)₂-,-N(R₆)C(O)O-, -N=C(R₆)- or -N(R₆)C(Y)N(R₇)-;
R₁ is C₁₋₆ alkyl or C₃₋₆ alkenyl;
R₂ is hydrogen or acyl;
R₃ is hydrogen or halogen;
X is a bond, a C₁₋₁₀ alkylene, a C₂₋₁₀ alkenylene or a C₂₋₁₀ alkynylene chain wherein said chains are:
i) optionally interrupted by a bivalent radical group selected from -O-, -N(R₈)-,-C(O)-, -N(R₈)C(Y)N(R₉)-, -S(O)m-, -N(R₈)C(O)-, -C(O)N(R₈)-, -N(R₈)C(O)C(O)-, -C(O)O- or -C(NOR₆)- and/or
ii) optionally substituted by one or two groups selected from:
C₁₋₄alkyl, oxo, C₁₋₄ alkoxy, halogen, cyano, phenoxy, hydroxy, NR₈R₉, N(R₈)C(O)R₉, =NOR₆, NR₈C(Y)NR₉ or optionally substituted phenyl;
R₄ is selected from:
hydrogen,
optionally substituted phenyl,
optionally substituted C₃₋₇ cycloalkyl,
optionally substituted 9 to 10 membered fused bicyclic carbocyclic,
optionally substituted 5 or 6 membered heteroaryl in which the 5-membered heteroaryl contains at least one heteroatom selected from oxygen, sulphur or nitrogen and the 6-membered heteroaryl group contains from 1 to 3 nitrogen atoms,
optionally substituted 5-6 membered heterocyclic,
or
R₄ is an optionally substituted 9 or 10 membered fused bicyclic heterocyclic having at least one heteroatom selected from oxygen, sulphur or nitrogen,
wherein
the term optionally substituted phenyl, optionally substituted 5-6 membered heterocyclic group, optionally substituted 9 to 10 membered fused bicyclic carbocyclic group, optionally substituted 9 to 10 membered fused bicyclic heterocyclic group or optionally substituted 5 or 6 membered heteroaryl group refers to a 5-6 membered heterocyclic, a 9 to 10 membered fused bicyclic carbocyclic, a 9 to 10 membered used bicyclic heterocyclic or 5 or 6 membered heteroaryl as defined above which is substituted by 1 to 4 groups, which may be the same or different, selected from (CH₂)ₚR₁₀ group wherein p is zero or an integer from 1 to 4 and R₁₀ is selected from:
halogen,
C₁₋₄alkoxy,
C₁₋₄alkyl,
hydroxy,
cyano,
nitro,
oxo,
trifluoromethyl,
carboxy,
NR₈R₉,
COR₈,
CONR₈R₉,
NHCOR₈,
NHSO₂R₈,
S(O)qR₆ (wherein q is 0 or an integer from 1 to 2),
phenyl (optionally substituted by halogen, C₁₋₄alkoxy or NR₈R₉);
phenoxy;
5-membered heteroaryl containing at least 1 heteroatom selected from oxygen, sulphur or nitrogen and a 6-membered heteroaryl group containing at least 1 nitrogen atom which 5-6 membered heteroaryl may be substituted by C₁₋₄alkyl or cyano,
or
9 or 10 membered fused bicyclic heterocyclic, and
the term optionally substituted C₃₋₇ cycloalkyl refers to a group optionally substituted by 1 or 2 substituents which may be the same or different and are selected from C₁₋₄ alkyl, halogen, cyano, nitro, trifluoromethyl and NR₆R₇;
R₅ is a 5 or 6 membered heterocyclic containing at least one nitrogen, optionally substituted by one or two groups selected from oxo or 9 or 10 membered fused bicyclic heterocyclic having at least one heteroatom selected from oxygen, sulphur or nitrogen;
R₆ and R₇ are independently hydrogen, C₁₋₄ alkyl or phenyl which is optionally substituted by one or two C₁₋₄ alkyl groups;
R₈ and R₉ are independently hydrogen, phenyl (which may be substituted by one or two C₁₋₄ alkyl) or R₈ and R₉ are independently C₁₋₄ alkyl which is optionally substituted by 1 or 2 groups selected from:
phenyl, C₁₋₄ alkoxy,
cyano,
5-membered heteroaryl containing 1 or 2 heteroatoms selected from oxygen, sulphur or nitrogen or 6-membered heteroaryl group containing from 1 to 3 nitrogen atoms,
hydroxy,
oxo,
carboxy;
Y is an oxygen or a sulphur atom;
n is 0 or an integer from 1 to 3;
m is 0, 1 or 2;
and pharmaceutically acceptable salts and solvates thereof.

2. A compound as claimed in claim 1 wherein R₁ is methyl or 2-propenyl.

3. A compound as claimed in claim 1 or 2 wherein R₂ is hydrogen.

4. A compound as claimed in any claims 1 to 3 wherein R₃ is hydrogen or fluorine.

5. A compound as claimed in any claims 1 to 4 wherein A is selected from-NH-, -NHC(O)- or -NHC(Y)NH-.

6. A compound as claimed in any claims 1 to 5 wherein X is a C₁₋₄ alkylene chain which is optionally interrupted by a bivalent radical selected from -O-, -NH-, -C(O)-,-NHC(O)-, -S(O)₂- and -S- and /or such a C₁₋₄ alkylene chain is optionally substituted by one group selected from NH₂, C₁₋₄ alkyl, oxo or N-OH.

7. A compound selected from:
(11S,21S)-3-decladinosyl-11,12-dideoxy-6-O-methyl-3-oxo-12,11-[oxycarbonyl-(2-(4-(pyridin-3-yl)-imidazol-1-yl)-ethylamino)-methylene]-erythromycin A;
(11S,21S)-3-decladinosyl-11,12-dideoxy-6-O-methyl-3-oxo-12,11-[oxycarbonyl-(2-(quinolin-4-yl)-ethylamino)-methylene]-erythromycin A;
(11S,21S)-3-decladinosyl-11,12-dideoxy-6-O-methyl-3-oxo-12,11-[oxycarbonyl-(3-(4-(pyridin-3-yl)-imidazol-1-yl)-propylamino)-methylene]-erythromycin A;
(11S,21S)-3-decladinosyl-11,12-dideoxy-6-O-methyl-3-oxo-12,11-[oxycarbonyl-(2-(quinoxalin-2-yl-sulfanyl)-acetamido)-methylene]-erythromycin A;
(11S, 21S)-3-decladinosyl-11,12-dideoxy-6-O-methyl-3-oxo-12,11-[oxycarbonyl-((4-(2,3-dihydro-benzo[1,4]dioxin-6-yl)-4-oxo)-butyramido)-methylene]-erythromycin A;
(11S,21S)-3-decladinosyl-11,12-dideoxy-6-O-methyl-3-oxo-12,11-[oxycarbonyl-(4-(4-methoxy-3-nitro-phenyl)-4-oxo-butyramido)-methylene]-erythromycin A;
(11S,21S)-3-decladinosyl-11,12-dideoxy-6-O-methyl-3-oxo-12,11-[oxycarbonyl-(4-(2-hydroxy-4,5-dimethoxy-phenyl)-4-oxo-butyramido)-methylene]-erythromycin A;
(11S,21S)-3-decladinosyl-11,12-dideoxy-6-O-methyl-3-oxo-12,11-[oxycarbonyl-(4-(4-oxo-4,5,6,7-tetrahydro-benzo[b]thiophen-2-yl)-4-oxo-butyramido)-methylene]-erythromycin A;
(11S,21S)-3-decladinosyl-11,12-dideoxy-6-O-methyl-3-oxo-12,11-[oxycarbonyl-(3-(4-quinolin-2-yl-1H-pyrazol-1-yl)propylamino)-methylene]-erythromycin A;
(11S,21S)-3-decladinosyl-11,12-dideoxy-6-O-methyl-3-oxo-12,11-[oxycarbonyl-(4-(3,4-dimethoxy-phenyl)-4-oxo-butyramido)-methylene]-erythromycin A;
(11S, 21S)-3-decladinosyl-11,12-dideoxy-6-O-methyl-3-oxo-12,11-[oxycarbonyl-(4-(4-hydroxy-3-methoxy-phenyl)-4-oxo-butyramido)-methylene]-erythromycin A; and
(11S, 21S)-3-decladinosyl-11,12-dideoxy-6-O-methyl-3-oxo-12,11-[oxycarbonyl-(3-(4-(thiophen-2-yl)-imidazol-1-yl)-propylamino)-methylene]-erythromycin A.

8. A process for the preparation of a compound of formula (I) or a pharmaceutically acceptable salt or solvate thereof
which comprises one of:
a) reacting a compound of formula (II) 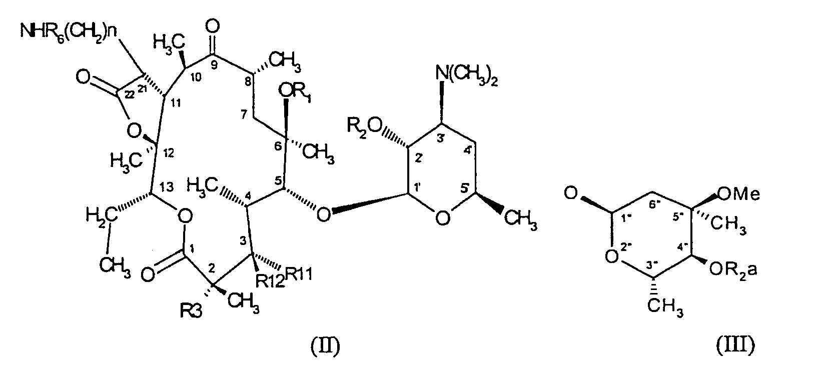 wherein R₁, R₂, R₃ and R₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a suitable activated derivative of the acid HOC(O)XR₄ (IV) or with a suitable activated derivative of the sulfonic acid (V) HOS(O)₂XR₄ (V) respectively wherein R₄ and X have the meanings defined in claim 1, to produce a compound of formula (I) wherein R is (CH₂)ₙA-X-R₄, A is -N(R₆)C(O)- or-N(R₆)S(O)₂-, and X and n have the meanings defined in claim 1; or
b) reacting a compound of formula (II) wherein R₁, R₂, R₃ and F₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a compound of formula R₄XN(R₇)C(Y)L (VI), wherein L is a suitable leaving group, R₇ is phenyl or C₁₋₄ alkyl, and R₄ and X have the meanings defined in claim 1, to produce a compound of formula (I) wherein R is (CH₂)ₙA-X-R₄, A is -N(R₆)C(Y)N(R₇)-, and X and n have the meanings defined in claim 1; or
c) reacting a compound of formula (II) wherein R₁, R₂, R₃ and R₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a compound of formula R₄XN=C(Y) (VII), wherein R₄ , Y and X have the meanings defined in claim 1, to produce a compound of formula (I) wherein R is (CH₂)ₙA-X-R₄, A is-N(R₆)C(Y)NH-, X and n have the meanings defined in claim 1; or
d) reacting a compound of formula (II) wherein R₁, R₂, R₃ and R₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a compound of formula R₄XL (VIII), wherein R₄ and X have the meanings defined in claim 1 and L is suitable leaving group, to produce a compound of formula (I) wherein R is (CH₂)ₙAX-R₄, A is -N(R₆)-, X and n have the meanings defined in claim 1; or
e) reacting a compound of formula (II) wherein R₁, R₂, R₃ and R₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a compound of formula R₄XOC(O)L (IX) wherein L is a suitable leaving group, and R₄ and X have the meanings defined in claim 1, to produce a compound of formula (I) wherein R is (CH₂)ₙA-X-R₄, A is N(R₆)C(O)O, X and n have the meanings defined in claim 1; or
f) reacting a compound of formula (II) wherein R₁, R₂, R₃ and R₆ have the meanings defined in claim 1, R₁₁ is a cladinose derivative of formula (III), in which R₂ₐ is a hydroxy protecting group, R₁₂ is hydrogen or R₁₁ together with R₁₂ is an oxygen atom, with a compound of formula R₄XCHO (X), wherein R₄ and X have the meanings defined in claim 1, to produce a compound of formula (I) wherein R is (CH₂)ₙA-X-R₄, A is N=C(R₆), X and n have the meanings defined in claim 1; or
g) reacting a compound of formula (XI), wherein R₁, R₂, R₃, R₄ and X have the meanings defined in claim 1, R₁₁ is a cladinose derivative of formula (III), in which R₂ₐ is a hydroxy protecting group, and R₁₂ is hydrogen, with a compound of formula R₆COOH (XIa) wherein R₆ has the meaning defined in claim 1, 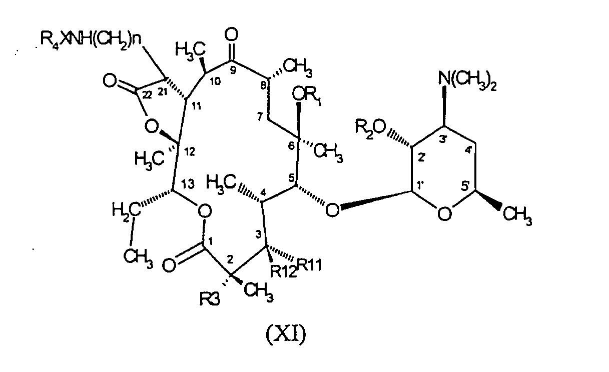 to produce a compound of formula (I) wherein R is (CH₂)ₙA-X-R₄, A is [NC(O)R₆], X and n have the meanings defined in claim 1; or
h) reacting a compound of formula (XVII), wherein R₁, R₂ and R₃ have the meanings defined in claim 1, R₁₁ is a cladinose derivative of formula (III), in which R₂ₐ is a hydroxy protecting group, and R₁₂ is hydrogen and R₁₃ is C₁₋₄ alkyl, with an aldehyde of formula 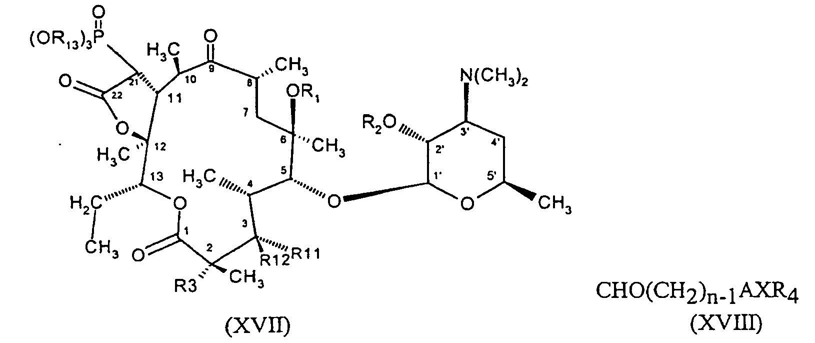 wherein R₄, X and n have the meanings defined in claim 1, to produce a compound of formula (I) wherein R is (CH₂)ₙAXR₄, and A and X have the meanings defined in claim 1; and n is an integer from 2 or 3; or
i) reacting a compound of formula (I) in which R₁ has the meanings defined in claim 1, R₃ is hydrogen and R₂ is hydroxy protecting group with a halogenating agent to produce a compound of formula (I) wherein R₃ is halogen;
j) cyclisation of a compound of formula (XXXII), wherein L is suitable leaving group such as halogen (i.e chlorine or bromine), R₁, R₂, R₃ have the meanings defined in claim 1, R₁₁ is a cladinose derivative of formula (III), in which R₂ₐ is a hydroxy protecting group, and R₁₂ is hydrogen, X is C₄₋₅ alkylene chain optionally substituted by one or two groups selected from oxo, 9 or 10 membered fused bicyclic heterocyclic having at least one heteroatom selected from oxygen, sulphur or nitrogen, 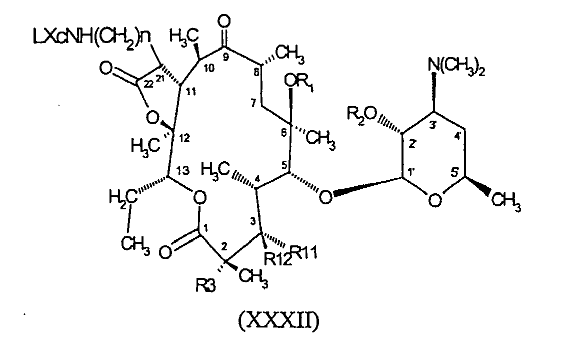
to produce a compound of formula (I) wherein R₁ is (CH₂)ₙR₅;
and thereafter, if required, subjecting the resulting compound to one or more of the following operations:
i) hydrolysis of the cladinose derivative (III);
ii) conversion of the 3-hydroxy group into the 3-oxo;
iii) removal of the protecting group R₂ and
iv) conversion of the resultant compound of formula (I) into a pharmaceutically acceptable salt and solvates thereof.

9. A compound as claimed in any claims 1 to 7 for use in therapy.

10. The use of a compound as claimed in any claims 1 to 7 in the preparation of a medicament for use in the therapy of systemic or topical bacterial infections in a human or animal body.

11. A pharmaceutical composition comprising a compound as claimed in any claims 1 to 7 in admixture with one or more pharmaceutically acceptable carriers or excipients.

## Patentansprüche

1. Verbindung der Formel (I) 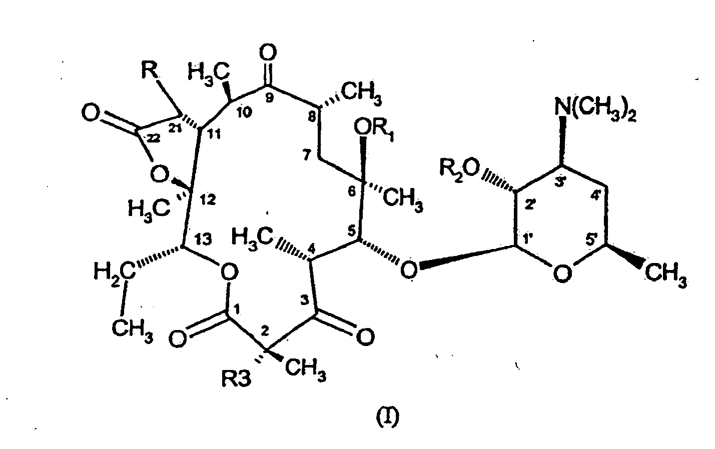 wobei
R für (CH₂)ₙA-X-R₄ oder (CH₂)ₙR₅ steht;
A ein Rest ausgewählt aus -N(R₆)-, -N[C(O)R₆]-, -N(R₆)C(O)-, -N(R₆)S(O)₂-, -N(R₆)C(O)O-, -N=C(R₆)- oder -N(R₆)C(Y)N(R₇)- ist;
R₁ für C₁₋₆-Alkyl oder C₃₋₆-Alkenyl steht;
R₂ ein Wasserstoffatom oder Acyl ist;
R₃ ein Wasserstoffatom oder Halogenatom ist;
X eine Bindung, eine C₁₋₁₀-Alkylen-, eine C₂₋₁₀-Alkenylen- oder eine C₂₋₁₀-Alkinylenkette ist, wobei die Ketten
i) gegebenenfalls durch einen zweiwertigen Rest, ausgewählt aus -O-,-N(R₈)-, -C(O)-, -N(R₈)C(Y)N(R₉)-, -S(O)m-, N(R₈)C(O)-, -C(O)N(R₈)-, -N(R₈)C(O)C(O)-, -C(O)O- oder -C(NOR₆)- unterbrochen sind, und/oder
ii) gegebenenfalls mit einem oder zwei Resten ausgewählt aus:
C₁₋₄-Alkyl, Oxo, C₁₋₄-Alkoxy, Halogen, Cyano, Phenoxy, Hydroxy, NR₈R₉, N(R₈)C(O)R₉, =NOR₆, NR₈C(Y)NR₉ oder gegebenenfalls substituiertem Phenyl, substituiert sind;
R₄ ausgewählt ist aus:
Wasserstoff,
gegebenenfalls substituiertem Phenyl,
gegebenenfalls substituiertem C₃₋₇-Cycloalkyl,
einem gegebenenfalls substituierten 9- bis 10-gliedrigen kondensierten bicyclischen carbocyclischen Rest,
einem gegebenenfalls substituierten 5- oder 6-gliedrigen Heteroarylrest, wobei der 5-gliedrige Heteroarylrest mindestens ein Heteroatom ausgewählt aus Sauerstoff, Schwefel oder Stickstoff enthält, und der 6-gliedrige Heteroarylrest 1 bis 3 Stickstoffatome enthält,
einem gegebenenfalls substituierten 5- bis 6-gliedrigen heterocyclischen Rest,
oder
R₄ ein gegebenenfalls substituierter 9- oder 10-gliedriger kondensierter bicyclischer heterocyclischer Rest mit mindestens einem Heteroatom ausgewählt aus Sauerstoff, Schwefel oder Stickstoff ist,
wobei
der Begriff "gegebenenfalls substituiertes Phenyl, gegebenenfalls substituierter 5- bis 6-gliedriger heterocyclischer Rest, gegebenenfalls substituierter 9- bis 10-gliedriger kondensierter bicyclischer carbocyclischer Rest, gegebenenfalls substituierter 9- bis 10-gliedriger kondensierter bicyclischer heterocyclischer Rest oder gegebenenfalls substituierter 5- oder 6-gliedriger Heteroarylrest sich auf einen 5- bis 6-gliedrigen heterocyclischen Rest, einen 9- bis 10-gliedrigen kondensierten bicyclischen carbocyclischen Rest, einen 9- bis 10-gliedrigen kondensierten bicyclischen heterocyclischen Rest oder einen 5- oder 6-gliedrigen Heteroarylrest wie oben stehend definiert bezieht, welcher mit 1 bis 4 Resten substituiert ist, die gleich oder verschieden sein können, ausgewählt aus einem Rest (CH₂)ₚR₁₀, wobei p gleich 0 oder eine ganze Zahl von 1 bis 4 ist und R₁₀ ausgewählt ist aus:
Halogen,
C₁₋₄-Alkoxy,
C₁₋₄-Alkyl,
Hydroxy,
Cyano,
Nitro,
Oxo,
Tri fluormethyl,
Carboxy,
NR₈R₉,
COR₈,
CONR₈R₉,
NHCOR₈,
NHSO₂R₈,
S(O)qR₆ (wobei q gleich 0 oder eine ganze Zahl von 1 bis 2 ist),
Phenyl (gegebenenfalls substituiert mit Halogen, C₁₋₄-Alkoxy oder NR₈R₉), Phenoxy,
einem 5-gliedrigen Heteroarylrest, welcher mindestens 1 Heteroatom ausgewählt aus Sauerstoff, Schwefel oder Stickstoff enthält und einem 6-gliedrigen Heteroarylrest, welcher mindestens 1 Stickstoffatom enthält, wobei der 5- bis 6-gliedrige Heteroarylrest mit C₁₋₄-Alkyl oder Cyano substituiert sein kann,
oder
einem 9- oder 10-gliedrigen kondensierten bicyclischen heterocyclischen Rest, und
der Begriff gegebenenfalls substituierter C₃₋₇-Cycloalkylrest sich auf einen Rest bezieht, welcher gegebenenfalls mit 1 oder 2 Substituenten substituiert ist, welche gleich oder verschieden sein können und aus C₁₋₄-Alkyl, Halogen, Cyano, Nitro, Trifluormethyl und NR₆R₇ ausgewählt sind;
R₅ ein 5- oder 6-gliedriger heterocyclischer Rest ist, welcher mindestens ein Stickstoffatom enthält, gegebenenfalls substituiert mit einem oder zwei Resten ausgewählt aus Oxo, oder einem 9- oder 10-gliedrigen kondensierten bicyclischen heterocyclischen Rest mit mindestens einem Heteroatom ausgewählt aus Sauerstoff, Schwefel oder Stickstoff;
R₆ und R₇ unabhängig Wasserstoff, C₁₋₄-Alkyl oder Phenyl, gegebenenfalls substituiert mit einem oder zwei C₁₋₄-Alkylresten, darstellen;
R₈ und R₉ unabhängig Wasserstoff, Phenyl (welches gegebenenfalls mit einem oder zwei C₁₋₄-Alkylresten substituiert sein kann) darstellen oder R₈ und R₉ unabhängig C₁₋₄-Alkyl darstellen, welches gegebenenfalls mit 1 oder 2 Resten ausgewählt aus:
Phenyl, C₁₋₄-Alkoxy,
Cyano,
einem 5-gliedrigen Heteroarylrest, welcher 1 oder 2 Heteroatome ausgewählt aus Sauerstoff, Schwefel oder Stickstoff enthält oder 6-gliedrigen Heteroarylrest, welcher 1 bis 3 Stickstoffatome enthält,
Hydroxy,
Oxo,
Carboxy,
substituiert ist;
Y ein Sauerstoff- oder Schwefelatom darstellt;
n gleich 0 oder eine ganze Zahl von 1 bis 3 ist;
m gleich 0, 1 oder 2 ist;
und pharmazeutisch verträgliche Salze und Solvate davon.

2. Verbindung gemäß Anspruch 1, wobei R₁ Methyl oder 2-Propenyl ist.

3. Verbindung gemäß Anspruch 1 oder 2, wobei R₂ ein Wasserstoffatom ist.

4. Verbindung gemäß einem der Ansprüche 1 bis 3, wobei R₃ Wasserstoff oder Fluor ist.

5. Verbindung gemäß einem der Ansprüche 1 bis 4, wobei A ausgewählt ist aus -NH-, -NHC(O)- oder -NHC(Y)NH-.

6. Verbindung gemäß einem der Ansprüche 1 bis 5, wobei X eine C₁₋₄-Alkylenkette darstellt, welche gegebenenfalls durch einen zweiwertigen Rest ausgewählt aus -O-, -NH-, -C(O)-, -NHC(O)-, -S(O)₂- und -S- unterbrochen ist, und/oder diese C₁₋₄₋Alkylenkette gegebenenfalls mit einem Rest ausgewählt aus NH₂, C₁₋₄Alkyl, Oxo oder N-OH substituiert ist.

7. Verbindung ausgewählt aus:
(11S,21S)-3-Decladinosyl-11,12-dideoxy-6-O-methyl-3-oxo-12,11-[oxycarbonyl-(2-(4-(pyridin-3-yl)imidazol-1-yl)ethylamino)methylen]erythromycin A;
(11S,21S)-3-Decladinosyl-11,12-dideoxy-6-O-methyl-3-oxo-12,11-[oxycarbonyl-(2-(chinolin-4-yl)ethylamino)methylen]erythromycin A;
(11S,21S)-3-Decladinosyl-11,12-dideoxy-6-O-methyl-3-oxo-12,11-[oxycarbonyl-(3-(4-(pyridin-3-yl)imidazol-1-yl)propylamino)methylen]erythromycin A;
(11S,21S)-3-Decladinosyl-11,12-dideoxy-6-O-methyl-3-oxo-12,11-[oxycarbonyl-(2-(chinoxalin-2-yl-sulfanyl)acetamido)methylen]erythromycin A;
(11S,21S)-3-Decladinosyl-11,12-dideoxy-6-O-methyl-3-oxo-12,11-[oxycarbonyl-((4-(2,3-dihydro-benzo[1,4]dioxin-6-yl)-4-oxo)butyramido)methylen]erythromycin A;
(11S,21S)-3-Decladinosyl-11,12-dideoxy-6-O-methyl-3-oxo-12,11-[oxycarbonyl-(4-(4-methoxy-3-nitro-phenyl)-4-oxo-butyramido)methylen]erythromycin A;
(11S,21S)-3-Decladinosyl-11,12-dideoxy-6-O-methyl-3-oxo-12,11-[oxycarbonyl-(4-(2-hydroxy-4,5-dimethoxy-phenyl)-4-oxo-butyramido)methylen]erythromycin A;
(11S,21S)-3-Decladinosyl-11,12-dideoxy-6-O-methyl-3-oxo-12,11-[oxycarbonyl-(4-(4-oxo-4,5,6,7-tetrahydro-benzo[b]thiophen-2-yl)-4-oxo-butyramido)methylen]erythromycin A;
(11S,21S)-3-Deladinosyl-11,12-dideoxy-6-O-methyl-3-oxo-12,11-[oxycarbonyl-(3-(4-chinolin-2-yl-1H-pyrazol-1-yl)propylamino)methylen]erythromycin A;
(11S,21S)-3-Dedadinosyl-11,12-dideoxy-6-O-methyl-3-oxo-12,11-[oxycarbonyl-(4-(3,4-dimethoxy-phenyl)-4-oxo-butyramido)methylen]erythromycin A;
(11S,21S)-3-Decladinosyl-11,12-dideoxy-6-O-methyl-3-oxo-12,11-[oxycarbonyl-(4-(4-hydroxy-3-methoxy-phenyl)-4-oxo-butyramido)methylen]erythromycin A; und
(11S,21S)-3-Decladinosyl-11,12-dideoxy-6-O-methyl-3-oxo-12,11-[oxycarbonyl-(3-(4-(thiophen-2-yl)imidazol-1-yl)propylamino)methylen]erythromycin A.

8. Verfahren zur Herstellung einer Verbindung der Formel (I) oder eines pharmazeutisch verträglichen Salzes oder Solvats davon
welches eines umfasst von
a) Umsetzen einer Verbindung der Formel (II) 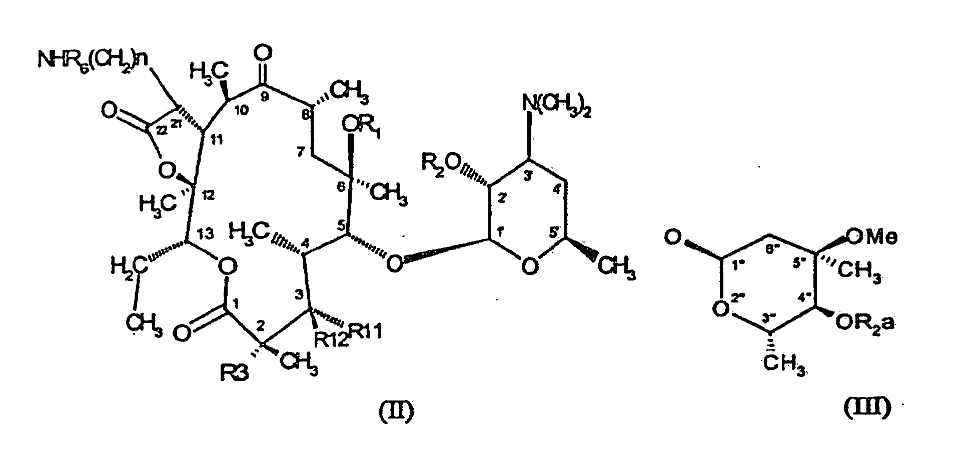 wobei R₁, R₂, R₃ und R₆ die in Anspruch 1 definierte Bedeutung haben, R₁₁ ein Cladinosederivat der Formel (III) ist, in welchem R₂ₐ eine Hydroxyschutzgruppe darstellt, R₁₂ ein Wasserstoffatom ist oder R₁₁ zusammen mit R₁₂ ein Sauerstoffatom ist, mit einem geeigneten aktivierten Derivat der Säure HOC(O)XR₄ (IV) oder mit einem geeigneten aktivierten Derivat der Sulfonsäure (V) HOS(O)₂XR₄ (V), wobei R₄ und X die in Anspruch 1 definierten Bedeutungen haben, um eine Verbindung der Formel (I) herzustellen, wobei R für (CH₂)ₙA-X-R₄ steht, A für -N(R₆)C(O)- oder -N(R₆)S(O)₂- steht, X und n die in Anspruch 1 definierten Bedeutungen haben; oder
b) Umsetzen einer Verbindung der Formel (II), wobei R₁, R₂, R₃ und R₆ die in Anspruch 1 definierten Bedeutungen haben, R₁₁ ein Cladinosederivat der Formel (III) ist, in welchem R₂ₐ eine Hydroxyschutzgruppe ist, R₁₂ ein Wasserstoffatom ist oder R₁₁ zusammen mit R₁₂ ein Sauerstoffatom ist, mit einer Verbindung der Formel R₄XN(R₇)C(Y)L (VI), wobei L eine geeignete Abgangsgruppe ist, R₇ ein Phenyl- oder C₁₋₄-Alkylrest ist, und R₄ und X die in Anspruch 1 definierten Bedeutungen haben, um eine Verbindung der Formel (I) herzustellen, wobei R für (CH₂)ₙA-X-R₄ steht, A für -N(R₆)C(Y)N(R₇)- steht und X und n die in Anspruch 1 definierten Bedeutungen haben; oder
c) Umsetzen einer Verbindung der Formel (II), wobei R₁, R₂, R₃ und R₆ die in Anspruch 1 definierten Bedeutungen haben, R₁₁ ein Cladinosederivat der Formel (III) ist, in welchem R₂ₐ eine Hydroxyschutzgruppe ist, R₁₂ ein Wasserstoffatom ist oder R₁₁ zusammen mit R₁₂ ein Sauerstoffatom ist, mit einer Verbindung der Formel R₄XN=C(Y) (VII), wobei R₄, Y und X die in Anspruch 1 definierten Bedeutungen haben, um eine Verbindung der Formel (I) herzustellen, wobei R für (CH₂)ₙA-X-R₄ steht, A für -N(R₆)C(Y)NH- steht, X und n die in Anspruch 1 definierten Bedeutungen haben; oder
d) Umsetzen einer Verbindung der Formel (II), wobei R₁, R₂, R₃ und R₆ die in Anspruch 1 definierten Bedeutungen haben, R₁₁ ein Cladinosederivat der Formel (III) ist, in welchem R₂ₐ eine Hydroxyschutzgruppe ist, R₁₂ ein Wasserstoffatom ist oder R₁₁ zusammen mit R₁₂ ein Sauerstoffatom ist, mit einer Verbindung der Formel R₄XL (VIII), wobei R₄ und X die in Anspruch 1 definierten Bedeutungen haben und L eine geeignete Abgangsgruppe ist, um eine Verbindung der Formel (I) herzustellen, wobei R für (CH₂)ₙA-X-R₄ steht, A für -N(R₆)- steht, X und n die in Anspruch 1 definierten Bedeutungen haben; oder
e) Umsetzen einer Verbindung der Formel (II), wobei R₁, R₂, R₃ und R₆ die in Anspruch 1 definierten Bedeutungen haben, R₁₁ ein Cladinosederivat der Formel (III) ist, in welchem R₂ₐ eine Hydroxyschutzgruppe ist, R₁₂ ein Wasserstoffatom ist oder R₁₁ zusammen mit R₁₂ ein Sauerstoffatom ist, mit einer Verbindung der Formel R₄XOC(O)L (IX), wobei L eine geeignete Abgangsgruppe ist, und R₄ und X die in Anspruch 1 definierten Bedeutungen haben, um eine Verbindung der Formel (I) herzustellen, wobei R für (CH₂)ₙA-X-R₄ steht, A für N(R₆)C(O)O steht, X und n die in Anspruch 1 definierten Bedeutungen haben; oder
f) Umsetzen einer Verbindung der Formel (II), wobei R₁, R₂, R₃ und R₆ die in Anspruch 1 definierten Bedeutungen haben, R₁₁ ein Cladinosederivat der Formel (III) ist, in welchem R₂ₐ eine Hydroxyschutzgruppe ist, R₁₂ ein Wasserstoffatom ist oder R₁₁ zusammen mit R₁₂ ein Sauerstoffatom ist, mit einer Verbindung der Formel R₄XCHO (X), wobei R₄ und X die in Anspruch 1 definierten Bedeutungen haben, um eine Verbindung der Formel (I) herzustellen, wobei R für (CH₂)ₙA-X-R₄ steht, A für N=C(R₆) steht, X und n die in Anspruch 1 definierten Bedeutungen haben; oder
g) Umsetzen einer Verbindung der Formel (XI), wobei R₁, R₂, R₃, R₄ und X die in Anspruch 1 definierten Bedeutungen haben, R₁₁ ein Cladinosederivat der Formel (III) ist, in welchem R₂ₐ eine Hydroxyschutzgruppe ist, und R₁₂ ein Wasserstoffatom ist, mit einer Verbindung der Formel R₆COOH (XIa) wobei R₆ die Anspruch 1 definierte Bedeutung hat, 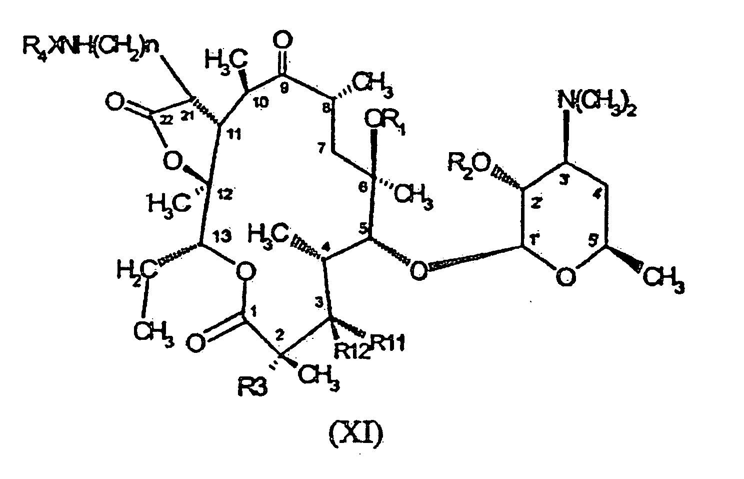 um eine Verbindung der Formel (I) herzustellen, wobei R für (CH₂)ₙA-X-R₄ steht, A für [NC(O)R₆] steht, X und n die in Anspruch 1 definierten Bedeutungen haben; oder
h) Umsetzen einer Verbindung der Formel (XVII), wobei R₁, R₂ und R₃ die in Anspruch 1 definierten Bedeutungen haben, R₁₁ ein Cladinosederivat der Formel (III) ist, in welchem R₂ₐ eine Hydroxyschutzgruppe ist, und R₁₂ ein Wasserstoffatom ist, und R₁₃ ein C₁₋₄-Alkylrest ist, mit einem Aldehyd der Formel 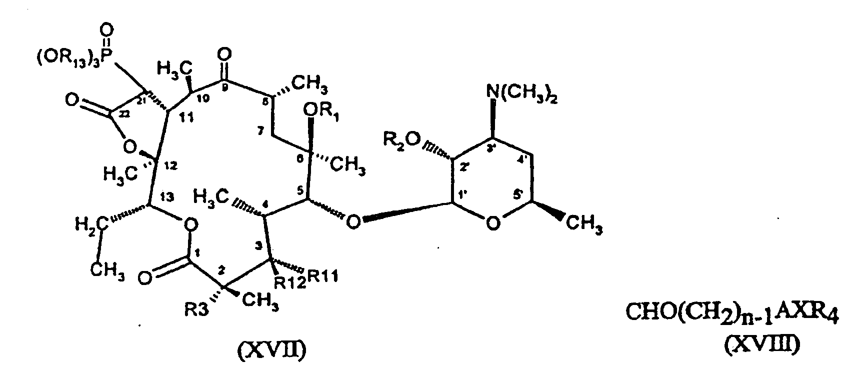 wobei R₄, X und n die in Anspruch 1 definierten Bedeutungen haben, um eine Verbindung der Formel (I) herzustellen, wobei R für (CH₂)ₙAXR₄ steht und A und X die in Anspruch 1 definierten Bedeutungen haben;
und n eine ganze Zahl von 2 oder 3 ist; oder
i) Umsetzen einer Verbindung der Formel (I), wobei R₁ die in Anspruch 1 definierten Bedeutungen hat, R₃ ein Wasserstoffatom ist und R₂ eine Hydroxyschutzgruppe ist, mit einem Halogenierungsmittel, um eine Verbindung der Formel (I) herzustellen, wobei R₃ ein Halogenatom ist;
j) Cyclisieren einer Verbindung der Formel (XXXII), wobei L eine geeignete Abgangsgruppe ist, wie Halogen (d.h. Chlor oder Brom), R₁, R₂, R₃ die in Anspruch 1 definierten Bedeutungen haben, R₁₁ ein Cladinosederivat der Formel (III) ist, in welchem R₂ₐ eine Hydroxyschutzgruppe ist, und R₁₂ ein Wasserstoffatom ist, X eine C₄₋₅₋Alkylenkette ist, welche gegebenenfalls mit einem oder zwei Resten ausgewählt aus Oxo, einem 9- oder 10-gliedrigen kondensierten bicyclischen heterocyclischen Rest mit mindestens einem Heteroatom ausgewählt aus Sauerstoff, Schwefel oder Stickstoff substituiert ist, 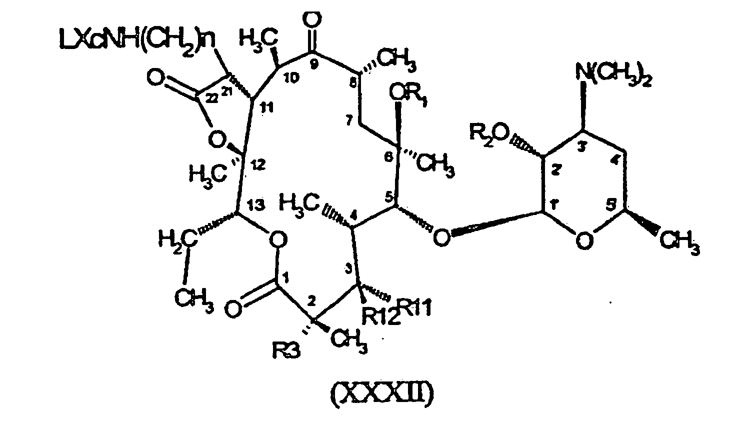
um eine Verbindung der Formel (I) herzustellen, wobei R₁ für (CH₂)ₙR₅ steht;
und danach, falls notwendig, Unterwerfen der resultierenden Verbindung einem oder mehreren der folgenden Abläufe:
i) Hydrolyse des Cladinosederivats (III);
ii) Umwandeln der 3-Hydroxygruppe in 3-Oxo ;
iii) Entfernen der Schutzgruppe R₂, und
iv) Umwandeln der resultierenden Verbindung der Formel (I) in ein pharmazeutisch verträgliches Salz und Solvat davon.

9. Verbindung gemäß einem der Ansprüche 1 bis 7 zur Verwendung in der Therapie.

10. Verwendung einer Verbindung gemäß einem der Ansprüche 1 bis 7 zur Herstellung eines Medikaments zur Verwendung in der Therapie von systemischen oder topischen bakteriellen Infektionen bei einem menschlichen oder tierischen Körper.

11. Arzneimittel, umfassend eine Verbindung gemäß einem der Ansprüche 1 bis 7 im Gemisch mit einem oder mehreren pharmazeutisch verträglichen Trägem oder Exzipienten.

## Revendications

1. Composé de formule (I) 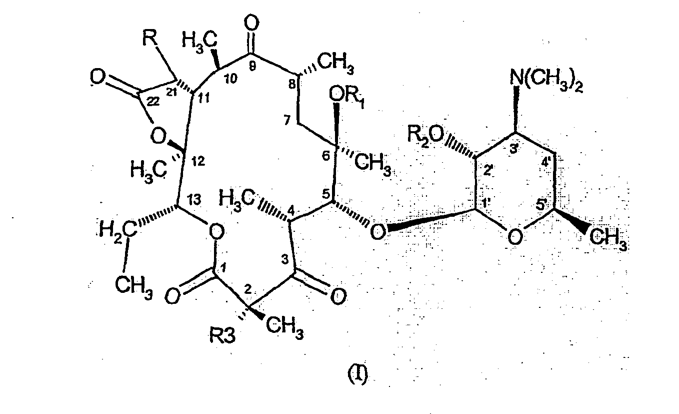 dans laquelle
R est (CH₂)ₙA-X-R₄ ou (CH₂)ₙR₅ ;
A est un groupe choisi parmi -N(R₆)-, -N[C(O)R₆]-, -N(R₆)C(O)-, -N(R₆)S(O)₂-, -N(R₆)C(O)O-, -N=C(R₆)- ou -N(R₆)C(Y)N(R₇)- ;
R₁ est un groupe alkyle en C₁ à C₆ ou un groupe alcényle en C₃ à C₆ ;
R₂ est l'hydrogène ou un groupe acyle ;
R₃ est l'hydrogène ou un halogène ;
X est une liaison, une chaîne alkylène en C₁ à C₁₀, alcénylène en C₂ à C₁₀ ou alcynylène en C₂ à C₁₀ où lesdites chaînes sont :
i) éventuellement interrompues par un groupe radical bivalent choisi parmi -O-, -N(R₈)-, -C(O)-, -N(R₈)C(Y)N(R₉)-, -S(O)m-, -N(R₈)C(O)-, -C(O)N(R₈)-, -N(R₈)C(O)C(O)-, -C(O)O- ou -C(NOR₆)- et/ou
ii) éventuellement substituées par un ou deux groupes choisis parmi :
un groupe alkyle en C₁ à C₄, un groupe oxo, un groupe alcoxy en C₁ à C₄, un halogène, un groupe cyano, un groupe phénoxy, un groupe hydroxy, NR₈R₉, N(R₈)C(O)R₉, =NOR₆, NR₈C(Y)NR₉ ou un groupe phényle éventuellement substitué ;
R₄ est choisi parmi :
l'hydrogène,
un groupe phényle éventuellement substitué,
un groupe cycloalkyle en C₃ à C₇ éventuellement substitué,
un groupe carbocyclique bicyclique condensé à 9 à 10 chaînons éventuellement substitué,
un groupe hétéroaryle à 5 ou 6 chaînons éventuellement substitué, le groupe hétéroaryle à 5 chaînons contenant au moins un hétéroatome choisi parmi l'oxygène, le soufre ou l'azote et le groupe hétéroaryle à 6 chaînons contenant de 1 à 3 atomes d'azote,
un groupe hétérocyclique à 5 à 6 chaînons éventuellement substitué,
ou
R⁴ est un groupe hétérocyclique bicyclique condensé à 9 ou 10 chaînons éventuellement substitué ayant au moins un hétéroatome choisi parmi l'oxygène, le soufre ou l'azote,
dans lequel
l'expression groupe phényle éventuellement substitué, groupe hétérocyclique à 5 à 6 chaînons éventuellement substitué, groupe carbocyclique bicyclique condensé à 9 à 10 chaînons éventuellement substitué, groupe hétérocyclique bicyclique condensé à 9 à 10 chaînons éventuellement substitué, ou groupe hétéroaryle à 5 à 6 chaînons éventuellement substitué se réfère à un groupe hétérocyclique à 5 à 6 chaînons, un groupe carbocyclique bicyclique condensé à 9 à 10 chaînons, un groupe hétérocyclique bicyclique condensé à 9 à 10 chaînons, ou un groupe hétéroaryle à 5 ou 6 chaînons tel que défini ci-dessus qui est substitué par 1 à 4 groupes, qui peuvent être identiques ou différents, choisis parmi un groupe (CH₂)ₚR₁₀ où p est zéro ou un nombre entier de 1 à 4 et R₁₀ est choisi parmi :
un halogène,
un groupe alcoxy en C₁ à C₄,
un groupe alkyle en C₁ à C₄,
un groupe hydroxy,
un groupe cyano,
un groupe nitro,
un groupe oxo,
un groupe trifluorométhyle,
un groupe carboxy,
NR₈R₉,
COR₈,
CONR₈R₉,
NHCOR₈,
NHSO₂R₈,
S(O)qR₆, (où q est 0 ou un nombre entier de 1 à 2),
un groupe phényle (éventuellement substitué par un halogène, un groupe alcoxy en C₁ à C₄ ou NR₈R₉) ;
un groupe phénoxy ;
un groupe hétéroaryle à 5 chaînons contenant au moins un hétéroatome choisi parmi l'oxygène, le soufre ou l'azote et un groupe hétéroaryle à 6 chaînons contenant au moins un atome d'azote, lequel groupe hétéroaryle à 5 à 6 chaînons peut être substitué par un groupe alkyle en C₁ à C₄ ou un groupe cyano,
ou
un groupe hétérocyclique bicyclique condensé à 9 ou 10 chaînons, et
l'expression groupe cycloalkyle en C₃ à C₇ éventuellement substitué se réfère à un groupe éventuellement substitué par 1 ou 2 substituants qui peuvent être identiques ou différents et sont choisis parmi un groupe alkyle en C₁ à C₄, un halogène, un groupe cyano, un groupe nitro, un groupe trifluorométhyle et NR₆R₇ ;
R₅ est un groupe hétérocyclique à 5 ou 6 chaînons contenant au moins un atome d'azote, éventuellement substitué par 1 ou 2 groupes choisis parmi un groupe oxo ou un groupe hétérocyclique bicyclique condensé à 9 ou 10 chaînons ayant au moins un hétéroatome choisi parmi l'oxygène, le soufre ou l'azote ;
R₆ et R₇ sont indépendamment l'hydrogène, un groupe alkyle en C₁ à C₄ ou un groupe phényle qui est éventuellement substitué par un ou deux groupes alkyle en C₁ à C₄ ;
R₈ et R₉ sont indépendamment l'hydrogène, un groupe phényle (qui peut être substitué par un ou deux groupes alkyle en C₁ à C₄) ou R₈ et R₉ sont indépendamment un groupe alkyle en C₁ à C₄ qui est éventuellement substitué par 1 ou 2 groupes choisis parmi :
un groupe phényle, un groupe alcoxy en C₁ à C₄,
un groupe cyano,
un groupe hétéroaryle à 5 chaînons contenant 1 ou 2 hétéroatomes choisis parmi l'oxygène, le soufre ou l'azote ou le groupe hétéroaryle à 6 chaînons contient de 1 à 3 atomes d'azote,
un groupe hydroxy,
un groupe oxo,
un groupe carboxy ;
Y est un atome d'oxygène ou un atome de soufre ;
n est 0 ou un nombre entier de 1 à 3 ;
m est 0, 1 ou 2 ;
et des sels et des produits de solvatation de ceux-ci acceptables d'un point de vue pharmaceutique.

2. Composé selon la revendication 1, dans lequel R₁ est un groupe méthyle ou 2-propényle.

3. Composé selon la revendication 1 ou 2, dans lequel R₂ est l'hydrogène.

4. Composé selon l'une quelconque des revendications 1 à 3, dans lequel R₃ est l'hydrogène ou le fluor.

5. Composé selon l'une quelconque des revendications 1 à 4, dans lequel A est choisi parmi -NH-, -NHC(O)- ou -NHC(Y)NH-.

6. Composé selon l'une quelconque des revendications 1 à 5, dans lequel X est une chaîne alkylène en C₁ à C₄ qui est éventuellement interrompue par un radical bivalent choisi parmi -O-, -NH-, -C(O)-, -NHC(O)-, -S(O)₂- -S- et/ou une telle chaîne alkylène en C₁ à C₄ est éventuellement substituée par un groupe choisi parmi NH₂, alkyle en C₁ à C₄, oxo ou N-OH.

7. Composé choisi parmi :
(11S,21S)-3-décladinosyl-11,12-didéoxy-6-O-méthyl-3-oxo-12,11-[oxycarbonyl-2-(4-pyridin-3-yl)-imidazol-1-yl)-éthylamino)-méthylène]-érythromycine A ;
(11S,21S)-3-décladinosyl-11,12-didéoxy-6-O-méthyl-3-oxo-12,11-[oxycarbonyl-2(quinolin-4-yl)-éthylamino)-méthylène]-érythromycine A ;
(11S,21S)-3-décladinosyl-11,12-didéoxy-6-O-méthyl-3-oxo-12,11-[oxycarbonyl-(3-(4-(pyridin-3-yl)-imidazol-1-yl)-propylamino)-méthylène]-érythromycine A ;
(11S,21S)-3-décladinosyl-11,12-didéoxy-6-O-méthyl-3-oxo-12,11-[oxycarbonyl-(2(quinoxalin-2-ylsulfanyl)-acétamido)-méthylène]-érythromycine A ;
(11S, 21S)-3-décladinosyl-11,12-didéoxy-6-O-méthyl-3-oxo-12,11-[oxycarbonyl-((4-(2,3-dihydro-benzo[1,4]dioxin-6-yl)-4-oxo)-butyramido)-méthylène]-érythromycine A ;
(11S,21S)-3-décladinosyl-11,12-didéoxy-6-O-méthyl-3-oxo-12,11-[oxycarbonyl-(4-(4-méthoxy-3-nitro-phényl)-4-oxo-butyramido)-méthylène]-érythromycine A ;
(11S,21S)-3-décladinosyl-11,12-didéoxy-6-O-méthyl-3-oxo-12,11-[oxycarbonyl-(4-(2-hydroxy-4,5-diméthoxy-phényl)-4-oxo-butyramido)-méthylène]-érythromycine A ;
(11S,21S)-3-décladinosyl-11,12-didéoxy-6-O-méthyl-3-oxo-12,11-[oxycarbonyl-(4-(4-oxo-4,5,6,7-tétrahydro-benzo[b]thiophen-2-yl)-4-oxo-butyramido)-méthylène]-érythromycine A ;
(11S,21S)-3-décladinosyl-11,12-didéoxy-6-O-méthyl-3-oxo-12,11-[oxycarbonyl-3-(4-quinolin-2-yl-1H-pyrazol-1-yl)-propylamino)-méthylène]-érythromycine A ;
(11S,21S)-3-décladinosyl-11,12-didéoxy-6-O-méthyl-3-oxo-12,11-[oxycarbonyl-(4-(3,4-diméthoxy-phényl)-4-oxo-butyramido)-méthylène]-érythromycine A ;
(11S,21S)-3-décladinosyl-11,12-didéoxy-6-O-méthyl-3-oxo-12,11-[oxycarbonyl-(4-(4-hydroxy-3-méthoxy-phényl)-4-oxo-butyramido)-méthylène]-érythromycine A ; et
(11S,21S)-3-décladinosyl-11,12-didéoxy-6-O-méthyl-3-oxo-12,11-[oxycarbonyl-(3-(4-(thiophén-2-yl)-imidazol-1-yl)-propylamino)-méthylène]-érythromycine A.

8. Procédé pour la préparation d'un composé de formule (I) ou d'un sel ou produit de solvatation de celui-ci acceptable d'un point de vue pharmaceutique
qui comprend une étape parmi :
a) la réaction d'un composé de formule (II) 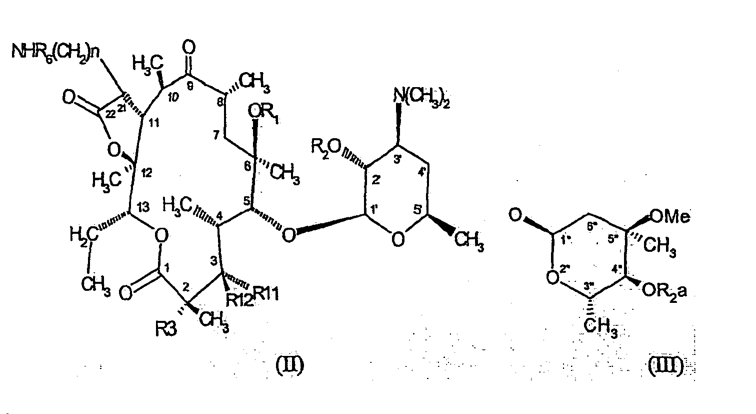 dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome oxygène, avec un dérivé activé approprié de HOC(O)XR₄ acide (IV) ou avec un dérivé activé approprié de l'acide sulfonique (V) HOS(O)₂XR₄ (V) respectivement, où R₄ et X ont les significations définies dans la revendication 1, pour produire un composé de formule (I) dans laquelle R est (CH₂)ₙA-X-R₄, A est -N(R₆)C(O)- ou -N(R₆)S(O)₂-X et n ont les significations définies dans la revendication 1 ; ou
b) la réaction d'un composé de formule (II) dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome d'oxygène, avec un composé de formule R₄XN(R₇)C(Y)L (VI), dans laquelle L est un groupe partant approprié, R₇ est un groupe phényle ou un groupe alkyle en C₁ à C₄, R₄ et X ont les significations définies dans la revendication 1, pour produire un composé de formule (I) dans laquelle R est (CH₂)ₙA-X-R₄, A est -N(R₆)C(Y)N(R₇)- et X et n ont les significations définies dans la revendication 1 ; ou
c) la réaction d'un composé de formule (II) dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome oxygène, avec un composé de formule R₄XN=C(Y) (VII), où R₄, Y et X ont les significations définies dans la revendication 1, pour produire un composé de formule (I) dans laquelle R est (CH₂)ₙA-X-R₄, A est -N(R₆)C(Y)NH-, X et n ont les significations définies dans la revendication 1 ; ou
d) la réaction d'un composé de formule (II) dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome d'oxygène, avec un composé de formule R₄XL (VIII), dans laquelle R₄ et X ont les significations définies dans la revendication 1 et L est un groupe partant approprié, pour produire un composé de formule (I) dans laquelle R est (CH₂)ₙA-X-R₄, A est -N(R₆)-, X et n ont les significations définies dans la revendication 1 ; ou
e) la réaction d'un composé de formule (II) dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome d'oxygène, avec un composé de formule R₄XOC(O)L (IX), dans laquelle L est un groupe partant approprié, et R₄ et X ont les significations définies dans la revendication 1, pour produire un composé de formule (I) dans laquelle R est (CH₂)ₙA-X-R₄, A est N(R₆)C(O)O, X et n ont les significations définies dans la revendication 1 ; ou
f) la réaction d'un composé de formule (II) dans laquelle R₁, R₂, R₃ et R₆ ont les significations définies dans la revendication 1, R₁₁ est un dérivé de cladinose de formule (III), dans laquelle R₂ₐ est un groupe protecteur hydroxy, R₁₂ est l'hydrogène ou R₁₁ conjointement avec R₁₂ est un atome d'oxygène, avec un composé de formule R₄XCHO (X), dans laquelle R₄, et X ont les significations définies dans la revendication 1, pour produire un composé de formule (I) dans laquelle R est (CH₂)ₙA-X-R₄, A est N=C(R₆), X et n ont les significations définies dans la revendication 1 ; ou
g) la réaction d'un composé de formule (XI) dans laquelle R₁, R₂, R₃, R₄ et X ont les significations définies dans la revendication 1, R₁₁ est un dérivé de cladinose de formule (III), dans laquelle R₂ₐ est un groupe protecteur hydroxy, et R₁₂ est l'hydrogène, avec un composé de formule R₆COOH (XIa) dans laquelle R₆ a la signification définie dans la revendication 1, 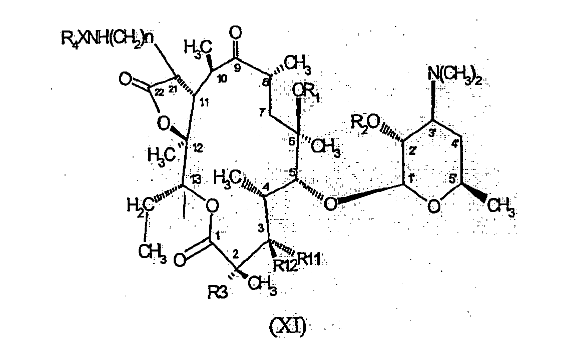 pour produire un composé de formule (I) dans laquelle R est (CH₂)ₙA-X-R₄, A est [NC(O)(R₆], X et n ont les significations définies dans la revendication 1 ; ou
h) la réaction d'un composé de formule (XVII), dans laquelle R₁, R₂ et R₃ ont les significations définies dans la revendication 1, R₁₁ est un dérivé de cladinose de formule (III), dans laquelle R₂ₐ est un groupe protecteur hydroxy, et R₁₂ est l'hydrogène et R₁₃ est un groupe alkyle en C₁ à C₄ avec un aldéhyde de formule 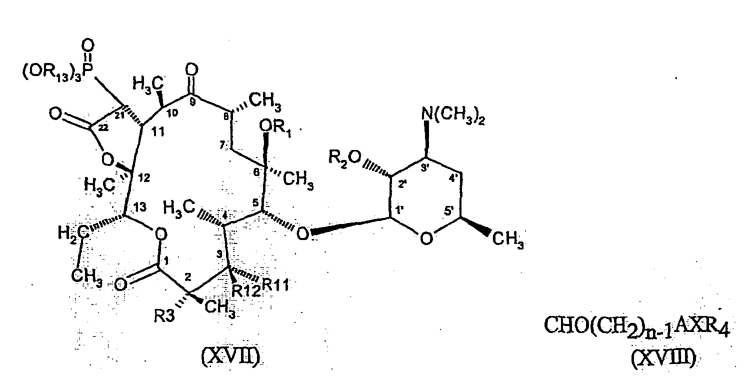 dans laquelle R₄, X et n ont les significations définies dans la revendication 1, pour produire un composé de formule (I), dans laquelle R est (CH₂)ₙAXR₄, A et X ont les significations définies dans la revendication 1 ; et n est un nombre entier de 2 ou 3 ; ou
i) la réaction d'un composé de formule (I) dans laquelle R₁ a les significations définies dans la revendication 1, R₃ est l'hydrogène et R₂ est un groupe protecteur hydroxy avec un agent d'halogénation pour produire un composé de formule (I) dans laquelle R₃ est un halogène ;
j) la cyclisation d'un composé de formule (XXXII), dans laquelle L est un groupe partant approprié tel qu'un halogène (c'est-à-dire le chlore ou le brome), R₁, R₂, R₃ ont les significations définies dans la revendication 1, R₁₁ est un dérivé de cladinose de formule (III), dans laquelle R₂ₐ est un groupe protecteur hydroxy, et R₁₂ est l'hydrogène, X est une chaîne alkylène en C₄ à C₅ éventuellement substituée par un ou deux groupes choisis parmi un groupe oxo, un groupe hétérocyclique bicyclique condensé à 9 ou 10 chaînons ayant au moins un hétéroatome choisi parmi l'oxygène, le soufre ou l'azote, 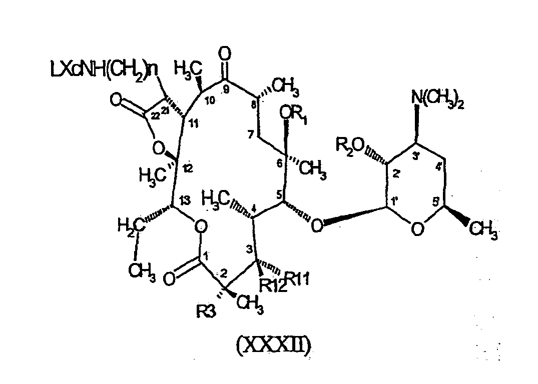
pour produire un composé de formule (I) dans laquelle R₁ est (CH₂)ₙR₅ ;
et par la suite, si nécessaire, la soumission du composé résultant à une ou plusieurs des opérations suivantes :
i) une hydrolyse du dérivé de cladinose (III) ;
ii) la transformation du groupe 3-hydroxy en groupe 3-oxo,
iii) le retrait du groupe protecteur R₂ et
iv) la transformation du composé résultant de formule (I) en un sel et des produits de solvatation de celui-ci acceptables d'un point de vue pharmaceutique.

9. Composé selon l'une quelconque des revendications 1 à 7 pour une utilisation en thérapie.

10. Utilisation d'un composé selon l'une quelconque des revendications 1 à 7 dans la préparation d'un médicament pour une utilisation dans la thérapie d'infections bactériennes systémiques ou topiques chez un humain ou un animal.

11. Composition pharmaceutique comprenant un composé selon l'une quelconque des revendications 1 à 7 en mélange avec un ou plusieurs supports ou excipients acceptables d'un point de vue pharmaceutique.
